# EUROPEAN PATENT APPLICATION

(11) **EP 4 317 171 A1**
(43) Date of publication of application: **07.02.2024**
(21) Application number: 22867732.4
(22) Date of filing: 08.09.2022
(51) Int. Cl.: C07K 5/117, A61P 29/00, A61P 1/00, A61K 38/00, A23L 33/18

(54) **COMPOSITION FOR PREVENTING OR TREATING INFLAMMATORY BOWEL DISEASE CONTAINING NOVEL COMPOUND**

(30) Priority: 09.09.2021 KR 20210120647
(71) Applicant: Anygen Co., Ltd., Gwangju 61008 (KR)
(72) Inventor: KIM, Jae-Il, Gwangju 62254 (KR); KIM, Gyeong Min, Gunsan-si Jeollabuk-do 54139 (KR); RYU, Jae Ha, Gwangju 62278 (KR); JOO, Sang Hyun, Gwangju 61258 (KR); PARK, Ye Ga, Seoul 07778 (KR)
(74) Representative: HGF
(86) International application number: PCT/KR2022/013535
(87) International publication number: WO 2023/038464

(57) **Abstract**

A compound according to the present invention or a pharmaceutically acceptable salt thereof selectively and specifically acts as an agonist for GPR39, promotes the proliferation of intestinal tissue cells and enterohepatic association, promotes cell differentiation, and alleviates inflammation in inflamed intestinal tissue. Thus, the compound can be utilized in a composition for alleviating and mitigating, preventing, or treating inflammatory bowel disease.

## Description

### Technical Field

The present invention relates to a novel compound and a composition for preventing or treating inflammatory bowel disease, comprising the same as an active ingredient.

### Background Art

About 880 G protein coupled receptors (GPCRs), which are the most abundant membrane proteins in all mammals, are currently known. Of these, about 60% are related to the senses of sight, smell, taste, and the like, and the remaining about 400 GPCRs, including orphan GPCRs, are regulated by various ligands (e.g., proteins, peptide hormones, amino acids, amines and lipids) in the body and are involved in various biological phenomena. In particular, GPCRs are associated with diseases such as metabolic diseases, cardiovascular diseases, degenerative diseases and carcinogenesis. Therefore, it is an important drug target for new drug development, and more than 50% of the drugs developed to date are directly or indirectly related to the activity of GPCR.

On the other hand, GPR39 (G protein coupled receptor 39) is one of the orphan GPCRs, for which an intrinsic ligand has not been discovered to date. GPR39 belongs to the Ghrelin receptor (GHSR, growth hormone secretagogue receptor) family, and is known to be distributed in the stomach, intestine, pancreas, liver, kidney, reproductive and adipose tissues (Cell. Mol. Life Sci. 2011, 68:85-95). GPR39, also known as a zinc sensing receptor, is mainly expressed in gastrointestinal tract tissues and is known to promote gastric emptying and enhance secretion of gastric juice through studies on GPR39 deficient mice (Gastroenterology, 2006, 131:1131-1141). In addition, it is known that GPR39 promotes the expression of factors for tight junction in a model of ulcerative colitis, one of inflammatory bowel diseases (IBD) (Phil. Trans. R. Soc. B., 2016, 371:20150420).

The above results suggest that GPR39 plays an important role in the function of the gastrointestinal tract in the body, and a modulator for GPR39 is expected to be a new drug candidate for the treatment of inflammatory bowel disease.

### Detailed Description of Invention

### Technical Problem

The present inventors have completed the present invention by confirming that a peptide consisting of a specific amino acid sequence or a variant thereof effectively regulates the activity of GPR39 to activate intestinal function and exhibit anti-inflammatory action.

Therefore, an object of the present invention is to provide a compound represented by Formula 1 below or a pharmaceutically acceptable salt thereof.

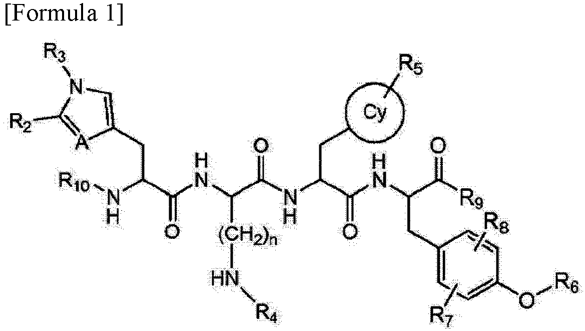

### Solution to Problem

In order to achieve the above object, in one aspect of the present invention, there is provided a compound represented by Formula 1 below or a pharmaceutically acceptable salt thereof 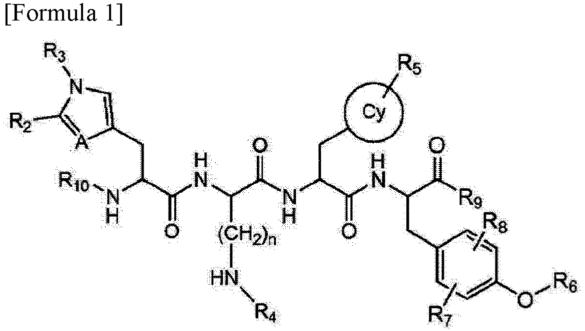 in Formula 1
A is -C(-R₀)-, -N= or -N(-R₁)-,
Cy is C₆₋₁₄ aryl or 5 to 6-membered heteroaryl,
R₁ and R₃ are each independently hydrogen, Ra, an amine protecting group, C₁₋₆ alkyl substituted with 1 to 3 Ra, C₃₋₁₀ cycloalkyl, or 5 to 6-membered heterocyclyl, wherein said heterocyclyl has or does not have 1 to 3 substituents selected from phenylethyl and cyclohexylethyl,
R₂ is hydrogen, Ra, or 5 to 6-membered heterocyclyl, and R₀ is hydrogen, or R₀ and R₂ are linked to each other to form a benzene ring together with the two carbon atoms to which they are attached,
Ra is each independently C₃₋₁₀ cycloalkyl or C₆₋₁₄ aryl, wherein said aryl has or does not have one or more substituents selected from the group consisting of halogen, -OH, C₆₋₁₄ aryl substituted with 5 to 6-membered heteroaryl, C₁₋₆ alkyl and C₁₋₆ alkoxy,
n is 1 to 10,
R₄ is hydrogen, C₁₋₁₀ alkyl or C₁-C₂₀ alkylcarbonyl,
R₅ is hydrogen, halogen, CF₃ or C₁₋₆ alkyl,
R₆ is hydrogen, C₁₋₁₀ alkyl or -S(=O)₂OH,
R₇ and R₈ are each independently hydrogen, halogen, nitro, amine or C₁₋₆ alkyl,
R₉ is -OH or -NH₂, and
R₁₀ is hydrogen, an amine protecting group or biotin, wherein said heterocyclyl and heteroaryl each independently contain at least one heteroatom selected from the group consisting of N, S and O.

In another aspect of the present invention, there is provided a pharmaceutical composition for preventing or treating inflammatory bowel disease, comprising the compound or pharmaceutically acceptable salt thereof as an active ingredient.

In another aspect of the present invention, there is provided a health functional food composition for preventing or mitigating inflammatory bowel disease, comprising the compound or pharmaceutically acceptable salt thereof as an active ingredient.

### Effects of Invention

A compound according to the present invention or a pharmaceutically acceptable salt thereof selectively and specifically acts as an agonist for GPR39, promotes the proliferation of intestinal tissue cells and tight junction, promotes cell differentiation, and alleviates inflammation in inflamed intestinal tissue. Thus, the compound can be utilized in a composition for alleviating and mitigating, preventing, or treating inflammatory bowel disease.

### Brief Description of Drawings

Fig. 1 illustrates the results obtained by confirming the purity of HKFY-1 prepared according to one embodiment of the present invention through HPLC.
Fig. 2 illustrates the results obtained by confirming the purity of HKFY-2 prepared according to one embodiment of the present invention through HPLC.
Fig. 3 illustrates the results obtained by confirming the purity of HKFY-3 prepared according to one embodiment of the present invention through HPLC.
Fig. 4 illustrates the results obtained by confirming the purity of HKFY-4 prepared according to one embodiment of the present invention through HPLC.
Fig. 5 illustrates the results obtained by confirming the purity of HKFY-5 prepared according to one embodiment of the present invention through HPLC.
Fig. 6 illustrates the results obtained by confirming the purity of HKFY-6 prepared according to one embodiment of the present invention through HPLC.
Fig. 7 illustrates the results obtained by confirming the purity of HKFY-7 prepared according to one embodiment of the present invention through HPLC.
Fig. 8 illustrates the results obtained by confirming the purity of HKFY-8 prepared according to one embodiment of the present invention through HPLC.
Fig. 9 illustrates the results obtained by confirming the purity of HKFY-9 prepared according to one embodiment of the present invention through HPLC.
Fig. 10 illustrates the results obtained by confirming the purity of HKFY-10 prepared according to one embodiment of the present invention through HPLC.
Fig. 11 illustrates the results obtained by confirming the purity ofHKFY-11 prepared according to one embodiment of the present invention through HPLC.
Fig. 12 illustrates the results obtained by confirming the purity of HKFY-12 prepared according to one embodiment of the present invention through HPLC.
Fig. 13 illustrates the results obtained by confirming the purity of HKFY-13 prepared according to one embodiment of the present invention through HPLC.
Fig. 14 illustrates the results obtained by confirming the purity of HKFY-14 prepared according to one embodiment of the present invention through HPLC.
Fig. 15 illustrates the results obtained by confirming the purity of HKFY-15 prepared according to one embodiment of the present invention through HPLC.
Fig. 16 illustrates the results obtained by confirming the purity of HKFY-16 prepared according to one embodiment of the present invention through HPLC.
Fig. 17 illustrates the results obtained by confirming the purity of HKFY-17 prepared according to one embodiment of the present invention through HPLC.
Fig. 18 illustrates the results obtained by confirming the purity of HKFY-18 prepared according to one embodiment of the present invention through HPLC.
Fig. 19 illustrates the results obtained by confirming the purity of HKFY-19 prepared according to one embodiment of the present invention through HPLC.
Fig. 20 illustrates the results obtained by confirming the purity of HKFY-20 prepared according to one embodiment of the present invention through HPLC.
Fig. 21 illustrates the results obtained by confirming the purity of HKFY-21 prepared according to one embodiment of the present invention through HPLC.
Fig. 22 illustrates the results obtained by confirming the purity of HKFY-22 prepared according to one embodiment of the present invention through HPLC.
Fig. 23 illustrates the results obtained by confirming the purity of HKFY-23 prepared according to one embodiment of the present invention through HPLC.
Fig. 24 illustrates the results obtained by confirming the purity of HKFY-24 prepared according to one embodiment of the present invention through HPLC.
Fig. 25 illustrates the results obtained by confirming the purity of HKFY-25 prepared according to one embodiment of the present invention through HPLC.
Fig. 26 illustrates the results obtained by confirming the purity of HKFY-26 prepared according to one embodiment of the present invention through HPLC.
Fig. 27 illustrates the results obtained by confirming the purity of HKFY-27 prepared according to one embodiment of the present invention through HPLC.
Fig. 28 illustrates the results obtained by confirming the purity of HKFY-28 prepared according to one embodiment of the present invention through HPLC.
Fig. 29 illustrates the results obtained by confirming the purity of HKFY-29 prepared according to one embodiment of the present invention through HPLC.
Fig. 30 illustrates the results obtained by confirming the purity of HKFY-30 prepared according to one embodiment of the present invention through HPLC.
Fig. 31 illustrates the results obtained by confirming the purity of HKFY-31 prepared according to one embodiment of the present invention through HPLC.
Fig. 32 illustrates the results obtained by confirming the purity of HKFY-32 prepared according to one embodiment of the present invention through HPLC.
Fig. 33 illustrates the results obtained by confirming the purity of HKFY-33 prepared according to one embodiment of the present invention through HPLC.
Fig. 34 illustrates the results obtained by confirming the purity of HKFY-34 prepared according to one embodiment of the present invention through HPLC.
Fig. 35 illustrates the results obtained by confirming the purity of HKFY-35 prepared according to one embodiment of the present invention through HPLC.
Fig. 36 illustrates the results obtained by confirming the purity of HKFY-36 prepared according to one embodiment of the present invention through HPLC.
Fig. 37 illustrates the results obtained by confirming the molecular weight of HKFY-1 prepared according to one embodiment of the present invention through Ion-Mass.
Fig. 38 illustrates the results obtained by confirming the molecular weight of HKFY-2 prepared according to one embodiment of the present invention through Ion-Mass.
Fig. 39 illustrates the results obtained by confirming the molecular weight of HKFY-3 prepared according to one embodiment of the present invention through Ion-Mass.
Fig. 40 illustrates the results obtained by confirming the molecular weight of HKFY-4 prepared according to one embodiment of the present invention through Ion-Mass.
Fig. 41 illustrates the results obtained by confirming the molecular weight of HKFY-5 prepared according to one embodiment of the present invention through Ion-Mass.
Fig. 42 illustrates the results obtained by confirming the molecular weight of HKFY-6 prepared according to one embodiment of the present invention through Ion-Mass.
Fig. 43 illustrates the results obtained by confirming the molecular weight of HKFY-7 prepared according to one embodiment of the present invention through Ion-Mass.
Fig. 44 illustrates the results obtained by confirming the molecular weight of HKFY-8 prepared according to one embodiment of the present invention through Ion-Mass.
Fig. 45 illustrates the results obtained by confirming the molecular weight of HKFY-9 prepared according to one embodiment of the present invention through Ion-Mass.
Fig. 46 illustrates the results obtained by confirming the molecular weight of HKFY-10 prepared according to one embodiment of the present invention through Ion-Mass.
Fig. 47 illustrates the results obtained by confirming the molecular weight of HKFY-11 prepared according to one embodiment of the present invention through Ion-Mass.
Fig. 48 illustrates the results obtained by confirming the molecular weight of HKFY-12 prepared according to one embodiment of the present invention through Ion-Mass.
Fig. 49 illustrates the results obtained by confirming the molecular weight of HKFY-13 prepared according to one embodiment of the present invention through Ion-Mass.
Fig. 50 illustrates the results obtained by confirming the molecular weight of HKFY-14 prepared according to one embodiment of the present invention through Ion-Mass.
Fig. 51 illustrates the results obtained by confirming the molecular weight of HKFY-15 prepared according to one embodiment of the present invention through Ion-Mass.
Fig. 52 illustrates the results obtained by confirming the molecular weight of HKFY-16 prepared according to one embodiment of the present invention through Ion-Mass.
Fig. 53 illustrates the results obtained by confirming the molecular weight of HKFY-17 prepared according to one embodiment of the present invention through Ion-Mass.
Fig. 54 illustrates the results obtained by confirming the molecular weight of HKFY-18 prepared according to one embodiment of the present invention through Ion-Mass.
Fig. 55 illustrates the results obtained by confirming the molecular weight of HKFY-19 prepared according to one embodiment of the present invention through Ion-Mass.
Fig. 56 illustrates the results obtained by confirming the molecular weight of HKFY-20 prepared according to one embodiment of the present invention through Ion-Mass.
Fig. 57 illustrates the results obtained by confirming the molecular weight of HKFY-21 prepared according to one embodiment of the present invention through Ion-Mass.
Fig. 58 illustrates the results obtained by confirming the molecular weight of HKFY-22 prepared according to one embodiment of the present invention through Ion-Mass.
Fig. 59 illustrates the results obtained by confirming the molecular weight of HKFY-23 prepared according to one embodiment of the present invention through Ion-Mass.
Fig. 60 illustrates the results obtained by confirming the molecular weight of HKFY-24 prepared according to one embodiment of the present invention through Ion-Mass.
Fig. 61 illustrates the results obtained by confirming the molecular weight of HKFY-25 prepared according to one embodiment of the present invention through Ion-Mass.
Fig. 62 illustrates the results obtained by confirming the molecular weight of HKFY-26 prepared according to one embodiment of the present invention through Ion-Mass.
Fig. 63 illustrates the results obtained by confirming the molecular weight of HKFY-27 prepared according to one embodiment of the present invention through Ion-Mass.
Fig. 64 illustrates the results obtained by confirming the molecular weight of HKFY-28 prepared according to one embodiment of the present invention through Ion-Mass.
Fig. 65 illustrates the results obtained by confirming the molecular weight of HKFY-29 prepared according to one embodiment of the present invention through Ion-Mass.
Fig. 66 illustrates the results obtained by confirming the molecular weight of HKFY-30 prepared according to one embodiment of the present invention through Ion-Mass.
Fig. 67 illustrates the results obtained by confirming the molecular weight of HKFY-31 prepared according to one embodiment of the present invention through Ion-Mass.
Fig. 68 illustrates the results obtained by confirming the molecular weight of HKFY-32 prepared according to one embodiment of the present invention through Ion-Mass.
Fig. 69 illustrates the results obtained by confirming the molecular weight of HKFY-33 prepared according to one embodiment of the present invention through Ion-Mass.
Fig. 70 illustrates the results obtained by confirming the molecular weight of HKFY-34 prepared according to one embodiment of the present invention through Ion-Mass.
Fig. 71 illustrates the results obtained by confirming the molecular weight of HKFY-35 prepared according to one embodiment of the present invention through Ion-Mass.
Fig. 72 illustrates the results obtained by confirming the molecular weight of HKFY-36 prepared according to one embodiment of the present invention through Ion-Mass.
Fig. 73 illustrates the results obtained by analyzing the specific binding of HKFY-34 to the GPR39 receptor using a luciferase assay system (top) and a calcium influx assay system (bottom).
Fig. 74 illustrates the results obtained by analyzing the binding capacity of each intrinsic ligand for NTSR1, NTSR2, GHSR, MTLR, NMUR1, NMUR2 and TRHR belonging to the GHSR family and HKFY-34, a HKFY derivative, using a luciferase assay system.
Fig. 75 is a graph showing the results obtained by measuring the cell proliferation according to treatment with HKFY-34 at different concentrations in HT-29 cells, an intestinal epithelial cell line.
Fig. 76 is a graph showing the results obtained by measuring the calcium concentration in the cells according to treatment with HKFY-34 (10 µM) in HT-29 cells, an intestinal epithelial cell line, through Fluo-8 staining.
Fig. 77 is a graph showing the results obtained by confirming the mRNA expression levels of occludin and ZO-1 according to treatment with HKFY-34 at different concentrations in HT-29 cells, an intestinal epithelial cell line, through quantitative RT-PCR. *p represents a significant difference (*p <0.05 and ***p<0.001) in the group treated with HKFY-34 relative to the untreated group (vehicle).
Fig. 78 is a graph and diagram showing the results obtained by confirming the protein expression levels of occludin and ZO-1 according to treatment with HKFY-34 at different concentrations in HT-29 cells, an intestinal epithelial cell line, through Western blot. *p represents a significant difference (*p<0.05) in the group treated with HKFY-34 relative to the untreated group (vehicle).
Fig. 79 is a graph and diagram showing the results obtained by confirming the phosphorylation of AMPK according to treatment with HKFY-34 at different concentrations in HT-29 cells, an intestinal epithelial cell line, through Western blot. *p represents a significant difference (***p<0.001) in the group treated with HKFY-34 relative to the untreated group (vehicle).
Fig. 80 is a graph and diagram showing the results obtained by confirming the phosphorylation of AMPK according to treatment with HKFY-34 after pretreatment with an AMPK inhibitor (STO-609, 1 µM), in HT-29 cells, an intestinal epithelial cell line, through Western blot. *p represents a significant difference (***p<0.001) in the group treated with HKFY-34 relative to the group treated with STO-609 alone, and ^{###}p represents a significant difference (^{###}p<0.001) in the group treated with the combination of HKFY-34 and STO-609 relative to the group treated with STO-609 alone.

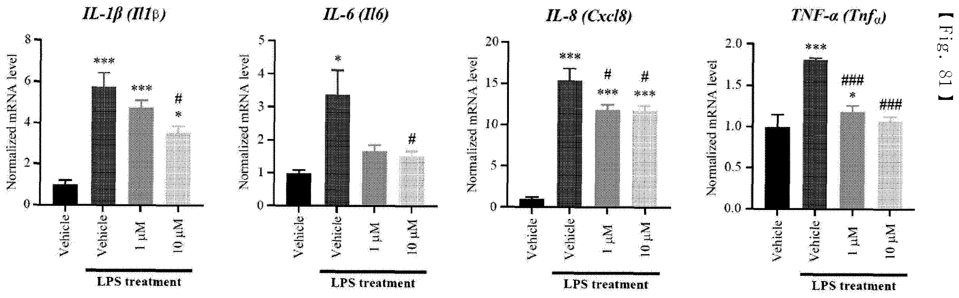

Fig. 81 is a graph showing the results obtained by confirming the expression levels of pro-inflammatory cytokines (interleukin (IL)-1β, IL-6, IL-8 and TNF-α) associated with ulcerative colitis according to treatment with HKFY-34 at different concentrations through quantitative RT-PCR, after inflammation was induced with 1 µg/mL LPS (lipopolysaccharide) in HT-29 cells, an intestinal epithelial cell line. *p represents a significant difference (*p<0.05 and ***p<0.001) relative to the untreated group (vehicle), and ^{#}p represents a significant difference (^{#}p<0.05 and ^{###}p<0.001) relative to the group treated with LPS alone.

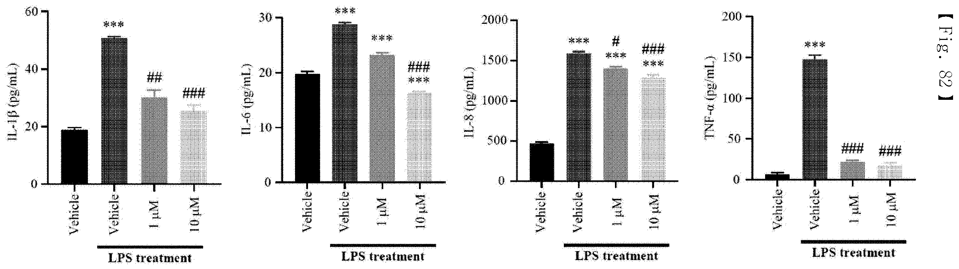

Fig. 82 is a graph showing the results obtained by confirming the extracellular secretion levels of pro-inflammatory cytokines (IL-1β, IL-6, IL-8 and TNF-α) associated with ulcerative colitis according to treatment with HKFY-34 at different concentrations through ELISA (enzymatic immunosorbent assay), after inflammation was induced with 1 µg/mL LPS in HT-29 cells, an intestinal epithelial cell line. *p represents a significant difference (***p<0.001) relative to the untreated group (vehicle), and ^{#}p represents a significant difference (^{#}p<0.05, ^{##}p<0.01 and ^{###}p<0.001) relative to the group treated with LPS alone.
Fig. 83 is a graph showing the results obtained by measuring the change in body weight during (left) and after (right) intraperitoneal administration of HKFY-34 at doses of 0.1, 1 and 10 mg/kg for 5 days to model mice in which ulcerative colitis was induced by DSS intake (left: daily cumulative body weight loss (%), right: final body weight loss (%)). *p represents a significant difference (***p<0.001) relative to the control group (vehicle), and ^{#}p represents a significant difference (^{#}p<0.05 and ^{##}p<0.01) relative to the negative control group (treated with DSS alone).
Fig. 84 is a graph and diagram showing the results obtained by measuring the length of the colon in model mice in which ulcerative colitis was induced by DSS after intraperitoneal administration of HKFY-34 at doses of 0.1, 1 and 10 mg/kg for 5 days to model mice in which ulcerative colitis was induced by DSS intake. *p represents a significant difference (***p<0.001) relative to the control group (vehicle), and ^{#}p represents a significant difference (^{###}p<0.001) relative to the negative control group (treated with DSS alone).
Fig. 85 is a graph showing the degree of the stool consistency (diarrhea) as a score by observing stool during intraperitoneal administration of HKFY-34 at doses of 0.1, 1 and 10 mg/kg to model mice in which ulcerative colitis was induced by DSS intake. *p represents a significant difference (***p<0.001) relative to the control group (vehicle), and ^{#}p represents a significant difference (^{#}p<0.05) relative to the negative control group (treated with DSS alone).
Fig. 86 is a graph showing the degree of blood in stool as a score by observing stool during intraperitoneal administration of HKFY-34 at doses of 0.1, 1 and 10 mg/kg to model mice in which ulcerative colitis was induced by DSS intake. *p represents a significant difference (***p<0.001) relative to the control group (vehicle), and ^{#}p represents a significant difference (^{#}p<0.05) relative to the negative control group (treated with DSS alone).

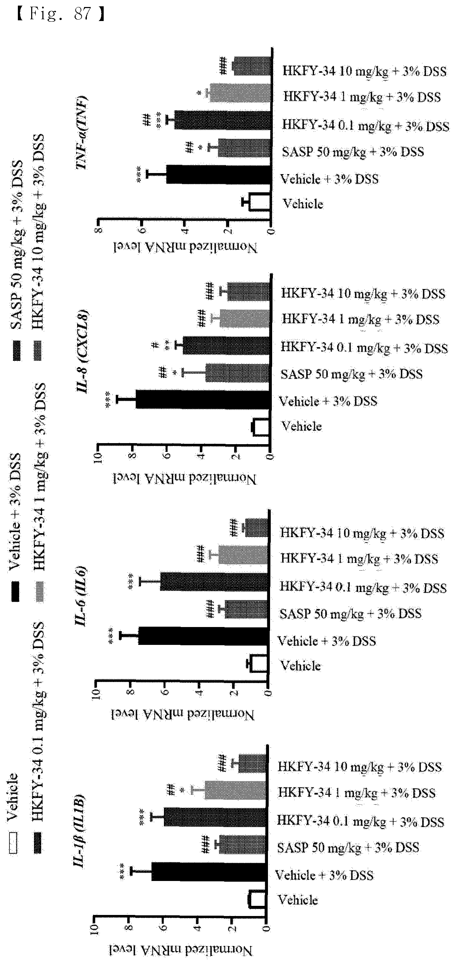

Fig. 87 is a graph showing the results obtained by confirming the mRNA expression levels of IL-1β, IL-6, IL-8 and TNF-α in the colon tissue of model mice in which ulcerative colitis was induced by DSS through quantitative RT-PCR, after intraperitoneal administration of HKFY-34 at doses of 0.1, 1 and 10 mg/kg for 5 days to model mice in which ulcerative colitis was induced by DSS intake. *p represents a significant difference (*p<0.05, **p<0.01 and ***p<0.001) relative to the control group (vehicle), and ^{#}p represents a significant difference (^{#}p<0.05, ^{##}p<0.01 and ^{###}p<0.001) relative to the negative control group (treated with DSS alone).

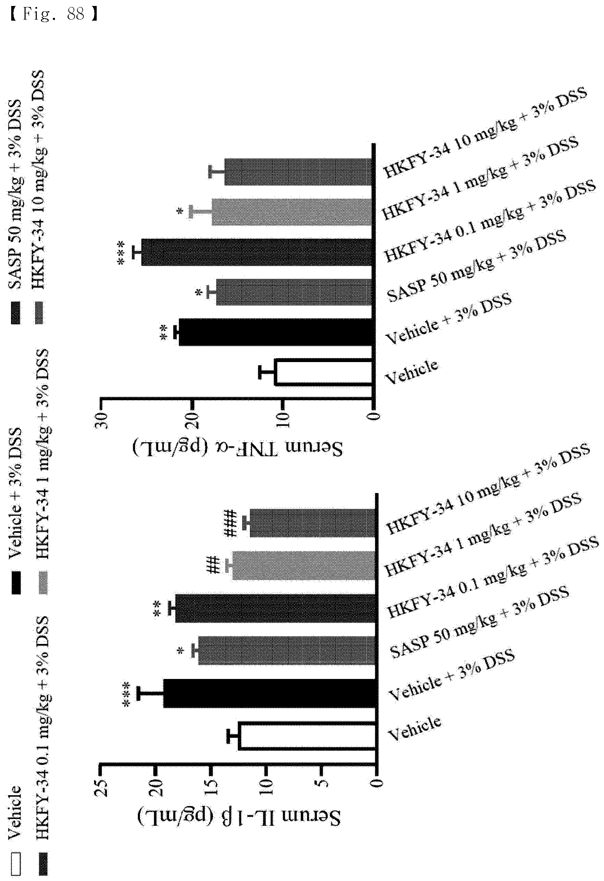

Fig. 88 is a graph showing the results obtained by confirming the concentration of IL-1β and TNF-α in the blood of model mice in which ulcerative colitis was induced by DSS through ELISA, after intraperitoneal administration of HKFY-34 at doses of 0.1, 1 and 10 mg/kg for 5 days to model mice in which ulcerative colitis was induced by DSS intake. *p represents a significant difference (*p<0.05, **p<0.01 and ***p<0.001) relative to the control group (vehicle), and ^{#}p represents a significant difference (^{##}p<0.01 and ^{###}p<0.001) relative to the negative control group (treated with DSS alone).

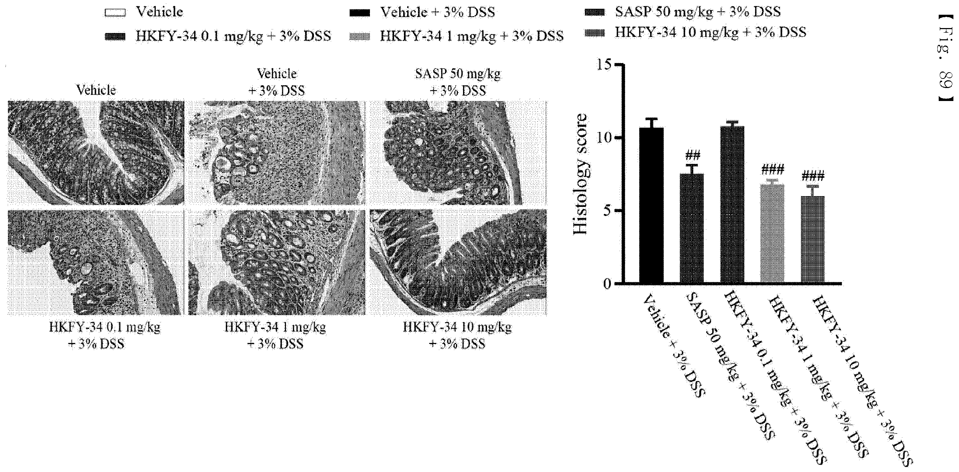

Fig. 89 is a graph and diagram showing the results (left) obtained by H&E staining of the colon tissue of model mice in which ulcerative colitis was induced by DSS and the results (right) obtained by analyzing and quantifying the degree of inflammatory damage (macrophage infiltration rate, mucus secreting cell loss rate, villus structural collapse rate and secreting gland loss rate) compared to the control group, after intraperitoneal administration of HKFY-34 at doses of 0.1, 1 and 10 mg/kg for 5 days to model mice in which ulcerative colitis was induced by DSS intake. ^{#}p represents a significant difference (^{##}p<0.01 and ^{###}p<0.001) relative to the negative control group (treated with DSS alone).

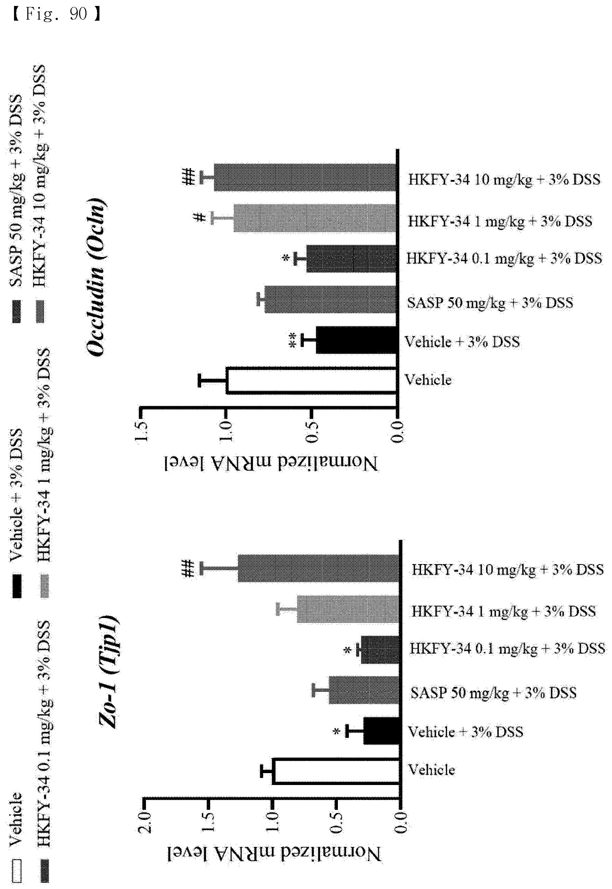

Fig. 90 is a graph showing the results obtained by confirming the mRNA expression levels of occludin and ZO-1 in the colon tissue of model mice in which colitis was induced by DSS through quantitative RT-PCR, after intraperitoneal administration of HKFY-34 at doses of 0.1, 1 and 10 mg/kg for 5 days to model mice in which ulcerative colitis was induced by DSS intake. *p represents a significant difference (*p<0.05, **p<0.01 and ***p<0.001) relative to the control group (vehicle), and ^{#}p represents a significant difference (^{##}p<0.01 and ^{###}p<0.001) relative to the negative control group (treated with DSS alone).

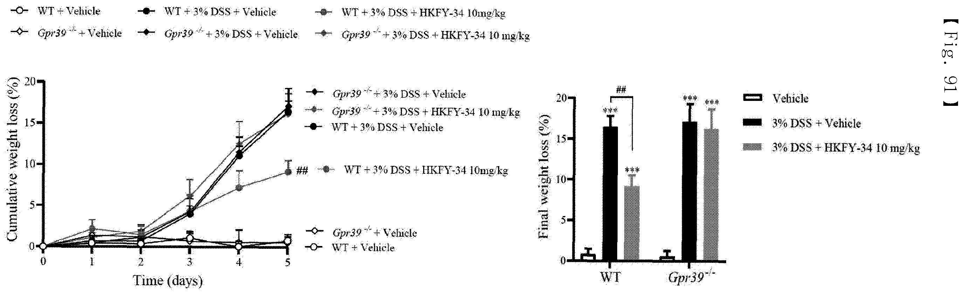

Fig. 91 is a graph showing the results obtained by measuring the change in body weight during (left) and after (right) intraperitoneal administration of HKFY-34 at a dose of 10 mg/kg to wild type and GPR39 genetically deficient *(Gpr39* ^{-/-}) model mice in which ulcerative colitis was induced by DSS intake (left: daily cumulative body weight loss (%), right: final body weight loss (%)). *p represents a significant difference (**p<0.01 and ***p<0.001) relative to the control group (vehicle), and ^{#}p represents a significant difference (^{##}p<0.01) relative to the negative control group (treated with DSS alone).
Fig. 92 is a graph and diagram showing the results obtained by measuring the length of the colon in model mice in which ulcerative colitis was induced by DSS after intraperitoneal administration of HKFY-34 at a dose of 10 mg/kg for 5 days to wild type and GPR39 genetically deficient *(Gpr39* ^{-/-}) model mice in which ulcerative colitis was induced by DSS intake. *p represents a significant difference (***p<0.001) relative to the control group (vehicle), and ^{#}p represents a significant difference (^{##}p<0.01) relative to the negative control group (treated with DSS alone).
Fig. 93 is a graph showing the degree of the stool consistency (diarrhea) as a score by observing stool during intraperitoneal administration of HKFY-34 at a dose of 10 mg/kg to wild type and GPR39 genetically deficient *(Gpr39* ^{-/-}) model mice in which ulcerative colitis was induced by DSS intake. ^{#}p represents a significant difference (^{#}p<0.05) relative to the negative control group (treated with DSS alone).
Fig. 94 is a graph showing the degree of blood in stool as a score by observing stool during intraperitoneal administration of HKFY-34 at a dose of 10 mg/kg to wild type and GPR39 genetically deficient *(Gpr39* ^{-/-}) model mice in which ulcerative colitis was induced by DSS intake.

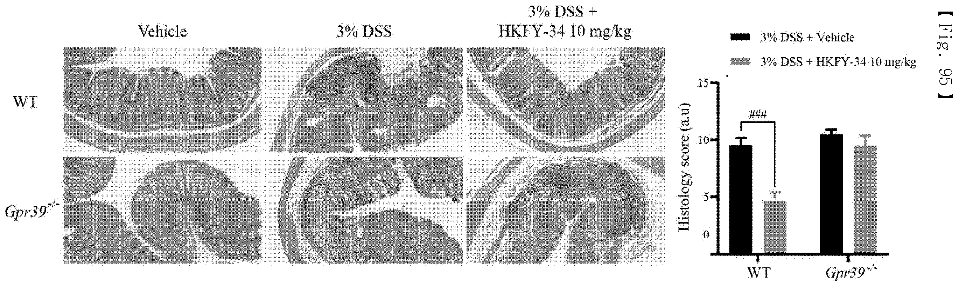

Fig. 95 is a graph and diagram showing the results (left) obtained by H&E staining of the colon tissue of model mice in which ulcerative colitis was induced by DSS and the results (right) obtained by analyzing and quantifying the degree of inflammatory damage (macrophage infiltration rate, mucus secreting cell loss rate, villus structural collapse rate and secreting gland loss rate) compared to the control group, after intraperitoneal administration of HKFY-34 at a dose of 10 mg/kg for 5 days to wild type and GPR39 genetically *(Gpr39* ^{-/-}) deficient model mice in which ulcerative colitis was induced by DSS intake.
Fig. 96 illustrates the results obtained by confirming ¹H NMR of HKFY-1 prepared according to one embodiment of the present invention.
Fig. 97 illustrates the results obtained by confirming ¹H NMR of HKFY-2 prepared according to one embodiment of the present invention.
Fig. 98 illustrates the results obtained by confirming ¹H NMR of HKFY-34 prepared according to one embodiment of the present invention.
Fig. 99 illustrates the results obtained by confirming the purity of HKFY-37 prepared according to one embodiment of the present invention through HPLC.
Fig. 100 illustrates the results obtained by confirming the molecular weight of HKFY-37 prepared according to one embodiment of the present invention through Ion-Mass.
Fig. 101 illustrates the results obtained by confirming the purity of HKFY 38 prepared according to one embodiment of the present invention through HPLC.
Fig. 102 illustrates the results obtained by confirming the molecular weight of HKFY-38 prepared according to one embodiment of the present invention through Ion-Mass.
Fig. 103 illustrates the results obtained by confirming the purity of HKFY-39 prepared according to one embodiment of the present invention through HPLC.
Fig. 104 illustrates the results obtained by confirming the molecular weight of HKFY-39 prepared according to one embodiment of the present invention through Ion-Mass.
Fig. 105 illustrates the results obtained by confirming the purity of HKFY-40 prepared according to one embodiment of the present invention through HPLC.
Fig. 106 illustrates the results obtained by confirming the molecular weight of HKFY-40 prepared according to one embodiment of the present invention through Ion-Mass.
Fig. 107 illustrates the results obtained by confirming the purity of HKFY-41 prepared according to one embodiment of the present invention through HPLC.
Fig. 108 illustrates the results obtained by confirming the molecular weight of HKFY-41 prepared according to one embodiment of the present invention through Ion-Mass.
Fig. 109 illustrates the results obtained by confirming the purity of HKFY-42 prepared according to one embodiment of the present invention through HPLC.
Fig. 110 illustrates the results obtained by confirming the molecular weight of HKFY-42 prepared according to one embodiment of the present invention through Ion-Mass.
Fig. 111 illustrates the results obtained by confirming the purity of HKFY-43 prepared according to one embodiment of the present invention through HPLC.
Fig. 112 illustrates the results obtained by confirming the molecular weight of HKFY-43 prepared according to one embodiment of the present invention through Ion-Mass.
Fig. 113 illustrates the results obtained by confirming the purity of HKFY-44 prepared according to one embodiment of the present invention through HPLC.
Fig. 114 illustrates the results obtained by confirming the molecular weight of HKFY-44 prepared according to one embodiment of the present invention through Ion-Mass.
Fig. 115 illustrates the results obtained by confirming the purity of HKFY-45 prepared according to one embodiment of the present invention through HPLC.
Fig. 116 illustrates the results obtained by confirming the molecular weight of HKFY-45 prepared according to one embodiment of the present invention through Ion-Mass.
Fig. 117 illustrates the results obtained by confirming the purity of HKFY-46 prepared according to one embodiment of the present invention through HPLC.
Fig. 118 illustrates the results obtained by confirming the molecular weight of HKFY-46 prepared according to one embodiment of the present invention through Ion-Mass.
Fig. 119 illustrates the results obtained by confirming the purity of HKFY-47 prepared according to one embodiment of the present invention through HPLC.
Fig. 120 illustrates the results obtained by confirming the molecular weight of HKFY-47 prepared according to one embodiment of the present invention through Ion-Mass.
Fig. 121 illustrates the results obtained by confirming the purity of HKFY-48 prepared according to one embodiment of the present invention through HPLC.
Fig. 122 illustrates the results obtained by confirming the molecular weight of HKFY-48 prepared according to one embodiment of the present invention through Ion-Mass.
Fig. 123 illustrates the results obtained by confirming the purity of HKFY-49 prepared according to one embodiment of the present invention through HPLC.
Fig. 124 illustrates the results obtained by confirming the molecular weight of HKFY-49 prepared according to one embodiment of the present invention through Ion-Mass.
Fig. 125 illustrates the results obtained by confirming the purity of HKFY-50 prepared according to one embodiment of the present invention through HPLC.
Fig. 126 illustrates the results obtained by confirming the molecular weight of HKFY-50 prepared according to one embodiment of the present invention through Ion-Mass.
Fig. 127 illustrates the results obtained by confirming the purity of HKFY-51 prepared according to one embodiment of the present invention through HPLC.
Fig. 128 illustrates the results obtained by confirming the molecular weight of HKFY-51 prepared according to one embodiment of the present invention through Ion-Mass.
Fig. 129 illustrates the results obtained by confirming the purity of HKFY-52 prepared according to one embodiment of the present invention through HPLC.
Fig. 130 illustrates the results obtained by confirming the molecular weight of HKFY-52 prepared according to one embodiment of the present invention through Ion-Mass.
Fig. 131 illustrates the results obtained by confirming the purity of HKFY-53 prepared according to one embodiment of the present invention through HPLC.
Fig. 132 illustrates the results obtained by confirming the molecular weight of HKFY-53 prepared according to one embodiment of the present invention through Ion-Mass.
Fig. 133 illustrates the results obtained by confirming the purity of HKFY-54 prepared according to one embodiment of the present invention through HPLC.
Fig. 134 illustrates the results obtained by confirming the molecular weight of HKFY-54 prepared according to one embodiment of the present invention through Ion-Mass.
Fig. 135 illustrates the results obtained by confirming the purity of HKFY-55 prepared according to one embodiment of the present invention through HPLC.
Fig. 136 illustrates the results obtained by confirming the molecular weight of HKFY-55 prepared according to one embodiment of the present invention through Ion-Mass.
Fig. 137 illustrates the results obtained by confirming the purity of HKFY-56 prepared according to one embodiment of the present invention through HPLC.
Fig. 138 illustrates the results obtained by confirming the molecular weight of HKFY-56 prepared according to one embodiment of the present invention through Ion-Mass.
Fig. 139 illustrates the results obtained by confirming the purity of HKFY-57 prepared according to one embodiment of the present invention through HPLC.
Fig. 140 illustrates the results obtained by confirming the molecular weight of HKFY-57 prepared according to one embodiment of the present invention through Ion-Mass.
Fig. 141 illustrates the results obtained by confirming the purity of HKFY-58 prepared according to one embodiment of the present invention through HPLC.
Fig. 142 illustrates the results obtained by confirming the molecular weight of HKFY-58 prepared according to one embodiment of the present invention through Ion-Mass.
Fig. 143 illustrates the results obtained by confirming the purity of HKFY-59 prepared according to one embodiment of the present invention through HPLC.
Fig. 144 illustrates the results obtained by confirming the molecular weight of HKFY-59 prepared according to one embodiment of the present invention through Ion-Mass.
Fig. 145 illustrates the results obtained by confirming the purity of HKFY-60 prepared according to one embodiment of the present invention through HPLC.
Fig. 146 illustrates the results obtained by confirming the molecular weight of HKFY-60 prepared according to one embodiment of the present invention through Ion-Mass.
Fig. 147 illustrates the results obtained by confirming the purity of HKFY-61 prepared according to one embodiment of the present invention through HPLC.
Fig. 148 illustrates the results obtained by confirming the molecular weight of HKFY-61 prepared according to one embodiment of the present invention through Ion-Mass.
Fig. 149 illustrates the results obtained by confirming the purity of HKFY-62 prepared according to one embodiment of the present invention through HPLC.
Fig. 150 illustrates the results obtained by confirming the molecular weight of HKFY-62 prepared according to one embodiment of the present invention through Ion-Mass.
Fig. 151 illustrates the results obtained by confirming the purity of HKFY-63 prepared according to one embodiment of the present invention through HPLC.
Fig. 152 illustrates the results obtained by confirming the molecular weight of HKFY-63 prepared according to one embodiment of the present invention through Ion-Mass.
Fig. 153 illustrates the results obtained by confirming the purity of HKFY-64 prepared according to one embodiment of the present invention through HPLC.
Fig. 154 illustrates the results obtained by confirming the molecular weight of HKFY-64 prepared according to one embodiment of the present invention through Ion-Mass.
Fig. 155 illustrates the results obtained by confirming the purity of HKFY-65 prepared according to one embodiment of the present invention through HPLC.
Fig. 156 illustrates the results obtained by confirming the molecular weight of HKFY-65 prepared according to one embodiment of the present invention through Ion-Mass.
Fig. 157 illustrates the results obtained by confirming the purity of HKFY-66 prepared according to one embodiment of the present invention through HPLC.
Fig. 158 illustrates the results obtained by confirming the molecular weight of HKFY-66 prepared according to one embodiment of the present invention through Ion-Mass.
Fig. 159 illustrates the results obtained by confirming the purity of HKFY-67 prepared according to one embodiment of the present invention through HPLC.
Fig. 160 illustrates the results obtained by confirming the molecular weight of HKFY-67 prepared according to one embodiment of the present invention through Ion-Mass.
Fig. 161 illustrates the results obtained by confirming the purity of HKFY-68 prepared according to one embodiment of the present invention through HPLC.
Fig. 162 illustrates the results obtained by confirming the molecular weight of HKFY-68 prepared according to one embodiment of the present invention through Ion-Mass.
Fig. 163 illustrates the results obtained by confirming the purity of HKFY-69 prepared according to one embodiment of the present invention through HPLC.
Fig. 164 illustrates the results obtained by confirming the molecular weight of HKFY-69 prepared according to one embodiment of the present invention through Ion-Mass.
Fig. 165 illustrates the results obtained by confirming the purity of HKFY-70 prepared according to one embodiment of the present invention through HPLC.
Fig. 166 illustrates the results obtained by confirming the molecular weight of HKFY-70 prepared according to one embodiment of the present invention through Ion-Mass.
Fig. 167 illustrates the results obtained by confirming the purity of HKFY-71 prepared according to one embodiment of the present invention through HPLC.
Fig. 168 illustrates the results obtained by confirming the molecular weight of HKFY-71 prepared according to one embodiment of the present invention through Ion-Mass.
Fig. 169 illustrates the results obtained by confirming the purity of HKFY-72 prepared according to one embodiment of the present invention through HPLC.
Fig. 170 illustrates the results obtained by confirming the molecular weight of HKFY-72 prepared according to one embodiment of the present invention through Ion-Mass.
Fig. 171 illustrates the results obtained by confirming the purity of HKFY-73 prepared according to one embodiment of the present invention through HPLC.
Fig. 172 illustrates the results obtained by confirming the molecular weight of HKFY-73 prepared according to one embodiment of the present invention through Ion-Mass.
Fig. 173 illustrates the results obtained by confirming the purity of HKFY-74 prepared according to one embodiment of the present invention through HPLC.
Fig. 174 illustrates the results obtained by confirming the molecular weight of HKFY-74 prepared according to one embodiment of the present invention through Ion-Mass.
Fig. 175 illustrates the results obtained by confirming the purity of HKFY-75 prepared according to one embodiment of the present invention through HPLC.
Fig. 176 illustrates the results obtained by confirming the molecular weight of HKFY-75 prepared according to one embodiment of the present invention through Ion-Mass.
Fig. 177 illustrates the results obtained by confirming the purity of HKFY-76 prepared according to one embodiment of the present invention through HPLC.
Fig. 178 illustrates the results obtained by confirming the molecular weight of HKFY-76 prepared according to one embodiment of the present invention through Ion-Mass.
Fig. 179 illustrates the results obtained by confirming the purity of HKFY-77 prepared according to one embodiment of the present invention through HPLC.
Fig. 180 illustrates the results obtained by confirming the molecular weight of HKFY-77 prepared according to one embodiment of the present invention through Ion-Mass.
Fig. 181 illustrates the results obtained by confirming the purity of HKFY-78 prepared according to one embodiment of the present invention through HPLC.
Fig. 182 illustrates the results obtained by confirming the molecular weight of HKFY-78 prepared according to one embodiment of the present invention through Ion-Mass.
Fig. 183 illustrates the results obtained by confirming the purity of HKFY-79 prepared according to one embodiment of the present invention through HPLC.
Fig. 184 illustrates the results obtained by confirming the molecular weight of HKFY-79 prepared according to one embodiment of the present invention through Ion-Mass.

### Best Mode for Carrying out the Invention

Hereinafter, the present invention will be described in more detail.

In one aspect of the present invention, there is provided a compound represented by Formula 1 below or a pharmaceutically acceptable salt thereof. 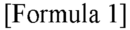 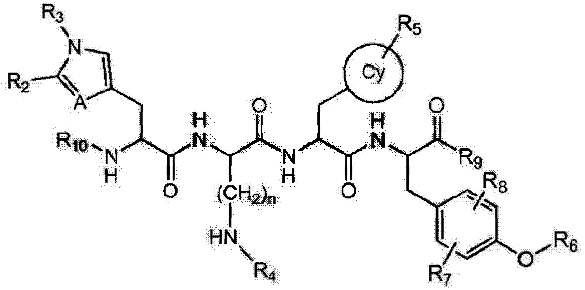 in Formula 1
A is -C(-R₀)-, -N= or -N(-R₁)-,
Cy is C₆₋₁₄aryl or 5 to 6-membered heteroaryl,
R₁ and R₃ are each independently hydrogen, Ra, an amine protecting group, C₁₋₆ alkyl substituted with 1 to 3 Ra, C₃₋₁₀ cycloalkyl, or 5 to 6-membered heterocyclyl, wherein said heterocyclyl has or does not have 1 to 3 substituents selected from phenylethyl and cyclohexylethyl,
R₂ is hydrogen, Ra, or 5 to 6-membered heterocyclyl, and
R₀ is hydrogen, or R₀ and R₂ are linked to each other to form a benzene ring together with the two carbon atoms to which they are attached,
Ra is each independently C₃₋₁₀ cycloalkyl or C₆₋₁₄ aryl, wherein said aryl has or does not have one or more substituents selected from the group consisting of halogen, -OH, C₆₋₁₄ aryl substituted with 5 to 6-membered heteroaryl, C₁₋₆ alkyl and C₁₋₆ alkoxy,
n is 1 to 10,
R₄ is hydrogen, C₁₋₁₀ alkyl or C₁-C₂₀ alkylcarbonyl,
R₅ is hydrogen, halogen, CF₃ or C₁₋₆ alkyl,
R₆ is hydrogen, C₁₋₁₀ alkyl or -S(=O)zOH,
R₇ and R₈ are each independently hydrogen, halogen, nitro, amine or C₁₋₆ alkyl,
R₉ is -OH or -NH₂, and
R₁₀ is hydrogen, an amine protecting group or biotin,
wherein said heterocyclyl and heteroaryl each independently contain at least one heteroatom selected from the group consisting of N, S and O.

In one embodiment of the present invention, the compound may be a peptide, and the peptide may be a derivative of a peptide having a sequence of four amino acids, and the sequence of four amino acids may be His-Lys-Phe-Tyr (SEQ ID NO: 1).

In accordance with the conventional use in the art, in the Formula herein, 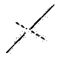 is used to denote a bond that is the point of attachment of a moiety or substituent to a core or skeletal structure.

As used herein, the term "alkyl" is a hydrocarbon having primary, secondary, tertiary or cyclic carbon atoms. For example, an alkyl group may have 1 to 20 carbon atoms (i.e., C₁-C₂₀ alkyl), 1 to 10 carbon atoms (i.e., C₁-C₁₀ alkyl), or 1 to 6 carbon atoms (i.e., C₁-C₆ alkyl). Examples of suitable alkyl groups include methyl (Me, -CH₃), ethyl (Et, -CH₂CH₃), 1-propyl (n-Pr, n-propyl, -CH₂CH₂CH₃), 2-propyl (i-Pr, i-propyl, -CH(CH₃)₂), 1-butyl (n-Bu, n-butyl, - CH₂CH₂CH₂CH₃), 2-methyl-1-propyl (i-Bu, i-butyl, -CH₂CH(CH₃)₂), 2-butyl (s-Bu, s-butyl, -CH(CH₃)CH₂CH₃), 2-methyl-2-propyl (t-Bu, t-butyl, -C(CH₃)₃), 1-pentyl (n-pentyl, - CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃, and octyl (-(CH₂)₇CH₃), but are not limited thereto.

As used herein, the term "cycloalkyl" refers to a saturated monocycle or poly-cycle containing only carbon atoms in the ring. A cycloalkyl may have 3 to 7 carbon atoms as a monocycle, 7 to 12 carbon atoms as a bicyclic cycloalkyl, and up to about 20 carbon atoms as a poly-cycle. A monocyclic cycloalkyl has 3 to 6 ring atoms, and more typically 5 or 6 ring atoms. A bicyclic cycloalkyl has 7 to 12 ring atoms arranged in a bicyclo [4,5], [5,5], [5,6] or [6,6] system, or 9 to 10 ring atoms arranged in a bicyclo [5,6] or [6,6] system or a spiro-fused ring. Non-limiting examples of monocyclic cycloalkyls may include cyclopropyl, cyclobutyl, cyclopentyl, and cyclohexyl (each of which may be substituted or unsubstituted).

As used herein, the term "heterocyclyl" includes the radicals of heterocycles described in the literature [Paquette, Leo A.; Principles of Modern Heterocyclic Chemistry (W.A. Benjamin, New York, 1968), specifically Chapters 1, 3, 4, 6, 7 and 9; The Chemistry of Heterocyclic Compounds, A Series of Monographs (John Wiley & Sons, New York, from 1950 to present), specifically Volumes 13, 14, 16, 19 and 28; and J. Am. Chem. Soc. (1960) 82:5566], but is not limited thereto. The term "heterocyclyl" refers to an aromatic or nonaromatic ring radical that is formed as saturated or unsaturated single or multiple rings and has one or more heteroatoms. For example, "5 to 6-membered heterocyclyl" may refer to a heterocyclyl having a total of 5 to 6 heteroatoms and/or carbon atoms. As used herein, when "heterocyclyl" is described as a substituent, it can be used as a term encompassing "heterocycloalkyl" and "heteroaryl." Herein, "heterocycloalkyl" refers to a saturated ring radical that is formed as single or multiple rings and has one or more heteroatoms, and "heteroaryl" refers to an aromatic ring radical that is formed as single or multiple rings and has one or more heteroatoms, and "heteroatom" may be selected from N, O and S. Non-limiting examples of heteroaryl rings include all those listed in the definition of "heterocyclyl," including pyridinyl, pyrrolyl, oxazolyl, indolyl, isoindolyl, purinyl, furanyl, thienyl, benzofuranyl, benzothiophenyl, carbazolyl, imidazolyl, thiazolyl, isoxazolyl, pyrazolyl, isothiazolyl, quinolyl, isoquinolyl, pyridazyl, pyrimidyl, pyrazyl (which may be substituted or unsubstituted), and the like.

As used herein, the term "heteroaryl" refers to an aromatic heterocyclyl having one or more heteroatoms in the ring. Non-limiting examples of suitable heteroatoms that may be included in the aromatic ring include oxygen, sulfur and nitrogen. Non-limiting examples of heteroaryl rings include all those listed in the definition of "heterocyclyl," including pyridinyl, pyrrolyl, oxazolyl, indolyl, isoindolyl, purinyl, furanyl, thienyl, benzofuranyl, benzothiophenyl, carbazolyl, imidazolyl, thiazolyl, isoxazolyl, pyrazolyl, isothiazolyl, quinolyl, isoquinolyl, pyridazyl, pyrimidyl, pyrazyl (which may be substituted or unsubstituted), and the like.

As used herein, the term "alkoxy" refers to a group having the Formula -O-alkyl, in which an alkyl group as defined above is attached to a parent compound through an oxygen atom. The alkyl portion of the alkoxy group may have 1 to 20 carbon atoms (i.e., C₁-C₂₀ alkoxy), 1 to 12 carbon atoms (i.e., C₁-C₁₂ alkoxy), or 1 to 6 carbon atoms (i.e., C₁-C₆ alkoxy). Examples of suitable alkoxy groups include methoxy (-O-CH₃ or -OMe), ethoxy (-OCH₂CH₃ or -OEt), t-butoxy (-O-C(CH₃)₃ or -O-tBu), and the like, but are not limited thereto.

As used herein, the term "alkylcarbonyl" refers to a group having the Formula - C(=O)-alkyl, in which an alkyl group as defined above is attached to a parent compound through a carbon atom. The alkyl portion of the alkylcarbonyl group may have 1 to 20 carbon atoms (i.e., C₁-C₂₀ alkylcarbonyl) or 5 to 17 carbon atoms (i.e., C₅-C₁₇ alkylcarbonyl). Examples of suitable alkylcarbonyl groups include caprylic (-C(=O)(CH₂)₆CH₃), capric (-C(=O)(CH₂)₈CH₃), lauric (-C(=O)(CH₂)₁₀CH₃), myristic (-C(=O)(CH₂)₁₂CH₃), palmitic (-C(=O)(CH₂)₁₄CH₃), and the like, but are not limited thereto.

The term "substituted" with respect to alkyl, aryl, heteroaryl, heterocyclyl, carbocyclyl (for example, cycloalkyl), and the like, for example, "substituted alkyl", "substituted aryl", "substituted heteroaryl", "substituted heterocyclyl" and "substituted carbocyclyl (for example, substituted cycloalkyl)" refer to alkyl, aryl, heteroaryl, heterocyclyl, carbocyclyl (for example, cycloalkyl) in which one or more hydrogen atoms are each independently substituted with a non-hydrogen substituent. Typical substituents include -X, -R, -O-, =O, -OR, -SR, -S-, -NR₂, -N+R₃, =NR, -C(X)₃, -CN, -OCN, -SCN, -N=C=O, - NCS, -NO, -NO₂, =N-OH, =N₂, -N₃, -NHC(=O)R, -C(=O)R, -C(=O)NRR -S(=O)₂O-, - S(=O)₂OH, -S(=O)₂R, -OS(=O)₂OR, -S(=O)₂NR, -S(=O)R, -OP (=O)(OR)₂, -C(=O)R, alkylene-C(=O)R, -C(S)R, -C(=O)OR, alkylene-C(=O)OR, -C(=O)O-, alkylene-C(=O)O-, - C(=S)OR, -C(=O)SR, -C(=S)SR, -C(=O)NRR, alkylene-C(=O)NRR, -C(=S)NRR, -C(-NR)NRR (wherein, each X is independently halogen: F, Cl, Br, or I, and R is independently H, alkyl, aryl, arylalkyl or heterocycle), but are not limited thereto. Alkylene, alkenylene, and alkynylene groups may also be similarly substituted.

In addition, the present invention includes pharmaceutically acceptable salts and addition salts of the compounds of the present invention, such as hydrochloride, hydrobromide or trifluoroacetate addition salts and sodium, potassium and magnesium salts, but is not limited thereto.

It will be appreciated by one of ordinary skill in the art that when a moiety such as "alkyl", "aryl", "heterocyclyl," etc. is substituted by one or more substituents, it may optionally be referred to as a moiety such as "alkylene", "arylene", "heterocyclylene" (i.e., meaning that one or more hydrogen atoms of the parent "alkyl", "aryl", "heterocyclyl" moiety are substituted with the substituents as stated above). When a moiety such as "alkyl", "aryl", "heterocyclyl," etc. is referred to herein as "substituted" or shown as substituted in the drawings (or optionally substituted, for example, when the number of substituents is from 0 to a positive number), the terms "alkyl", "aryl", "heterocyclyl" and the like should be understood as interchangeable with "alkylene", "arylene", "heterocyclylene" and the like.

It will be appreciated by one of ordinary skill in the art that the substituents and other moieties of the compound of Formula 1 should be selected to provide a compound that is sufficiently stable to provide a pharmaceutically useful compound that can be formulated into an acceptably stable pharmaceutical composition. The compound of Formula 1 having such stability is considered to be included in the scope of the present invention.

As used herein, the term "optionally substituted" refers to a particular moiety of a compound of Formula 1 having one, two, or more substituents.

In the present invention, the compound represented by Formula 1 may be an optical isomer of an L-form or a D-form.

In the compound represented by Formula 1 according to the present invention, A may be -C(-R₀)- or -N(-R₁)-, and R₁ may be hydrogen, 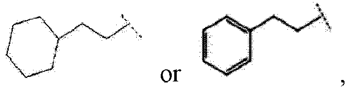 but is not limited thereto. In addition, R₀ may be hydrogen, and R₂ may be hydrogen, 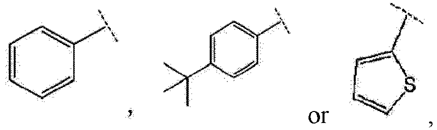 or R₀ and R₂ may be linked to each other to form a benzene ring together with the two carbon atoms to which they are attached, but is not limited thereto. In addition, R₃ may be one selected from the group consisting of 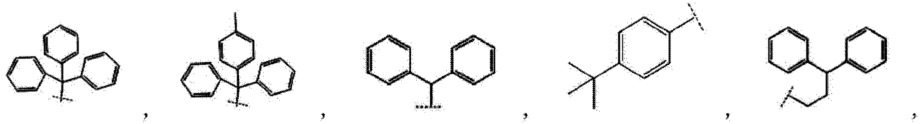 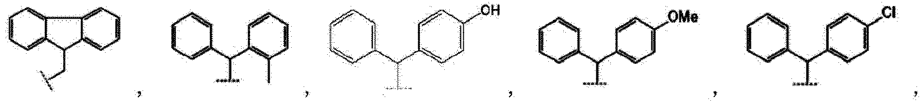 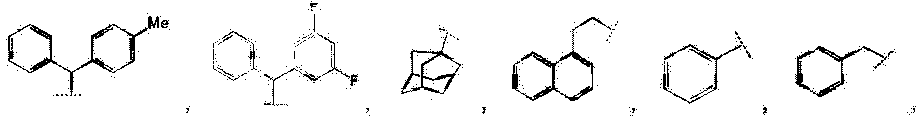 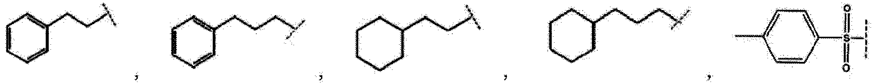 and 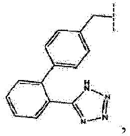 and preferably, it may be 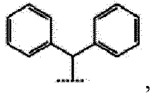 but is not limited thereto.

In addition, n may be 3 or 4, R₄ may be heptylcarbonyl, R₅ may be methyl or CF₃, R₆ may be hydrogen or S(=O)₂OH, and R₇ and R₈ may be each independently hydrogen or amine. In addition, R₇ and R₈ may be each independently hydrogen, R₉ may be -OH, and R₁₀ may be hydrogen, p-toluenesulfonyl (p-Ts), t-butoxycarbonyl (t-Boc) or biotin, but is not limited thereto. In addition, R₅, R₇ and R₈ are not particularly limited at positions to be substituted on the benzene ring. For example, R₅ may be substituted at an ortho (o-), meta (m-) or para (p-) position.

It may be any one selected from the group consisting of:
1) His(trt)-Lys(caprylic)-Phe-Tyr,
2) Biotin-His(trt)-Lys(caprylic)-Phe-Tyr,
3) His(benzyl)-Lys(caprylic)-Phe-Tyr,
4) d-His(trt)-Lys(caprylic)-Phe-Tyr,
5) His(4-methyltrityl)-Lys(caprylic)-Phe-Tyr,
6) His(DAMP-3)-Lys(caprylic)-Phe-Tyr,
7) His(DAMP-5)-Lys(caprylic)-Phe-Tyr,
8) His(DAMP-2)-Lys(caprylic)-Phe-Tyr,
9) His(2-phenylethyl)-Lys(caprylic)-Phe-Tyr,
10) His(2-naphthalen-1-ethyl)-Lys(caprylic)-Phe-Tyr,
11) His(2-cyclohexylethyl)-Lys(caprylic)-Phe-Tyr,
12) His(trt)-Lys(caprylic)-d-Phe-Tyr,
13) His(trt)-Lys(caprylic)-d-Phe(4-Cl)-Tyr,
14) d-His(trt)-Lys(caprylic)-d-Phe(4-Cl)-d-Tyr,
15) His(trt)-Lys(caprylic)-d-Phe(4-Cl)-d-Tyr,
16) His(trt)-d-Lys(caprylic)-d-Phe(4-Cl)-Tyr,
17) His(3,3-diphenylpropyl)-Lys(caprylic)-Phe-Tyr,
18) His(Fm)-Lys(caprylic)-Phe-Tyr,
19) His(phenylpropyl)-Lys(caprylic)-Phe-Tyr,
20) His(4-methoxylbenzhydryl)-Lys(caprylic)-Phe-Tyr,
21) His(4-chlorobenzhydryl)-Lys(caprylic)-Phe-Tyr,
22) His(4-methylbenzhydryl)-Lys(caprylic)-Phe-Tyr,
23) His(admantanmethyl)-Lys(caprylic)-Phe-Tyr,
24) His(trt)-Lys(capric)-Phe-Tyr,
25) His(trt)-Lys(lauric)-Phe-Tyr,
26) His(trt)-Lys(myristic)-Phe-Tyr,
27) His(trt)-Lys(palmitic)-Phe-Tyr,
28) His(trt)-Lys(caprylic)-d-Phe(F)-Tyr,
29) His(trt)-Lys(caprylic)-d-Phe(Br)-Tyr,
30) His(trt)-Lys(caprylic)-d-Phe(4-methyl)-Tyr,
31) d-His(trt)-Lys(caprylic)-d-Phe(4-methyl)-Tyr,
32) His(1,3-difluorobenzhydryl)-Lys(caprylic)-d-Phe(4-methyl)-Tyr,
33) His(benzhydryl)-Lys(caprylic)-Phe-Tyr,
34) His(benzhydryl)-Lys(caprylic)-d-Phe(4-methyl)-Tyr,
35) His(benzhydryl)-Lys(caprylic)-d-Phe(4-methyl)-Tyr(SO₃H),
36) His(benzhydryl)-Lys(caprylic)-d-Phe(4-methyl)-Tyr(2,6-di-methyl),
57) His(2-phenyl)-Lys(caprylic)-d-Phe(4-methyl)-Tyr,
58) His(1-phenyl)-Lys(caprylic)-d-Phe(4-methyl)-Tyr,
59) His(1,2-diphenyl)-Lys(caprylic)-d-Phe(4-methyl)-Tyr,
60) His(2-(4-tert-butyl)phenyl)-Lys(caprylic)-d-Phe(4-methyl)-Tyr,
61) His(1-(4-tert-butyl)phenyl)-Lys(caprylic)-d-Phe(4-methyl)-Tyr,
62) d-His(benzhydryl)-Lys(caprylic)-d-Phe(4-methyl)-Tyr,
63) His(thiophene)-Lys(caprylic)-d-Phe(4-methyl)-Tyr,
64) His(4-methoxybenzhydryl)-Lys(caprylic)-d-Phe(4-methyl)-Tyr, and
65) His(4-hydroxybenzhydryl)-Lys(caprylic)-d-Phe(4-methyl)-Tyr
66) His(benzhydryl)-Lys(caprylic)-d-Phe(4-methyl)-Tyr-NH₂
67) His(benzhydryl)-Lys(caprylic)-d-Phe(4-methyl)-Tyr(3-chloro)
68) His(benzhydryl)-Lys(caprylic)-d-Phe(4-methyl)-Tyr(3-nitro)
69) His(benzhydryl)-Lys(caprylic)-d-Phe(4-methyl)-Tyr(3,5-nitro)
70) His(benzhydryl)-Lys(caprylic)-d-Phe(4-methyl)-Tyr(3-amino)
71) His(benzhydryl)-Orn(caprylic)-d-Phe(4-methyl)-Tyr
72) Biotin-His(benzhydryl)-Lys(caprylic)-d-Phe(4-methyl)-Tyr
73) His(trt)-Lys(caprylic)-d-3Pal-Tyr
74) His(trt)-Lys(caprylic)-d-2Nal-Tyr
75) His(tosyl)-Lys(caprylic)-d-Phe(4-methyl)-Tyr
76) His(trt)-Lys(caprylic)-d-Phe(4-CF3)-Tyr
77) His(tbm)-Lys(caprylic)-d-Phe(4-CF3)-Tyr
78) Boc-Trp-Lys(caprylic)-d-Phe(4-methyl)-Tyr and
79) His(benzhydryl)Lys(caprylic).

In the present invention, the compound may be (X₁)-(X₂)-[His(trt)-Lys(caprylic)-Phe-Tyr], wherein X₁ and X₂ may be each independently any one amino acid selected from 20 amino acids or derivatives thereof, and specifically, it may be 37) Gly-Ser-His(trt)-Lys(caprylic)-Phe-Tyr (SEQ ID NO: 2).

In the compound represented by Formula 1 according to the present invention, 1 to 10 amino acids or derivatives thereof may be further bound to the C terminus of the compound. Specifically, the compound may be [His(trt)-Lys(caprylic)-Phe-Tyr]-(X₃)ₐ-(X₄)_{b}-(X₅)_{c}-(X₆)_{d}-(X₇)ₑ-(X₈)_{f}-(X₉)_{g}, wherein X₃ to X₉ may be each independently any one amino acid selected from 20 amino acids or derivatives thereof, and a to g may be 0 or 1, and at least one of them may be 1. More specifically, the compound may be any one selected from the group consisting of 38) His(trt)-Lys(caprylic)-Phe-Tyr-Ser-Asp-Gln-Gln-Ala-Arg-Phe (SEQ ID NO: 3), 39) His(trt)-Lys(caprylic)-Phe-Tyr-Ser-Gln-Asn-Gly-Ala-Arg-Phe (SEQ ID NO: 4), 40) His(trt)-Lys(caprylic)-Phe-Tyr-Ser-His-Gln-Gln-Ala-Arg-Phe (SEQ ID NO: 5), 41) His(trt)-Lys(caprylic)-Phe-Tyr-Gln-Asn-Gly-Ala-Arg-Phe (SEQ ID NO: 6), 42) His(trt)-Lys(caprylic)-Phe-Tyr-His-Gln-Gln-Ala-Arg-Phe (SEQ ID NO: 7), 43) His(trt)-Lys(caprylic)-Phe-Tyr-Gln-Asn-Gln-Ala-Arg-Phe (SEQ ID NO: 8), and 44) His(trt)-Lys(caprylic)-Phe-Tyr-Trp (SEQ ID NO: 9).

In another aspect of the present invention, there is provided a compound or a pharmaceutically acceptable salt thereof, wherein the compound consists of [His(trt)-Lys(caprylic)]-(X₁₀)ₕ-(X₁₁)ᵢ, wherein X₁₀ and X₁₁ may be each independently any one amino acid selected from 20 amino acids or derivatives thereof, and h and i may be 0 or 1, and at least one of them may be 1. Specifically, the compound may be any one selected from the group consisting of 45) Biotin-His(trt)-Lys(caprylic)-Bip-Tyr (SEQ ID NO: 10), 46) His(trt)-Lys(caprylic)-Phe-Trp (SEQ ID NO: 11), 47) His(trt)-Lys(caprylic)-Tyr-Phe (SEQ ID NO: 12), 48) His(trt)-Lys(caprylic)-Tyr-Tyr (SEQ ID NO: 13), 49) His(trt)-Lys(caprylic)-Phe, 50) His(trt)-Lys(caprylic)-Phe-Ser (SEQ ID NO: 14), and 51) His(trt)-Lys(caprylic)-Leu-Tyr (SEQ ID NO: 15).

In another aspect of the present invention, there is provided any one selected from the group consisting of 52) His(trt)-Phe-Tyr-Asp-Gln-Gln-Ala-Arg-Phe (SEQ ID NO: 16), 53) His(trt)-Gly-Ser-Lys(caprylic)-Phe-Tyr (SEQ ID NO: 17), 54) Lys(caprylic)-Phe-Tyr, 55) Lys(caprylic)-His(trt)-Phe-Tyr (SEQ ID NO: 18), 56) His(trt)-Lys(caproic)-Phe-Tyr-Asp-Gln-Gln-Ala-Arg-Phe (SEQ ID NO: 19) and 79) His(benzhydryl)Lys(caprylic).

In the present invention, the compound may be present in a mixture of L-form and D-form amino acids (for example, which may be any one amino acid selected from the group consisting of His, Lys, Phe, Tyr, Ser, Asp, Gln, Ala, Arg and Asn, but is not limited thereto), where "d" means a D-form amino acid.

In the present invention, "trt" is triphenylmethyl, "Boc" is t-butyloxycarbonyl, "tBu" is t-butyl, "Fmoc" is 9-fluorenylmethyloxycarbonyl, "dde" is 1-(4,4-dimethyl-2,6-dioxocyclohexylidene)ethyl, and "Bip" is biphenylalanine.

In the present invention, the "biotin" is a kind of vitamin B complex required for the growth of animals and plants. The natural one is a D-form isomer, in which the bond of the two pentagonal rings is in the cis configuration. The other seven isomers, such as L-biotin in cis form or allobiotin in trans form, do not exhibit coenzyme activity.

In the present invention, the "amine protecting group" may be a protecting group selected from the group consisting of t-butyloxycarbonyl (Boc), p-toluenesulfonyl (Ts), fluorenylmethyloxycarbonyl, benzyl, triphenylmethyl and carboxybenzyl, and it may be preferably p-toluenesulfonyl or t-butyloxycarbonyl.

In the present invention, when R₁₀ in the compound represented by Formula 1 is biotin, the biotin may be a compound represented by Formula 2 below.

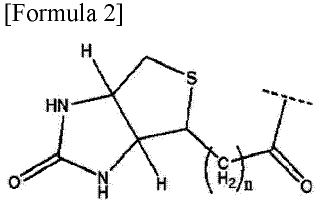

In Formula 2, n may be 1 to 10, and it may be preferably 4, but is not limited thereto.

In addition, the compound represented by Formula 1 according to the present invention may be represented by Formula 3 below, when A is -N=, R₂ is hydrogen, R₃ is 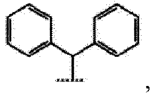 n is 4, R₄ is heptylcarbonyl, Cy is phenyl, R₅ is methyl, R₆ is S(=O)₂OH, R₇, R₈ and R₁₀ are each independently hydrogen, and R₉ is -OH, and the amino acid of SEQ ID NO: 1 may be a substituted form, such as His(benzhydryl)-Lys(caprylic)-d-Phe(4-methyl)-Tyr. The IUPAC name of the compound represented by Formula 3 below is ((R)-2-((S)-2-((S)-2-amino-3-(1-benzhydryl-1H-imidazol-4-yl)propanamido)-6-octanamidohexanamido)-3-(p-tolyl)propanoyl)-L-tyrosine, and in one embodiment of the present invention, it was named HKFY-34.

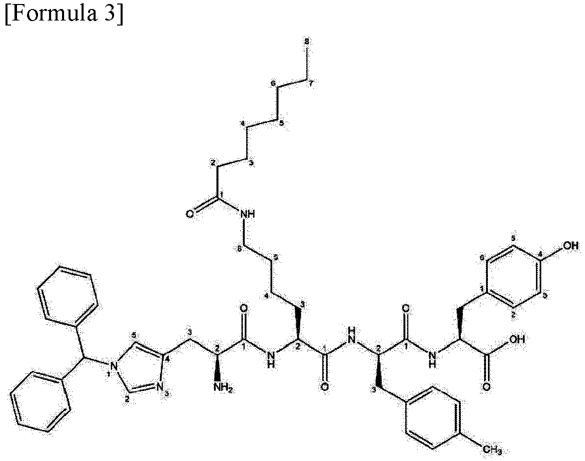

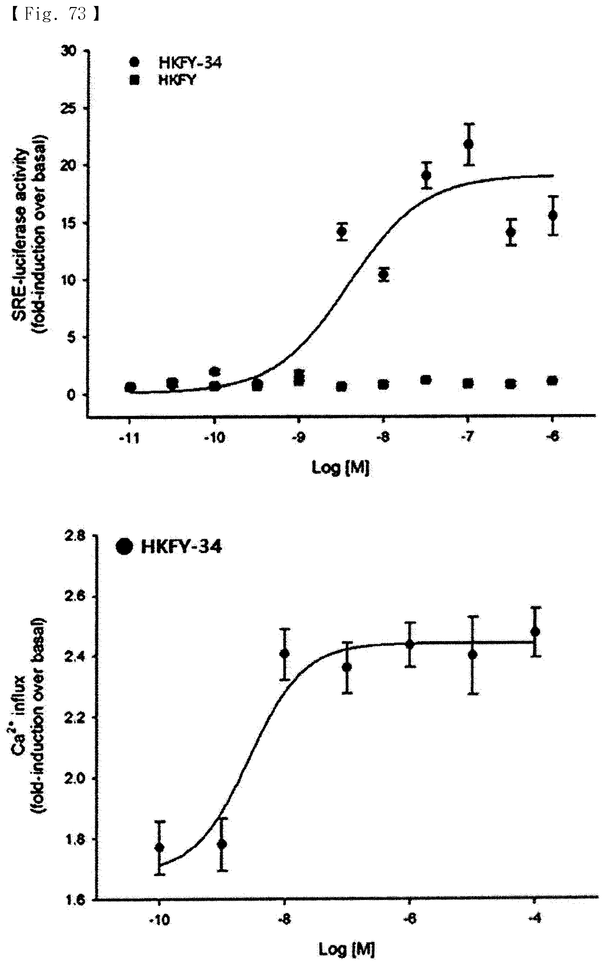

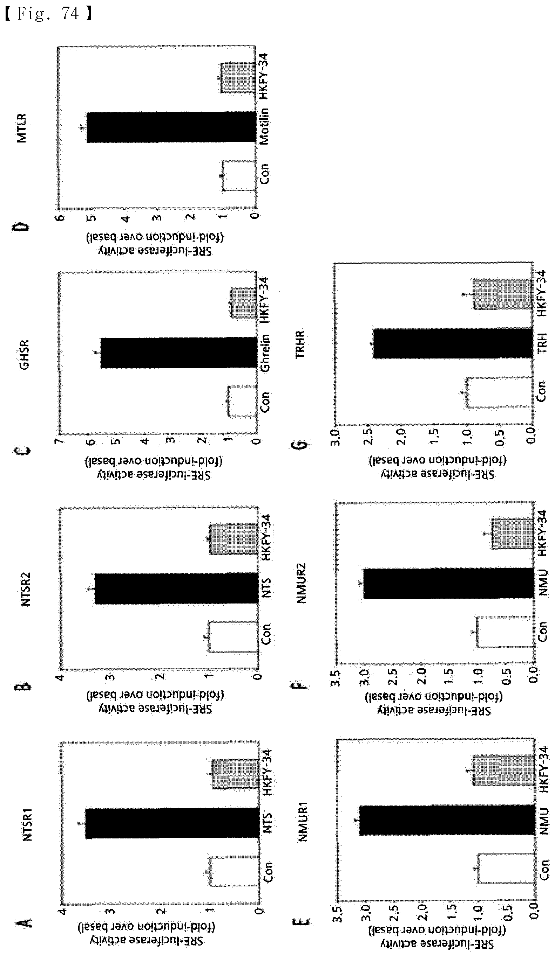

In one embodiment of the present invention, it was confirmed that the peptide derivative, which is a compound having the structure of Formula 1 according to the present invention, selectively and specifically binds to GPR39 (G protein coupled receptor 39), one of the orphan G protein coupled receptors (orphan GPCR), which is a membrane protein (Tables 3 to 5 and Figs. 73 and 74).

In another aspect of the present invention, there is provided a pharmaceutical composition for preventing or treating inflammatory bowel disease, comprising the compound or pharmaceutically acceptable salt thereof as an active ingredient.

As used herein, the term "inflammation" is a response to protect a living body against harmful factors, and includes a series of processes involving immune cells, blood vessels, and inflammatory mediators to inhibit cell damage, remove damaged tissue and necrotic cells, and regenerate tissue.

As used herein, the term "inflammatory bowel disease (IBD)" refers to a disease that causes chronic inflammation in the gastrointestinal tract, and may include both infectious enteritis such as bacterial, viral, amebic, and tuberculous enteritis, and inflammatory diseases occurring in all intestines, such as ischemic bowel disease and radiation enteritis. Although the cause of inflammatory bowel disease has not been clearly identified, it is known to be affected by genetic and environmental influences and mediated by immunological abnormalities. The inflammatory bowel disease may be Crohn's disease, ulcerative colitis, intestinal Behcet's disease and enteritis, but is not limited thereto.

The "Crohn's disease" is a chronic inflammatory bowel disease that affects the entire gastrointestinal tract from the oral cavity to the anus. It is an autoimmune disease, most of which occurs in the ileocaecal region, which is the border between the small intestine and the large intestine, and occurs frequently in the large intestine, terminal ileum, and small intestine. Crohn's disease is characterized by repeated symptomatic and asymptomatic phases showing symptoms such as abdominal pain and diarrhea. The cause of the disease is not clear, but it is assumed that mycobacterial infection, measles virus infection, and an excessive immune response to normal bacteria in the digestive tract are involved. "Ulcerative colitis" is a kind of diffuse nonspecific inflammation disease that mainly invades mucous membranes and forms rotting or ulcers. The cause of ulcerative colitis is not clearly identified, and it causes various systemic symptoms such as bloody diarrhea. "Behcet's disease" is a disease in which oral and genital ulcers and ocular inflammation appear repeatedly, and is a systemic and chronic disease that affects the cardiovascular system, nervous system, digestive tract, liver, spleen, kidney and lung. When Behcet's disease affects the intestinal tract, it is called "intestinal Behcet's disease". Intestinal Behcet's disease is a nonspecific, recurrent chronic inflammatory disease, which is accompanied by symptoms such as abdominal pain and diarrhea, and may be accompanied by complications such as bleeding and perforation.

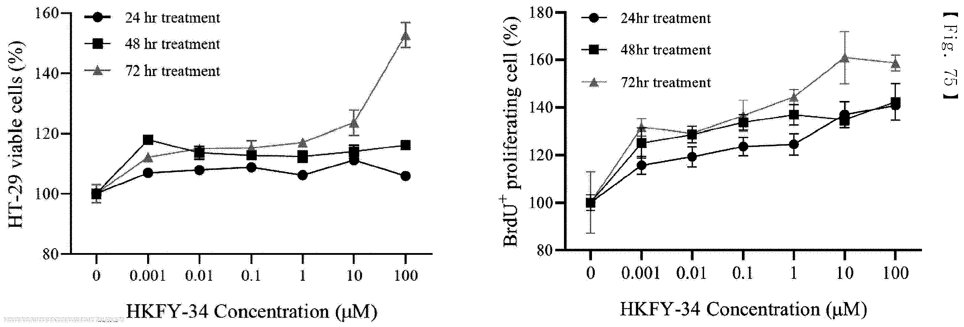

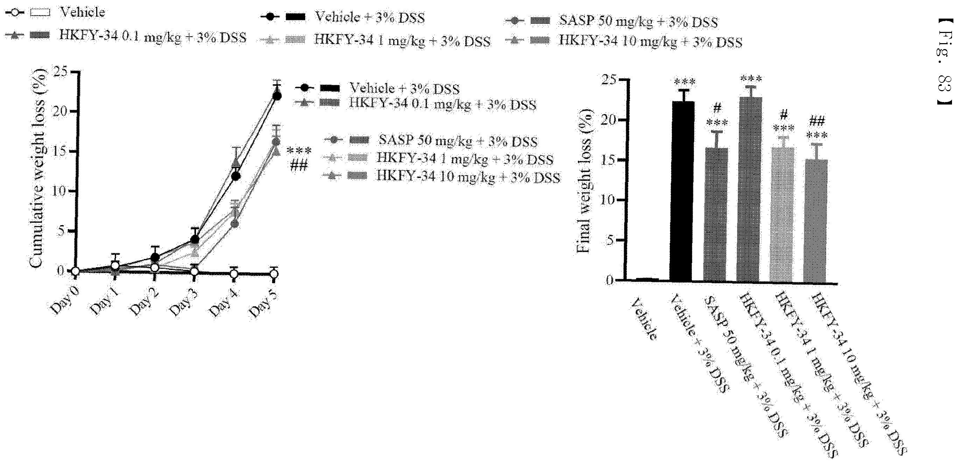

In one embodiment of the present invention, when HT-29 cells were treated with the peptide derivative according to the present invention, cell proliferation and binding were enhanced (Figs. 75 to 78). In addition, it alleviated inflammation in a cell model of inflammatory bowel disease (IBD) (Fig. 81). When an ulcerative colitis mouse model was treated with the peptide derivative according to the present invention, body weight loss and intestine length contraction, which are symptoms of ulcerative colitis, were inhibited, and diarrhea, blood in stool, and inflammatory response in intestinal tissue were alleviated. Therefore, it was confirmed that the therapeutic effect for inflammatory bowel disease was excellent (Figs. 83 to 89). In addition, when GPR39 deficient mice in which ulcerative colitis was induced were treated with the peptide derivative according to the present invention, no significant alleviating effect was observed (Figs. 91 to 95). Therefore, it was confirmed that the peptide derivative according to the present invention acts specifically on GPR39 against inflammatory bowel disease.

As used herein, the term "prevention" refers to any action that inhibits or delays the occurrence, spread, and recurrence of a target disease by administration of the composition. As used herein, the term "treatment" refers to any action that improves or beneficially changes the symptoms of a target disease by administration of the composition. Specifically, it includes curing, alleviating, avoiding, preventing, delaying or reducing symptoms caused by inflammatory bowel disease.

The pharmaceutical composition may further comprise suitable carriers, excipients and diluents commonly used in the preparation of pharmaceutical compositions. In addition, it can be formulated and used in the form of oral formulations such as powders, granules, tablets, capsules, suspensions, emulsions, syrups, aerosols, external preparations, suppositories, and sterile injection solutions according to conventional methods. Suitable preparations known in the art may be those disclosed in the literature (Remington's Pharmaceutical Science, Mack Publishing Company, Easton PA).

In addition, the pharmaceutical composition may comprise carriers, excipients and diluents including lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, mineral oil, and the like. When formulating the composition, it may be prepared using diluents or excipients such as fillers, extenders, binders, wetting agents, disintegrants, and surfactants, which are commonly used.

Solid preparations for oral administration of the "pharmaceutical composition" of the present invention may include tablets, pills, powders, granules, capsules, and the like. Such a solid preparation may be prepared by mixing the composition with at least one or more excipients, for example, starch, calcium carbonate, sucrose, lactose, gelatin, and the like. In addition to simple excipients, lubricants such as magnesium stearate and talc may also be used. Liquid preparations for oral administration may include suspensions, internal solutions, emulsions, syrups, and the like, and may comprise, in addition to water and liquid paraffin, which are commonly used simple diluents, various excipients such as wetting agents, sweeteners, flavouring agents, and preservatives.

Preparations for parenteral administration may include sterilized aqueous solutions, non-aqueous solvents, suspensions, emulsions, freeze-dried preparations, and suppositories. Propylene glycol, polyethylene glycol, vegetable oils such as olive oil, and injectable esters such as ethyl oleate may be used as non-aqueous solvents and suspending agents. As a base for the suppository, witepsol, macrogol, tween 61, cacao butter, laurin butter, glycerogelatin and the like may be used.

The composition is preferably formulated in a dosage form specific to a particular patient.

For each unit dose for oral administration, it is preferable to contain the compound of Formula 1 or a pharmaceutically acceptable salt thereof in an amount of 0.1 mg/kg to 500 mg/kg, 1 mg/kg to 100 mg/kg, or 10 mg/kg to 50 mg/kg.

A preferred dosage of the pharmaceutical composition varies depending on the condition and body weight of the patient, the severity of the disease, the type of drug, the route and duration of administration, but can be appropriately selected by one of ordinary skill in the art.

A daily administration dose suitable for oral administration is about 0.01 to 40 mg/kg of the compound of Formula 1 or a pharmaceutically acceptable salt thereof, and may be administered 1 to 6 times a day depending on the condition of the patient.

In addition, the pharmaceutical composition may be used alone or in combination with methods using surgery, hormone therapy, chemotherapy, and biological response modifiers for the prevention and treatment of inflammatory bowel disease.

In another aspect of the present invention, there is provided a method for preventing or treating inflammatory bowel disease, comprising administering the compound or pharmaceutically acceptable salt thereof to a mammal. In this case, the compound or a salt thereof, inflammatory bowel disease, administration, prevention and treatment are the same as described above.

In another aspect of the present invention, there is provided the use of the compound or pharmaceutically acceptable salt thereof for the manufacture of a medicament for preventing or treating inflammatory bowel disease. In this case, the compound or a salt thereof, inflammatory bowel disease, prevention and treatment are the same as described above.

In another aspect of the present invention, there is provided a health functional food composition for preventing or mitigating inflammatory bowel disease, comprising the compound or cytologically acceptable salt thereof as an active ingredient. In this case, the compound or a salt thereof, inflammatory bowel disease and prevention are the same as described above.

The health functional food according to the present invention may be in the form of powder, granule, tablet, capsule or beverage, and may be candy, chocolate, gum, tea, vitamin complex, health supplement food, and the like. The health functional food may be prepared in various forms such as tablets, capsules, powders, granules, liquids, pills, and the like in order to obtain useful effects for mitigating inflammatory bowel disease, but is not limited thereto.

In this case, the content of the compound or cytologically acceptable salt thereof according to the present invention included in the health functional food may be typically 0.01 to 50 % by weight, or 0.1 to 20 % by weight based on the total food weight. In addition, in the case of the health beverage composition, it may be included in an amount of 0.02 to 10 g, or 0.3 to 1 g, based on 100 mL of the health beverage composition.

The food may further comprise a cytologically acceptable food supplementary additive together with the compound or cytologically acceptable salt thereof according to the present invention.

The health functional food according to the present invention may comprise additives that are commonly used in food compositions to improve smell, taste, appearance, and the like. For example, vitamins A, C, D, E, B 1, B2, B6, B 12, niacin, biotin, folate, panthotenic acid, and the like may be included. In addition, minerals such as zinc (Zn), iron (Fe), calcium (Ca), chromium (Cr), magnesium (Mg), manganese (Mn), and copper (Cu) may be included. In addition, amino acids such as lysine, tryptophan, cysteine, and valine may be included.

In addition, food additives such as preservatives (potassium sorbate, sodium benzoate, salicylic acid, sodium dehydroacetate, etc.), disinfectants (bleaching powder, high bleaching powder, sodium hypochlorite, etc.), antioxidants (butylhydroxyanisole (BHA), butylhydroxytoluene (BHT), etc.), coloring agents (tar color, etc.), color formers (sodium nitrite, sodium nitrite, etc.), bleaching agents (sodium sulfite), seasonings (MSG sodium glutamate, etc.), sweeteners (dulcin, cyclemate, saccharin, sodium, etc.), flavors (vanillin, lactones, etc.), expanding agents (alum, D-potassium hydrogen tartrate, etc.), strengthening agents, emulsifiers, thickeners, coating agents, gum base agents, foam inhibitors, solvents, and modifiers may be added. The additive may be selected according to the type of food and used in an appropriate amount.

### Mode for Carrying out the Invention

Hereinafter, the present invention will be described in detail by way of Examples. However, the following examples are only for illustrating the present invention, and the present invention is not limited to the following examples.

### Example 1. Preparation of HKFY derivatives

### Example 1.1. Preparation of HKFY-1

In order to prepare His(trt)-Lys(caprylic)-Phe-Tyr (N6-octanoyl-N2-(Nt-trityl-L-histidyl)-L-lysyl-L-phenylalanyl-L-tyrosine, HKFY-1), Fmoc-Tyr(tBu) and DMF (dimethylformamide) were loaded onto the trityl resin to prepare Fmoc-Tyr(tBu)-trityl resin. DMF containing 20% piperidine and Fmoc-Phe-OH and HOBt (hydroxyl-benzotriazole) were added to Fmoc-Tyr(tBu)-trityl resin to prepare Fmoc-Phe-Tyr(tBu)-trityl resin.

DMF containing 2% NH₂NH₂ .H₂O was added to Fmoc-Lys(dde)-OH, and dde was removed to prepare Fmoc-Lys-OH. DMF containing caprylic acid and HOBt and DIC was added to the Fmoc-Lys-OH to prepare Fmoc-Lys(caprylic acid)-OH.

DMF containing 20% piperidine was added to the Fmoc-Phe-Tyr(tBu)-trityl resin, and HOBt (hydroxyl-benzotriazole) was added to the Fmoc-Lys(caprylic acid)-OH to prepare Fmoc-Lys(caprylic acid)-Phe-Tyr(tBu)-trityl resin.

DMF containing 20% piperidine was added to the Fmoc-Lys(caprylic acid)-Phe-Tyr(tBu)-trityl resin, and Boc-His(trt)-OH and HOBt (hydroxyl-benzotriazole) were added to prepare Boc-His(trt)-Lys(caprylic acid)-Phe-Tyr(tBu)-trityl resin.

The protecting group was cleaved from the Boc-His(trt)-Lys(caprylic acid)-Phe-Tyr(tBu)-trityl resin, and the resin was then purified to prepare His(trt)-Lys(caprylic acid)-Phe-Tyr.

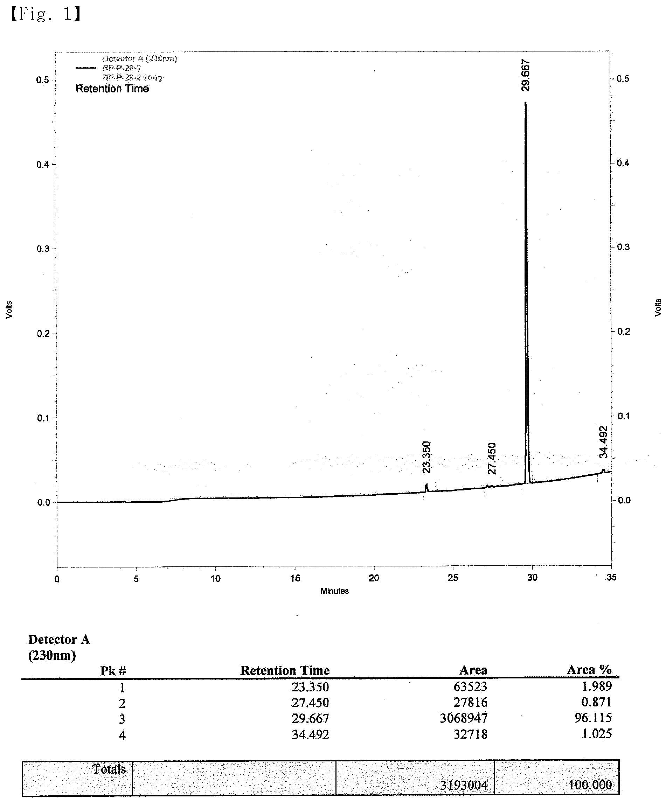

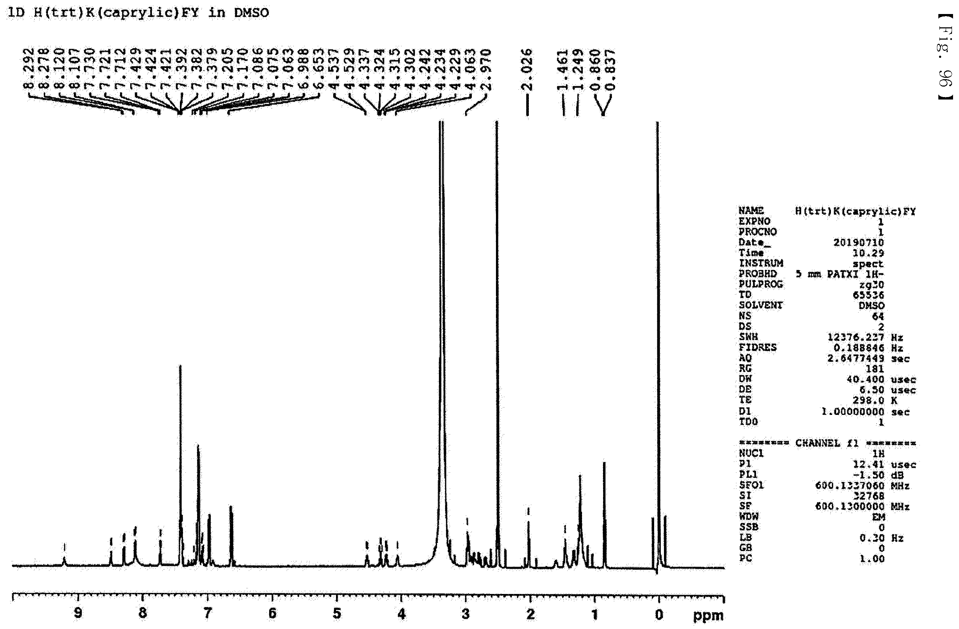

The purity, molecular weight and ¹H NMR of the HKFY-1 are shown in Figs. 1, 37 and 96, respectively.

### Example 1.2. Preparation of HKFY-2

In order to prepare biotin ((S)-3-([1,1'-biphenyl]-4-yl)-2-((S)-6-octanamido-2-((S)-2-(5-((3aS,4S,6aR)-2-oxohexahydro-1H-thieno[3,4-d]imidazol-4-yl)pentanamido)-3-(1-trityl-1H-imidazol-4-yl)propanamido)hexanamido)propanoyl)-L-tyrosine, DMF containing 20% piperidine was added to the Fmoc-Lys(caprylic acid)-Phe-Tyr(tBu)-trityl resin obtained from Example 1.1., and Fmoc-His(trt)-OH was added to prepare Fmoc-His(trt)-Lys(caprylic acid)-Phe-Tyr(tBu)-trityl resin. D-biotin was added to the resin in the same manner to prepare D-biotin-His(trt)-Lys(caprylic acid)-Phe-Tyr(tBu)-trityl resin.

The protecting group of the resin was cleaved, and the resin was then purified to prepare biotin-His(trt)-Lys(caprylic acid)-Phe-Tyr (N6-octanoyl-N2-(Na-(5-((3aS,4S,6aR)-2-oxohexahydro-1H-thieno[3,4-d]imidazol-4-yl)pentanoyl)-Nt-trityl-L-histidyl)-L-lysyl-L-phenylalanyl-L-tyrosine).

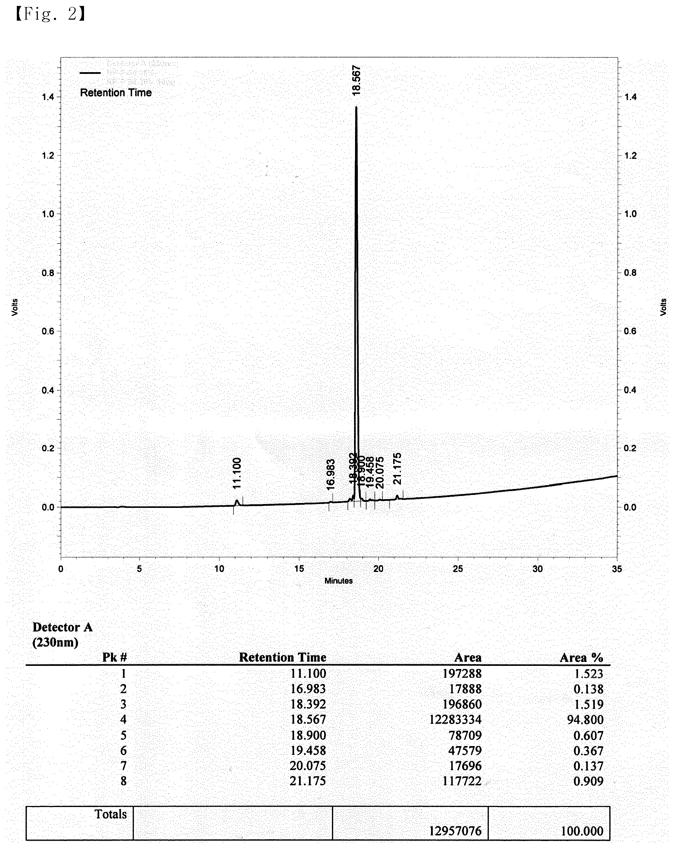

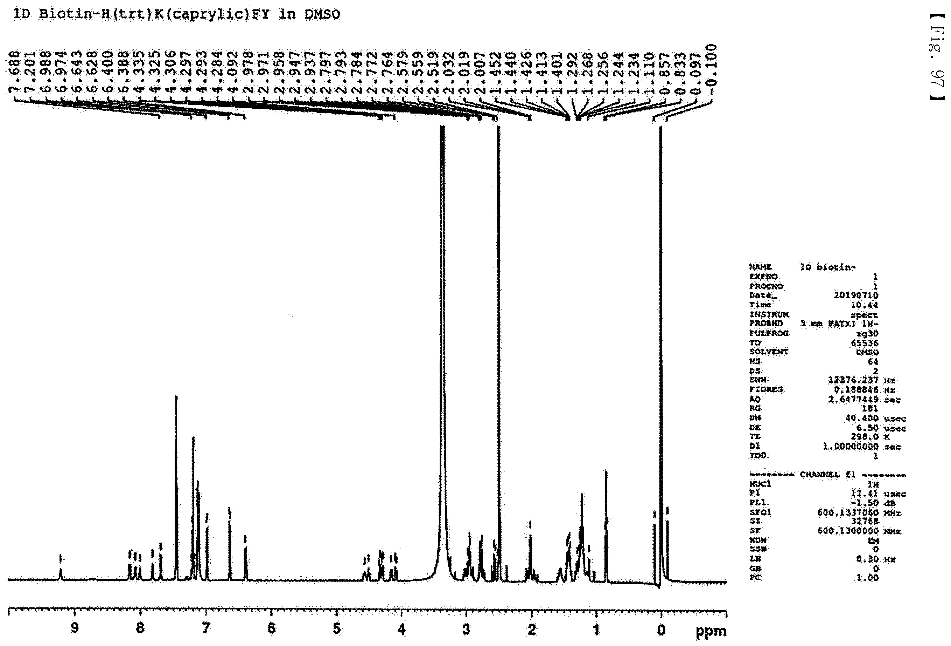

The purity, molecular weight and ¹H NMR of the HKFY-2 are shown in Figs. 2, 38 and 97, respectively.

### Example 1.3. Preparation of HKFY-3

His(benzyl)-Lys(caprylic)-Phe-Tyr (N2-(Nt-benzyl-L-histidyl)-N6-octanoyl-L-lysyl-L-phenylalanyl-L-tyrosine) was prepared in the same manner except that Boc-His(benzyl)-OH was used instead of Boc-His(trt)-OH obtained during the preparation process of Example 1.1.

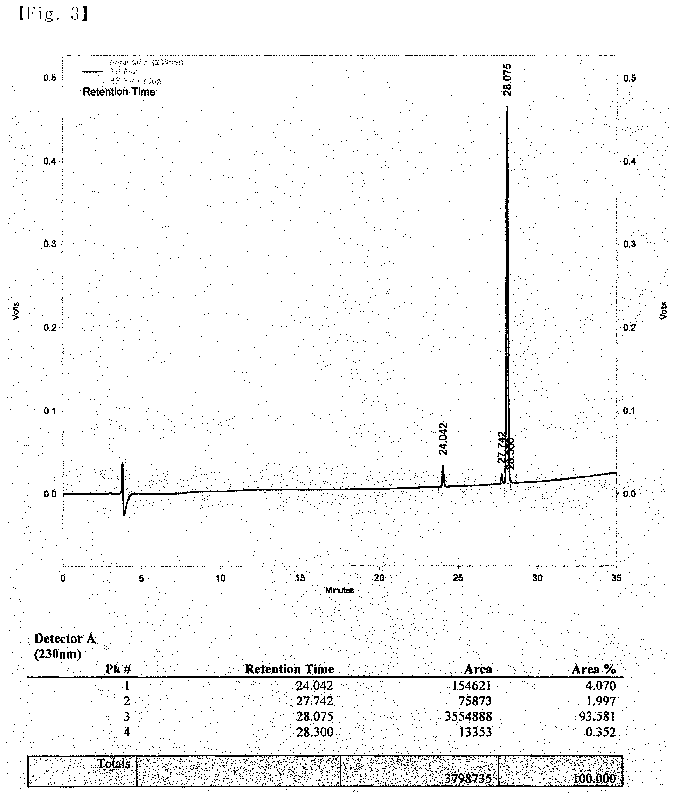

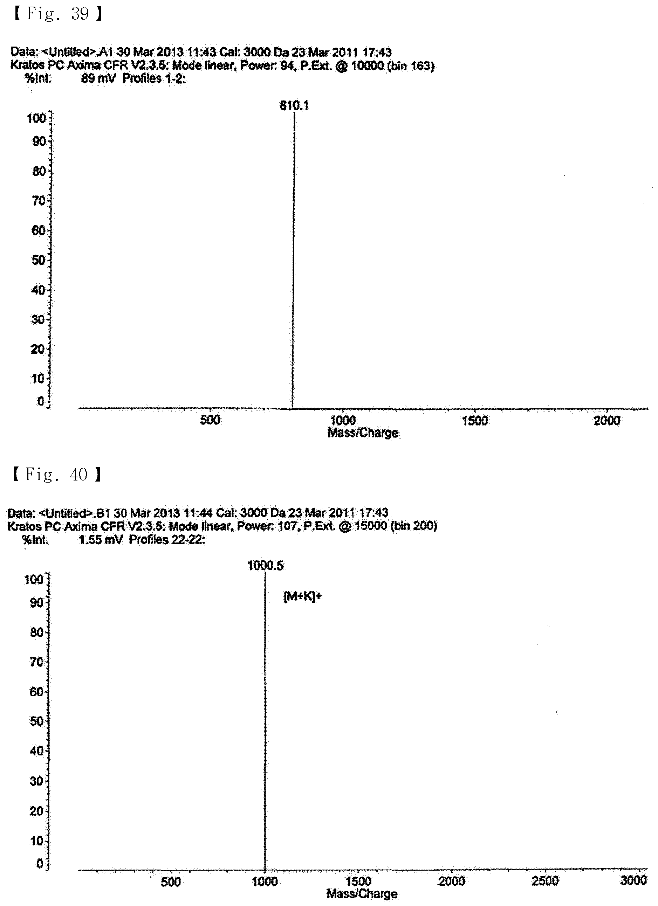

The purity and molecular weight of the HKFY-3 are shown in Figs. 3 and 39, respectively.

### Example 1.4. Preparation of HKFY-4

d-His(trt)-Lys(caprylic)-Phe-Tyr (N6-octanoyl-N2-(Nt-trityl-D-histidyl)-L-lysyl-L-phenylalanyl-L-tyrosine) was prepared in the same manner except that Boc-d-His(trt) was used instead of Boc-His(trt)-OH obtained during the preparation process of Example 1.1.

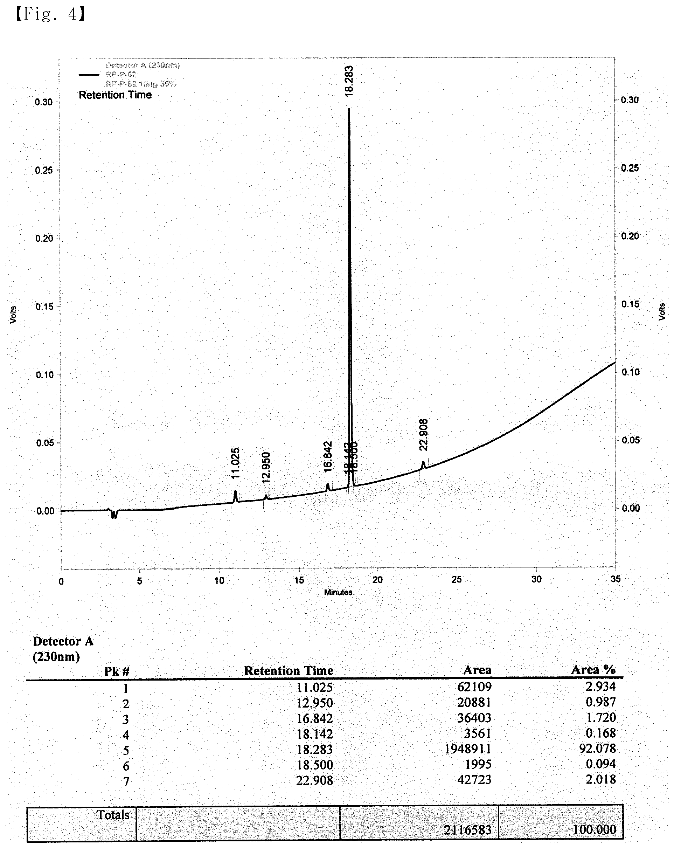

The purity and molecular weight of the HKFY-4 are shown in Figs. 4 and 40, respectively.

### Example 1.5. Preparation of HKFY-5

His(4-methyltrityl)-Lys(caprylic)-Phe-Tyr (N2-(Nt-(diphenyl (p-tolyl)methyl)-L-histidyl)-N6-octanoyl-L-lysyl-L-phenylalanyl-L-tyrosine) was prepared in the same manner except that Boc-His(4-methyltrityl)-OH was used instead of Boc-His(trt)-OH obtained during the preparation process of Example 1.1.

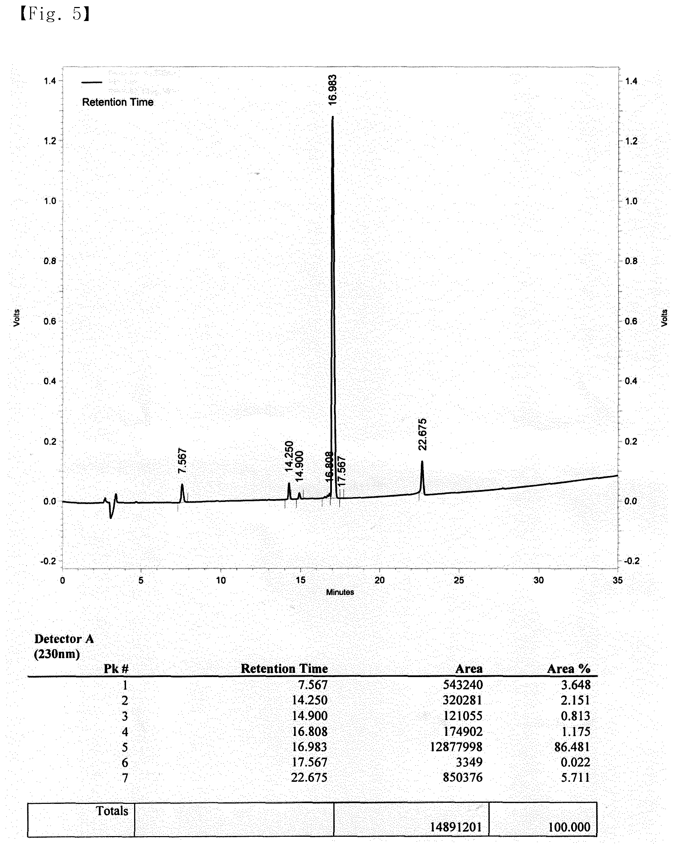

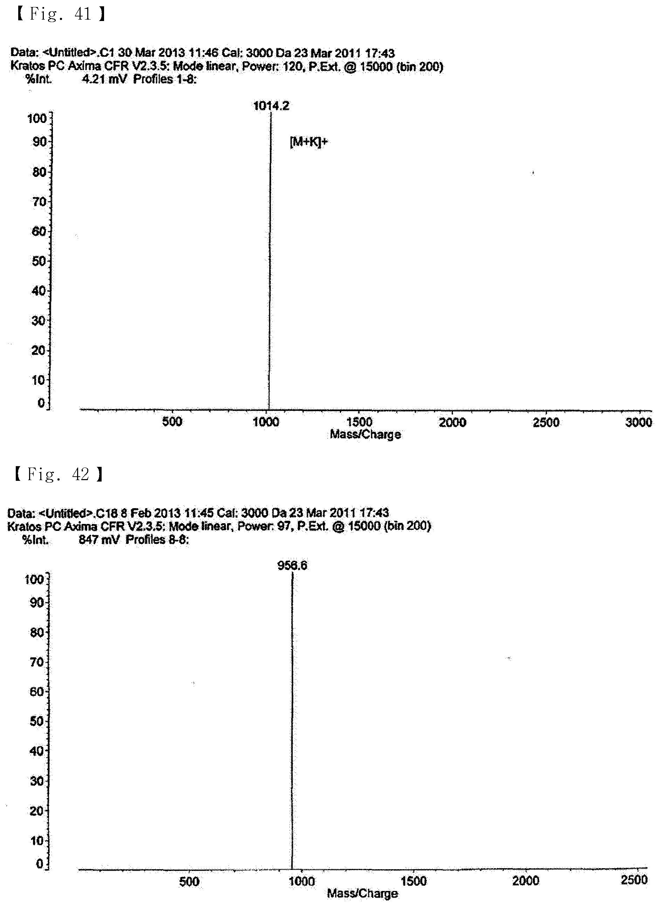

The purity and molecular weight of the HKFY-5 are shown in Figs. 5 and 41, respectively.

### Example 1.6. Preparation of HKFY-6

His(DAMP-3)-Lys(caprylic)-Phe-Tyr (N2-((S)-2-amino-3-(1,3-diphenethyl-2,3-dihydro-1H-imidazol-4-yl)propanoyl)-N6-octanoyl-L-lysyl-L-phenylalanyl-L-tyrosine) was prepared in the same manner except that Boc-His(DAMP-3)-OH was used instead of Boc-His(trt)-OH obtained during the preparation process of Example 1.1.

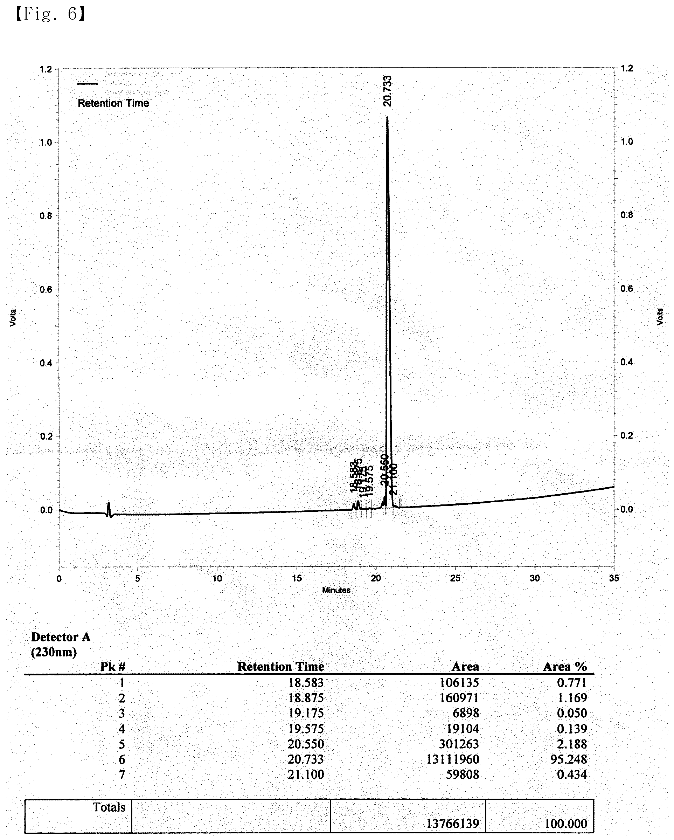

The purity and molecular weight of the HKFY-6 are shown in Figs. 6 and 42, respectively.

### Example 1.7. Preparation of HKFY-7

His(DAMP-5)-Lys(caprylic)-Phe-Tyr (N2-((S)-2-amino-3-(3-(2-cyclohexylethyl)-2,3-dihydro-1H-imidazol-4-yl)propanoyl)-N6-octanoyl-L-lysyl-L-phenylalanyl-L-tyrosine) was prepared in the same manner except that Boc-His(DAMP-5)-OH was used instead of Boc-His(trt)-OH obtained during the preparation process of Example 1.1.

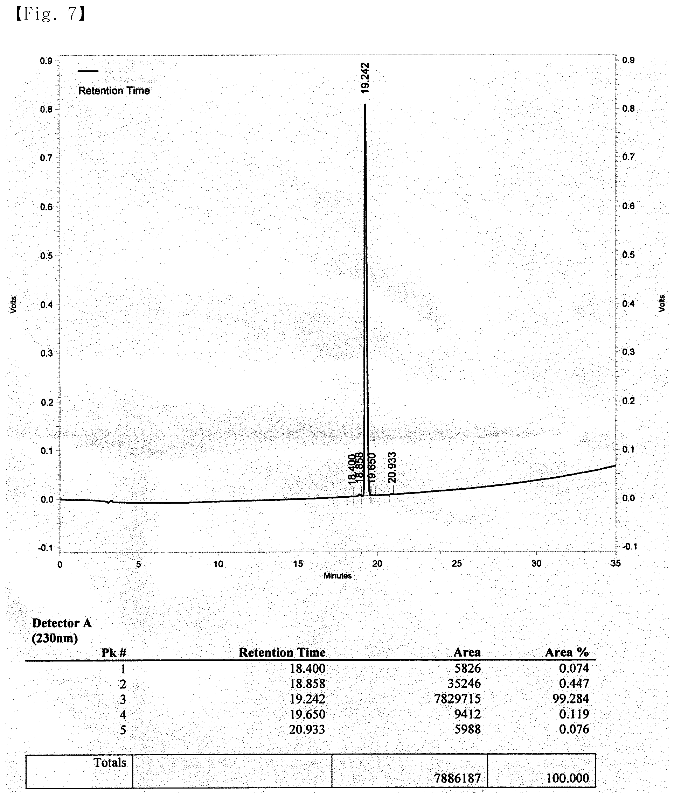

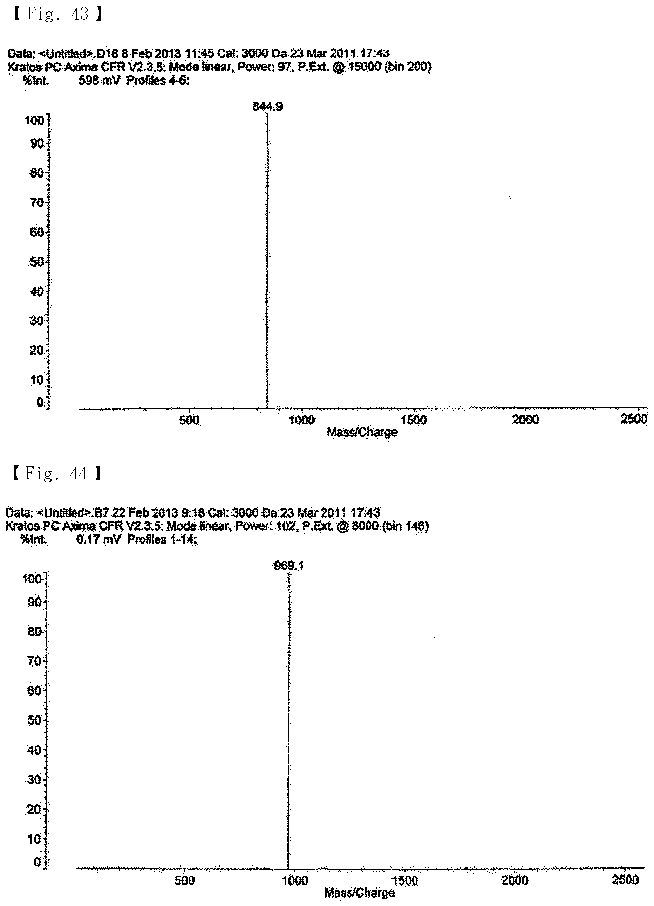

The purity and molecular weight of the HKFY-7 are shown in Figs. 7 and 43, respectively.

### Example 1.8. Preparation of HKFY-8

His(DAMP-2)-Lys(caprylic)-Phe-Tyr (N2-((S)-2-amino-3-(1,3-bis(2-cyclohexylethyl)-2,3-dihydro-1H-imidazol-4-yl)propanoyl)-N6-octanoyl-L-lysyl-L-phenylalanyl-L-tyrosine) was prepared in the same manner except that Boc-His(DAMP-2)-OH was used instead of Boc-His(trt)-OH obtained during the preparation process of Example 1.1.

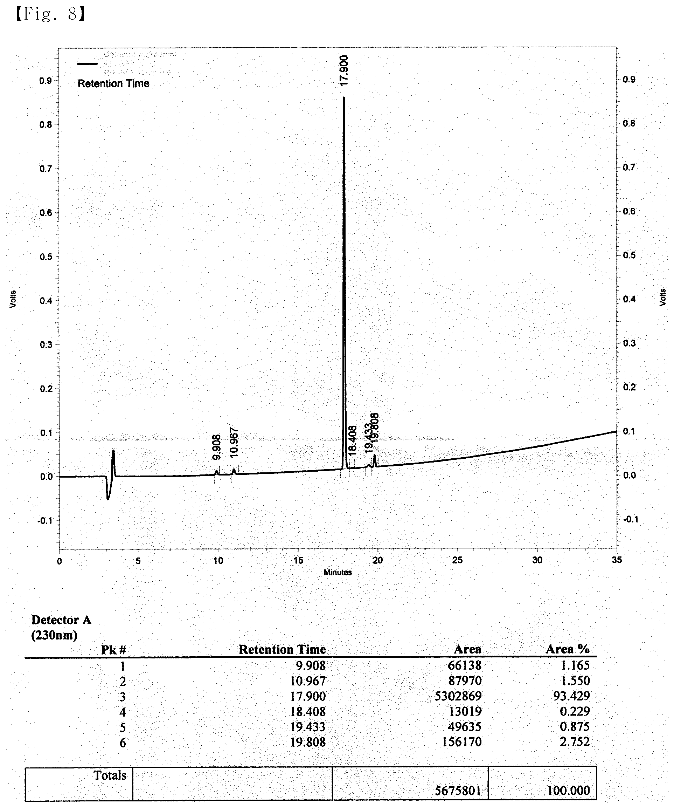

The purity and molecular weight of the HKFY-8 are shown in Figs. 8 and 44, respectively.

### Example 1.9. Preparation of HKFY-9

His(DAMP-2)-Lys(caprylic)-Phe-Tyr (N6-octanoyl-N2-(Nt-phenethyl-L-histidyl)-L-lysyl-L-phenylalanyl-L-tyrosine) was prepared in the same manner except that Boc-His(2-phenylethyl)-OH was used instead of Boc-His(trt)-OH obtained during the preparation process of Example 1.1.

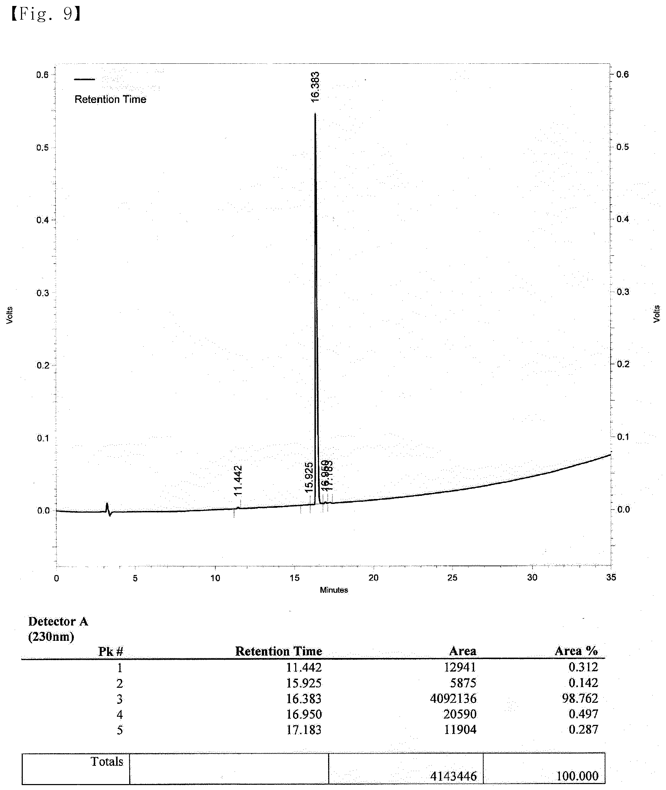

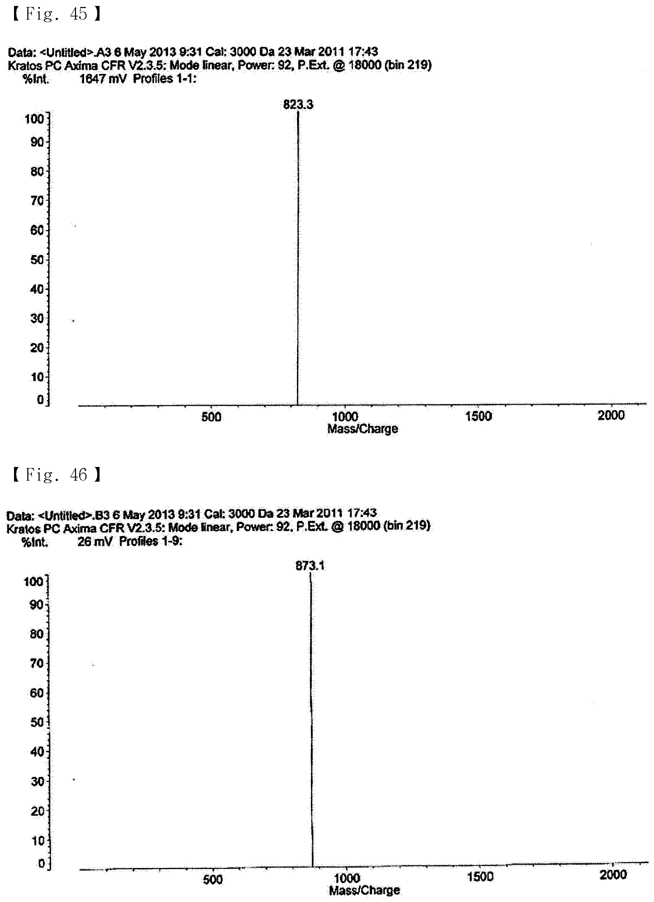

The purity and molecular weight of the HKFY-9 are shown in Figs. 9 and 45, respectively.

### Example 1.10. Preparation of HKFY-10

His(2-naphthalen-1 -ethyl)-Lys(caprylic)-Phe-Tyr (N2-(Nt-(naphthalen-1-ylmethyl)-L-histidyl)-N6-octanoyl-L-lysyl-L-phenylalanyl-L-tyrosine) was prepared in the same manner except that Boc-His(2-naphthalen-1-ethyl)-OH was used instead of Boc-His(trt)-OH obtained during the preparation process of Example 1.1.

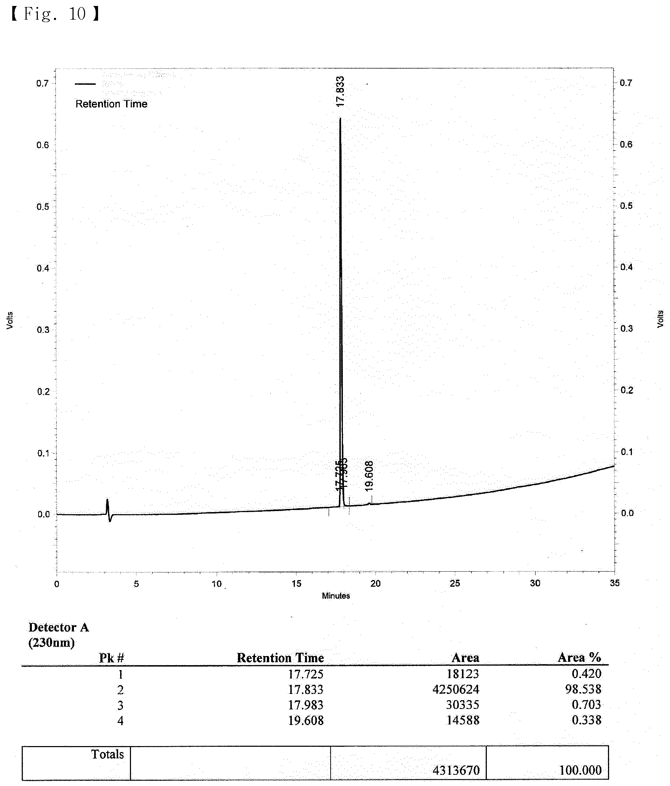

The purity and molecular weight of the HKFY-10 are shown in Figs. 10 and 46, respectively.

### Example 1.11. Preparation of HKFY-11

His(2-cyclohexylethyl)-Lys(caprylic acid)-Phe-Tyr (N2-(Nt-(2-cyclohexylethyl)-L-histidyl)-N6-octanoyl-L-lysyl-L-phenylalanyl-L-tyrosine) was prepared in the same manner except that Boc-His(2-cyclohexylethyl)-OH was used instead of Boc-His(trt)-OH obtained during the preparation process of Example 1.1.

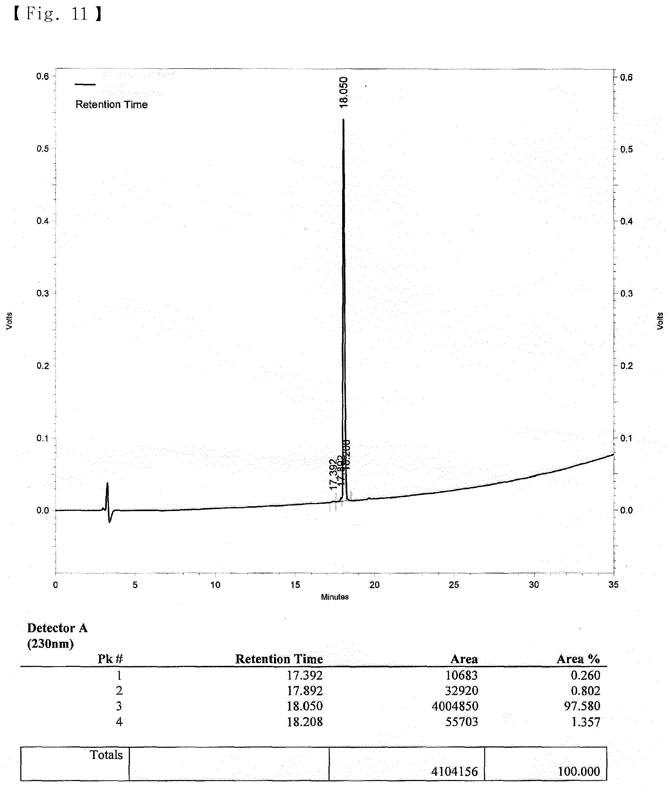

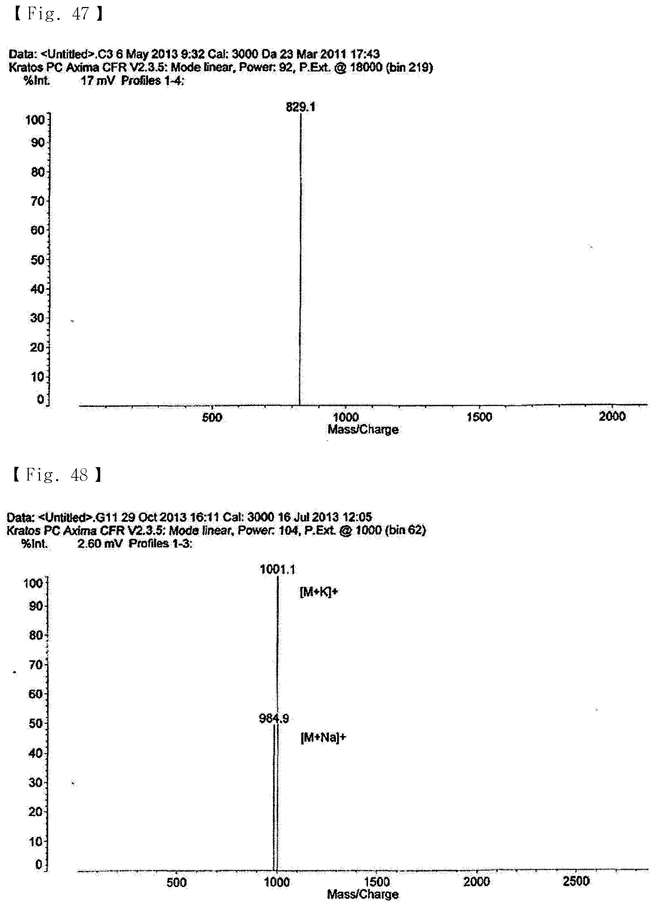

The purity and molecular weight of the HKFY-11 are shown in Figs. 11 and 47, respectively.

### Example 1.12. Preparation of HKFY-12

In order to prepare His(trt)-Lys(caprylic)-d-Phe-Tyr (N6-decanoyl-N2-(Nt-trityl-L-histidyl)-L-lysyl-D-phenylalanyl-L-tyrosine, HKFY-12), Fmoc-Tyr(tBu) and DMF (dimethylformamide) were loaded onto the trityl resin to prepare Fmoc-Tyr(tBu)-trityl resin. DMF containing 20% piperidine and Fmoc-d-Phe-OH of Formula 8 and HOBt (hydroxyl-benzotriazole) were added to Fmoc-Tyr(tBu)-trityl resin to prepare Fmoc-d-Phe-Tyr(tBu)-trityl resin.

Fmoc-Lys(caprylic acid)-OH and HOBt (hydroxyl-benzotriazole) were added to the Fmoc-d-Phe-Tyr(tBu)-trityl resin to prepare Fmoc-Lys(caprylic acid)-d-Phe-Tyr(tBu)-trityl resin. DMF containing 20% piperidine was added to Fmoc-Lys(caprylic acid)-d-Phe-Tyr(tBu)-trityl resin, and Boc-His(trt)-OH and HOBt (hydroxyl-benzotriazole) were added to prepare Boc-His(trt)-Lys(caprylic acid)-d-Phe-Tyr(tBu)-trityl resin.

The protecting group was cleaved from the Boc-His(trt)-Lys(caprylic acid)-d-Phe-Tyr(tBu)-trityl resin, and the resin was then purified to prepare His(trt)-Lys(caprylic)-d-Phe-Tyr.

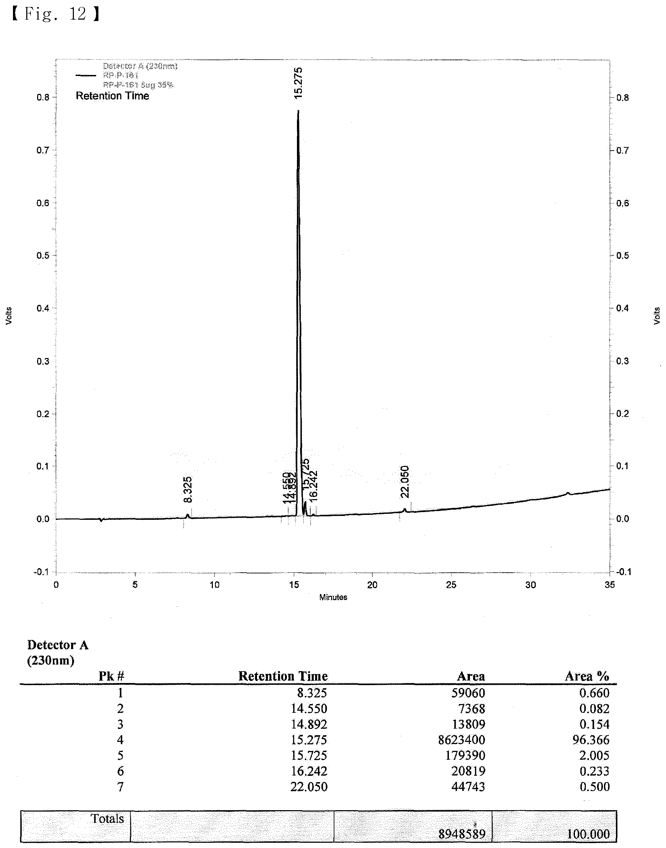

The purity and molecular weight of the HKFY-12 are shown in Figs. 12 and 48, respectively.

### Example 1.13. Preparation of HKFY-13

His(trt)-Lys(caprylic)-d-Phe(4-Cl)-Tyr (((R)-2-((S)-2-((S)-2-amino-3-(1-trityl-1H-imidazol-4-yl)propanamido)-6-(non-1-en-2-ylamino)hexanamido)-3-(4-chlorophenyl)propanoyl)-L-tyrosine, HKFY-13) was prepared in the same manner except that Fmoc-d-Phe(4-Cl)-OH was used instead of Fmoc-d-Phe-OH obtained during the preparation process of Example 1.6.

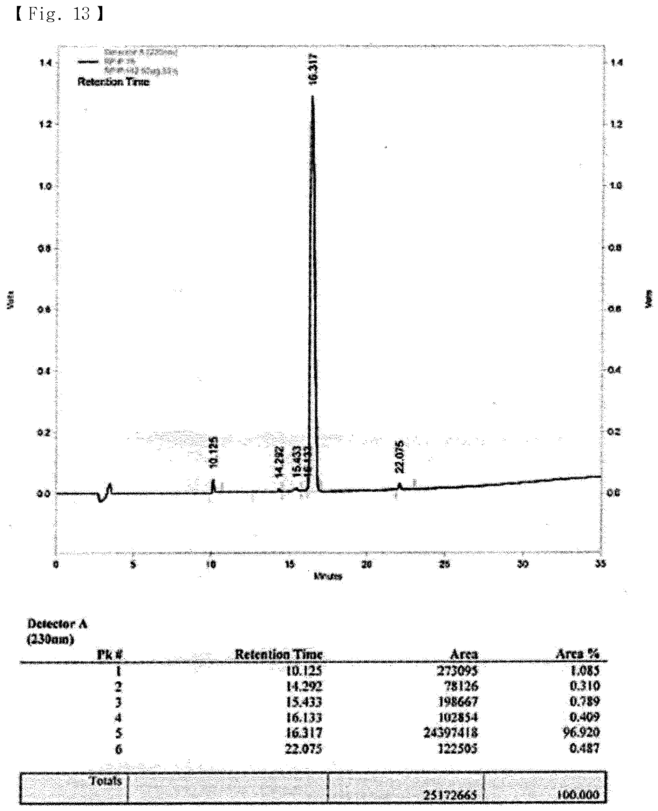

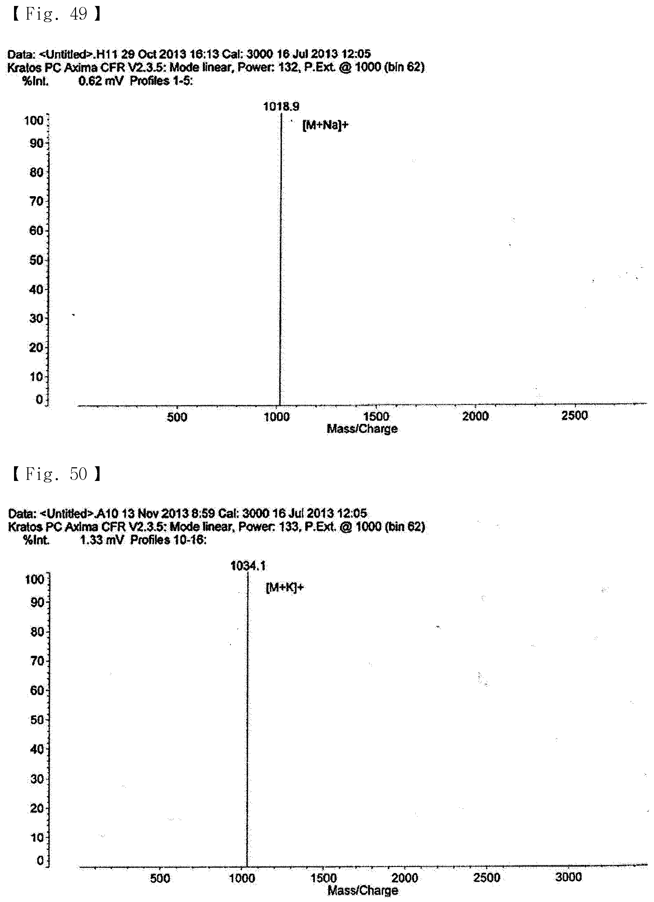

The purity and molecular weight of the HKFY-13 are shown in Figs. 13 and 49, respectively.

### Example 1.14. Preparation of HKFY-14

Fmoc-d-Tyr(tBu)-trityl resin was prepared in the same manner except that Fmoc-d-Tyr(tBu)-OH was used instead of Fmoc-Tyr(tBu)-OH obtained during the preparation process of Example 1.13. During the subsequent process, Boc-His(trt)-OH was changed to Boc-d-His(trt)-OH to prepare d-His(trt)-Lys(caprylic)-d-Phe(4-Cl)-d-Tyr (((R)-2-((S)-2-((R)-2-amino-3-(1-trityl-1H-imidazol-4-yl)propanamido)-6-octanamidohexanamido)-3-(4-chlorophenyl)propanoyl)-D-tyrosine).

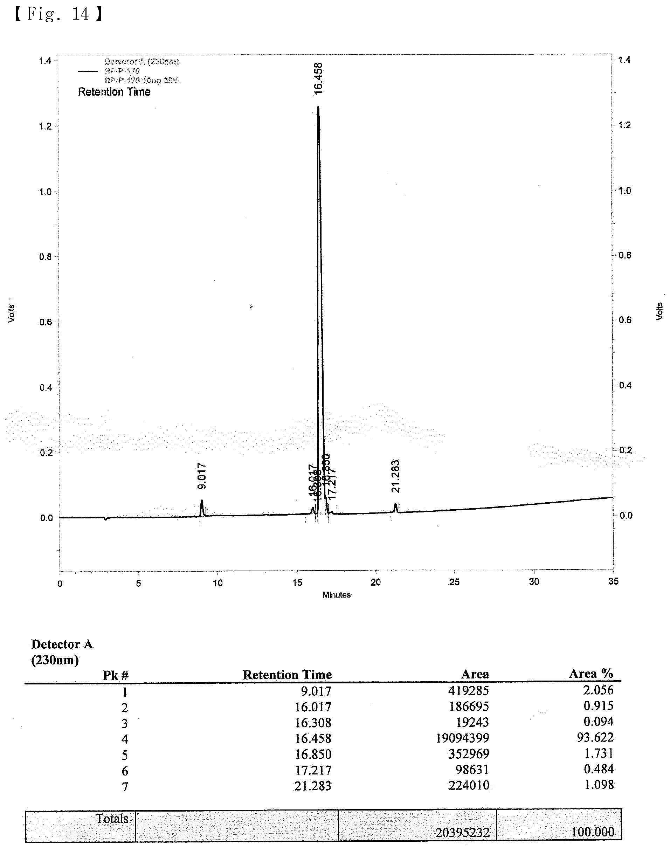

The purity and molecular weight of the HKFY-14 are shown in Figs. 14 and 50, respectively.

### Example 1.15. Preparation of HKFY-15

His(trt)-Lys(caprylic)-d-Phe(4-Cl)-d-Tyr (((R)-2-((S)-2-((S)-2-amino-3-(1 -trityl-1H-imidazol-4-yl)propanamido)-6-octanamidohexanamido)-3-(4-chlorophenyl)propanoyl)-D-tyrosine) was prepared in the same manner except that Boc-His(trt)-OH was used instead of Boc-d-His(trt)-OH obtained during the preparation process of Example 1.14.

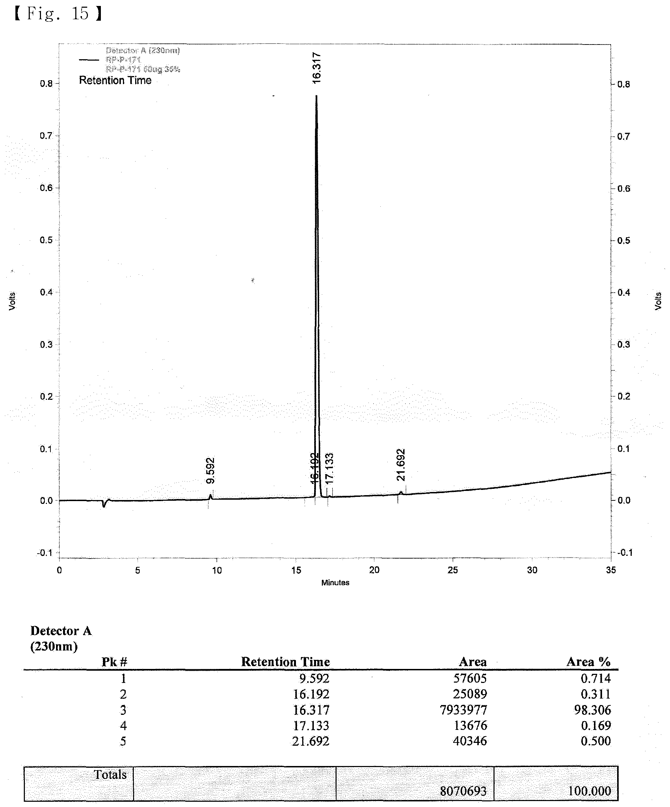

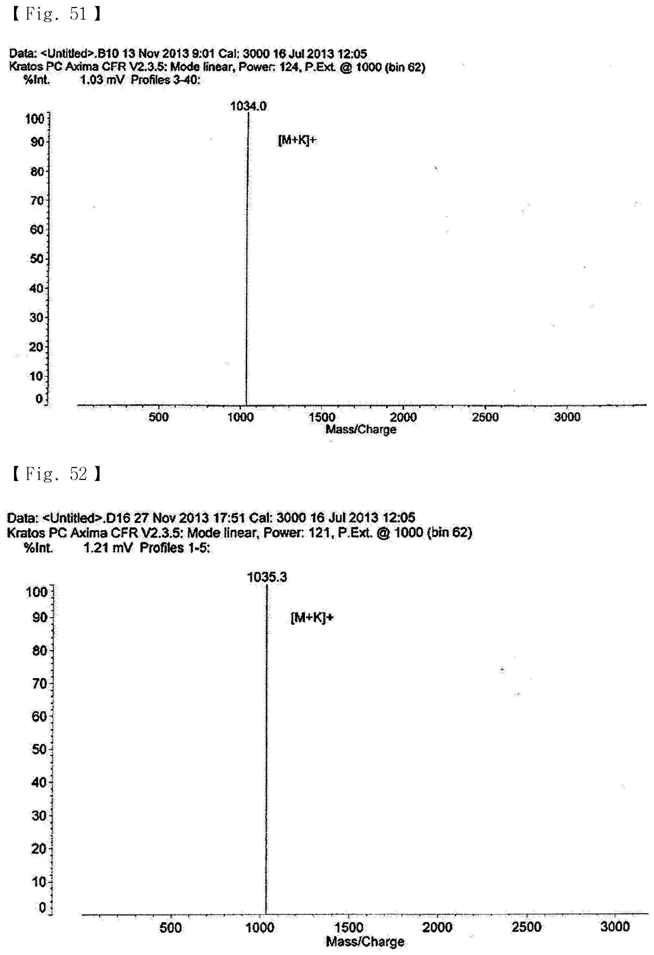

The purity and molecular weight of the HKFY-15 are shown in Figs. 15 and 51, respectively.

### Example 1.16. Preparation of HKFY-16

His(trt)-d-Lys(caprylic)-d-Phe(4-Cl)-Tyr (((R)-2-((R)-2-((S)-2-amino-3-(1 -trityl-1H-imidazol-4-yl)propanamido)-6-octanamidohexanamido)-3-(4-chlorophenyl)propanoyl)-L-tyrosine) was prepared in the same manner as in Example 1.13., except that Fmoc-d-Lys(caprylic)-OH was used by replacing Fmoc-Lys(dde)-OH with Fmoc-d-Lys(dde)-OH in the preparation process using Fmoc-Lys(caprylic)-OH instead of Fmoc-Lys(dde)-OH obtained during the preparation process of Example 1.13.

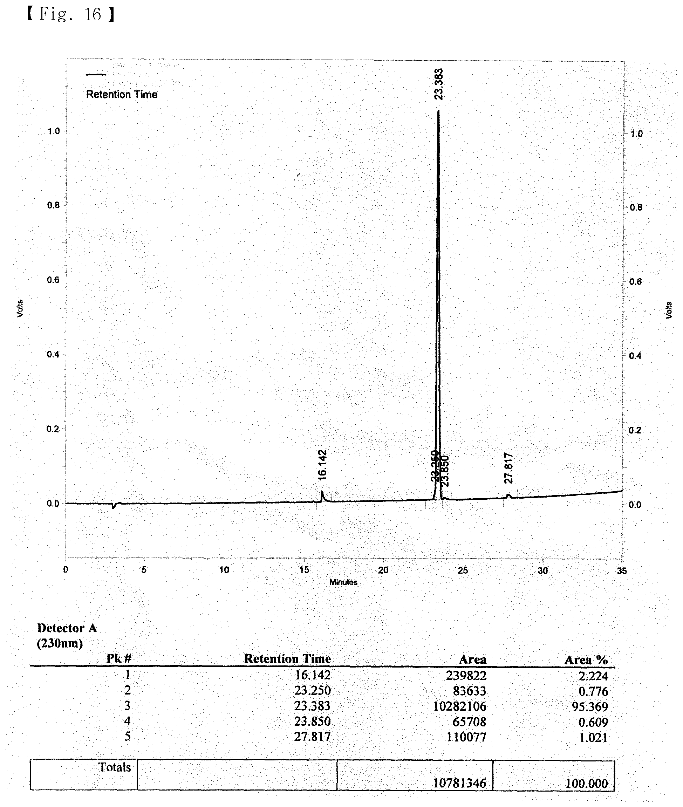

The purity and molecular weight of the HKFY-16 are shown in Figs. 16 and 52, respectively.

### Example 1.17. Preparation of HKFY-17

His(3,3-diphenylpropyl)-Lys(caprylic acid)-Phe-Tyr (N2-(Nt-(2,2-diphenylethyl)-L-histidyl)-N6-octanoyl-L-lysyl-L-phenylalanyl-L-tyrosine) was prepared in the same manner except that Boc-His(3,3-diphenylpropyl)-OH was used instead of Boc-His(trt)-OH obtained during the preparation process of Example 1.1.

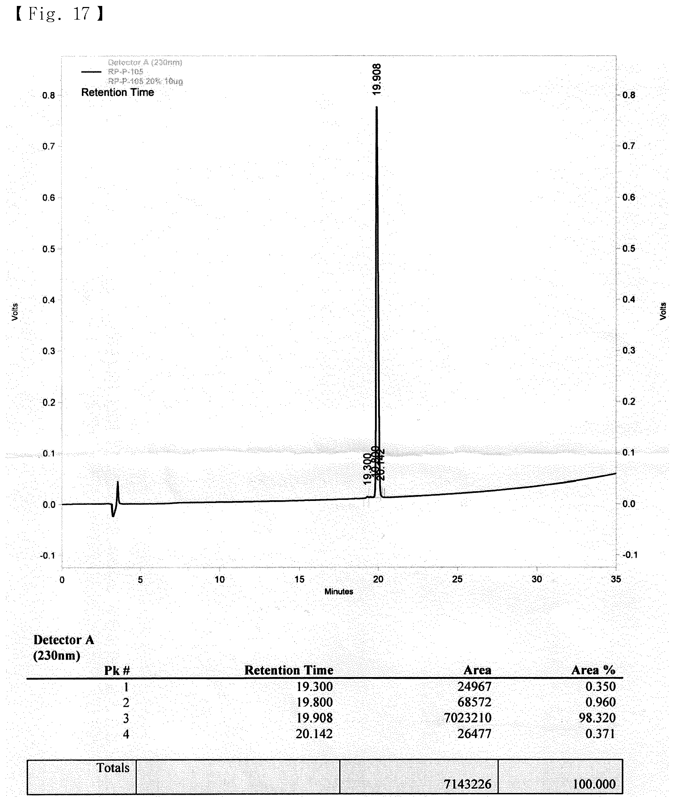

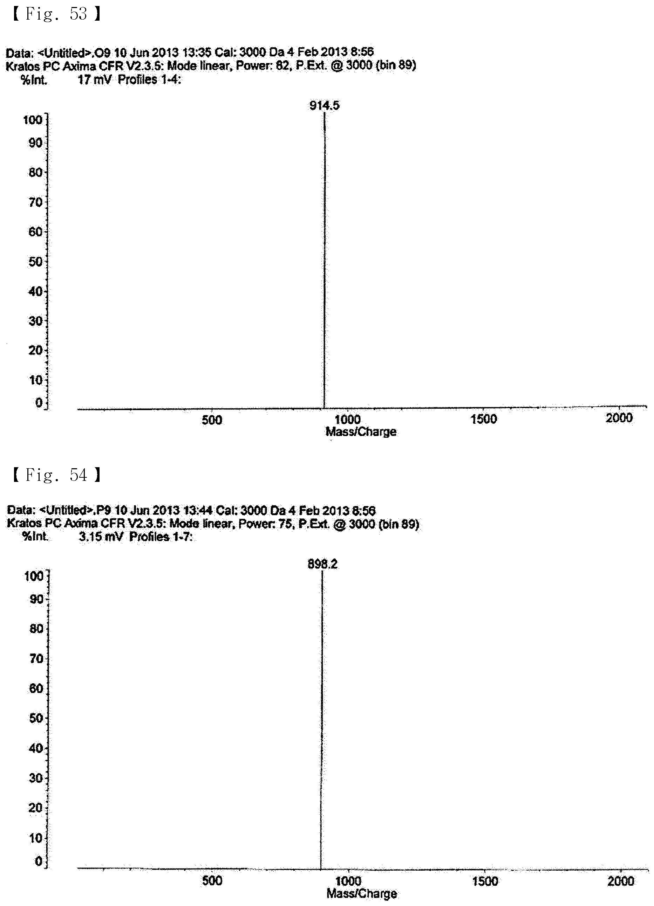

The purity and molecular weight of the HKFY-17 are shown in Figs. 17 and 53, respectively.

### Example 1.18. Preparation of HKFY-18

His(Fm)-Lys(caprylic acid)-Phe-Tyr (N2-(Nt-(9H-fluoren-9-yl)-L-histidyl)-N6-octanoyl-L-lysyl-L-phenylalanyl-L-tyrosine) was prepared in the same manner except that Boc-His(Fm)-OH was used instead of Boc-His(trt)-OH obtained during the preparation process of Example 1.1.

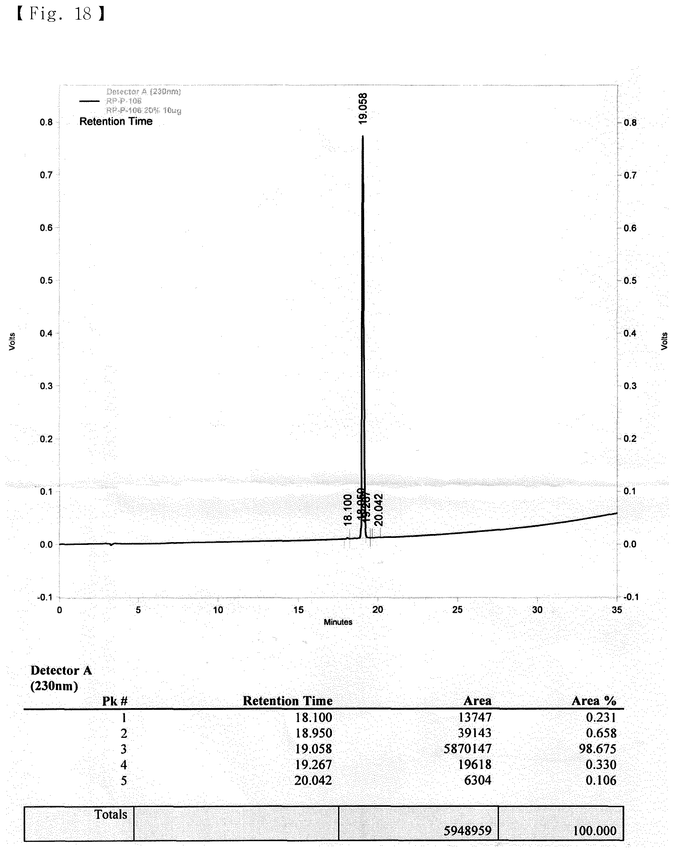

The purity and molecular weight of the HKFY-18 are shown in Figs. 18 and 54, respectively.

### Example 1.19. Preparation of HKFY-19

His(phenylpropyl)-Lys(caprylic acid)-Phe-Tyr (N6-octanoyl-N2-(Nt-phenethyl-L-histidyl)-L-lysyl-L-phenylalanyl-L-tyrosine) was prepared in the same manner except that Boc-His(phenylpropyl)-OH was used instead of Boc-His(trt)-OH obtained during the preparation process of Example 1.1.

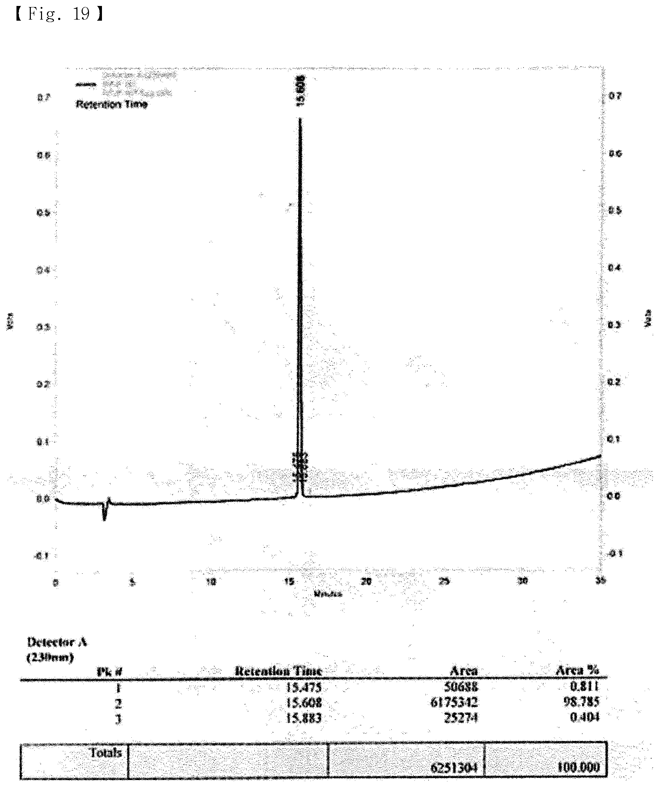

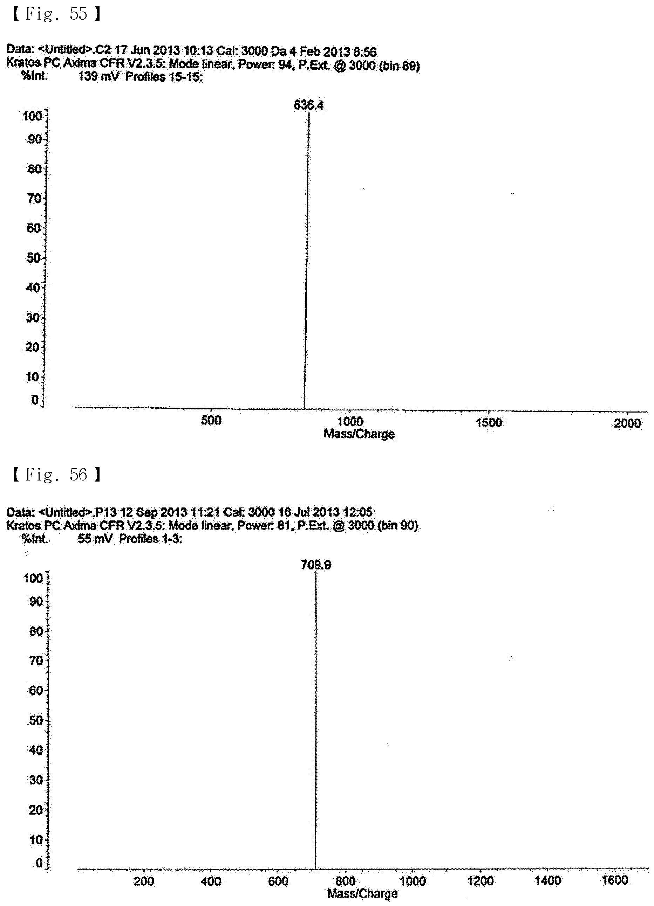

The purity and molecular weight of the HKFY-19 are shown in Figs. 19 and 55, respectively.

### Example 1.20. Preparation of HKFY-20

His(methoxylbenzhydryl)-Lys(caprylic acid)-Phe-Tyr (N2-(Nt-((4-methoxyphenyl)(phenyl)methyl)-L-histidyl)-N6-octanoyl-L-lysyl-L-phenylalanyl-L-tyrosine) was prepared in the same manner except that Boc-His(methoxylbenzhydryl)-OH was used instead of Boc-His(trt)-OH obtained during the preparation process of Example 1.1.

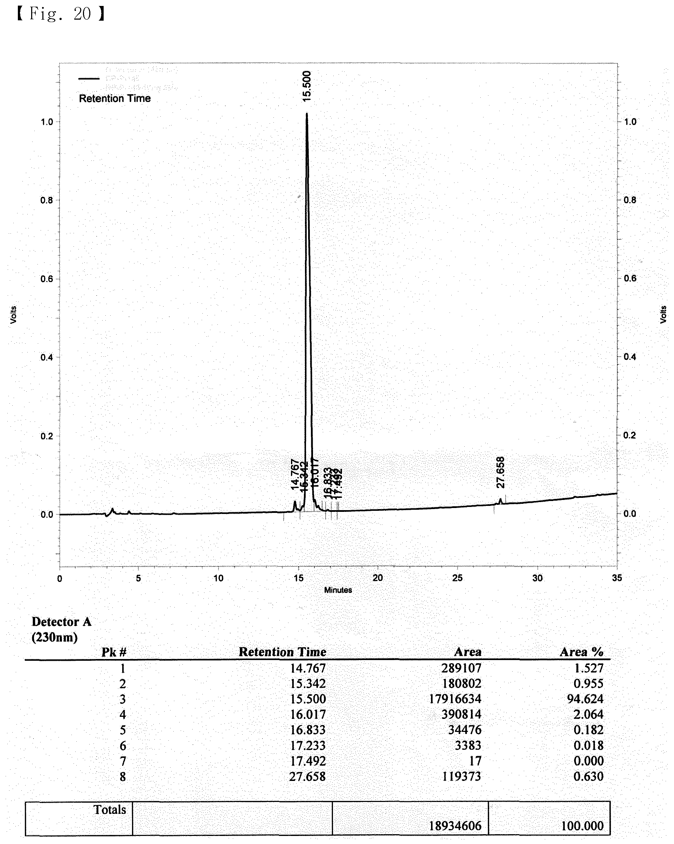

The purity and molecular weight of the HKFY-20 are shown in Figs. 20 and 56, respectively.

### Example 1.21. Preparation of HKFY-21

His(4-chlorobenzhydryl)-Lys(caprylic acid)-Phe-Tyr (N2-(Nt-((R)-(4-chlorophenyl)(phenyl)methyl)-L-histidyl)-N6-octanoyl-L-lysyl-L-phenylalanyl-L-tyrosine) was prepared in the same manner except that Boc-His(4-chlorobenzhydryl)-OH was used instead of Boc-His(trt)-OH obtained during the preparation process of Example 1.1.

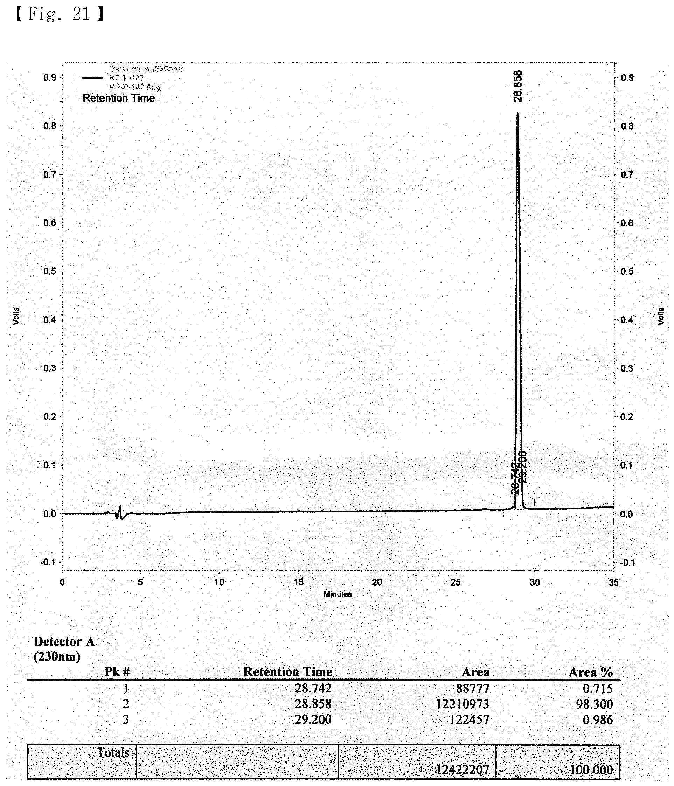

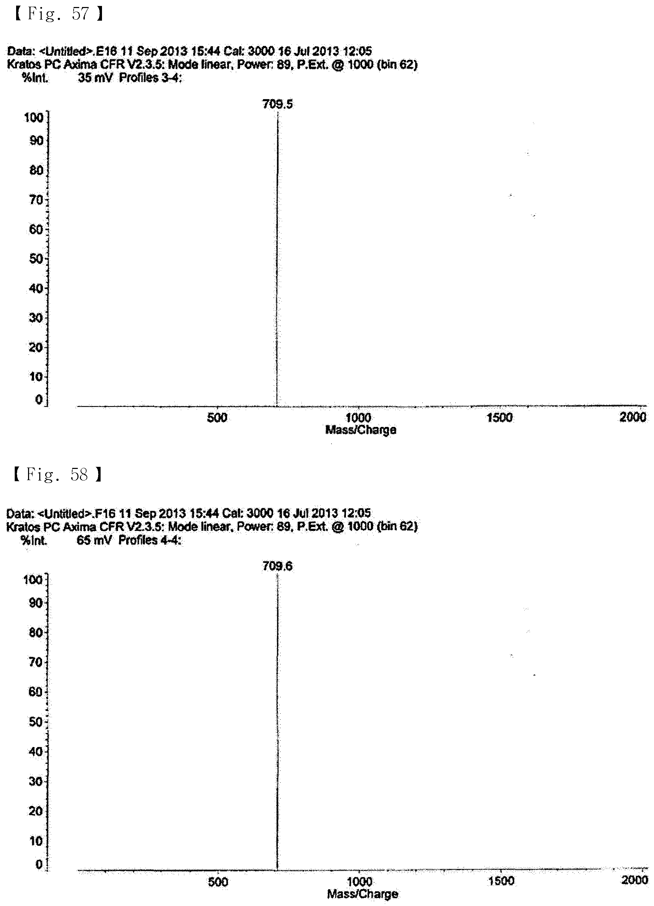

The purity and molecular weight of the HKFY-21 are shown in Figs. 21 and 57, respectively.

### Example 1.22. Preparation of HKFY-22

His(methylbenzhydryl)-Lys(caprylic acid)-Phe-Tyr (N6-octanoyl-N2-(Nt-((R)-phenyl (p-tolyl)methyl)-L-histidyl)-L-lysyl-L-phenylalanyl-L-tyrosine) was prepared in the same manner except that Boc-His(methylbenzhydryl)-OH was used instead of Boc-His(trt)-OH obtained during the preparation process of Example 1.1.

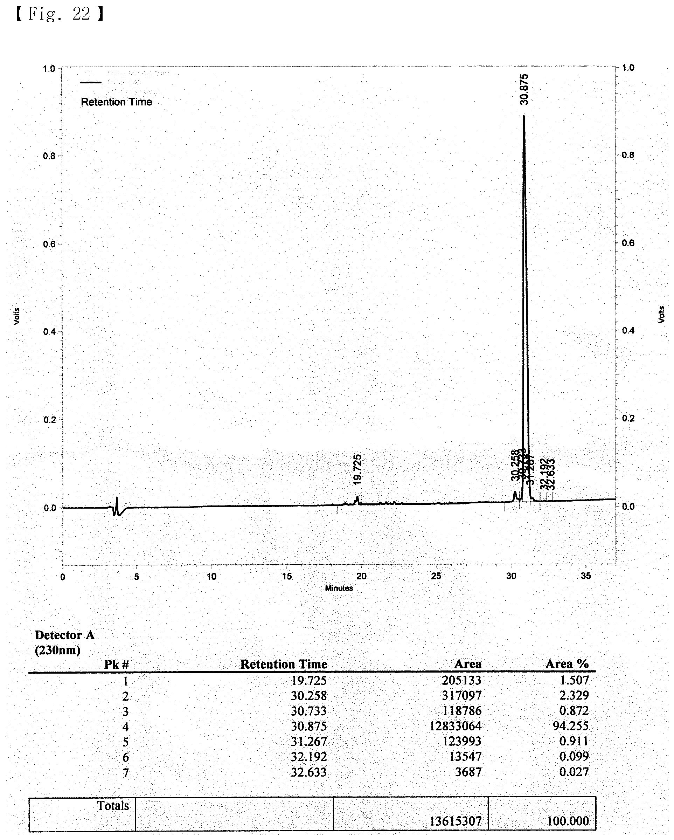

The purity and molecular weight of the HKFY-22 are shown in Figs. 22 and 58, respectively.

### Example 1.23. Preparation of HKFY-23

His(admantanmethyl)-Lys(caprylic acid)-Phe-Tyr (N2-(Nt-(adamantan-1 -yl)-L-histidyl)-N6-octanoyl-L-lysyl-L-phenylalanyl-L-tyrosine) was prepared in the same manner except that Boc-His(admantanmethyl)-OH was used instead of Boc-His(trt)-OH obtained during the preparation process of Example 1.1.

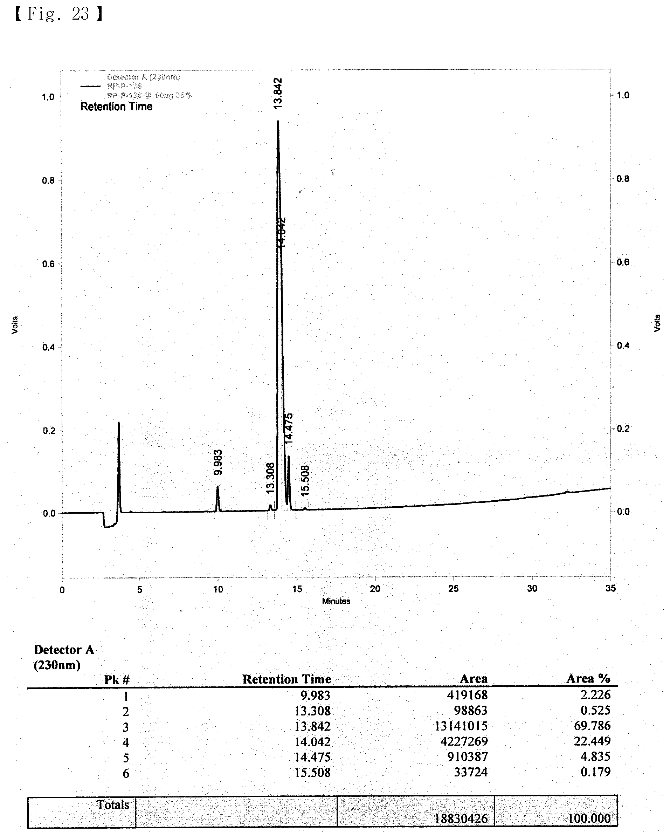

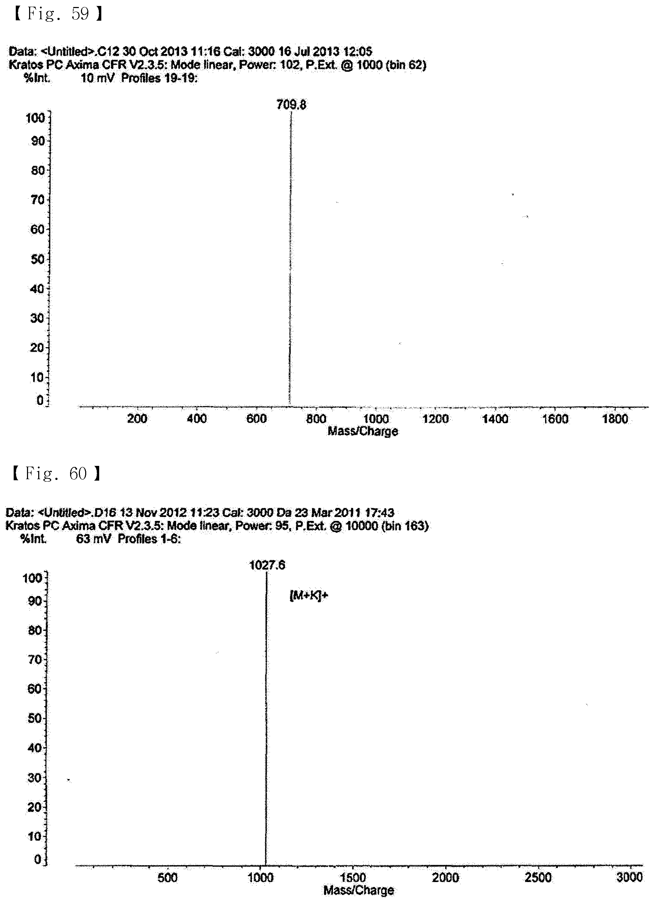

The purity and molecular weight of the HKFY-23 are shown in Figs. 23 and 59, respectively.

### Example 1.24. Preparation of HKFY-24

His(trt)-Lys(capric)-Phe-Tyr (N6-decanoyl-N2-(Nt-trityl-L-histidyl)-L-lysyl-L-phenylalanyl-L-tyrosine, HKFY-24) was prepared in the same manner except that capric acid instead of caprylic acid was added to Fmoc-Lys-OH obtained during the preparation process of Example 1.1.

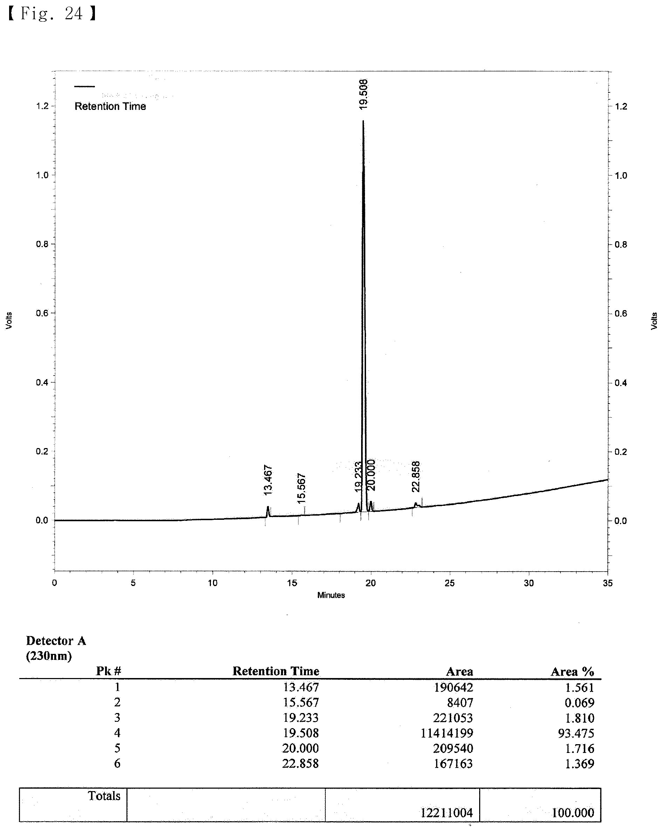

The purity and molecular weight of the HKFY-24 are shown in Figs. 24 and 60, respectively.

### Example 1.25. Preparation of HKFY-25

His(trt)-Lys(lauric)-Phe-Tyr (N6-dodecanoyl-N2-(Nt-trityl-L-histidyl)-L-lysyl-L-phenylalanyl-L-tyrosine, HKFY-25) was prepared in the same manner except that lauric acid instead of caprylic acid was added to Fmoc-Lys-OH obtained during the preparation process of Example 1.1.

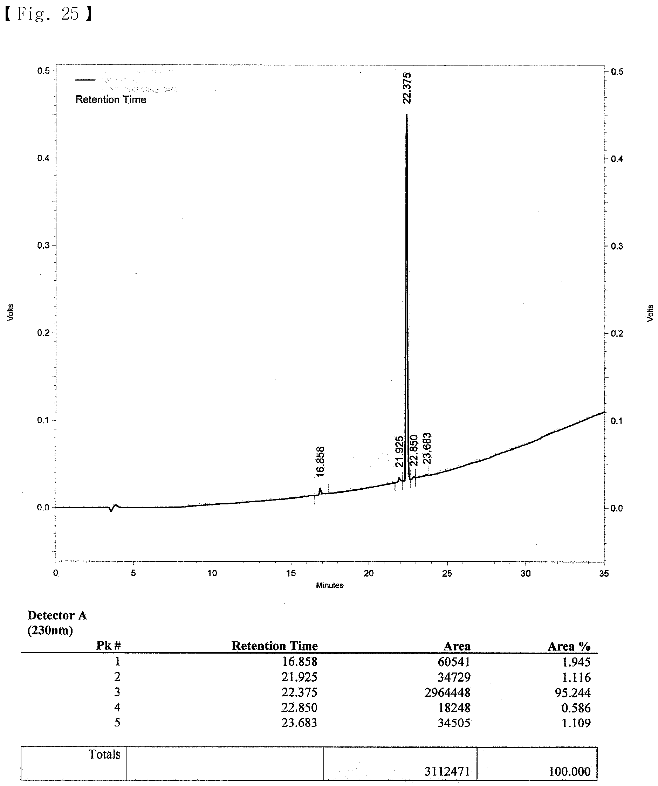

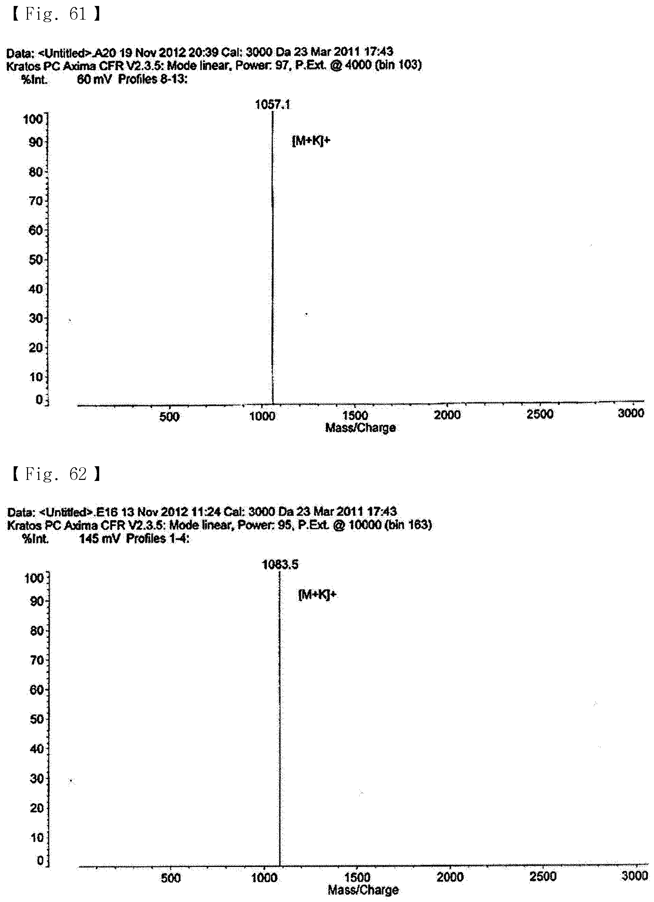

The purity and molecular weight of the HKFY-25 are shown in Figs. 25 and 61, respectively.

### Example 1.26. Preparation of HKFY-26

His(trt)-Lys(myristic)-Phe-Tyr (N6-tetradecanoyl-N2-(Nt-trityl-L-histidyl)-L-lysyl-L-phenylalanyl-L-tyrosine, HKFY-26) was prepared in the same manner except that myristic acid instead of caprylic acid was added to Fmoc-Lys-OH obtained during the preparation process of Example 1.1.

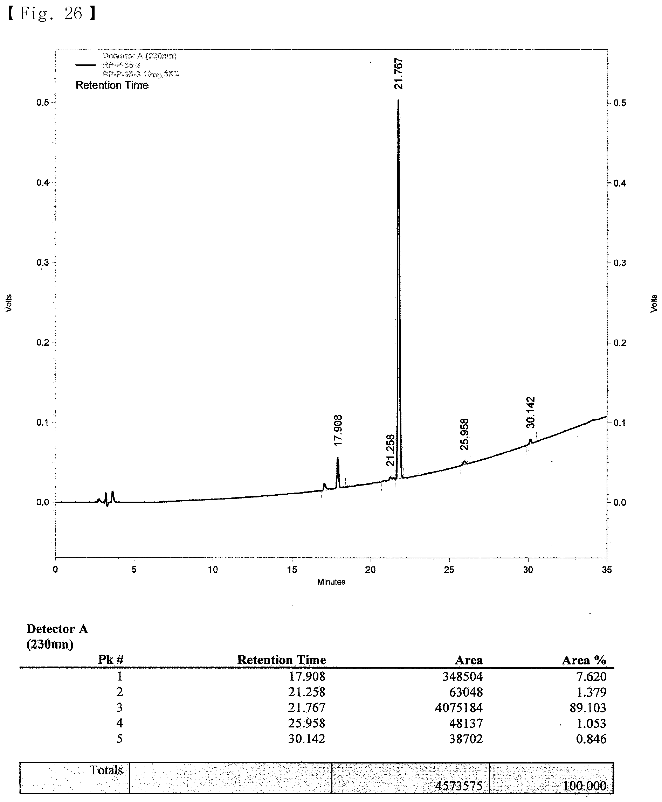

The purity and molecular weight of the HKFY-26 are shown in Figs. 26 and 62, respectively.

### Example 1.27. Preparation of HKFY-27

His(trt)-Lys(palmitic)-Phe-Tyr (N6-palmitoyl-N2-(Nt-trityl-L-histidyl)-L-lysyl-L-phenylalanyl-L-tyrosine, HKFY-27) was prepared in the same manner except that palmitic acid instead of caprylic acid was added to Fmoc-Lys-OH obtained during the preparation process of Example 1.1.

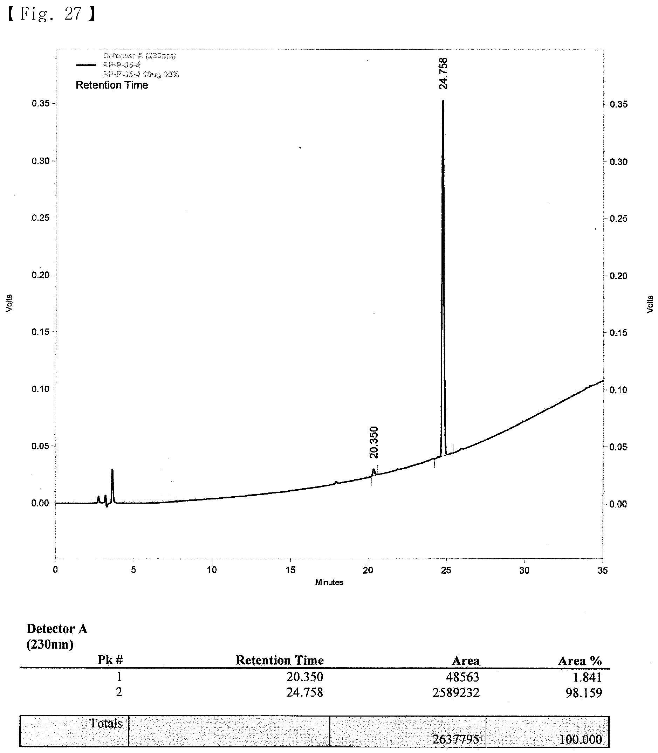

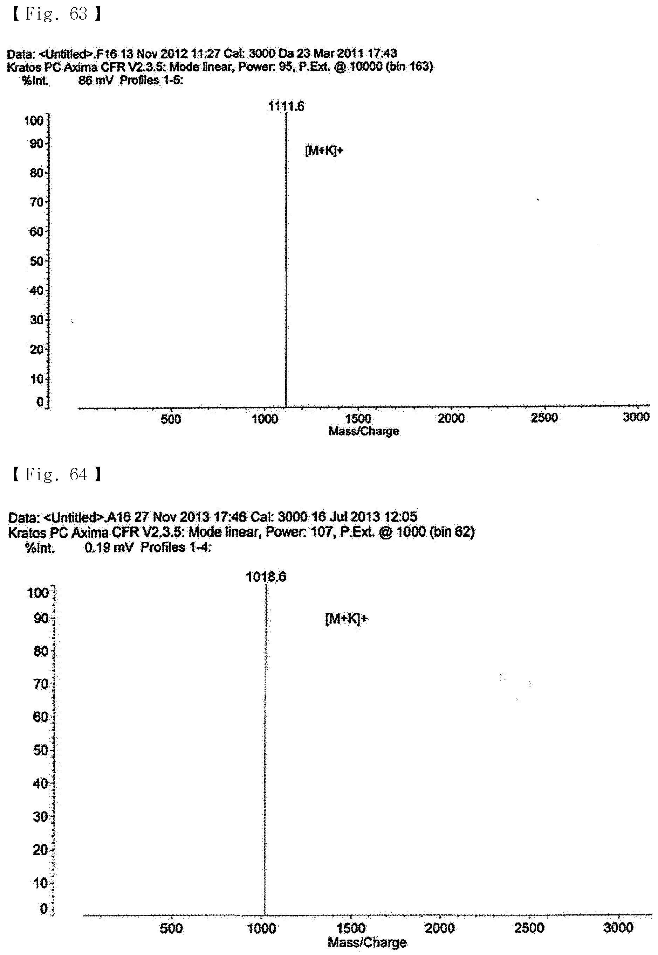

The purity and molecular weight of the HKFY-27 are shown in Figs. 27 and 63, respectively.

### Example 1.28. Preparation of HKFY-28

His(trt)-Lys(caprylic)-d-Phe(4-F)-Tyr (((R)-2-((S)-2-((S)-2-amino-3-(1-trityl-1H-imidazol-4-yl)propanamido)-6-octanamidohexanamido)-3-(4-fluorophenyl)propanoyl)-L-tyrosine, HKFY-28) was prepared in the same manner except that Fmoc-d-Phe(4-F)-OH was used instead of Fmoc-d-Phe-OH obtained during the preparation process of Example 1.6.

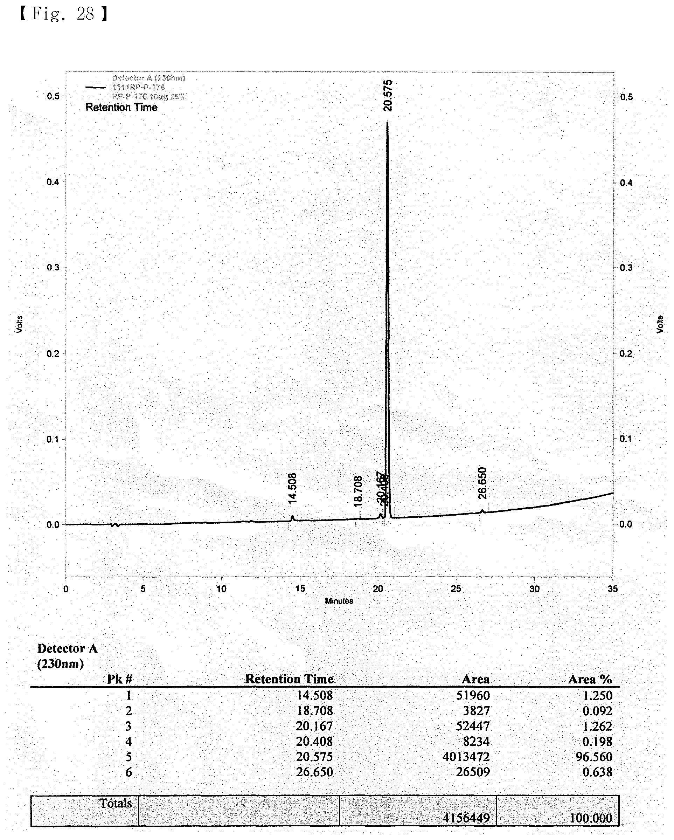

The purity and molecular weight of the HKFY-28 are shown in Figs. 28 and 64, respectively.

### Example 1.29. Preparation of HKFY-29

His(trt)-Lys(caprylic)-d-Phe(4-Br)-Tyr (((R)-2-((S)-2-((S)-2-amino-3-(1-trityl-1H-imidazol-4-yl)propanamido)-6-octanamidohexanamido)-3-(4-bromophenyl)propanoyl)-L-tyrosine, HKFY-29) was prepared in the same manner except that Fmoc-d-Phe(4-Br)-OH was used instead of Fmoc-d-Phe-OH obtained during the preparation process of Example 1.6.

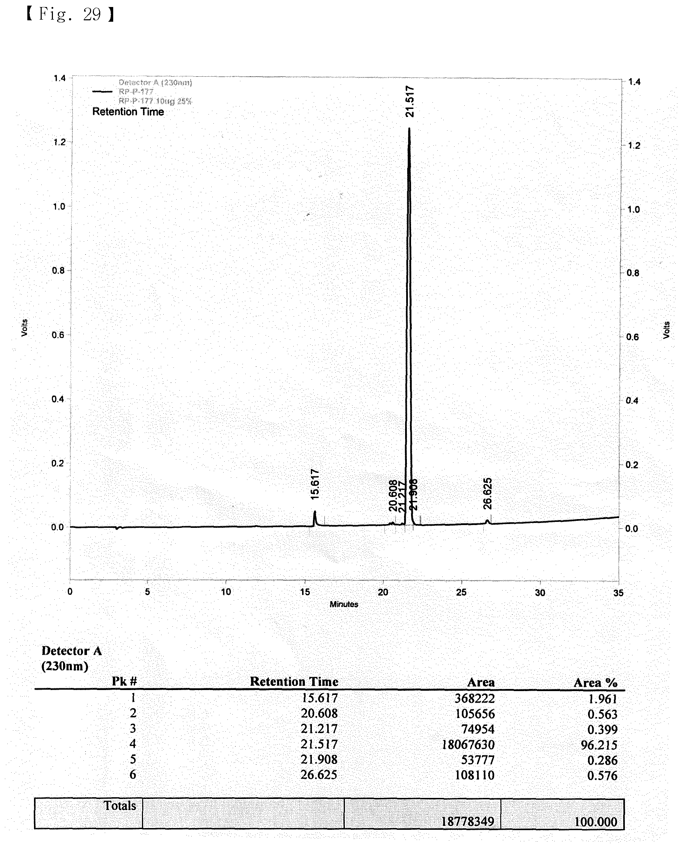

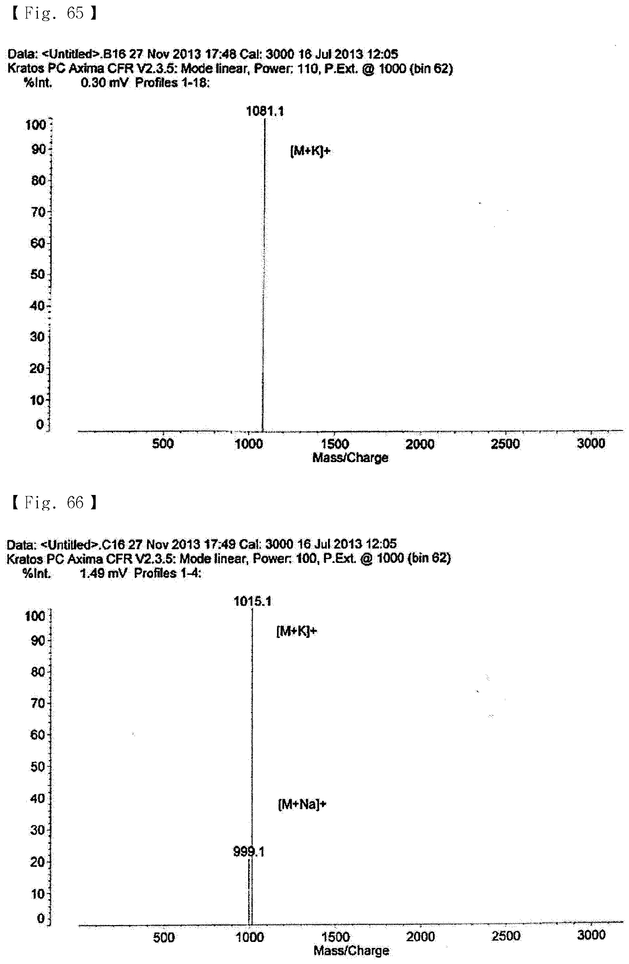

The purity and molecular weight of the HKFY-29 are shown in Figs. 29 and 65, respectively.

### Example 1.30. Preparation of HKFY-30

His(trt)-Lys(caprylic)-d-Phe(4-methyl)-Tyr (((R)-2-((S)-2-((S)-2-amino-3-(1 -trityl-1H-imidazol-4-yl)propanamido)-6-octanamidohexanamido)-3-(p-tolyl)propanoyl)-L-tyrosine, HKFY-30) was prepared in the same manner except that Fmoc-d-Phe(4-methyl)-OH was used instead of Fmoc-d-Phe-OH obtained during the preparation process of Example 1.6.

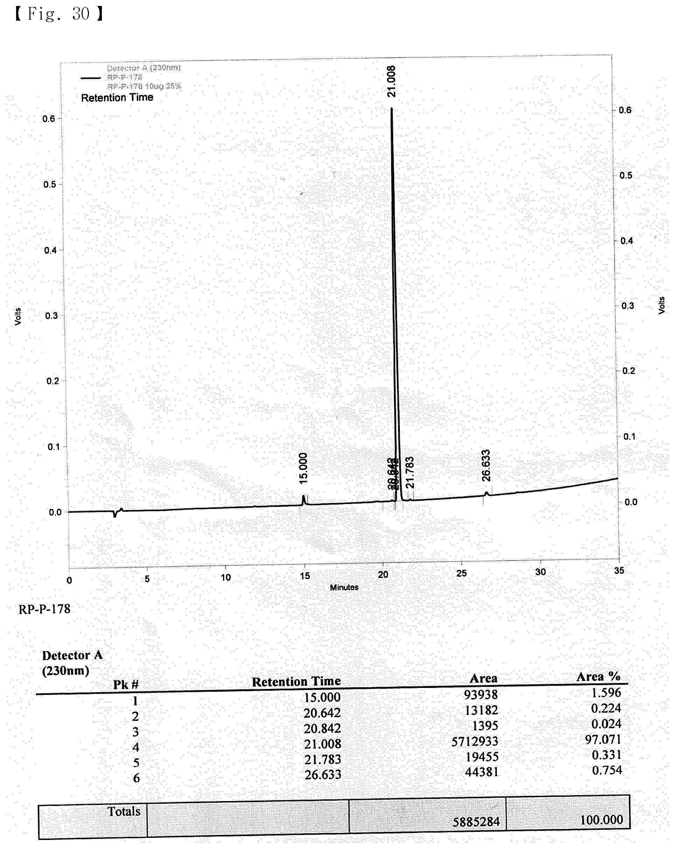

The purity and molecular weight of the HKFY-30 are shown in Figs. 30 and 66, respectively.

### Example 1.31. Preparation of HKFY-31

D-His(trt)-Lys(caprylic)-d-Phe(4-methyl)-Tyr ((R)-2-((S)-2-((R)-2-amino-3-(1-trityl-1H-imidazol-4-yl)propanamido)-6-octanamidohexanamido)-3-(p-tolyl)propanoyl)-L-tyrosine) was prepared in the same manner except that Boc-d-His(trt)-OH was used instead of Boc-His(trt)-OH obtained during the preparation process of Example 1.30.

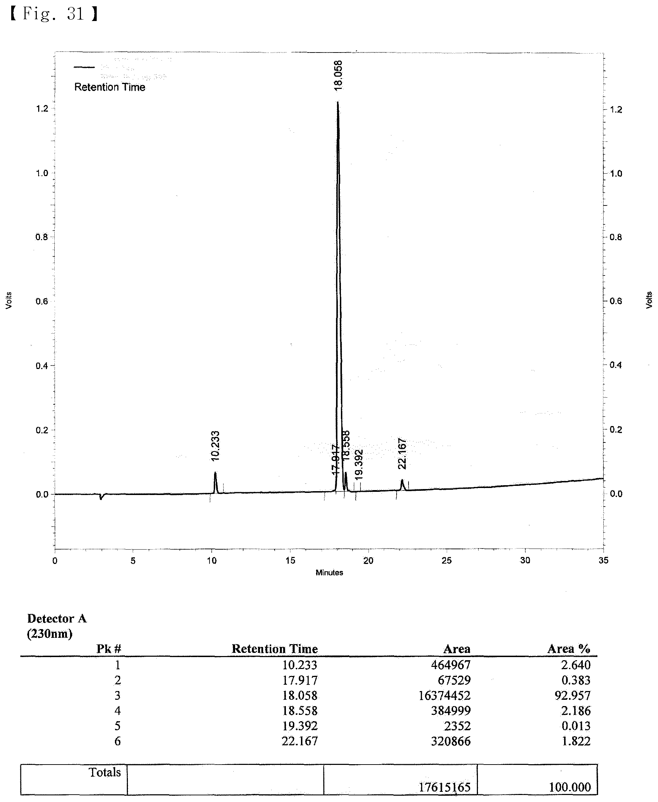

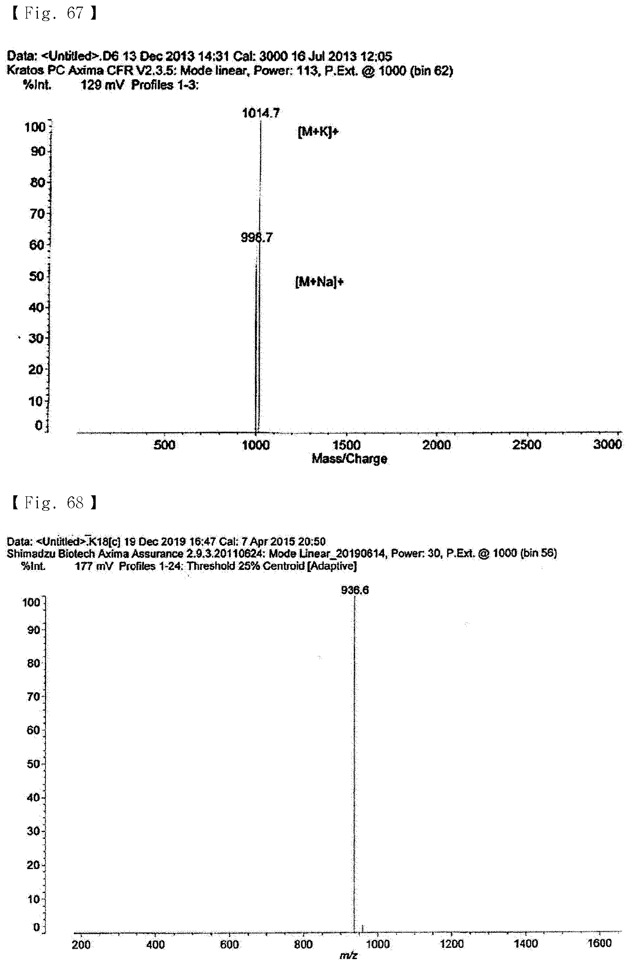

The purity and molecular weight of the HKFY-31 are shown in Figs. 31 and 67, respectively.

### Example 1.32. Preparation of HKFY-32

His(1,3-difluorobenzhydryl)-Lys(caprylic)-d-Phe(methyl)-Tyr (((2R)-2-((2S)-2-((2S)-2-amino-3-(1-((2,4-difluorophenyl)(phenyl)methyl)-1H-imidazol-4-yl)propanamido)-6-octanamidohexanamido)-3-(p-tolyl)propanoyl)-L-tyrosine) was prepared in the same manner except that Boc-His(1,3-difluorobenzhydryl)-OH was used instead of Boc-His(benzhydryl)-OH among the constituent amino acids of Example 1.34. below.

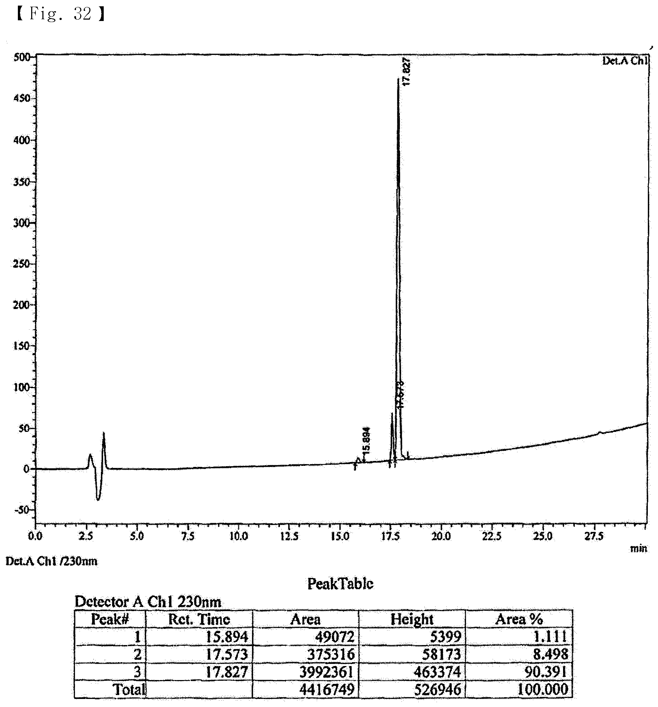

The purity and molecular weight of the HKFY-32 are shown in Figs. 32 and 68, respectively.

### Example 1.33. Preparation of HKFY-33

His(benzhydryl)-Lys(caprylic)-Phe-Tyr (N2-(Nt-benzhydryl-L-histidyl)-N6-octanoyl-L-lysyl-L-phenylalanyl-L-tyrosine) was prepared in the same manner except that Boc-His(benzhydryl)-OH was used instead of Boc-His(trt)-OH obtained during the preparation process of Example 1.1.

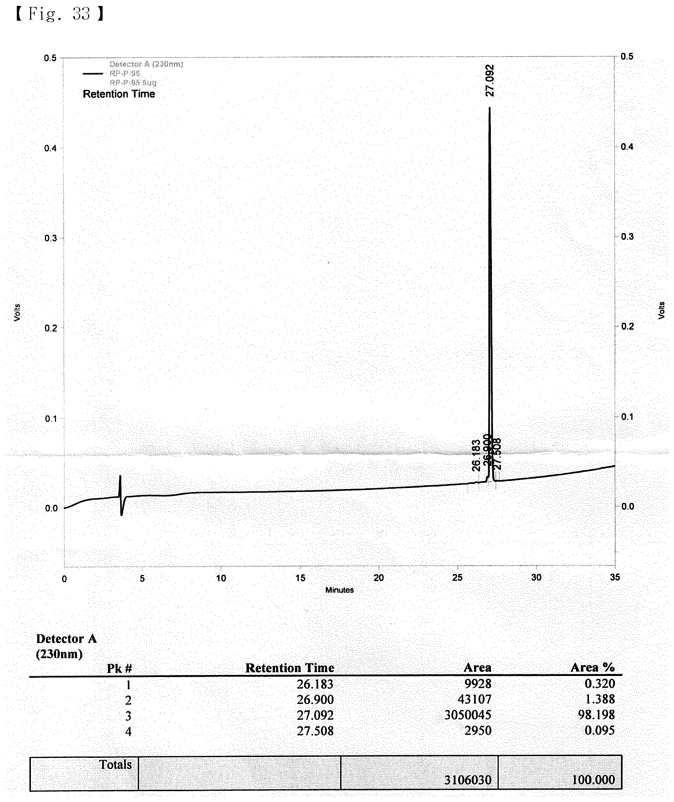

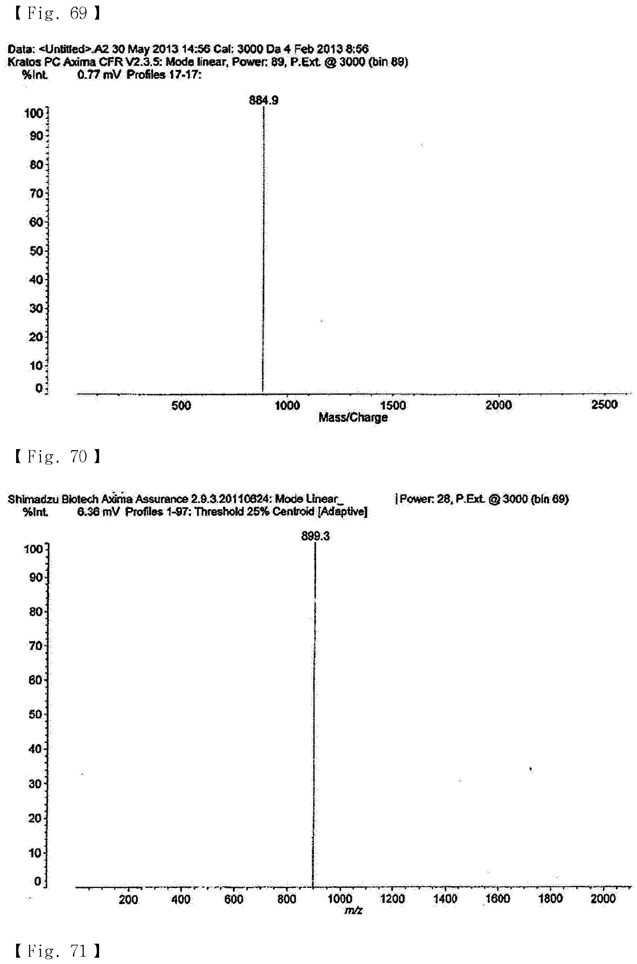

The purity and molecular weight of the HKFY-33 are shown in Figs. 33 and 69, respectively.

### Example 1.34. Preparation of HKFY-34

In order to prepare His(benzhydryl)-Lys(caprylic)-d-Phe(4-methyl)-Tyr (((R)-2-((S)-2-((S)-2-amino-3-(1-benzhydryl-1H-imidazol-4-yl)propanamido)-6-octanamidohexanamido)-3-(p-tolyl)propanoyl)-L-tyrosine, HKFY-34), Fmoc-Tyr(tBu) and DMF (dimethylformamide) were loaded onto the trityl resin to prepare Fmoc-Tyr(tBu)-trityl resin. DMF containing 20% piperidine and Fmoc-d-Phe(4-methyl)-OH and HOBt (hydroxyl-benzotriazole) were added to Fmoc-Tyr(tBu)-trityl resin to prepare Fmoc-d-Phe(4-methyl)-Tyr(tBu)-trityl resin.

DMF containing 2% NH₂NH₂ .H₂O was added to Fmoc-Lys(dde)-OH, and dde was removed to prepare Fmoc-Lys-OH. DMF containing caprylic acid, HOBt and DIC was added to the Fmoc-Lys-OH to prepare Fmoc-Lys(caprylic acid)-OH, and Boc-His(trt)-OH was used to prepare Boc-His(benzhydryl)-OH.

DMF containing 20% piperidine was added to the Fmoc-d-Phe(4-methyl)-Tyr(tBu)-trityl resin, and HOBt (hydroxyl-benzotriazole) was added to the Fmoc-Lys(caprylic acid)-OH to prepare Fmoc-Lys(caprylic acid)-d-Phe(4-methyl)-Tyr(tBu)-trityl resin. DMF containing 20% piperidine was added to Fmoc-Lys(caprylic acid)-d-Phe(4-methyl)-Tyr(tBu)-trityl resin, and Boc-His(benzhydryl)-OH and HOBt (hydroxyl-benzotriazole) were added to prepare a peptide of Boc-His(benzhydryl)-Lys(caprylic acid)-d-Phe(4-methyl)-Tyr(tBu)-trityl resin.

The protecting group was cleaved from the Boc-His(benzhydryl)-Lys(caprylic acid)-d-Phe(4-methyl)-Tyr(tBu)-trityl resin, and the resin was then purified to prepare His(benzhydryl)-Lys(caprylic acid)-d-Phe(4-methyl)-Tyr-OH.

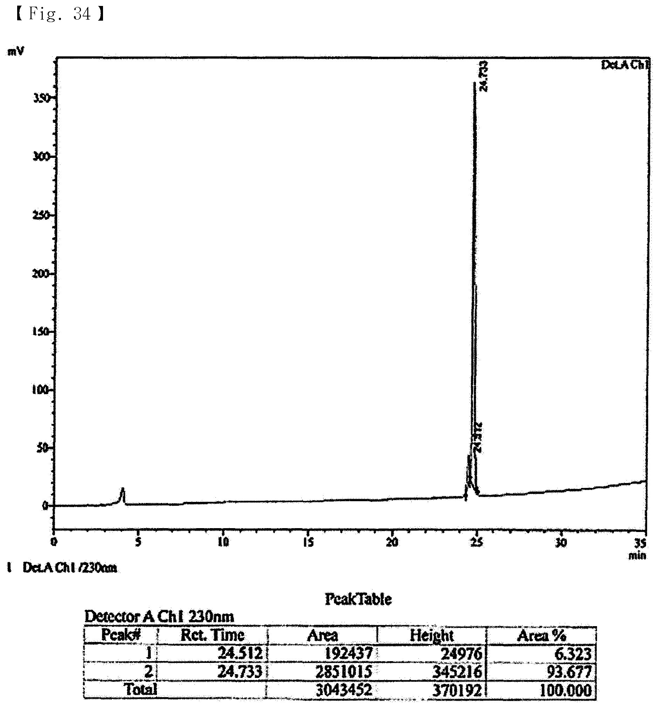

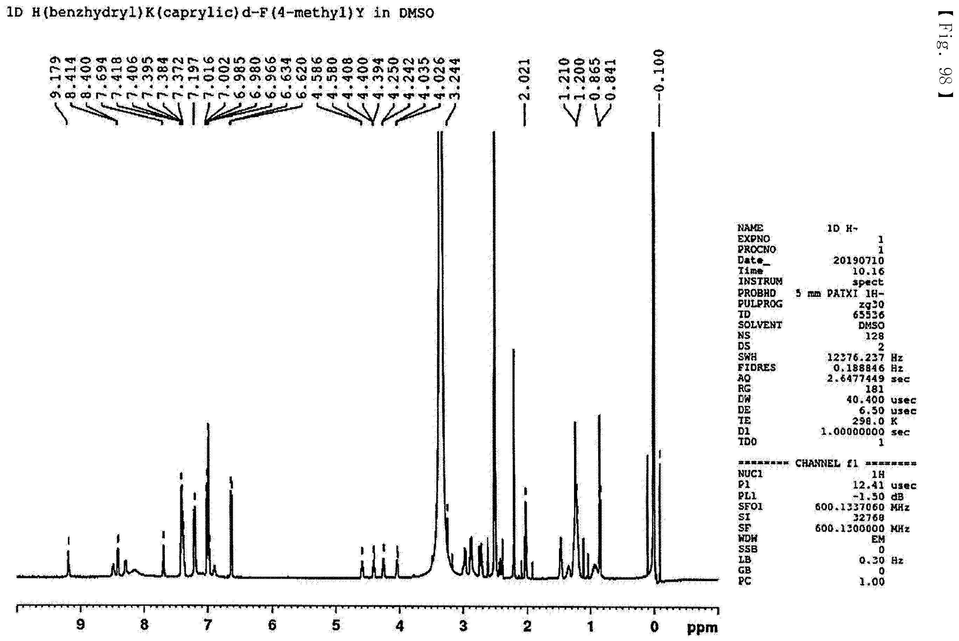

The purity, molecular weight and ¹H NMR of the HKFY-34 are shown in Figs. 34, 70 and 98, respectively.

### Example 1.35. Preparation of HKFY-35

His(benzhydryl)-Lys(caprylic acid)-d-Phe(4-methyl)-Tyr ((SO₃H)-OH (S)-2-((R)-2-((S)-2-((S)-2-amino-3-(1-benzhydryl-1H-imidazol-4-yl)propanamido)-6-octanamidohexanamido)-3-(p-tolyl)propanamido)-3-(4-(sulfooxy)phenyl)propanoic acid) was prepared in the same manner except that Fmoc-Tyr(SO3H)-OH was used instead of Fmoc-Tyr(tBu)-OH obtained during the preparation process of Example 1.34.

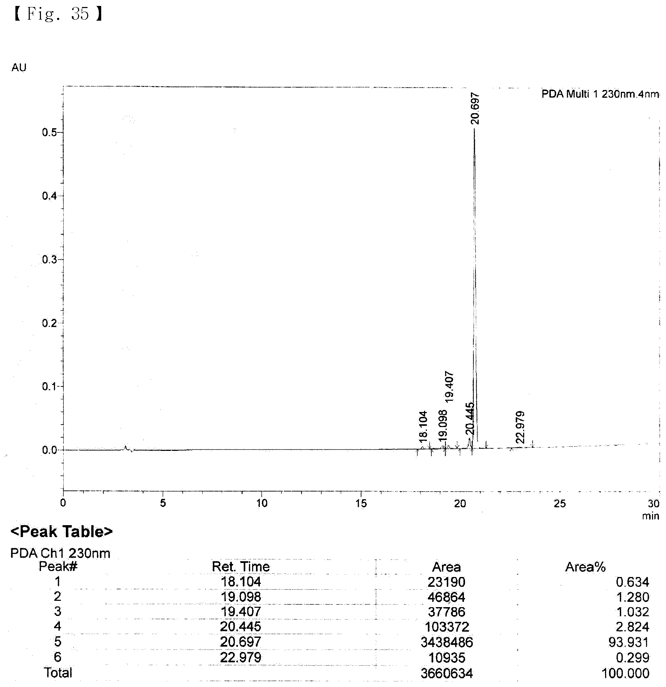

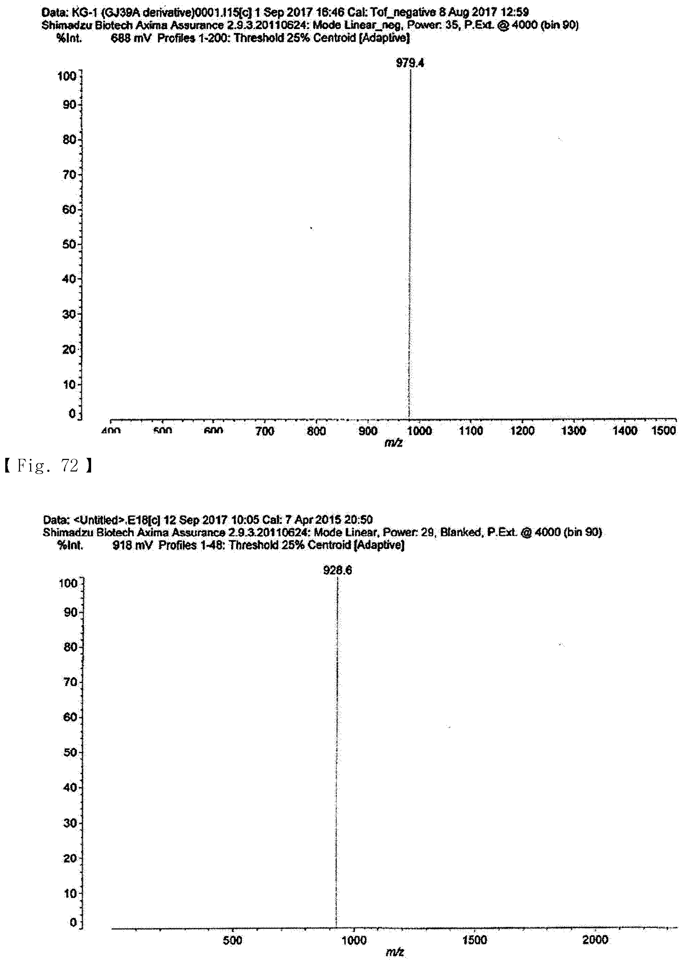

The purity and molecular weight of the HKFY-35 are shown in Figs. 35 and 71, respectively.

### Example 1.36. Preparation of HKFY-36

His(benzhydryl)-Lys(caprylic acid)-d-Phe(4-methyl)-Tyr ((2.6-di-methyl)-OH (S)-2-((R)-2-((S)-2-((S)-2-amino-3-(1-benzhydryl-1H-imidazol-4-yl)propanamido)-6-octanamidohexanamido)-3-(p-tolyl)propanamido)-3-(4-hydroxy-2,6-dimethylphenyl)propanoic acid) was prepared in the same manner except that Fmoc-Tyr(2.6-di-methyl)-OH was used instead of Fmoc-Tyr(tBu)-OH obtained during the preparation process of Example 1.34.

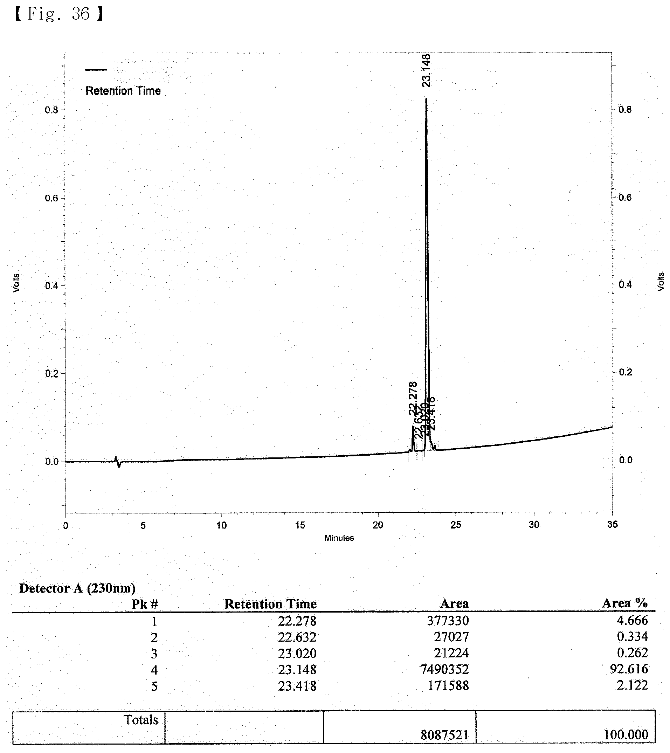

The purity and molecular weight of the HKFY-36 are shown in Figs. 36 and 72, respectively.

### Example 1.37. Preparation of HKFY-37

DMF containing 20% piperidine was added to Fmoc-His(trt)-Lys(caprylic)-Phe-Tyr-OH-trityl resin obtained during the preparation process of Example 1.1., and HOBt (hydroxyl-benzotriazole) was added to Fmoc-Ser(tBu)-OH to prepare Fmoc-Ser(tBu)-His(trt)-Lys(caprylic)-Phe-Tyr-OH-trityl resin. DMF containing 20% piperidine was added to the resin, and HOBt (hydroxyl-benzotriazole) was added to Fmoc-Gly-OH to prepare Fmoc-Gly-Ser(tBu)-His(trt)-Lys(caprylic)-Phe-Tyr-OH-trityl resin.

The protecting group was cleaved from the Fmoc-Gly-Ser(tBu)-His(trt)-Lys(caprylic)-Phe-Tyr-OH-trityl resin, and the resin was then purified to prepare Gly-Ser-His(trt)-Lys(caprylic acid)-Phe-Tyr (N2-(Na-glycyl-L-seryl-Nt-trityl-L-histidyl)-N6-octanoyl-L-lysyl-L-phenylalanyl-L-tyrosine).

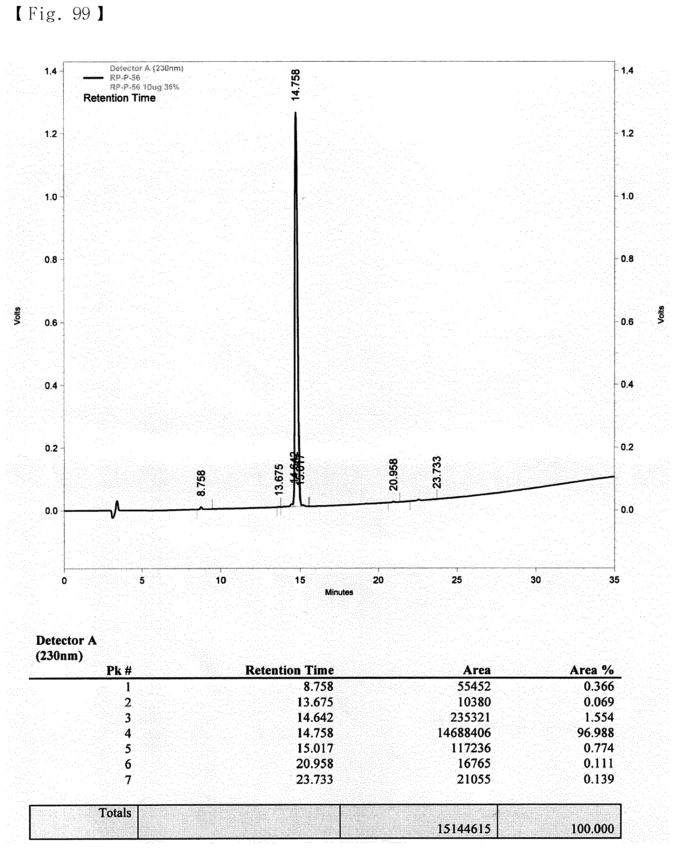

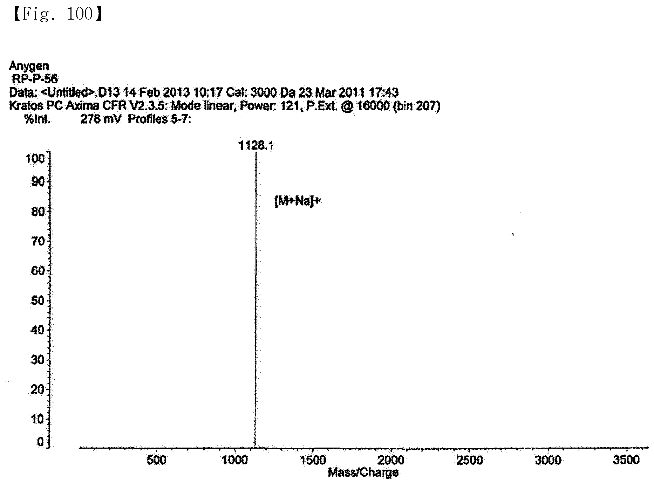

The purity and molecular weight of the HKFY-37 are shown in Figs. 99 and 100, respectively.

### Example 1.38. Preparation of HKFY-38

In order to prepare HKFY-38 ((3S,6S,9S,12S,15S)-15-((S)-2-amino-3-(1-trityl-1H-imidazol-4-yl)propanamido)-12-benzyl-3-(((6S,9S,12S,15S)-1,18-diamino-6-(((S)-1-amino-1-oxo-3-phenylpropan-2-yl)carbamoyl)-12-(3-amino-3-oxopropyl)-1-imino-9-methyl-8,11,14,18-tetraoxo-2,7,10,13 -tetraazaoctadecan-15-yl)carbamoyl)-9-(4-hydroxybenzyl)-6-(hydroxymethyl)-5,8,11,14,21-pentaoxo-4,7,10,13,20-pentaazaoctacosanoic acid), Fmoc-Phe-OH and DMF (dimethylformamide) were loaded onto the trityl resin to prepare Fmoc-Phe-trityl resin. DMF containing 20% piperidine and Fmoc-Arg(pbf)-OH and HOBt (hydroxyl-benzotriazole) were added to the Fmoc-Phe-trityl resin to prepare Fmoc-Arg(pbf)-Phe-trityl resin. DMF containing 20% piperidine and Fmoc-Ala-OH and HOBt (hydroxyl-benzotriazole) were added to Fmoc-Arg(pbf)-Phe-trityl resin to prepare Fmoc-Ala-Arg(pbf)-Phe-trityl resin. Fmoc-Gln(trt)-OH, Fmoc-Gln(trt)-OH, Fmoc-Asp(tBu)-OH, Fmoc-Ser(tBu)-OH, Fmoc-Tyr(tbu)-OH, Fmoc-Phe-OH were sequentially added in the same manner as above to prepare Fmoc-Phe-Tyr(tBu)-Ser(tBu)-Asp(tBu)-Gln(trt)-Gln(trt)-Ala-Arg(pbf)-Phe-trityl resin.

DMF containing 20% piperidine and Fmoc-Lys(caprylic)-OH obtained from Example 1.1. and HOBt (hydroxyl-benzotriazole) were added to the resin to prepare Fmoc-Lys(caprylic)-Phe-Tyr(tBu)-Ser(tBu)-Asp(tBu)-Gln(trt)-Gln(trt)-Ala-Arg(pbf)-Phe-trityl resin.

DMF containing 20% piperidine and Fmoc-His(trt)-OH obtained from Example 1.1. and HOBt (hydroxyl-benzotriazole) were added to the resin to prepare Fmoc-His(trt)-Lys(caprylic)-Phe-Tyr (tBu)-Ser(tBu)-Asp(tBu)-Gln(trt)-Gln(trt)-Ala-Arg(pbf)-Phe-trityl resin.

The protecting group was cleaved from the Fmoc-His(trt)-Lys(caprylic)-Phe-Tyr (tBu)-Ser(tBu)-Asp(tBu)-Gln(trt)-Gln(trt)-Ala-Arg(pbf)-Phe-trityl resin, and the resin was then purified to prepare His(trt)-Lys(caprylic)-Phe-Tyr-Ser-Asp-Gln-Gln-Ala-Arg-Phe.

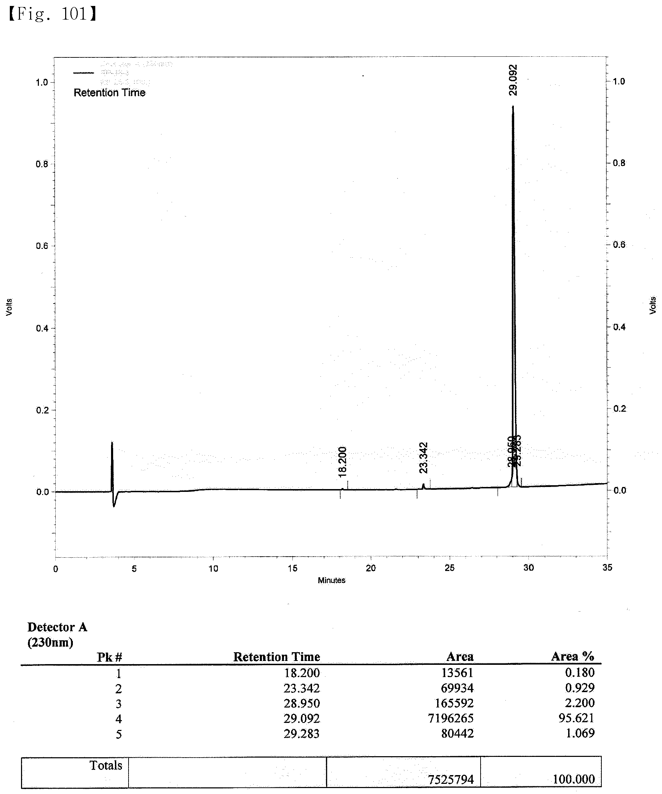

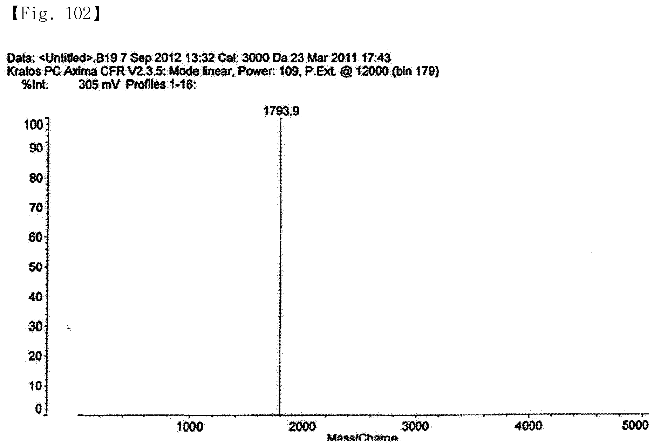

The purity and molecular weight of the HKFY-38 are shown in Figs. 101 and 102, respectively.

### Example 1.39. Preparation of HKFY-39

In order to prepare HKFY-39 ((3S,6S,9S,12S,15S,18S)-3-((2-(((S)-1-(((S)-1-(((S)-1-amino-1-oxo-3-phenylpropan-2-yl)amino)-5-guanidino-1-oxopentan-2-yl)amino)-1-oxopropan-2-yl)amino)-2-oxoethyl)carbamoyl)-18-((S)-2-amino-3-(1-trityl-1H-imidazol-4-yl)propanamido)-6-(3-amino-3-oxopropyl)-15-benzyl-12-(4-hydroxybenzyl)-9-(hydroxymethyl)-5,8,11,14,17,24-hexaoxo-4,7,10,13,16,23-hexaazahentriacontanoic acid), DMF containing 20% piperidine and Fmoc-Gly-OH and HOBt (hydroxyl-benzotriazole) were added to Fmoc-Ala-Arg(pbf)-Phe-trityl resin obtained from Example 1.38. to prepare Fmoc-Gly-Ala-Arg(pbf)-Phe-trityl resin. DMF containing 20% piperidine and Fmoc-Asn(trt)-OH and HOBt (hydroxyl-benzotriazole) were added to the Fmoc-Gly-Ala-Arg(pbf)-Phe-trityl resin to prepare Fmoc-Asn(trt)-Gly-Ala-Arg(pbf)-Phe-trityl resin. Fmoc-Gln(trt)-OH, Fmoc-Ser(tBu)-OH, Fmoc-Tyr(tbu)-OH, and Fmoc-Phe-OH were sequentially added in the same manner as above to prepare Fmoc-Phe-Tyr(tBu)-Ser(tBu)-Gly-Ala-Arg(pbf)-Phe-trityl resin. Fmoc-Lys(caprylic)-OH obtained from Example 1.1. and HOBt (hydroxyl-benzotriazole) were added to the resin to prepare Fmoc-Lys(caprylic)-Phe-Tyr(tBu)-Ser(tBu)-Gly-Ala-Arg(pbf)-Phe-trityl resin.

DMF containing 20% piperidine and Fmoc-His(trt)-OH obtained from Example 1.1. and HOBt (hydroxyl-benzotriazole) were added to the resin to prepare Fmoc-His(trt)-Lys(caprylic)-Phe-Tyr(tBu)-Ser(tBu)-Gly-Ala-Arg(pbf)-Phe-trityl resin.

The protecting group was cleaved from the Fmoc-His(trt)-Lys(caprylic)-Phe-Tyr(tBu)-Ser(tBu)-Gly-Ala-Arg(pbf)-Phe-trityl resin, and the resin was then purified to prepare His(trt)-Lys(caprylic)-Phe-Tyr-Ser-Gly-Ala-Arg-Phe.

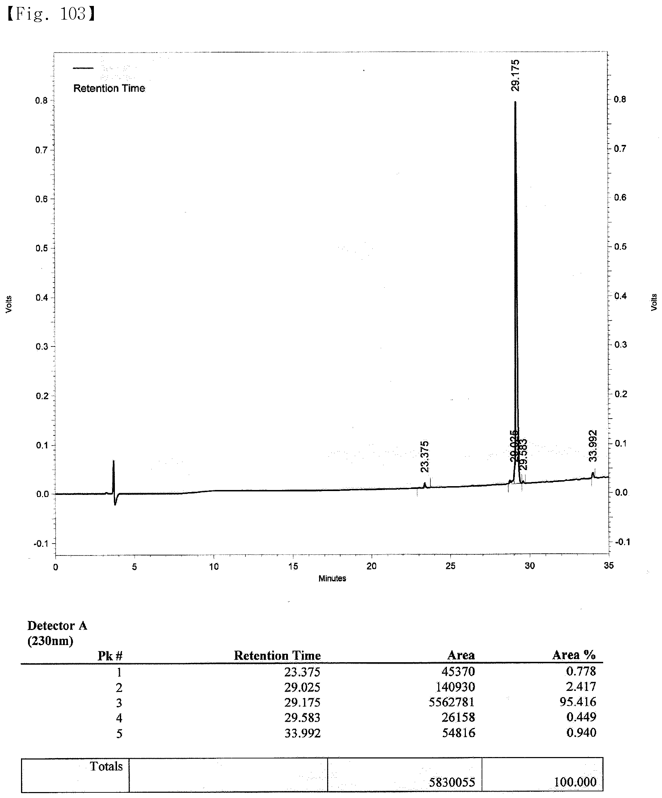

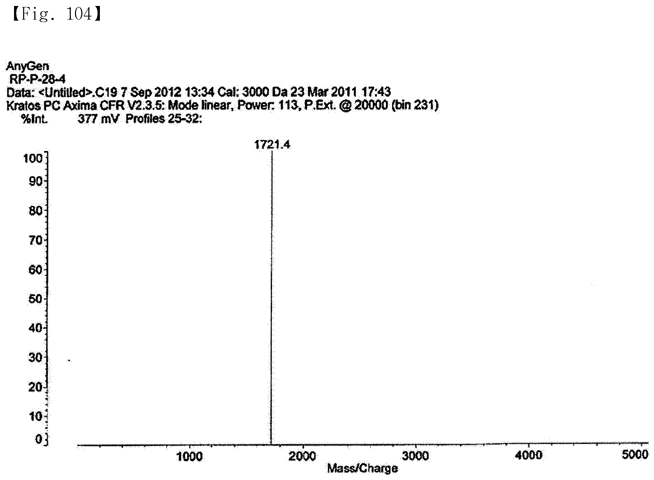

The purity and molecular weight of the HKFY-39 are shown in Figs. 103 and 104, respectively.

### Example 1.40. Preparation of HKFY-40

DMF containing 20% piperidine and Fmoc-Gln(trt)-OH and HOBt (hydroxyl-benzotriazole) were added to the Fmoc-Ala-Arg(pbf)-Phe-trityl resin obtained from Example 1.39. to prepare Fmoc-Gln(trt)-Ala-Arg(pbf)-Phe-trityl resin.

Fmoc-Gln(trt)-OH, Fmoc-His(trt)-OH, Fmoc-Ser(tBu)-OH, Fmoc-Tyr(tBu)-OH, Fmoc-Phe-OH were sequentially added in the same manner to prepare Fmoc-Phe-Tyr(tBu)-Ser(tBu)-His(trt)-Gln(trt)-Gln(trt)-Ala-Arg(pbf)-Phe-trityl resin.

Fmoc-Lys(caprylic)-OH obtained from Example 1.1. and Fmoc-His(trt)-OH were sequentially added to the resin in the same manner to prepare Fmoc-His(trt)-Lys(caprylic)-Phe-Tyr (tBu)-Ser(tBu)-His(trt)-Gln(trt)-Gln(trt)-Ala-Arg(pbf)-Phe-trityl resin.

The protecting group was cleaved from the Fmoc-His(trt)-Lys(caprylic)-Phe-Tyr (tBu)-Ser(tBu)-His(trt)-Gln(trt)-Gln(trt)-Ala-Arg(pbf)-Phe-trityl resin, and the resin was then purified to prepare His(trt)-Lys(caprylic)-Phe-Tyr-Ser-His-Gln-Gln-Ala-Arg-Phe ((S)-2-(2S,5S,8S,11S,14S)-2-((1H-imidazol-4-yl)methyl)-14-((S)-2-amino-3-(1-trityl-1H-imidazol-4-yl)propanamido)-11-benzyl-8-(4-hydroxybenzyl)-5-(hydroxymethyl)-4,7,10,13,20-pentaoxo-3,6,9,12,19-pentaazaheptacosanamido)-N1-((S)-5-amino-1-(((S)-1-(((S)-1-(((S)-1-amino-1-oxo-3-phenylpropan-2-yl)amino)-5-guanidino-1-oxopentan-2-yl)amino)-1-oxopropan-2-yl)amino)-1,5-dioxopentan-2-yl)pentanediamide).

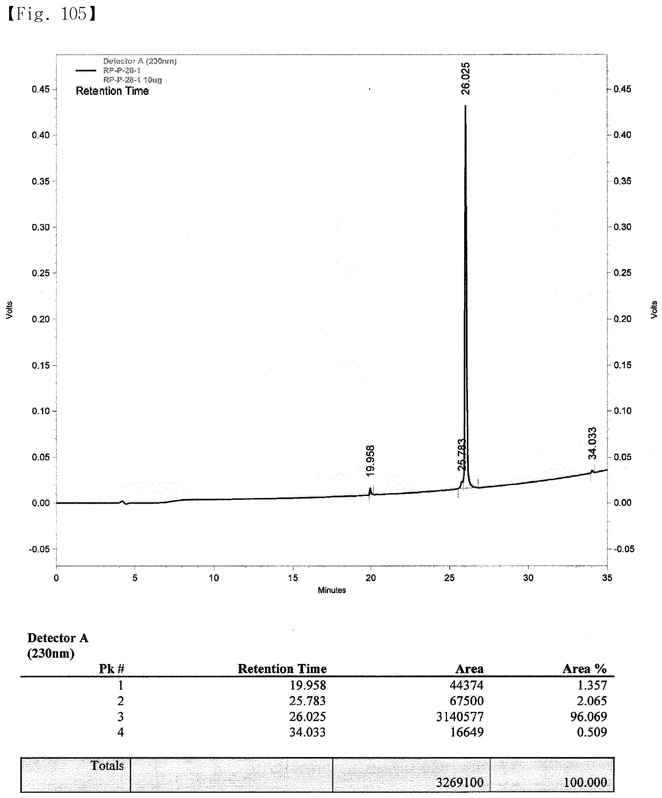

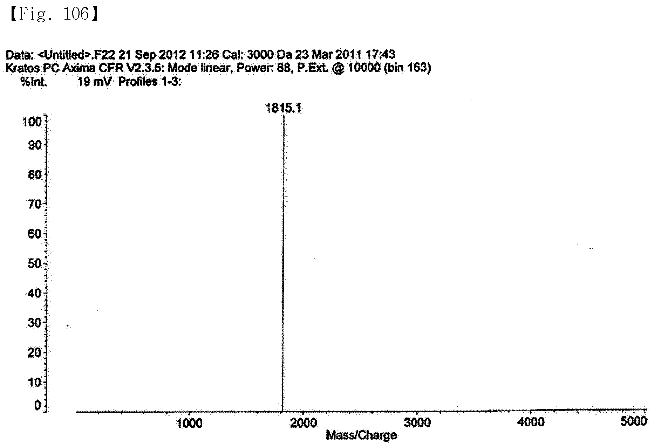

The purity and molecular weight of the HKFY-40 are shown in Figs. 105 and 106, respectively.

### Example 1.41. Preparation of HKFY-41

DMF containing 20% piperidine and Fmoc-Gly-OH and HOBt (hydroxyl-benzotriazole) were added to the Fmoc-Ala-Arg(pbf)-Phe-trityl resin obtained from Example 1.39. to prepare Fmoc-Gly-Ala-Arg(pbf)-Phe-trityl resin. Fmoc-Asn(trt)-OH, Fmoc-Gln(trt)-OH, Fmoc-Tyr(tBu)-OH, Fmoc-Phe-OH, Fmoc-Lys(caprylic)-OH obtained from Example 1.1., and Fmoc-His(trt)-OH were sequentially added to the resin in the same manner to prepare Fmoc-His(trt)-Lys(caprylic)-Phe-Tyr (tBu)-Gln(trt)-Asn(trt)-Gly-Ala-Arg(pbf)-Phe-trityl resin. The protecting group of the resin was cleaved to prepare His(trt)-Lys(caprylic)-Phe-Tyr-Gln-Asn-Gly-Ala-Arg-Phe ((3S,6S,9S,12S,15S,18S)-3-((2-(((S)-1-(((S)-1-(((S)-1-amino-1-oxo-3-phenylpropan-2-yl)amino)-5-guanidino-1-oxopentan-2-yl)amino)-1-oxopropan-2-yl)amino)-2-oxoethyl)carbamoyl)-18-((S)-2-amino-3-(1-trityl-1H-imidazol-4-yl)propanamido)-6-(3-amino-3-oxopropyl)-15-benzyl-12-(4-hydroxybenzyl)-9-(hydroxymethyl)-5,8,11,14,17,24-hexaoxo-4,7,10,13,16,23-hexaazahentriacontanoic acid).

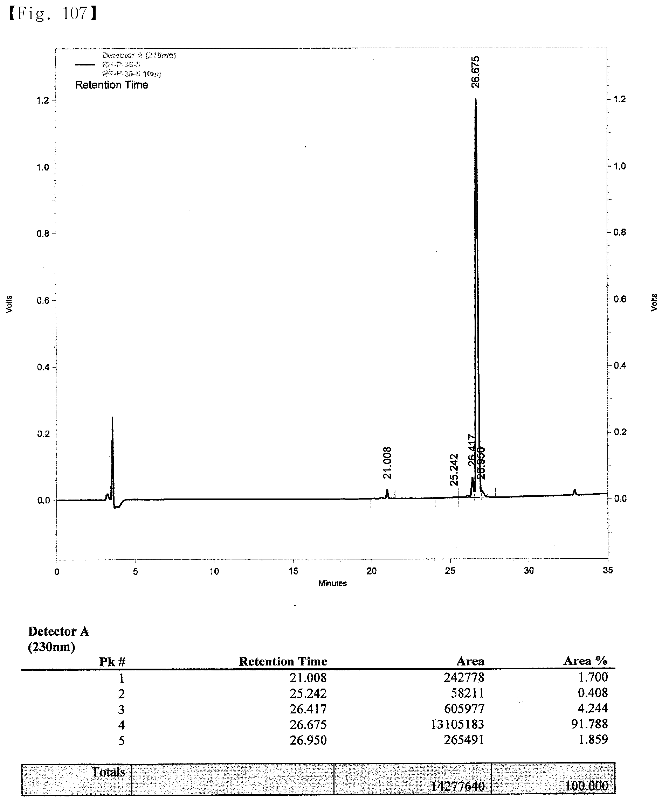

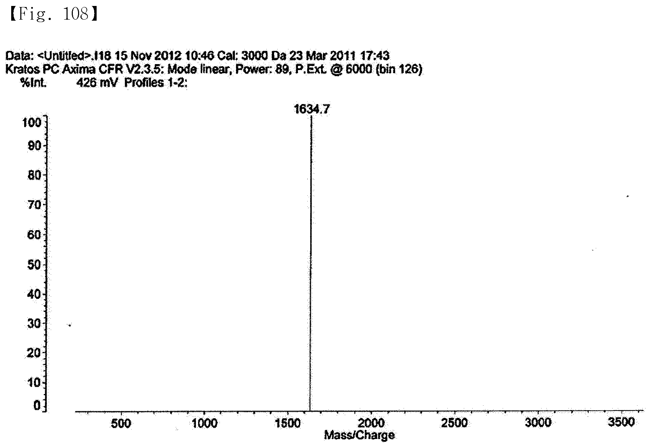

The purity and molecular weight of the HKFY-41 are shown in Figs. 107 and 108, respectively.

### Example 1.42. Preparation of HKFY-42

DMF containing 20% piperidine and Fmoc-Gln(trt) and HOBt (hydroxyl-benzotriazole) were added to the Fmoc-Ala-Arg(pbf)-Phe-trityl resin obtained from Example 1.39. to prepare Fmoc-Gly-Ala-Arg(pbf)-Phe-trityl resin. Fmoc-Gln(trt)-OH, Fmoc-His(trt)-OH, Fmoc-Tyr(tBu)-OH, Fmoc-Phe-OH, Fmoc-Lys(caprylic)-OH obtained from Example 1.1., and Fmoc-His(trt)-OH were sequentially added to the resin in the same manner to prepare Fmoc-His(trt)-Lys(caprylic)-Phe-Tyr(tBu)-His(trt)-Gln(trt)-Gln(trt)-Ala-Arg(pbf)-Phe-trityl resin. The protecting group of the resin was cleaved to prepare His(trt)-Lys(caprylic)-Phe-Tyr-His-Gln-Gln-Ala-Arg-Phe ((S)-2-((2S,5S,8S,11S)-2-((1H-imidazol-4-yl)methyl)-11-((S)-2-amino-3-(1-trityl-1H-imidazol-4-yl)propanamido)-8-benzyl-5-(4-hydroxybenzyl)-4,7,10,17-tetraoxo-3,6,9,16-tetraazatetracosanamido)-N1-((S)-5-amino-1-(((S)-1(((S)-1-(((S)-1-amino-1-oxo-3-phenylpropan-2-yl)amino)-5-guanidino-1-oxopentan-2-yl)amino)-1-oxopropan-2-yl)amino)-1,5-dioxopentan-2-yl)pentanediamide).

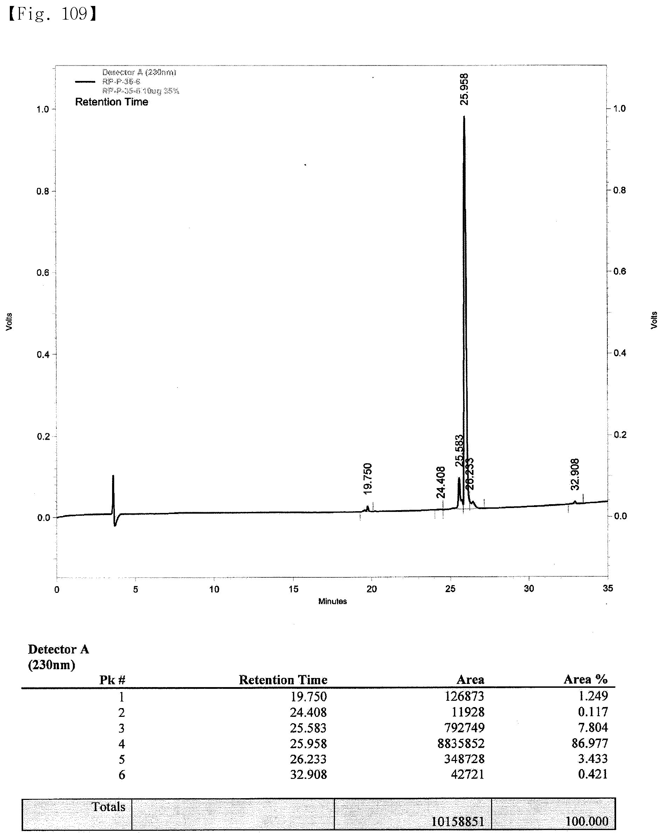

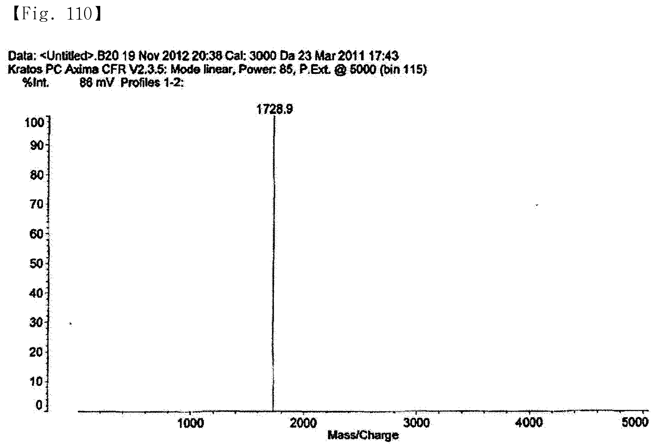

The purity and molecular weight of the HKFY-42 are shown in Figs. 109 and 110, respectively.

### Example 1.43. Preparation of HKFY-43

Fmoc-His(trt)-Lys(caprylic)-Phe-Tyr(tBu)-Gln(trt)-Asn(trt)-Gln(trt)-Ala-Arg(pbf)-Phe-trityl resin was prepared in the same manner except that Fmoc-Gln(trt)-OH was used instead of Fmoc-Gly-OH among the constituent amino acids of Example 1.41. The protecting group of the resin was cleaved to prepare His(trt)-Lys(caprylic)-Phe-Tyr-Gln-Asn-Gln-Ala-Arg-Phe ((S)-N1-((S)-1-(((S)-1-(((S)-1-amino-1-oxo-3-phenylpropan-2-yl)amino)-5-guanidino-1-oxopentan-2-yl)amino)-1-oxopropan-2-yl)-2-((2S,5S,8S,11S,14S)-2-(2-amino-2-oxoethyl)-14-((S)-2-amino-3-(1-trityl-1H-imidazol-4-yl)propanamido)-5-(3-amino-3-oxopropyl)-11-benzyl-8-(4-hydroxybenzyl)-4,7,10,13,20-pentaoxo-3,6,9,12,19-pentaazaheptacosanamido)pentanediamide).

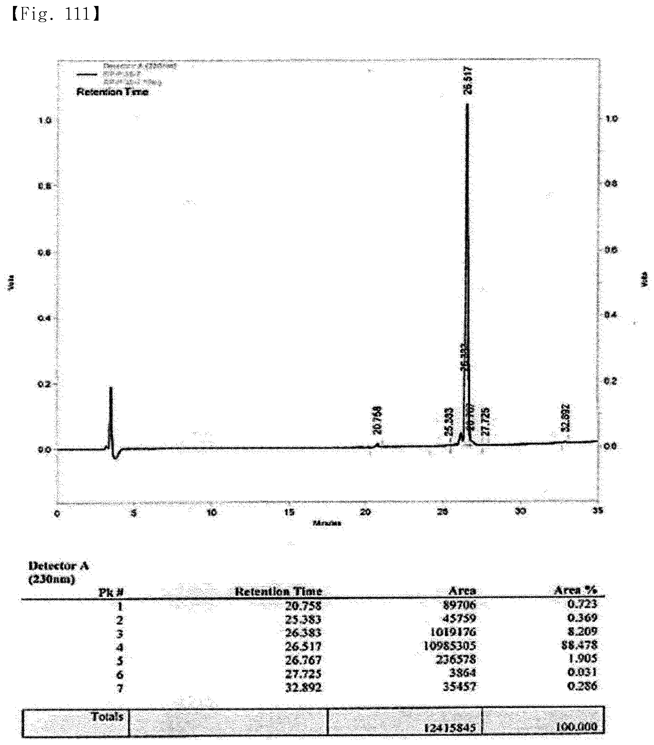

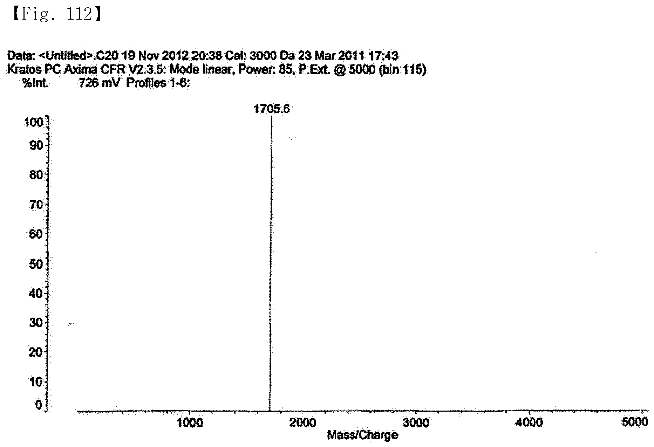

The purity and molecular weight of the HKFY-43 are shown in Figs. 111 and 112, respectively.

### Example 1.44. Preparation of HKFY-44

In order to prepare His(trt)-Lys(caprylic)-Phe-Tyr-Trp, Fmoc-Trp(Boc)-OH and DMF were loaded onto the trityl resin to prepare Fmoc-Trp(Boc)-trityl resin. DMF containing 20% piperidine and Fmoc-Tyr(tBu)-OH and HOBt (hydroxyl-benzotriazole) were added to the Fmoc-Trp(Boc)-trityl resin to prepare Fmoc-Tyr(tBu)-Trp(Boc)-trityl resin. Fmoc-Phe-OH, Fmoc-Lys(caprylic)-OH obtained from Example 1.1., and Fmoc-His(trt)-OH were sequentially added to the resin in the same manner to prepare Fmoc-His(trt)-Lys(caprylic)-Phe-Tyr (tBu)-Trp(Boc)-trityl resin. The protecting group of the resin was cleaved to prepare His(trt)-Lys(caprylic)-Phe-Tyr-Trp (N6-octanoyl-N2-(Nt-trityl-L-histidyl)-L-lysyl-L-phenylalanyl-L-lyrosyl-L-tryptophan).

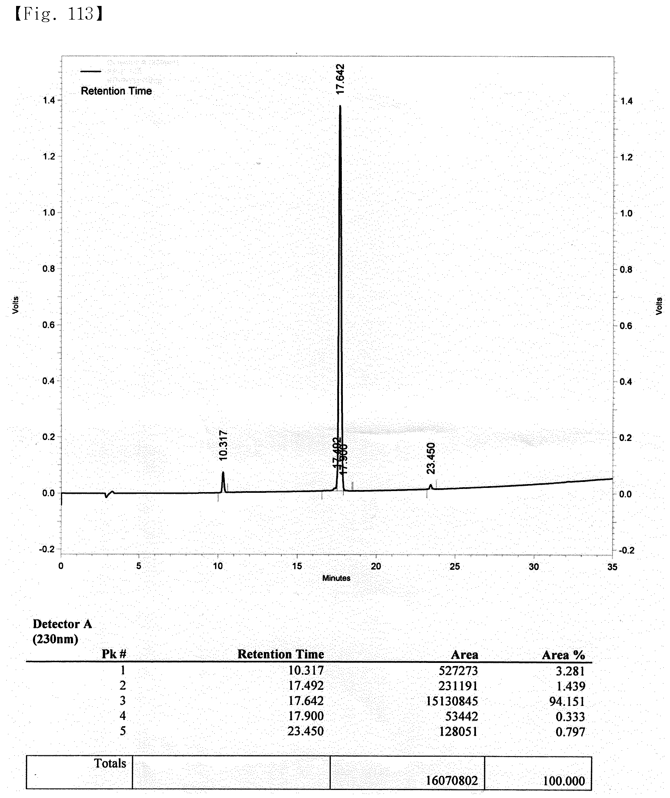

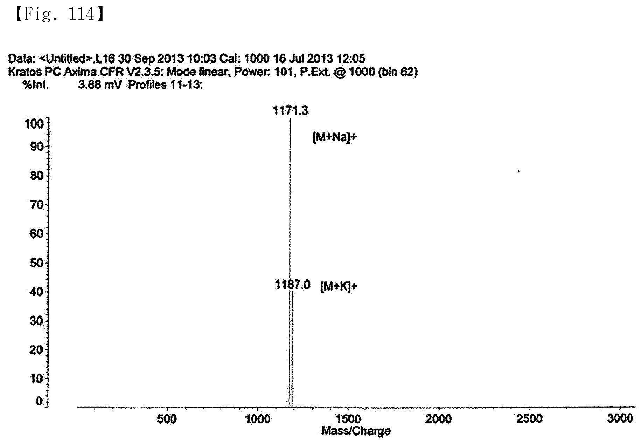

The purity and molecular weight of the HKFY-44 are shown in Figs. 113 and 114, respectively.

### Example 1.45. Preparation of HKFY-45

D-biotin-His(trt)-K(caprylic)-Bip-Tyr(Boc)-trityl resin was prepared in the same manner except that Fmoc-Bip-OH was used instead of Fmoc-Phe-OH during the preparation process of Example 1.2. The protecting group of the resin was cleaved to prepare biotin-His(trt)-K(caprylic)-Bip-Tyr (((S)-3-([1,1'-biphenyl]-4-yl)-2-((S)-6-octanamido-2-((S)-2-(5-((3aS,4S,6aR)-2-oxohexahydro-1H-thieno[3,4-d]imidazol-4-yl)pentanamido)-3-(1-trityl-1H-imidazol-4-yl)propanamido)hexanamido)propanoyl)-L-tyrosine).

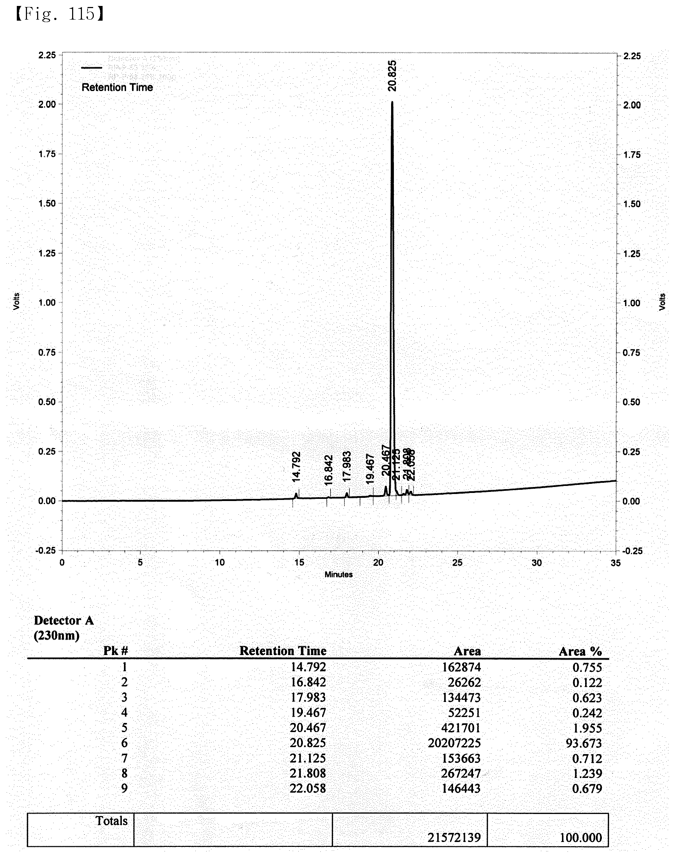

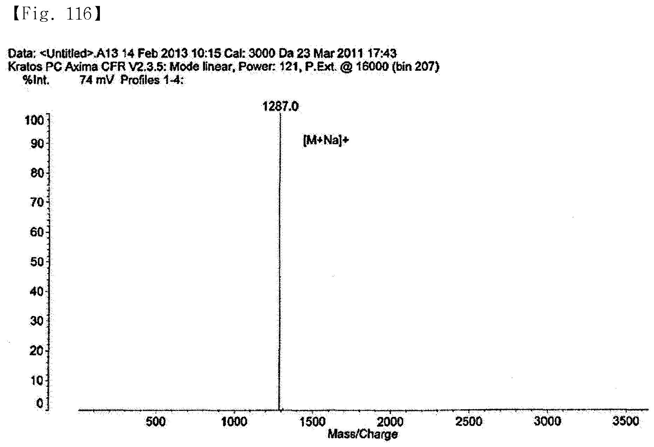

The purity and molecular weight of the HKFY-45 are shown in Figs. 115 and 116, respectively.

### Example 1.46. Preparation of HKFY-46

Fmoc-Phe-OH and DMF were loaded onto the Fmoc-Trp(Boc)-trityl resin obtained from Example 1.44. to prepare Fmoc-Trp(Boc)-trityl resin. Fmoc-Lys(caprylic)-OH obtained from Example 1.1., and Fmoc-His(trt)-OH were sequentially added to the resin in the same manner to prepare Fmoc-His(trt)-Lys(caprylic)-Phe-Trp(Boc)-trityl resin. The protecting group of the resin was cleaved to prepare His(trt)-Lys(caprylic)-Phe-Trp (N6-octanoyl-N2-(Nt-trityl-L-histidyl)-L-lysyl-L-phenylalanyl-L-tryptophan).

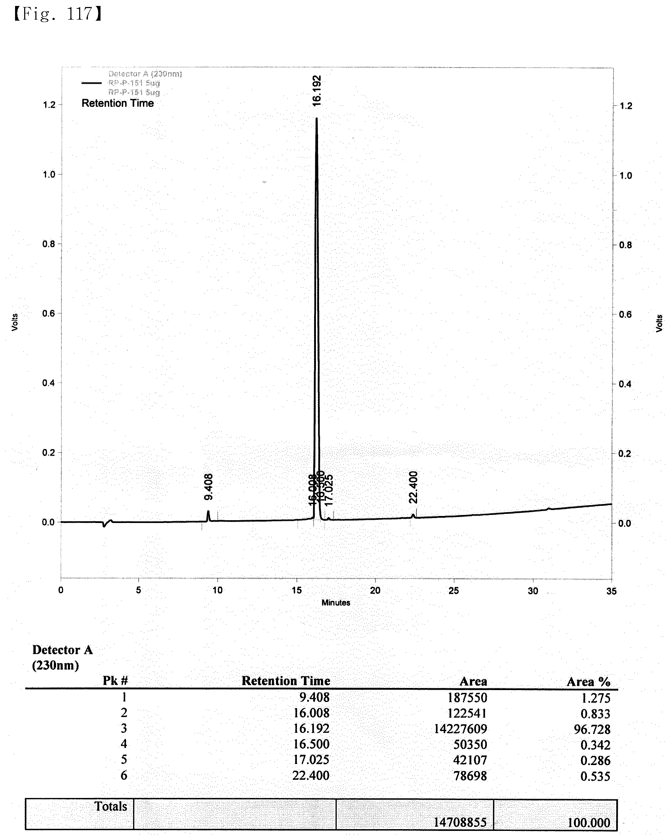

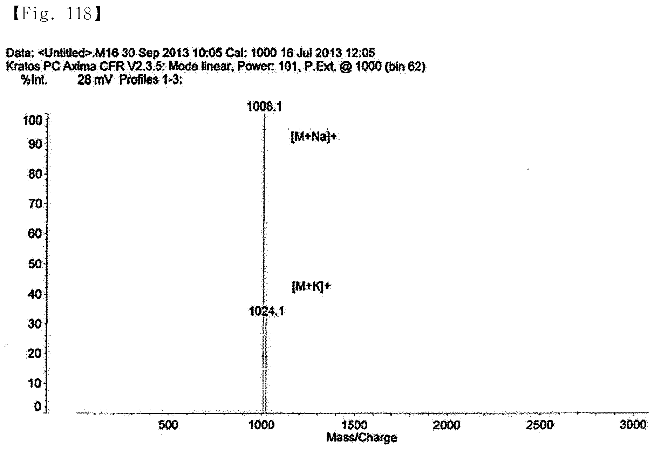

The purity and molecular weight of the HKFY-46 are shown in Figs. 117 and 118, respectively.

### Example 1.47. Preparation of HKFY-47

In order to prepare N6-octanoyl-N2-(Nt-trityl-L-histidyl)-L-lysyl-L-tyrosyl-L-phenylalanine, Fmoc-Phe-OH and DMF were loaded onto the trityl resin to prepare Fmoc-Phe-trityl resin. Fmoc-Tyr(Boc)-OH, Fmoc-Lys(caprylic)-OH obtained from Example 1.1., and Fmoc-His(trt)-OH were sequentially added to the resin in the same manner to prepare Fmoc-His(trt)-Lys(caprylic)-Tyr(Boc)-Phe-trityl resin. The protecting group of the resin was cleaved to prepare His(trt)-Lys(caprylic)-Tyr-Phe (N6-octanoyl-N2-(Nt-trityl-L-histidyl)-L-lysyl-L-tyrosyl-L-phenylalanine).

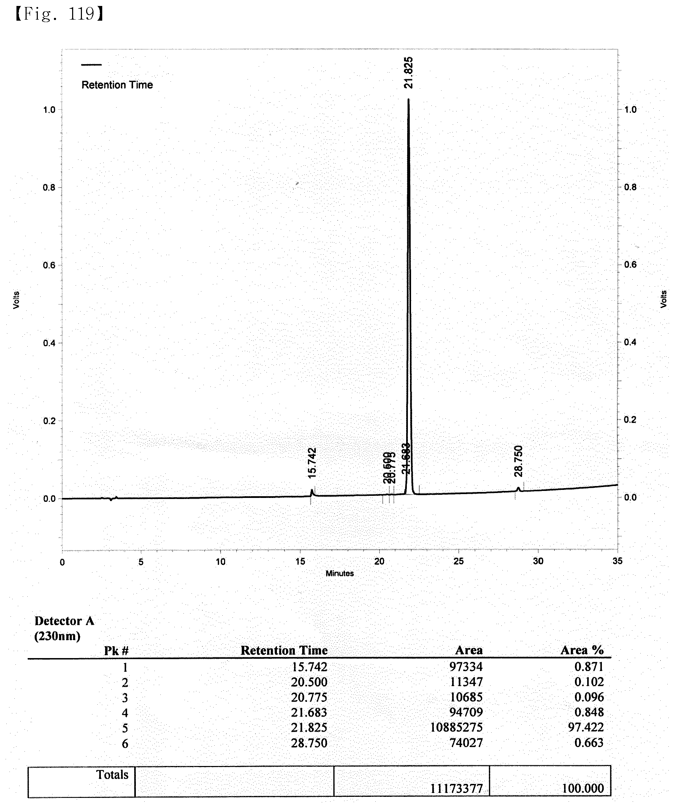

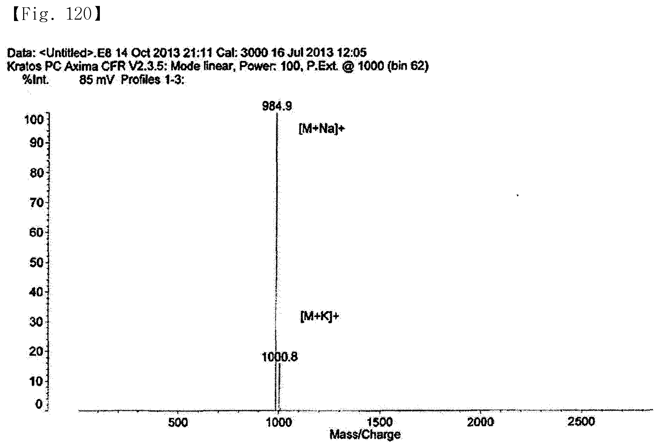

The purity and molecular weight of the HKFY-47 are shown in Figs. 119 and 120, respectively.

### Example 1.48. Preparation of HKFY 48

In order to prepare HKFY-48 (N6-octanoyl-N2-(Nt-trityl-L-histidyl)-L-lysyl-L-tyrosyl-L-tyrosine), Fmoc-Tyr(Boc)-OH and DMF were loaded onto the trityl resin to prepare Fmoc-Tyr(Boc)-trityl resin. Fmoc-Tyr(Boc)-OH, Fmoc-Lys(caprylic)-OH obtained from Example 1.1., and Fmoc-His(trt)-OH were sequentially added to the resin in the same manner to prepare Fmoc-His(trt)-Lys(caprylic)-Tyr(Boc)-Tyr(Boc)-trityl resin. The protecting group of the resin was cleaved to prepare His(trt)-Lys(caprylic)-Tyr-Tyr (N6-octanoyl-N2-(Nt-trityl-L-histidyl)-L-lysyl-L-tyrosyl-L-tyrosine).

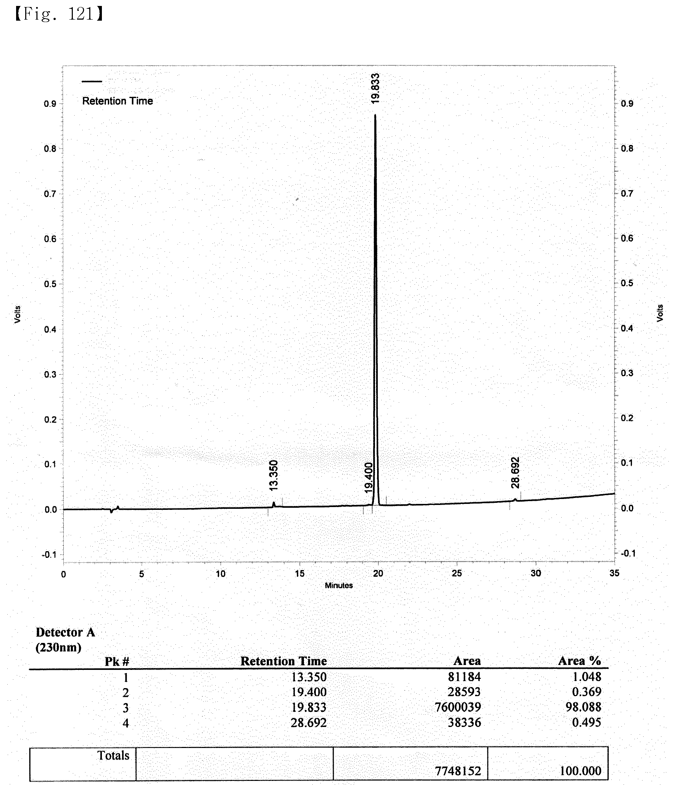

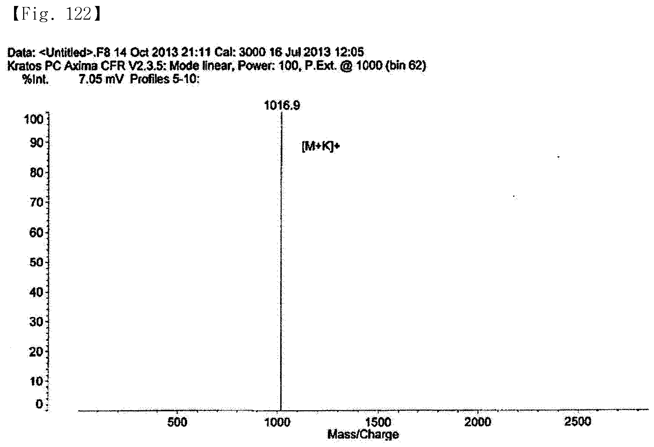

The purity and molecular weight of the HKFY-48 are shown in Figs. 121 and 122, respectively.

### Example 1.49. Preparation of HKFY-49

In order to prepare HKFY-49 (N6-octanoyl-N2-(Nt-trityl-L-histidyl)-L-lysyl-L-phenylalanine), Fmoc-Lys(caprylic)-OH obtained from Example 1.1. and Fmoc-His(trt)-OH were sequentially added to the Fmoc-Phe-trityl resin obtained from Example 1.47. in the same manner to prepare Fmoc-His(trt)-Lys(caprylic)-Phe-trityl resin. The protecting group of the resin was cleaved to prepare His(trt)-Lys(caprylic)-Phe.

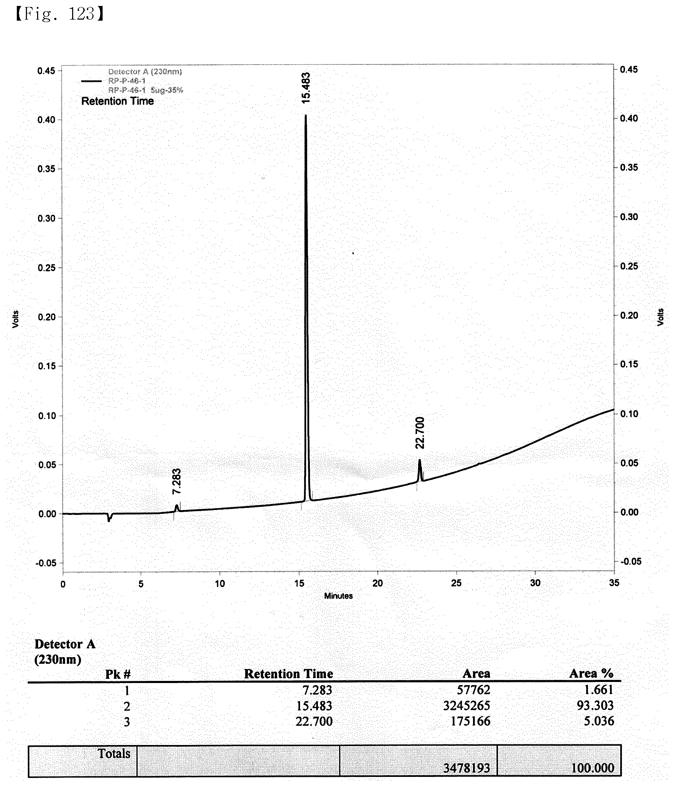

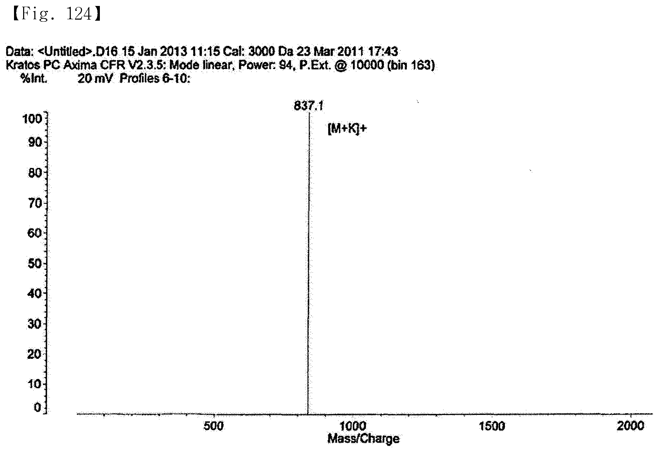

The purity and molecular weight of the HKFY-49 are shown in Figs. 123 and 124, respectively.

### Example 1.50. Preparation of HKFY-50

In order to prepare HKFY-50 (N6-octanoyl-N2-(Nt-trityl-L-histidyl)-L-lysyl-L-phenylalanyl-L-serine), Fmoc-Ser(tBu)-OH and DMF were loaded onto the trityl resin to prepare Fmoc-Ser(tBu)-trityl resin. Fmoc-Phe-OH, Fmoc-Lys(caprylic)-OH obtained from Example 1.1., and Fmoc-His(trt)-OH were sequentially added to the resin in the same manner to prepare Fmoc-His(trt)-Lys(caprylic)-Phe-Ser-trityl resin. The protecting group of the resin was cleaved to prepare His(trt)-Lys(caprylic)-Phe-Ser.

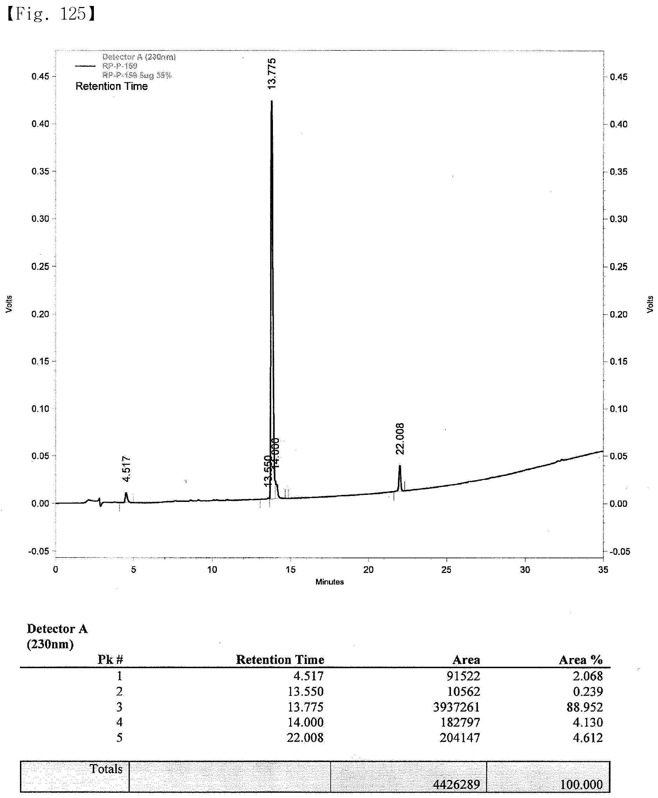

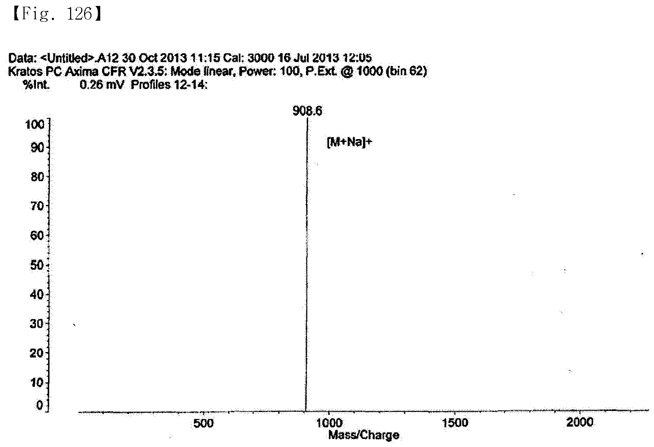

The purity and molecular weight of the HKFY-50 are shown in Figs. 125 and 126, respectively.

### Example 1.51. Preparation of HKFY-51

His(trt)-Lys(caprylic)-Leu-Tyr (N6-octanoyl-N2-(Nt-trityl-L-histidyl)-L-lysyl-L-leucyl-L-tyrosine) was prepared in the same manner except that Fmoc-Leu-OH was used instead of Fmoc-Tyr(Boc)-OH among the constituent amino acids of Example 1.48.

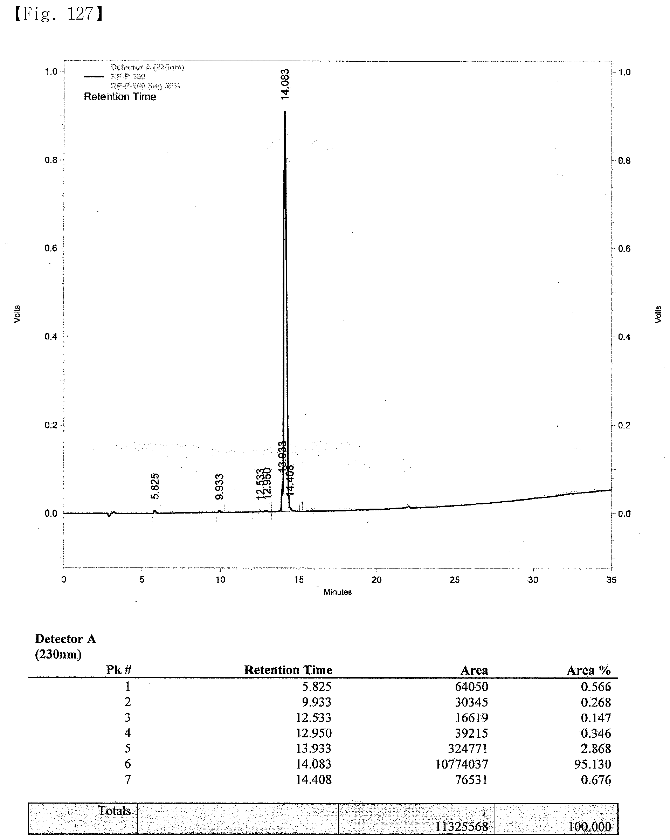

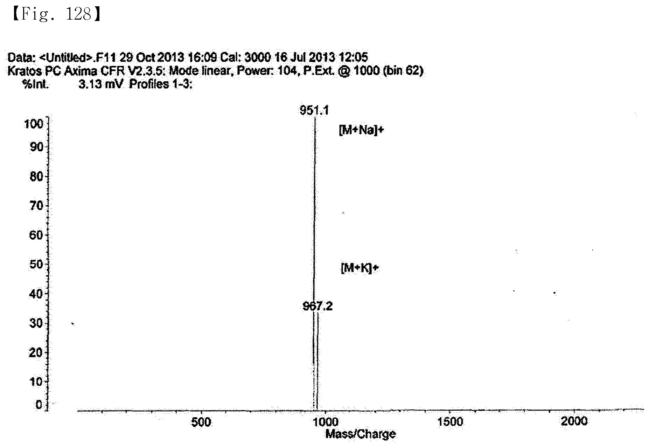

The purity and molecular weight of the HKFY-51 are shown in Figs. 127 and 128, respectively.

### Example 1.52. Preparation of HKFY-52

Fmoc-Asp(tBu)-OH, Fmoc-Tyr(Boc)-OH, Fmoc-Phe-OH, and Fmoc-His(trt)-OH were sequentially added to the Fmoc-Gln(trt)-Gln(trt)-Ala-Arg(pbf)-Phe-trityl resin obtained from Example 1.38. in the same manner to prepare Fmoc-His(trt)-Phe-Tyr(Boc)-Asp(tBu)-Gln(trt)-Gln(trt)-Ala-Arg(pbf)-Phe-trityl resin. The protecting group of the resin was cleaved to prepare His(trt)-Phe-Tyr-Asp-Gln-Gln-Ala-Arg-Phe ((6S,9S,12S,15S,18S)-1-amino-6-(((S)-1-amino-1-oxo-3-phenylpropan-2-yl)carbamoyl)-18-((S)-2-((S)-2-((S)-2-amino-3-(1-trityl-1H-imidazol-4-yl)propanamido)-3-phenylpropanamido)-3-(4-hydroxyphenyl)propanamido)-12,15-bis(3-amino-3-oxopropyl)-1-imino-9-methyl-8,11,14,17-tetraoxo-2,7,10,13,16-pentaazaicosan-20-oic acid).

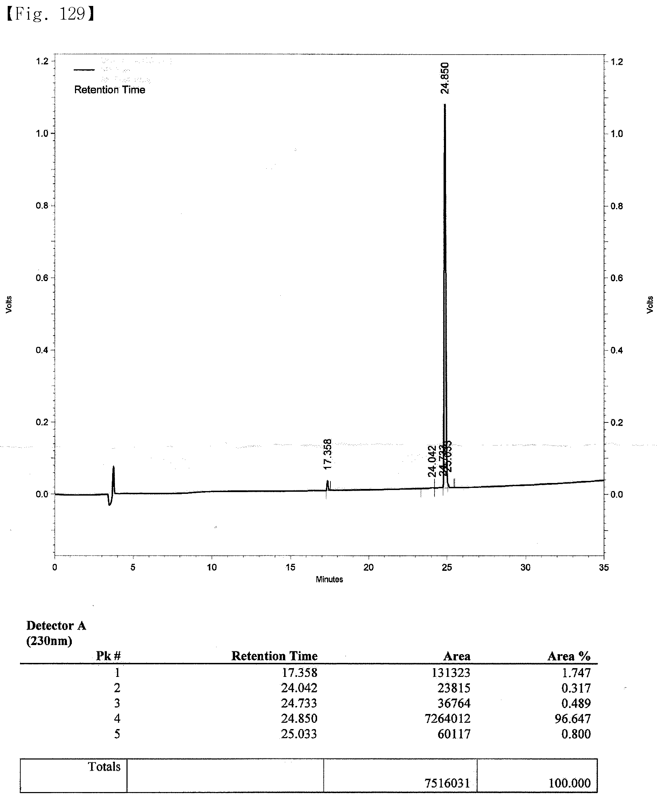

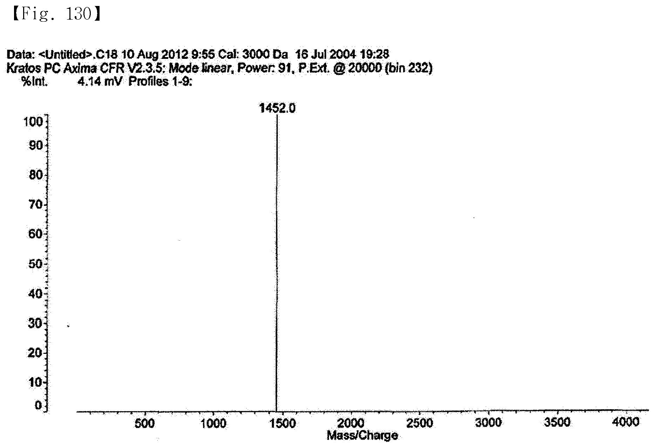

The purity and molecular weight of the HKFY-52 are shown in Figs. 129 and 130, respectively.

### Example 1.53. Preparation of HKFY-53

Fmoc-Lys(caprylic)-OH obtained from Example 1.1., Fmoc-Ser(tBu)-OH, Fmoc-Gly-OH, and Fmoc-His(trt)-OH were sequentially added to the Fmoc-Phe-Tyr(Boc)-trityl resin obtained from Example 1.47. in the same manner to prepare Fmoc-His(trt)-Gly-Ser(tBu)-Lys(caprylic)-Phe-Tyr(Boc)-trityl resin. The protecting group of the resin was cleaved to prepare His(trt)-Gly-Ser-Lys(caprylic)-Phe-Tyr (N6-octanoyl-N2-(Nt-trityl-L-histidylglycyl-L-seryl)-L-lysyl-L-phenylalanyl-L-tyrosine).

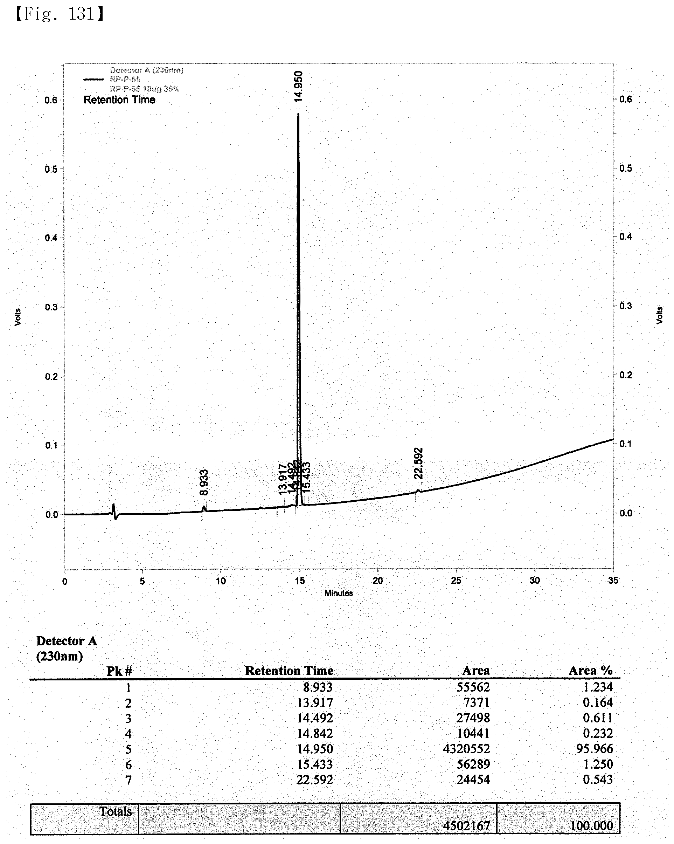

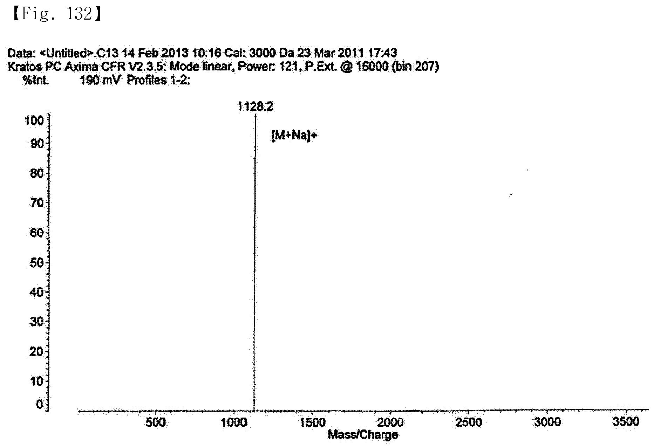

The purity and molecular weight of the HKFY-53 are shown in Figs. 131 and 132, respectively.

### Example 1.54. Preparation of HKFY-54

Fmoc-Lys(caprylic)-OH obtained from Example 1.1. and DMF and HOBt were added to the Fmoc-Phe-Tyr(Boc)-trityl resin obtained from Example 1.47. to prepare Fmoc-Lys(caprylic)-Phe-Tyr(Boc)-trityl resin. The protecting group of the resin was cleaved to prepare Lys(caprylic)-Phe-Tyr (N6-octanoyl-L-lysyl-L-phenylalanyl-L-tyrosine).

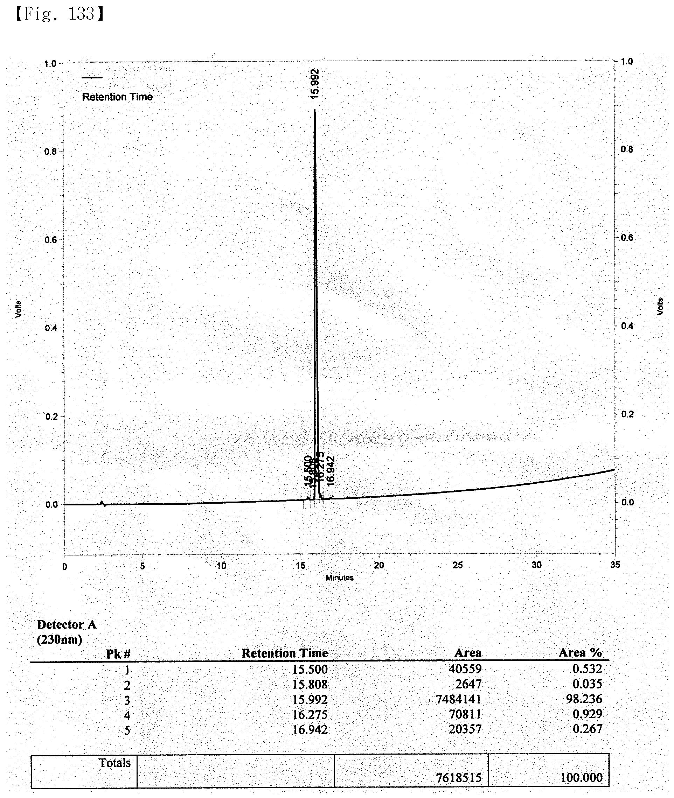

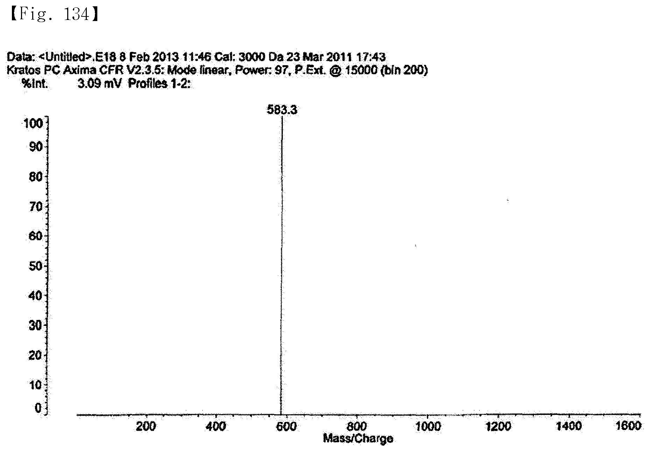

The purity and molecular weight of the HKFY-54 are shown in Figs. 133 and 134, respectively.

### Example 1.55. Preparation of HKFY-55

Fmoc-His(trt)-OH, and Fmoc-Lys(caprylic)-OH obtained from Example 1.1. were sequentially added to the Fmoc-Phe-Tyr(Boc)-trityl resin obtained from Example 1.47. in the same manner to prepare Fmoc-Lys(caprylic)-His(trt)-Phe-Tyr(Boc)-trityl resin. The protecting group of the resin was cleaved to prepare Lys(caprylic)-His(trt)-Phe-Tyr (Na-(N6-octanoyl-L-lysyl)-Nt-trityl-L-histidyl-L-phenylalanyl-L-tyrosine).

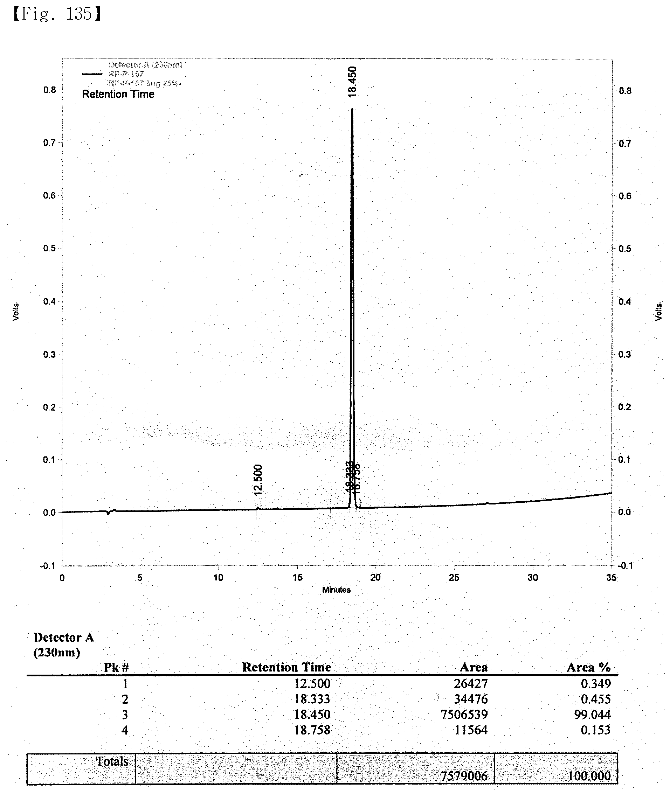

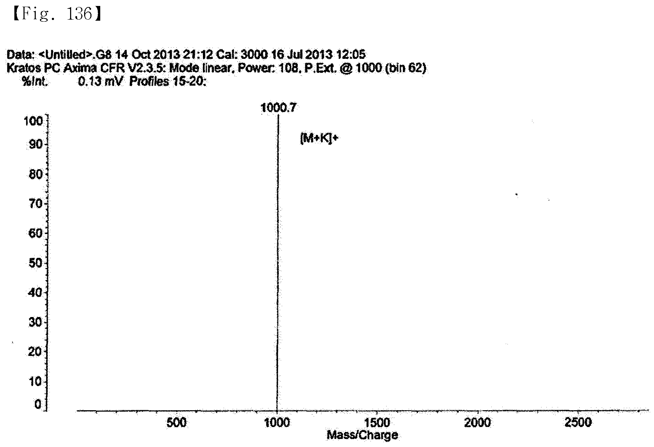

The purity and molecular weight of the HKFY-55 are shown in Figs. 135 and 136, respectively.

### Example 1.56. Preparation of HKFY-56

Fmoc-Asp(tBu)-Gln(trt)-Gln(trt)-Ala-Arg(pbf)-Phe-trityl resin was prepared in the same manner except that Fmoc-Asp(tBu)-OH was used instead of Fmoc-Ser(tBu)-OH during the preparation process of Example 1.38. DMF containing 2% NH₂NH₂ .H₂O was added to Fmoc-Lys(dde)-OH, and dde was removed to prepare Fmoc-Lys-OH. DMF containing caproic acid and HOBt and DIC was added to the Fmoc-Lys-OH to prepare Fmoc-Lys(caproic acid)-OH.

Fmoc-Lys(caproic)-OH and Fmoc-His(trt)-OH were sequentially added in the same manner to prepare Fmoc-His(trt)-Lys(caproic)-Phe-Tyr(Boc)-Asp(tBu)-Gln(trt)-Gln(trt)-Ala-Arg(pbf)-Phe-trityl resin. The protecting group of the resin was cleaved to prepare His(trt)-Lys(caproic)-Phe-Tyr-Asp-Gln-Gln-Ala-Arg-Phe ((6S,9S,12S,15S,18S)-1-amino-6-(((S)-1-amino-1-oxo-3-phenylpropan-2-yl)carbamoyl)-18-((S)-2-((S)-2-((S)-2-((S)-2-amino-3-(1-trityl-1H-imidazol-4-yl)propanamido)-6-(non-1-en-2-ylamino)hexanamido)-3-phenylpropanamido)-3-(4-hydroxyphenyl)propanamido)-12,15-bis(3-amino-3-oxopropyl)-1-imino-9-methyl-8,11,14,17-tetraoxo-2,7,10,13,16-pentaazaicosan-20-oic acid).

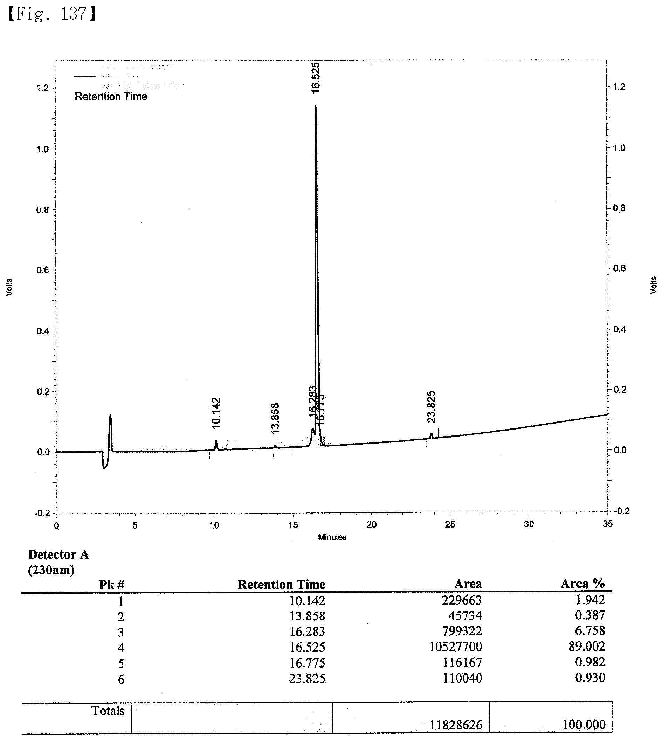

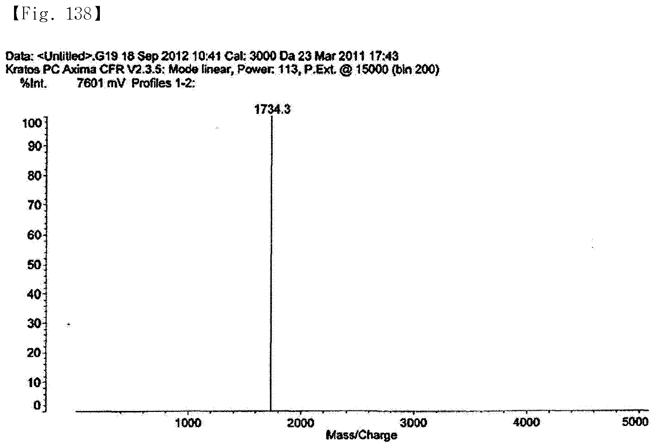

The purity and molecular weight of the HKFY-56 are shown in Figs. 137 and 138, respectively.

### Example 1.57. Preparation of HKFY-57

His(2-phenyl)-Lys(caprylic)-d-Phe(methyl)-Tyr (((R)-2-((S)-2-((S)-2-amino-3-(2-phenyl-1H-imidazol-4-yl)propanamido)-6-octanamidohexanamido)-3-(p-tolyl)propanoyl)-L-tyrosine) was prepared in the same manner except that Boc-His(2-phenyl)-OH was used instead of Boc-His(benzhydryl)-OH among the constituent amino acids of Example 1.34.

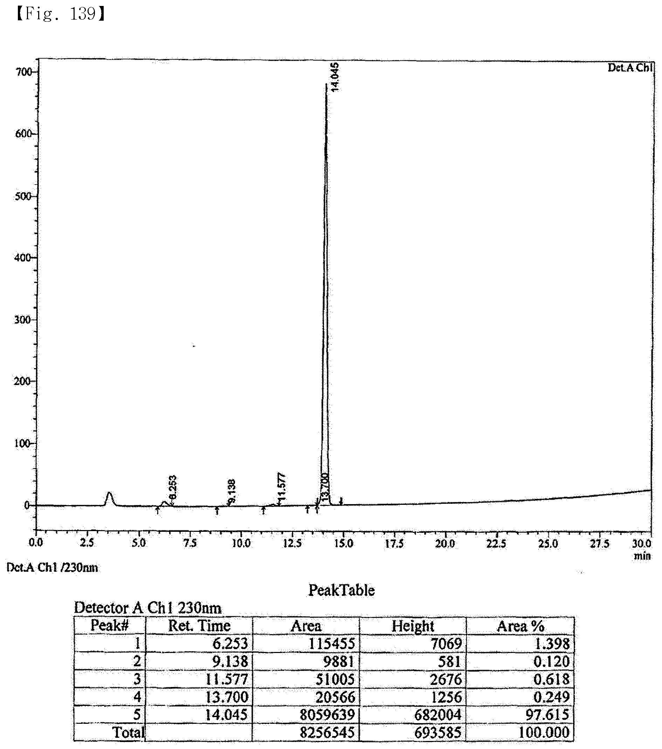

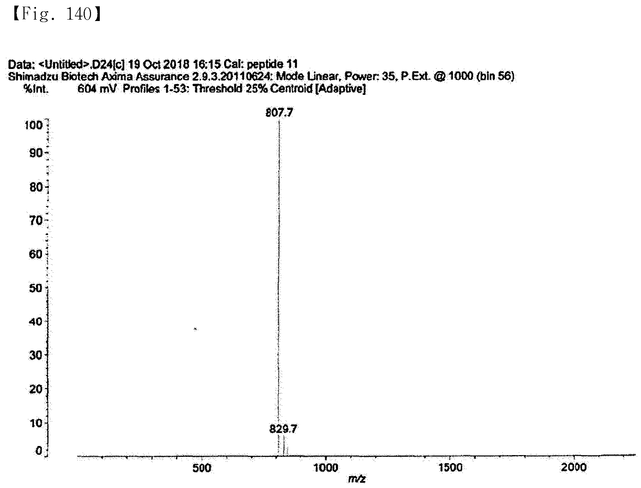

The purity and molecular weight of the HKFY-57 are shown in Figs. 139 and 140, respectively.

### Example 1.58. Preparation of HKFY-58

His(1-phenyl)-Lys(caprylic)-d-Phe(methyl)-Tyr (((R)-2-((S)-2-((S)-2-amino-3-(1-phenyl-1H-imidazol-4-yl)propanamido)-6-octanamidohexanamido)-3-(p-tolyl)propanoyl)-L-tyrosine) was prepared in the same manner except that Boc-His(1-phenyl)-OH was used instead of Boc-His(benzhydryl)-OH among the constituent amino acids of Example 1.34.

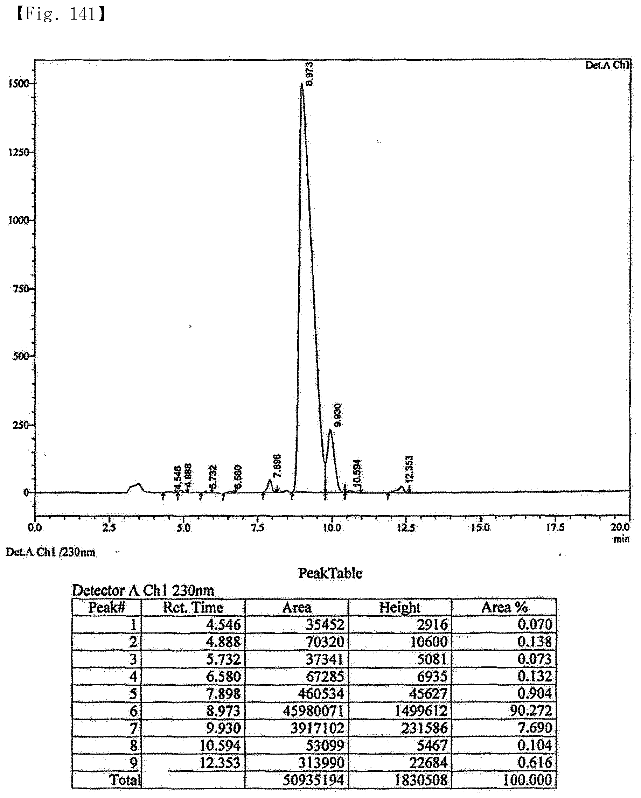

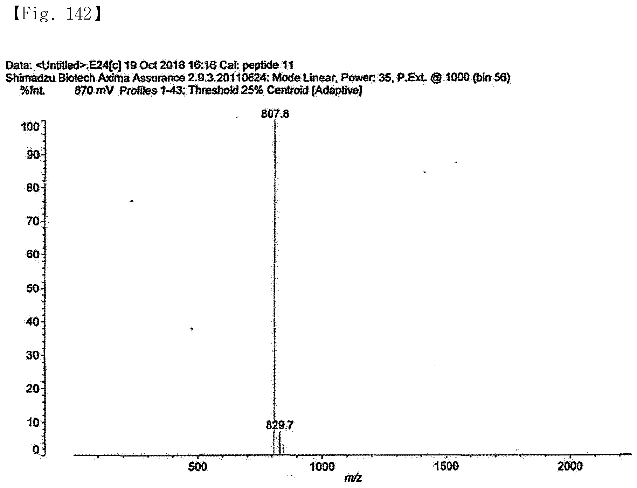

The purity and molecular weight of the HKFY-58 are shown in Figs. 141 and 142, respectively.

### Example 1.59. Preparation of HKFY-59

His(1,2-diphenyl)-Lys(caprylic)-d-Phe(methyl)-Tyr (((R)-2-((S)-2-((S)-2-amino-3-(12-diphenyl-1H-imidazol-4-yl)propanamido)-6-octanamidohexanamido)-3-(p-tolyl)propanoyl)-L-tyrosine) was prepared in the same manner except that Boc-His(1,2-diphenyl)-OH was used instead of Boc-His(benzhydryl)-OH among the constituent amino acids of Example 1.34.

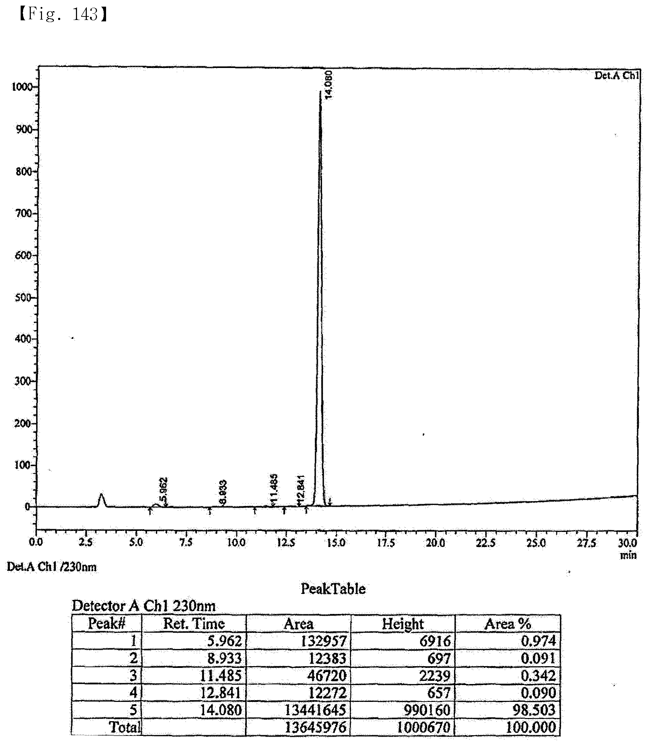

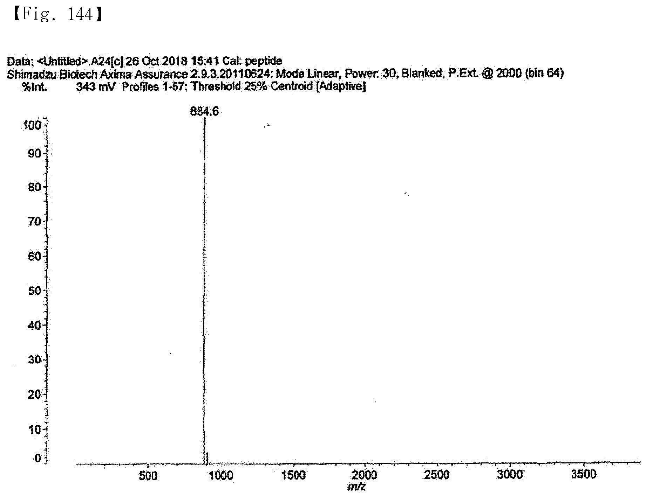

The purity and molecular weight of the HKFY-59 are shown in Figs. 143 and 144, respectively.

### Example 1.60. Preparation of HKFY-60

His(2-(4-tert-butyl)phenyl)-Lys(caprylic)-d-Phe(methyl)-Tyr (((R)-2-((S)-2-((S)-2-amino-3-(2-(4-(tert-butyl)phenyl)-1H-imidazol-4-yl)propanamido)-6-octanamidohexanamido)-3-(p-tolyl)propanoyl)-L-tyrosine) was prepared in the same manner except that Boc-His(2-(4-tert-butyl)phenyl)-OH was used instead of Boc-His(benzhydryl)-OH among the constituent amino acids of Example 1.34.

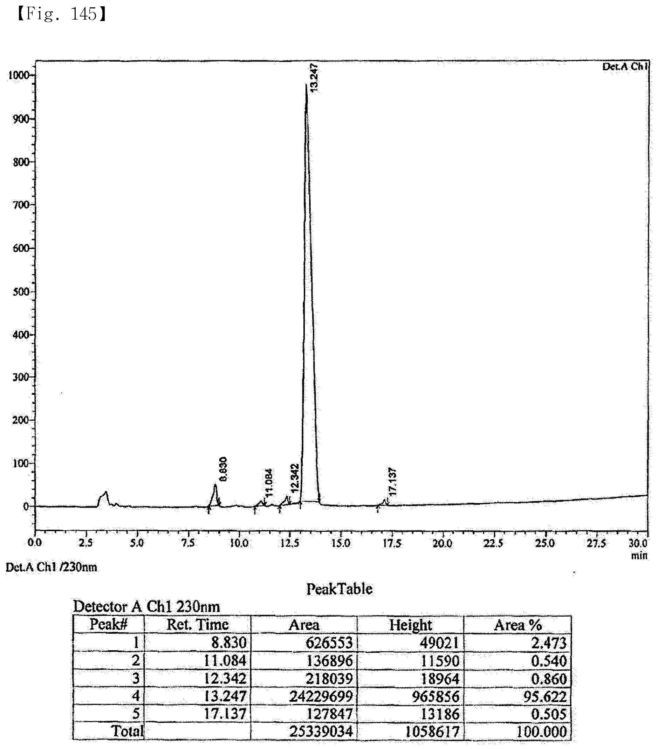

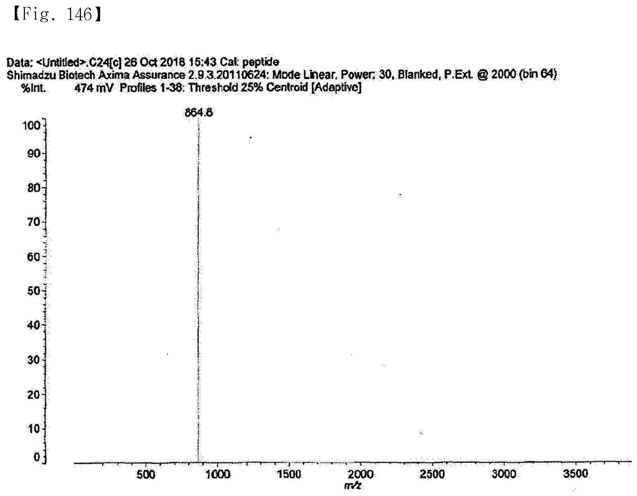

The purity and molecular weight of the HKFY-60 are shown in Figs. 145 and 146, respectively.

### Example 1.61. Preparation of HKFY-61

His(1-(4-tert-butyl)phenyl)-Lys(caprylic)-d-Phe(methyl)-Tyr(((R)-2-((S)-2-((S)-2-amino-3-(1-(4-(tert-butyl)phenyl)-1H-imidazol-4-yl)propanamido)-6-octanamidohexanamido)-3-(p-tolyl)propanoyl)-L-tyrosine) was prepared in the same manner except that Boc-His(1-(4-tert-butyl)phenyl)-OH was used instead of Boc-His(benzhydryl)-OH among the constituent amino acids of Example 1.34.

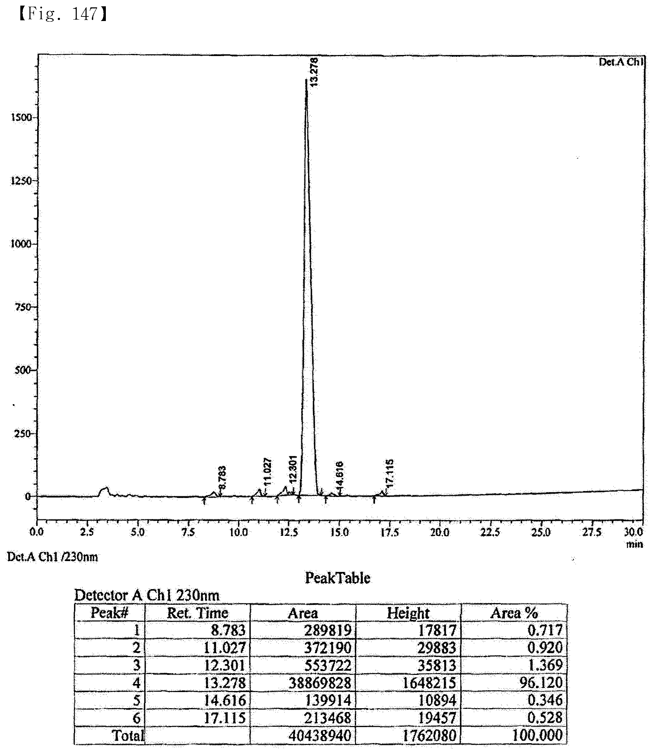

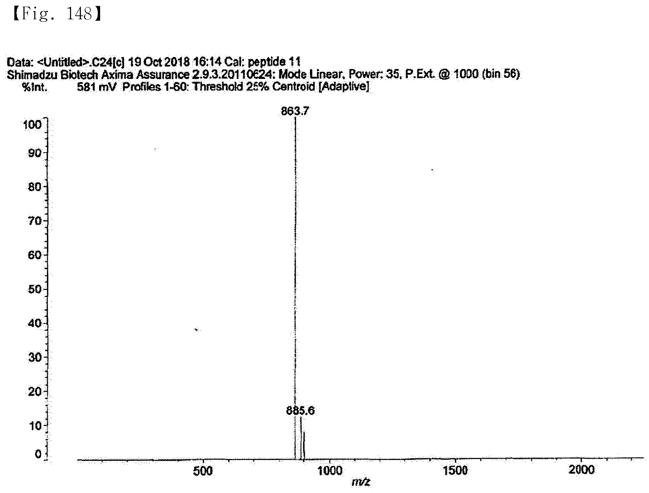

The purity and molecular weight of the HKFY-61 are shown in Figs. 147 and 148, respectively.

### Example 1.62. Preparation of HKFY-62

D-His(benzhydryl)-Lys(caprylic)-d-Phe(methyl)-Tyr (((R)-2-((S)-2-((R)-2-amino-3-(1-benzhydryl-1H-imidazol-4-yl)propanamido)-6-octanamidohexanamido)-3-(p-tolyl)propanoyl)-L-tyrosine) was prepared in the same manner except that Boc-d-His(benzhydryl)-OH was used instead of Boc-His(benzhydryl)-OH among the constituent amino acids of Example 1.34.

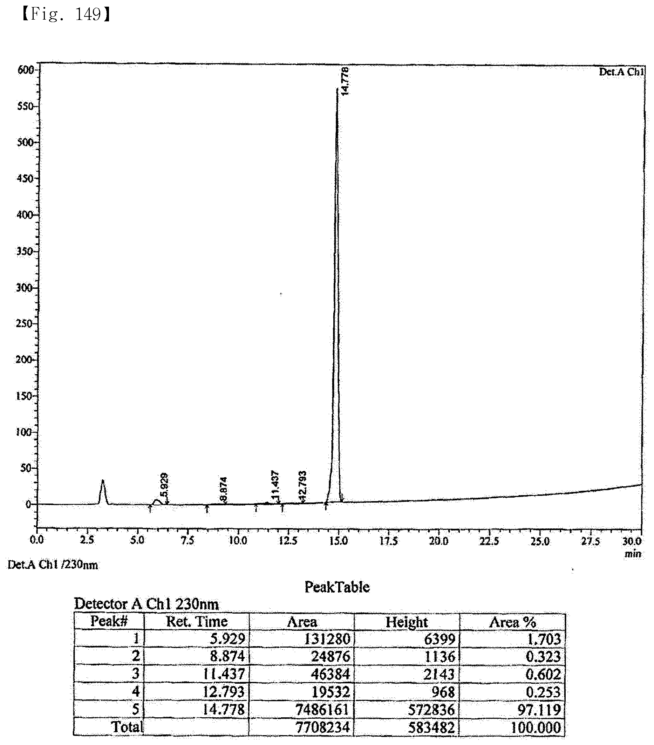

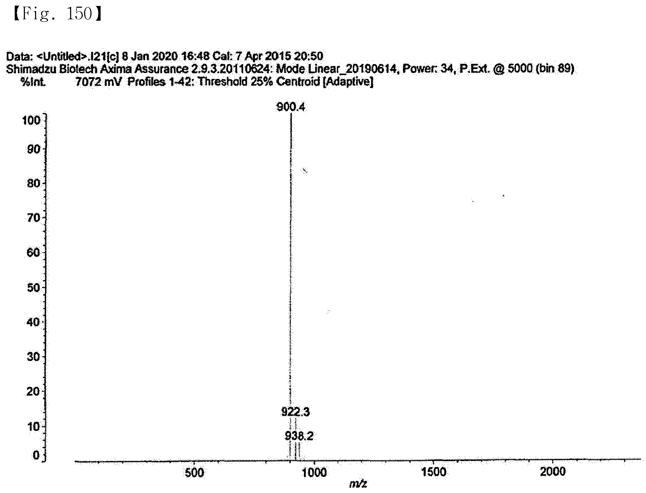

The purity and molecular weight of the HKFY-62 are shown in Figs. 149 and 150, respectively.

### Example 1.63. Preparation of HKFY-63

His(thiophene)-Lys(caprylic)-d-Phe(methyl)-Tyr (((R)-2-((S)-2-((S)-2-amino-3-(2-(thiophen-2-yl)-1H-imidazol-4-yl)propanamido)-6-octanamidohexanamido)-3-(p-tolyl)propanoyl)-L-tyrosine) was prepared in the same manner except that Boc-His(thiophene)-OH was used instead of Boc-His(benzhydryl)-OH among the constituent amino acids of Example 1.34.

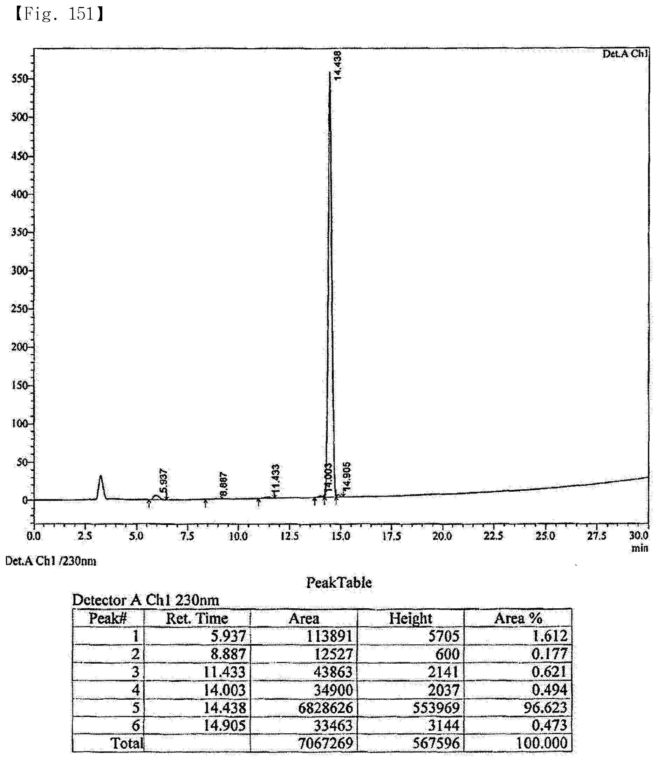

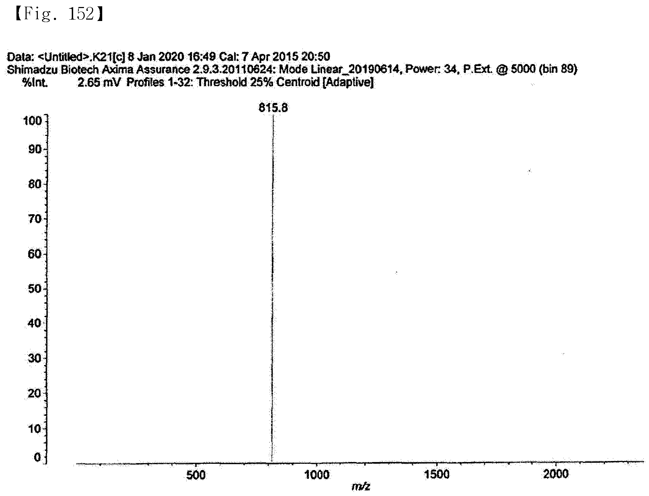

The purity and molecular weight of the HKFY-63 are shown in Figs. 151 and 152, respectively.

### Example 1.64. Preparation of HKFY-64

His(4-methoxybenzhydryl)-Lys(caprylic)-d-Phe(methyl)-Tyr (((2R)-2-((2S)-2-((2S)-2-amino-3-(1-((4-methoxyphenyl)(phenyl)methyl)-1H-imidazol-4-yl)propanamido)-6-octanamidohexanamido)-3-(p-tolyl)propanoyl)-L-tyrosine) was prepared in the same manner except that Boc-His(4-methoxybenzhydryl)-OH was used instead of Boc-His(benzhydryl)-OH among the constituent amino acids of Example 1.34.

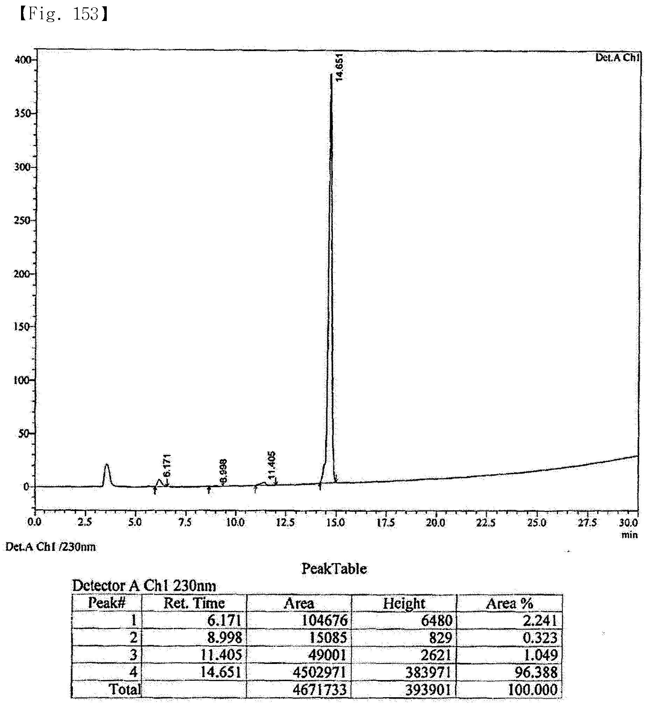

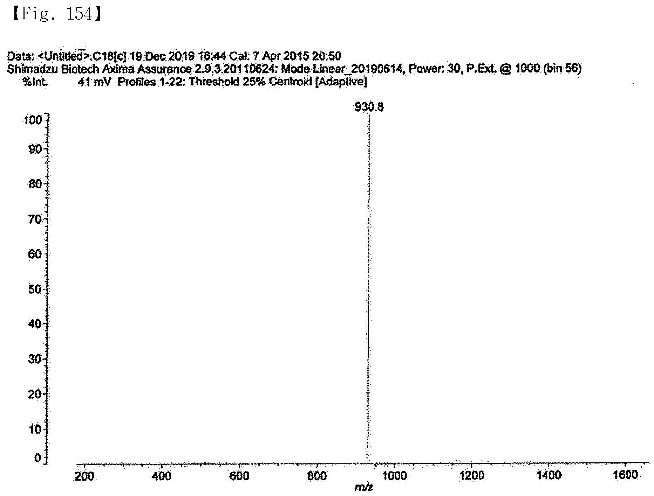

The purity and molecular weight of the HKFY-64 are shown in Figs. 153 and 154, respectively.

### Example 1.65. Preparation of HKFY-65

His(4-hydroxybenzhydryl)-Lys(caprylic)-d-Phe(methyl)-Tyr (((2R)-2-((2S)-2-((2S)-2-amino-3-(1-((4-hydroxyphenyl)(phenyl)methyl)-1H-imidazol-4-yl)propanamido)-6-octanamidohexanamido)-3-(p-tolyl)propanoyl)-L-tyrosine) was prepared in the same manner except that Boc-His(4-hydroxybenzhydryl)-OH was used instead of Boc-His(benzhydryl)-OH among the constituent amino acids of Example 1.34.

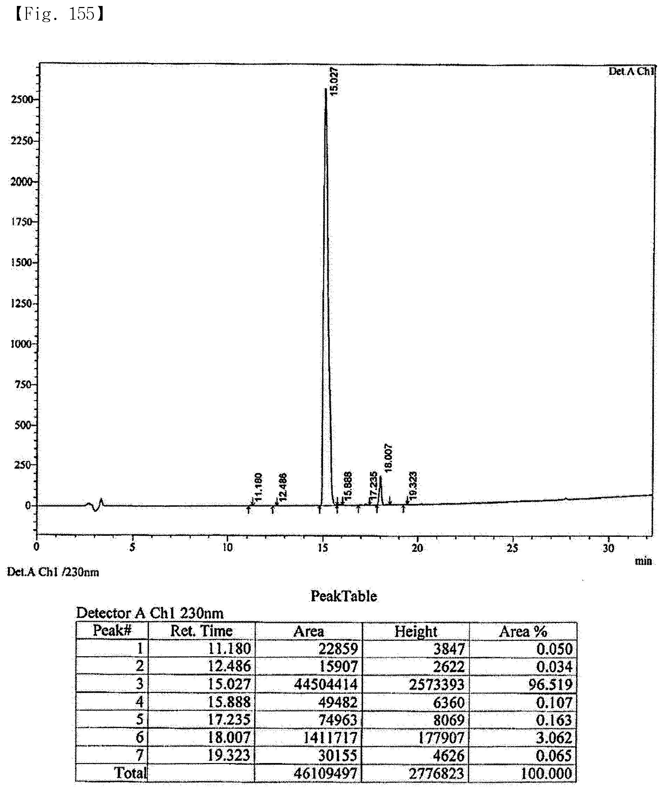

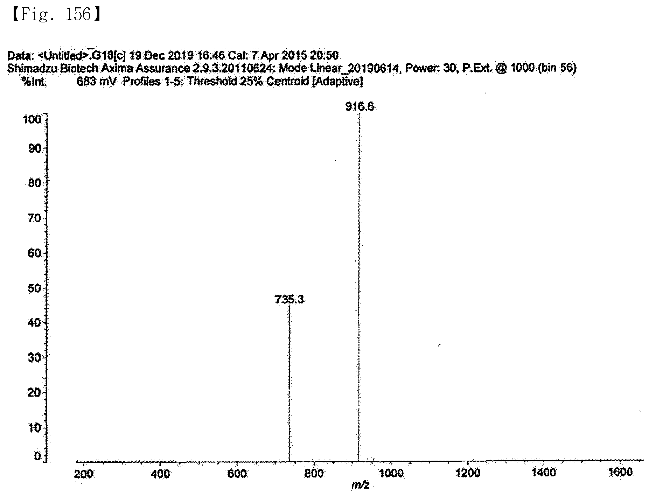

The purity and molecular weight of the HKFY-65 are shown in Figs. 155 and 156, respectively.

### Example 1.66. Preparation of HKFY-66

His(benzhydryl)-Lys(caprylic)-d-Phe(4-methyl)-Tyr-NH₂, (N-((S)-5-((S)-2-amino-3-(1-benzhydryl-1H-imidazol-4-yl)propanamido)-6-(((R)-1-(((S)-1-amino-3-(4-hydroxyphenyl)-1-oxopropan-2-yl)amino)-1-oxo-3-(p-tolyl)propan-2-yl)amino)-6-oxohexyl)octanamide) was prepared in the same manner except that Fmoc-Tyr-wang resin was used instead of the trityl resin among the constituent amino acids of Example 1.34.

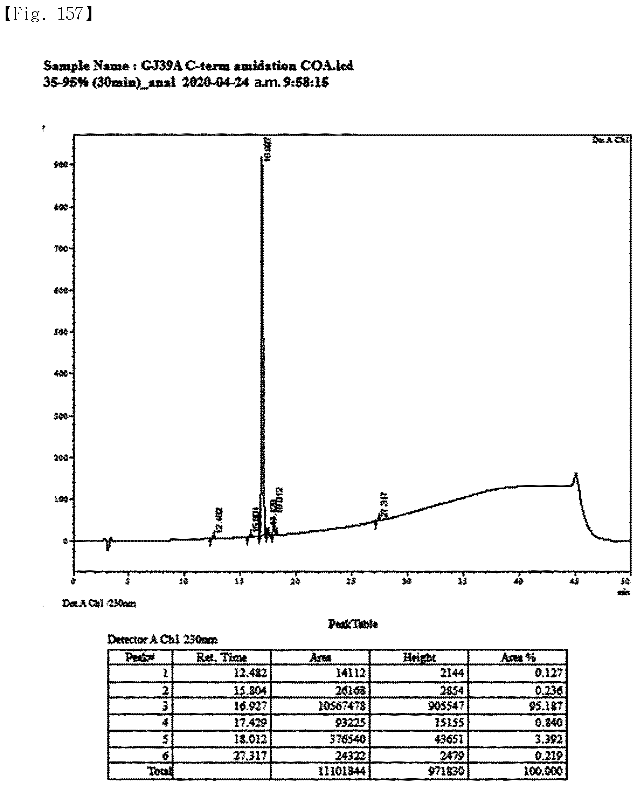

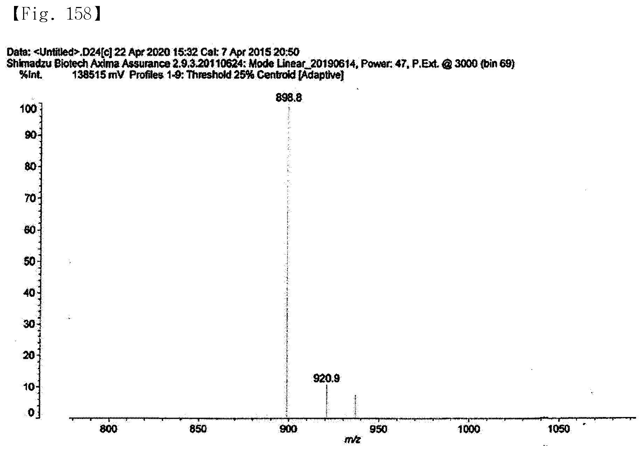

The purity and molecular weight of the HKFY-66 are shown in Figs. 157 and 158, respectively.

### Example 1.67. Preparation of HKFY-67

His(benzhydryl)-Lys(caprylic)-d-Phe(4-methyl)-Tyr(3-chloro) ((S)-2-((R)-2-((S)-2-((S)-2-amino-3-(1-benzhydryl-1H-imidazol-4-yl)propanamido)-6-octanamidohexanamido)-3-(p-tolyl)propanamido)-3-(3-chloro-4-hydroxyphenyl)propanoic acid) was prepared in the same manner except that Fmoc-Tyr(3-chloro) was used instead of Fmoc-Tyr(tBu) among the constituent amino acids of Example 1.34.

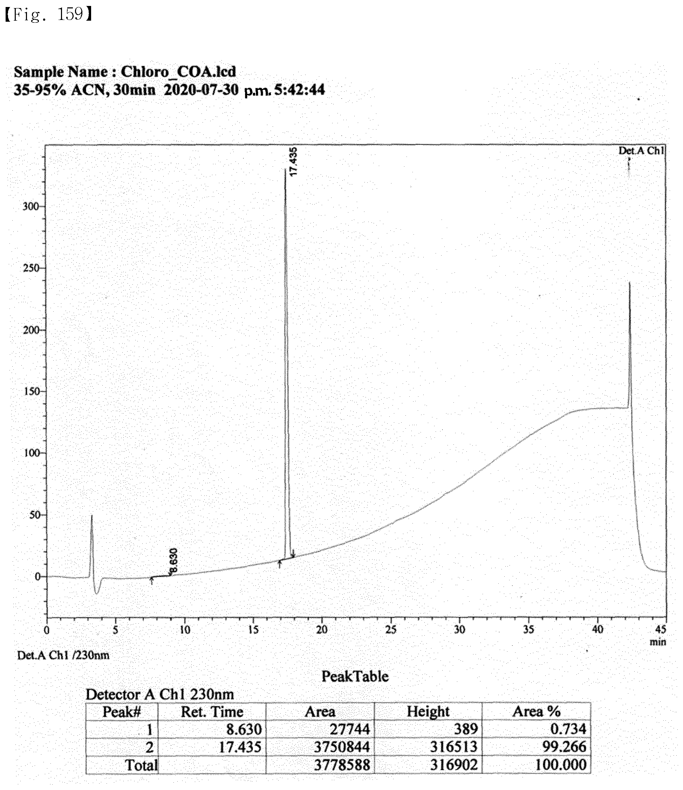

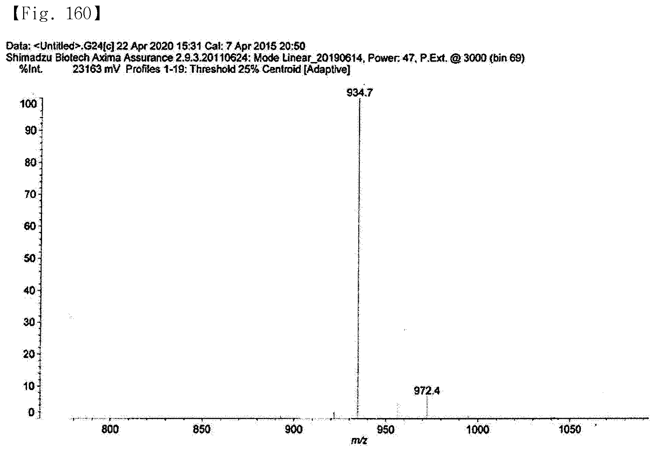

The purity and molecular weight of the HKFY-67 are shown in Figs. 159 and 160, respectively.

### Example 1.68. Preparation of HKFY-68

His(benzhydryl)-Lys(caprylic)-d-Phe(4-methyl)-Tyr(3-nitro) ((S)-2-((R)-2-((S)-2-((S)-2-amino-3-(1-benzhydryl-1H-imidazol-4-yl)propanamido)-6-octanamidohexanamido)-3-(p-tolyl)propanamido)-3-(4-hydroxy-3-nitrophenyl)propanoic acid) was prepared in the same manner except that Fmoc-Tyr(3-nitro) was used instead of Fmoc-Tyr(tBu) among the constituent amino acids of Example 1.34.

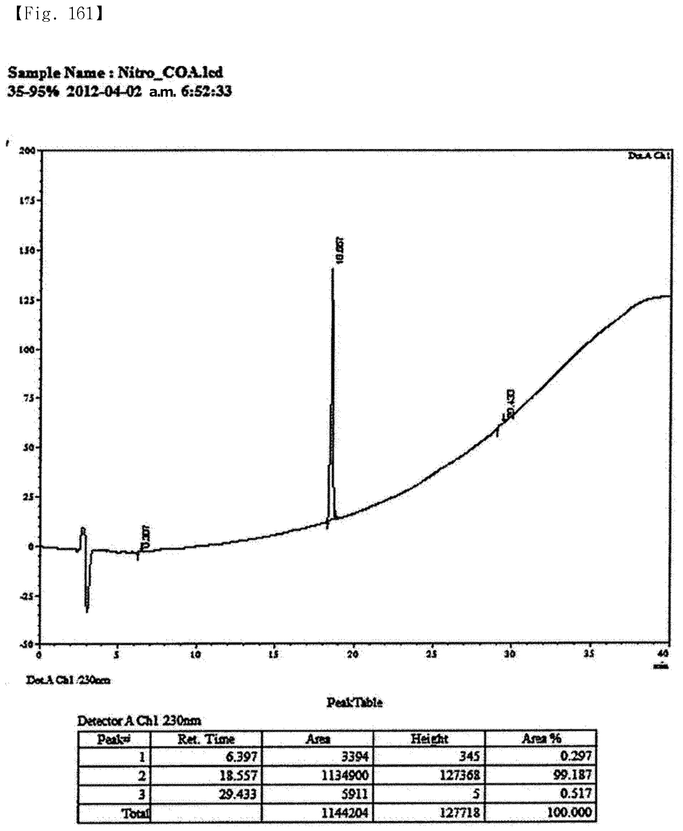

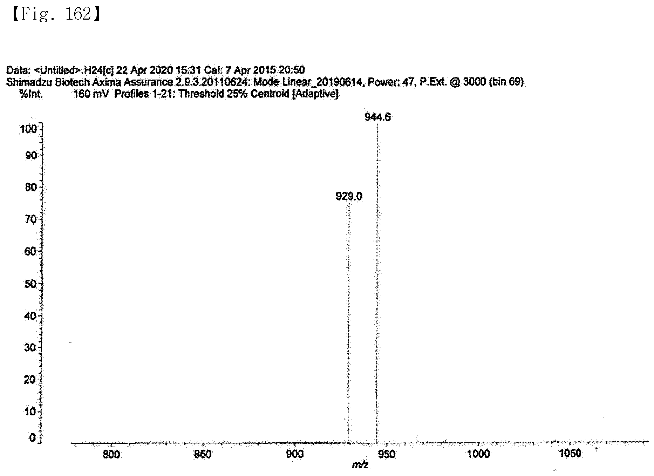

The purity and molecular weight of the HKFY-68 are shown in Figs. 161 and 162, respectively.

### Example 1.69. Preparation of HKFY-69

His(benzhydryl)-Lys(caprylic)-d-Phe(4-methyl)-Tyr(3,5-nitro) ((S)-2-((R)-2-((S)-2-((S)-2-amino-3-(1-benzhydryl-1H-imidazol-4-yl)propanamido)-6-octanamidohexanamido)-3-(p-tolyl)propanamido)-3-(4-hydroxy-3,5-dinitrophenyl)propanoic acid) was prepared in the same manner except that Fmoc-Tyr(3,5-nitro) was used instead of Fmoc-Tyr(tBu) among the constituent amino acids of Example 1.34.

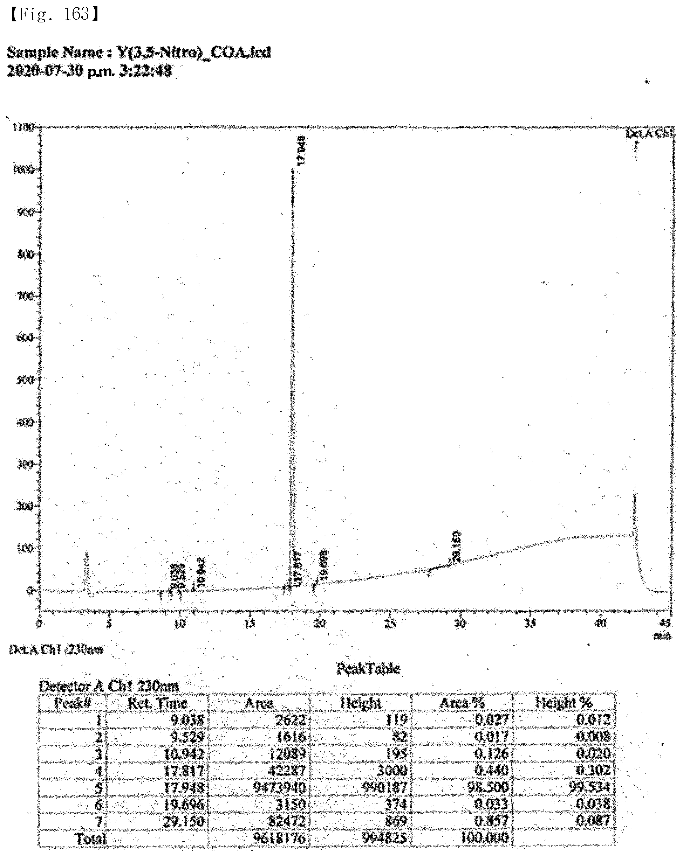

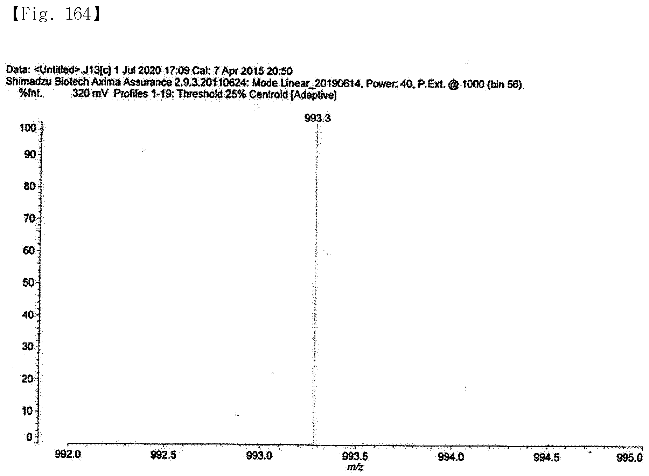

The purity and molecular weight of the HKFY-69 are shown in Figs. 163 and 164, respectively.

### Example 1.70. Preparation of HKFY-70

His(benzhydryl)-Lys(caprylic)-d-Phe(4-methyl)-Tyr(3-amino) ((S)-2-((R)-2-((S)-2-((S)-2-amino-3-(1-benzhydryl-1H-imidazol-4-yl)propanamido)-6-octanamidohexanamido)-3-(p-tolyl)propanamido)-3-(3-amino-4-hydroxyphenyl)propanoic acid) was prepared in the same manner except that Fmoc-Tyr(3-amino) was used instead of Fmoc-Tyr(tBu) among the constituent amino acids of Example 1.34.

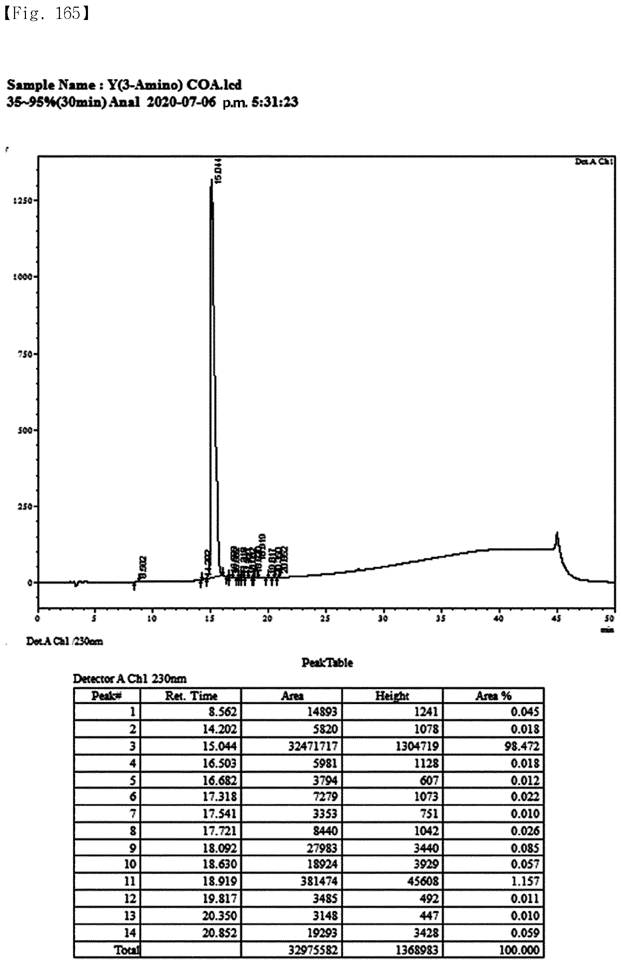

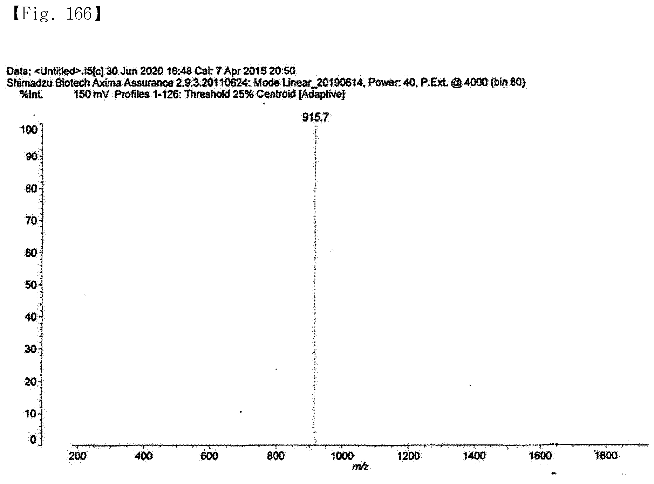

The purity and molecular weight of the HKFY-70 are shown in Figs. 165 and 166, respectively.

### Example 1.71. Preparation of HKFY-71

His(benzhydryl)-Orn(caprylic)-d-Phe(4-methyl)-Tyr (((R)-2-((S)-2-((S)-2-amino-3-(1-benzhydryl-1H-imidazol-4-yl)propanamido)-5-octanamidopentanamido)-3-(p-tolyl)propanoyl)-L-tyrosine) was prepared in the same manner except that Fmoc-Orn(dde) was used instead of Fmoc-Lys(dde) among the constituent amino acids of Example 1.34.

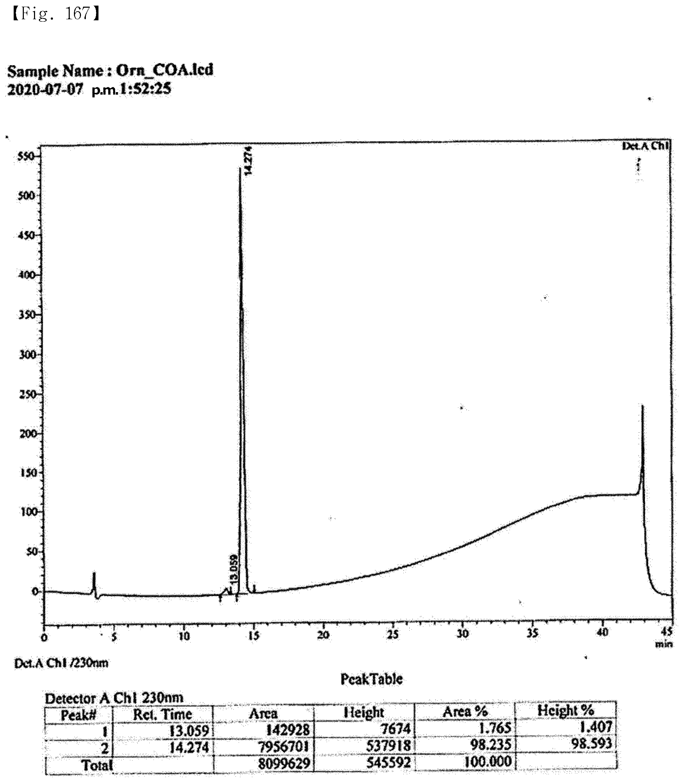

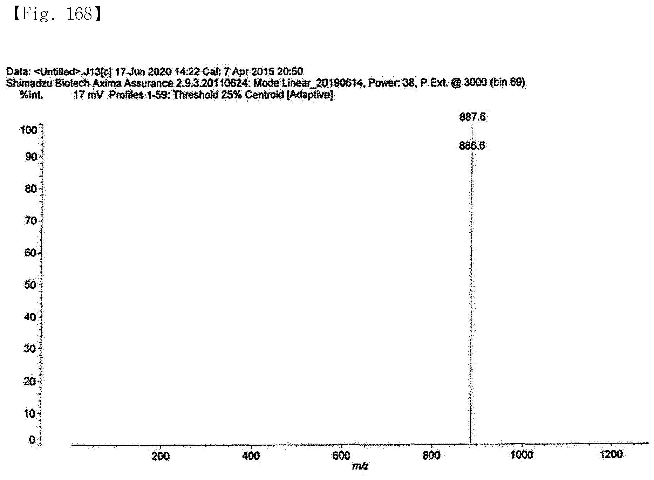

The purity and molecular weight of the HKFY-71 are shown in Figs. 167 and 168, respectively.

### Example 1.72. Preparation of HKFY-72

Biotin-His(benzhydryl)-Lys(caprylic)-d-Phe(4-methyl)-Tyr (((S)-2-((S)-2-((S)-3-(1-benzhydryl-1H-imidazol-4-yl)-2-(5-((3aS,4S,6aR)-2-oxohexahydro-1H-thieno[3,4-d1imidazol-4-yl)pentanamido)propanamido)-6-octanamidohexanamido)-3-(p-tolyl)propanoyl)-L-tyrosine) was prepared in the same manner as in the synthesis process of D-biotin of Example 1.2., using His(benzhydryl)-Lys(caprylic acid)-d-Phe(4-methyl)-Tyr-OH of Example 1.34.

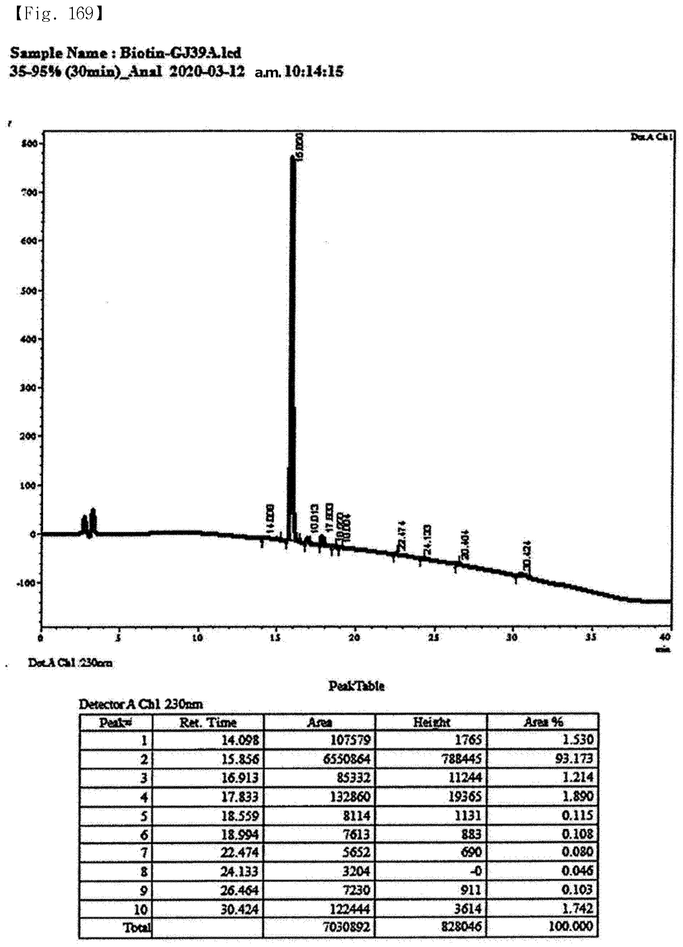

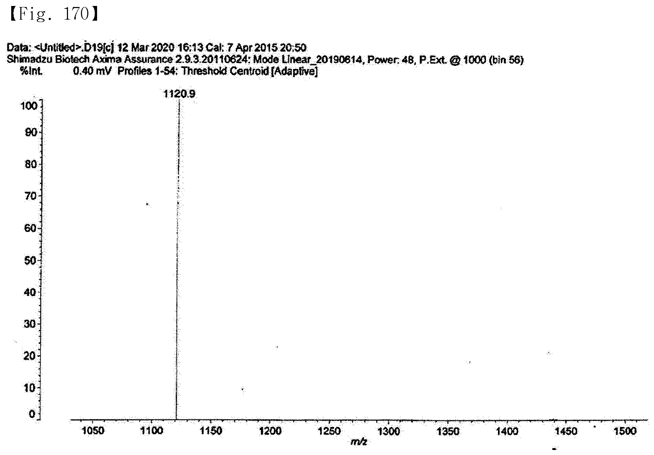

The purity and molecular weight of the HKFY-72 are shown in Figs. 169 and 170, respectively.

### Example 1.73. Preparation of HKFY-73

H(trt)Lys(caprylic)-d-3Pal-Tyr (((R)-2-((S)-2-((S)-2-amino-3-(T-trityl-1H-imidazol-4-yl)propanamido)-6-octanamidohexanamido)-3-(pyridin-3-yl)propanoyl)-L-tyrosine) was prepared in the same manner except that Fmoc-d-3Pal was used instead of Fmoc-d-Phe-OH among the constituent amino acids of Example 1.12.

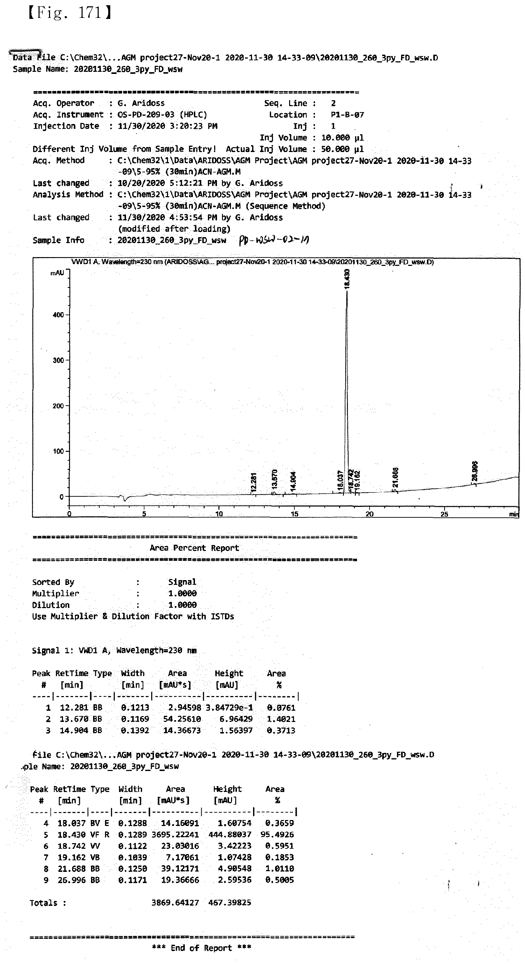

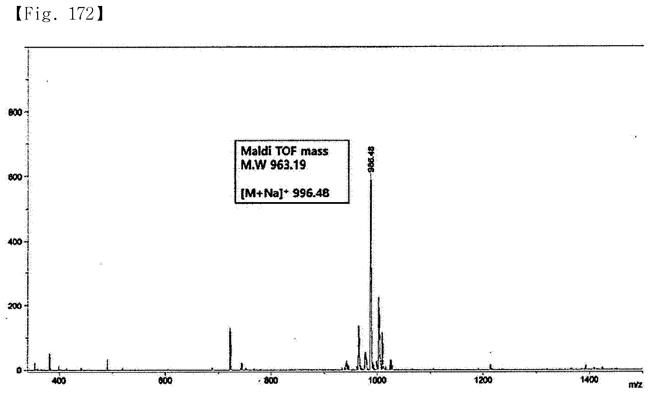

The purity and molecular weight of the HKFY-73 are shown in Figs. 171 and 172, respectively.

### Example 1.74. Preparation of HKFY-74

H(trt)Lys(caprylic)-d-2Nal-Tyr (((R)-2-((S)-2-((S)-2-amino-3-(1-trityl-1H-imidazol-4-yl)propanamido)-6-octanamidohexanamido)-3-(naphthalen-2-yl)propanoyl)-L-tyrosine) was prepared in the same manner except that Fmoc-d-2Nal was used instead of Fmoc-d-3Pal among the constituent amino acids of Example 1.73.

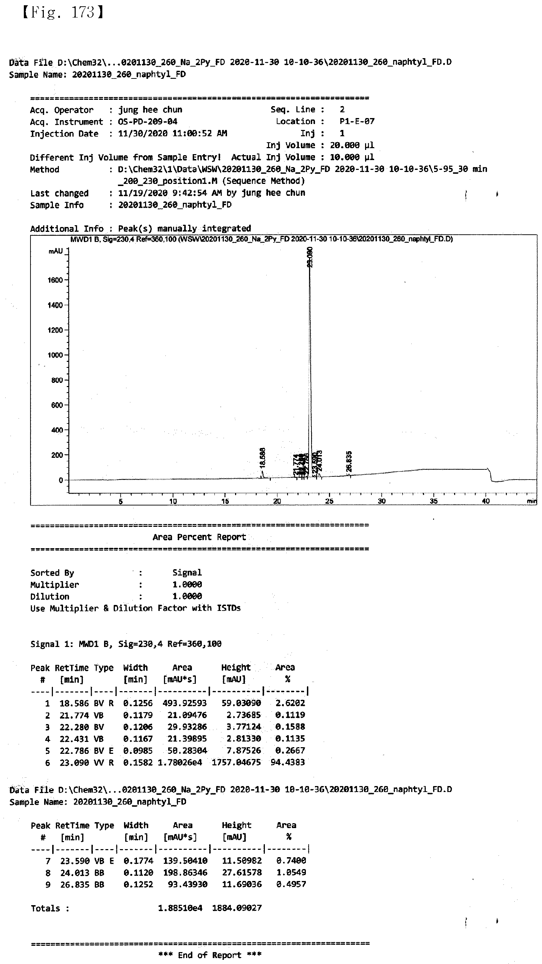

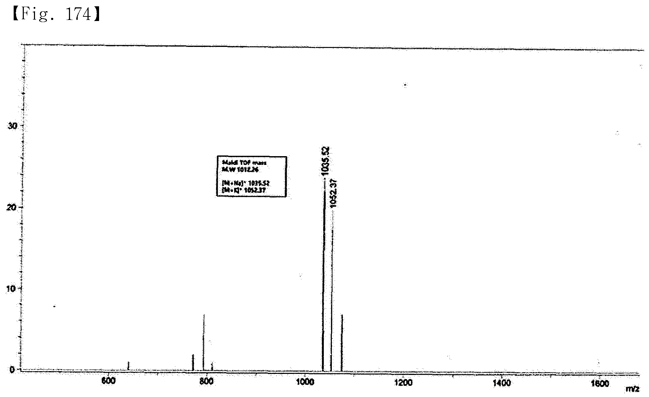

The purity and molecular weight of the HKFY-74 are shown in Figs. 173 and 174, respectively.

### Example 1.75. Preparation of HKFY-75

His(tosyl)-Lys(caprylic)-d-Phe(4-methyl)-Tyr (((R)-2-((S)-2-((S)-2-amino-3-(1-tosyl-1H-imidazol-4-yl)propanamido)-6-octanamidohexanamido)-3-(p-tolyl)propanoyl)-L-tyrosine) was prepared in the same manner except that Boc-His(tosyl)-OH was used instead of Boc-His(benzhydryl)-OH among the constituent amino acids of Example 1.34.

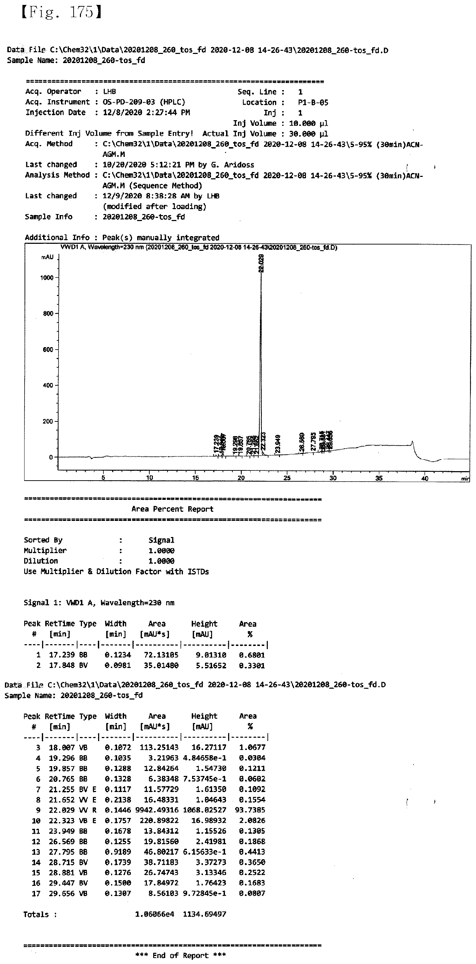

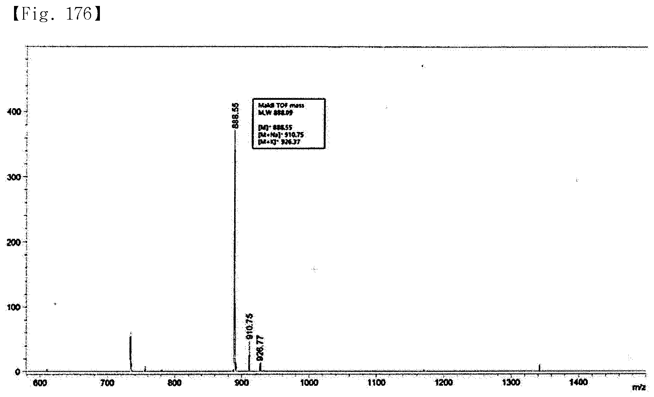

The purity and molecular weight of the HKFY-75 are shown in Figs. 175 and 176, respectively.

### Example 1.76. Preparation of HKFY-76

His(trityl)-Lys(caprylic)-d-Phe(4-CF3)-Tyr (((R)-2-((S)-2-((S)-2-amino-3-(1-trityl-1H-imidazol-4-yl)propanamido)-6-octanamidohexanamido)-3-(4-(trifluoromethyl)phenyl)propanoyl)-L-tyrosine) was prepared in the same manner except that Fmoc-d-Phe(4-CF3) was used instead of Fmoc-d-3Pal among the constituent amino acids of Example 1.73.

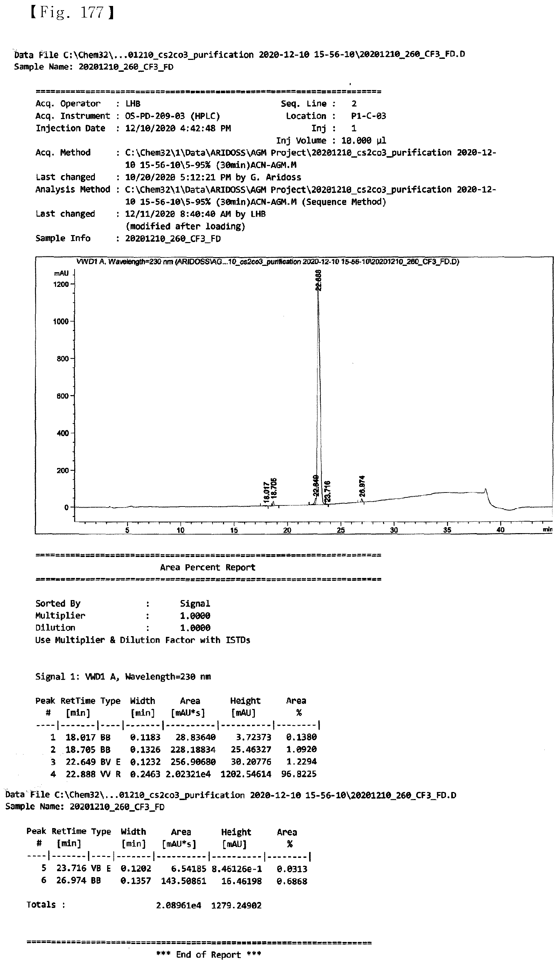

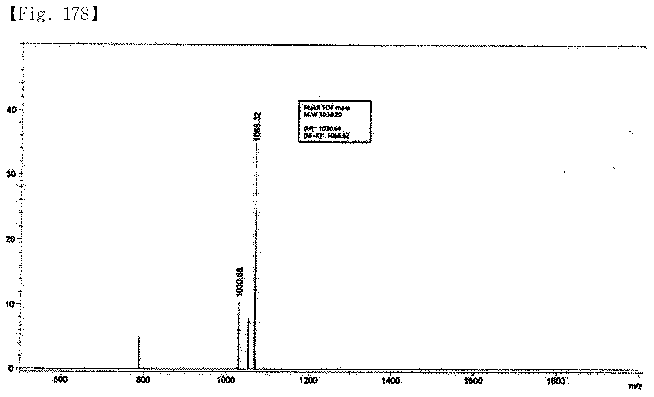

The purity and molecular weight of the HKFY-76 are shown in Figs. 177 and 178, respectively.

### Example 1.77. Preparation of HKFY-77

His(tbm)-Lys(caprylic)-d-Phe(4-CF3)-Tyr (((R)-2-((S)-2-((S)-3-(1-((2'-(1H-tetrazol-5-yl)-[1,1'-biphenyl]-4-yl)methyl)-1H-imidazol-4-yl)-2-aminopropanamido)-6-octanamidohexanamido)-3-(p-tolyl)propanoyl)-L-tyrosine) was prepared in the same manner except that Boc-His(tbm)-OH was used instead of Boc-His(benzhydryl)-OH among the constituent amino acids of Example 1.34.

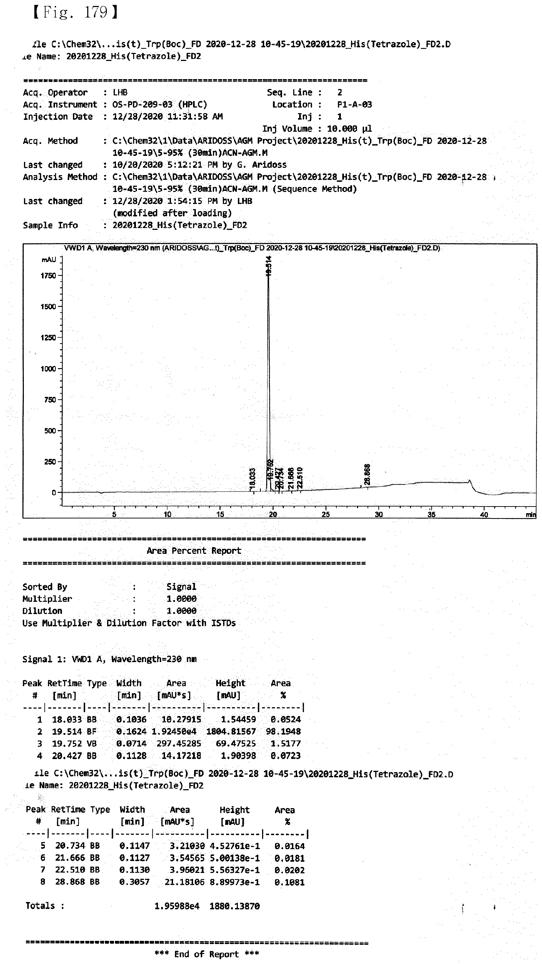

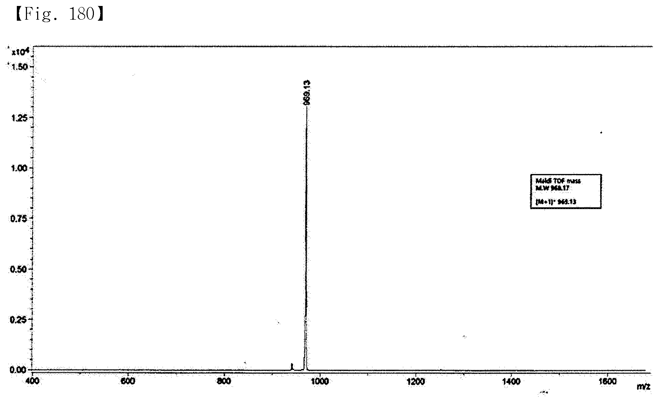

The purity and molecular weight of the HKFY-77 are shown in Figs. 179 and 180, respectively.

### Example 1.78. Preparation of HKFY-78

Boc-Trp-Lys(caprylic)-d-Phe(4-methyl)-Tyr (((R)-2-((S)-2-((S)-2-((tert-butoxycarbonyl)amino)-3-(1H-indol-3-yl)propanamido)-6-octanamidohexanamido)-3-(p-tolyl)propanoyl)-L-tyrosine) was prepared in the same manner except that Boc-Trp-OH was used instead of Boc-His(benzhydryl)-OH among the constituent amino acids of Example 1.34.

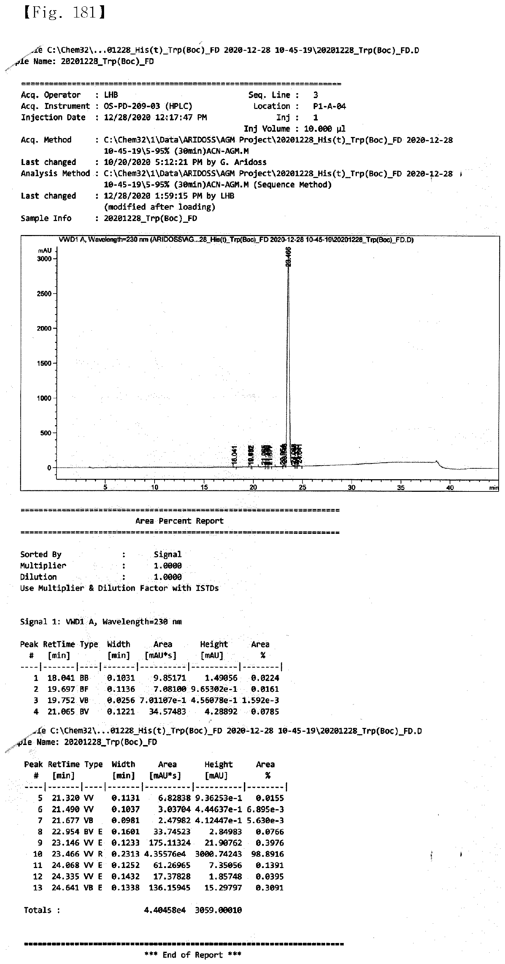

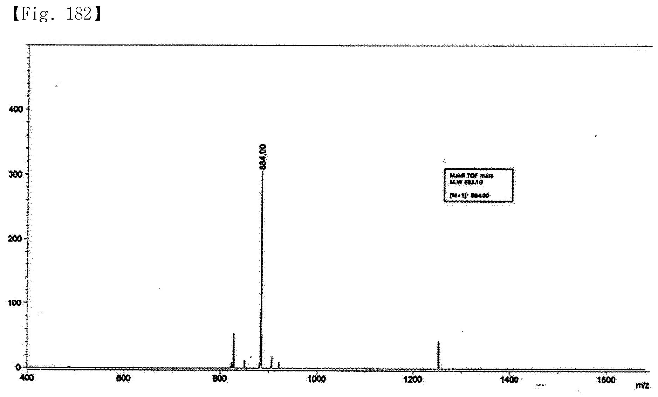

The purity and molecular weight of the HKFY-78 are shown in Figs. 181 and 182, respectively.

### Example 1.79. Preparation of HKFY-79

Fmoc-Lys(dde) and DMF (dimethylformamide) were loaded onto the trityl resin to prepare Fmoc-Lys(dde)-trityl resin. Fmoc-Lys(caprylic acid)-trityl resin was prepared in the same manner as in the synthesis process of Example 1.1. Thereafter, Boc-His(benzhdyryl)-OH was used to prepare His(benzhydryl)-Lys(caprylic) (N2-(Nt-benzhydryl-L-histidyl)-N6-octanoyl-L-lysine).

The HKFY peptide derivatives prepared by the above preparation method are shown in Table 1 below.

**[Table 1]**

| No. | Amino acid sequence | IUPAC |
|---|---|---|
| HKFY-1 | His(trt)- Lys(caprylic)-Phe-Tyr | N6-octanoyl-N2-(Nt-trityl-L-histidyl)-L-lysyl-L-phenylalanyl-L-tyrosine |
| HKFY-2 | Biotin-His(trt)- Lys(caprylic)-Phe-Tyr | N6-octanoyl-N2-(Na-(5-((3aS,4S,6aR)-2-oxohexahydro-1H-thieno[3,4-d]imidazol-4-yl)pentanoyl)-Nt-trityl-L-histidyl)-L-lysyl-L-phenylalanyl-L-tyrosine |
| HKFY-3 | His(benzyl)- Lys(caprylic)-Phe-Tyr | N2-(Nt-benzyl-L-histidyl)-N6-octanoyl-L-lysyl-L-phenylalanyl-L-tyrosine |
| HKFY-4 | d-His(trt)- Lys(caprylic)-Phe-Tyr | N6-octanoyl-N2-(Nt-trityl-D-histidyl)-L-lysyl-L-phenylalanyl-L-tyrosine |
| HKFY-5 | His(4-methyltrityl)- Lys(caprylic)-Phe-Tyr | N2-(Nt-(diphenyl(p-tolyl)methyl)-L-histidyl)-N6-octanoyl-L-lysyl-L-phenylalanyl-L-tyrosine |
| HKFY-6 | His(DAMP-3)- Lys(caprylic)-Phe-Tyr | N2-((S)-2-amino-3-(1,3-diphenethyl-2,3-dihydro-1H-imidazol-4-yl)propanoyl)-N6-octanoyl-L-lysyl-L-phenylalanyl-L-tyrosine |
| HKFY-7 | His(DAMP-5)- Lys(caprylic)-Phe-Tyr | N2-((S)-2-amino-3-(3-(2-cyclohexylethyl)-2,3-dihydro-1H-imidazol-4-yl)propanoyl)-N6-octanoyl-L-lysyl-L-phenylalanyl-L-tyrosine |
| HKFY-8 | His(DAMP-2)- Lys(caprylic)-Phe-Tyr | N2-((S)-2-amino-3-(1,3-bis(2-cyclohexylethyl)-2,3-dihydro-1H-imidazol-4-yl)propanoyl)-N6-octanoyl-L-lysyl-L-phenylalanyl-L-tyrosine |
| HKFY-9 | His(2-phenylethyl)- Lys(caprylic)-Phe-Tyr | N6-octanoyl-N2-(Nt-phenethyl-L-histidyl)-L-lysyl-L-phenylalanyl-L-tyrosine |
| HKFY-10 | His(2-naphthalen-1- methyl)- Lys(caprylic)-Phe-Tyr | N2-(Nt-(naphthalen-1-ylmethyl)-L-histidyl)-N6-octanoyl-L-lysyl-L-phenylalanyl-L-tyrosine |
| HKFY-11 | His(2- cyclohexylethyl)- Lys(caprylic)-Phe-Tyr | N2-(Nt-(2-cyclohexylethyl)-L-histidyl)-N6-octanoyl-L-lysyl-L-phenylalanyl-L-tyrosine |
| HKFY-12 | His(trt)- Lys(caprylic)-d-Phe- Tyr | N6-(non-1-en-2-yl)-N2-(Nt-trityl-L-histidyl)-L-lysyl-D-phenylalanyl-L-tyrosine |
| HKFY-13 | His(trt)- Lys(caprylic)-d- Phe(4-Cl)-Tyr | ((R)-2-((S)-2-((S)-2-amino-3-(1-trityl-1H-imidazol-4-yl)propanamido)-6-(non-1-en-2-ylamino)hexanamido)-3-(4-chlorophenyl)propanoyl)-L-tyrosine |
| HKFY-14 | d-His(trt)- Lys(caprylic)-d- Phe(4-Cl)-d-Tyr | ((R)-2-((S)-2-((R)-2-amino-3-(1-trityl-1H-imidazol-4-yl)propanamido)-6-octanamidohexanamido)-3-(4-chlorophenyl)propanoyl)-D-tyrosine |
| HKFY-15 | His(trt)- Lys(caprylic)-d- Phe(4-Cl)-d-Tyr | ((R)-2-((S)-2-((S)-2-amino-3-(1-trityl-1H-imidazol-4-yl)propanamido)-6-octanamidohexanamido)-3-(4-chlorophenyl)propanoyl)-D-tyrosine |
| HKFY-16 | His(trt)-d- Lys(caprylic)-d- Phe(4-Cl)-Tyr | ((R)-2-((R)-2-((S)-2-amino-3-(1-trityl-1H-imidazol-4-yl)propanamido)-6-octanamidohexanamido)-3-(4-chlorophenyl)propanoyl)-L-tyrosine |
| HKFY-17 | His(3,3- diphpenylethyl)- Lys(caprylic)-Phe-Tyr | N2-(Nt-(2,2-diphenylethyl)-L-histidyl)-N6-octanoyl-L-lysyl-L-phenylalanyl-L-tyrosine |
| HKFY-18 | His(Fm)- Lys(caprylic)-Phe-Tyr | N2-(Nt-(9H-fluoren-9-yl)-L-histidyl)-N6-octanoyl-L-lysyl-L-phenylalanyl-L-tyrosine |
| HKFY-19 | His(phenethyl)- Lys(caprylic)-Phe-Tyr | N6-octanoyl-N2-(Nt-phenethyl-L-histidyl)-L-lysyl-L-phenylalanyl-L-tyrosine |
| HKFY-20 | His(4- methoxylbenzhydryl) -Lys(caprylic)-Phe- Tyr | N2-(Nt-((4-methoxyphenyl)(phenyl)methyl)-L-histidyl)-N6-octanoyl-L-lysyl-L-phenylalanyl-L-tyrosine |
| HKFY-21 | His(4- chlorobenzhydryl)- Lys(caprylic)-Phe-Tyr | N2-(Nt-((R)-(4-chlorophenyl)(phenyl)methyl)-L-histidyl)-N6-octanoyl-L-lysyl-L-phenylalanyl-L-tyrosine |
| HKFY-22 | His(4- methylbenzhydryl)- Lys(caprylic)-Phe-Tyr | N6-octanoyl-N2-(Nt-((R)-phenyl(p-tolyl)methyl)-L-histidyl)-L-lysyl-L-phenylalanyl-L-tyrosine |
| HKFY-23 | His(adamatan-1-yl)- Lys(caprylic)-Phe-Tyr | N2-(Nt-(adamantan-1-yl)-L-histidyl)-N6-octanoyl-L-lysyl-L-phenylalanyl-L-tyrosine |
| HKFY-24 | His(trt)-Lys(capric)- Phe-Tyr | N6-decanoyl-N2-(Nt-trityl-L-histidyl)-L-lysyl-L-phenylalanyl-L-tyrosine |
| HKFY-25 | His(trt)-Lys(lauric)- Phe-Tyr | N6-dodecanoyl-N2-(Nt-trityl-L-histidyl)-L-lysyl-L-phenylalanyl-L-tyrosine |
| HKFY-26 | His(trt)- Lys(myristic)-Phe- Tyr | N6-tetradecanoyl-N2-(Nt-trityl-L-histidyl)-L-lysyl-L-phenylalanyl-L-tyrosine |
| HKFY-27 | His(trt)- Lys(palmitic)-Phe- Tyr | N6-palmitoyl-N2-(Nt-trityl-L-histidyl)-L-lysyl-L-phenylalanyl-L-tyrosine |
| HKFY-28 | His(trt)- Lys(caprylic)-d- Phe(4-F)-Tyr | ((R)-2-((S)-2-((S)-2-amino-3-(1-trityl-1H-imidazol-4-yl)propanamido)-6-octanamidohexanamido)-3-(4-fluorophenyl)propanoyl)-L-tyrosine |
| HKFY-29 | His(trt)- Lys(caprylic)-d- Phe(4-Br)-Tyr | ((R)-2-((S)-2-((S)-2-amino-3-(1-trityl-1H-imidazol-4-yl)propanamido)-6-octanamidohexanamido)-3-(4-bromophenyl)propanoyl)-L-tyrosine |
| HKFY-30 | His(trt)- Lys(caprylic)-d- Phe(4-methyl)-Tyr | ((R)-2-((S)-2-((S)-2-amino-3-(1-trityl-1H-imidazol-4-yl)propanamido)-6-octanamidohexanamido)-3-(p-tolyl)propanoyl)-L-tyrosine |
| HKFY-31 | d-His(trt)- Lys(caprylic)-d- Phe(4-methyl)-Tyr | ((R)-2-((S)-2-((R)-2-amino-3-(1-trityl-1H-imidazol-4-yl)propanamido)-6-octanamidohexanamido)-3-(p-tolyl)propanoyl)-L-tyrosine |
| HKFY-32 | His(1,3- difluorobenzhydryl)- Lys(caprylic)-d- Phe(4-methyl)-Tyr | ((2R)-2-((2S)-2-((2S)-2-amino-3-(1-((2,4-difluorophenyl)(phenyl)methyl)-1H-imidazol-4-yl)propanamido)-6-octanamidohexanamido)-3-(p-tolyl)propanoyl)-L-tyrosine |
| HKFY-33 | His(benzhydryl)- Lys(caprylic)-Phe-Tyr | N2-(Nt-benzhydryl-L-histidyl)-N6-octanoyl-L-lysyl-L-phenylalanyl-L-tyrosine |
| HKFY-34 | His(benzhydryl)- Lys(caprylic)-d- Phe(4-methyl)-Tyr | ((R)-2-((S)-2-((S)-2-amino-3-(1-benzhydryl-1H-imidazol-4-yl)propanamido)-6-octanamidohexanamido)-3-(p-tolyl)propanoyl)-L-tyrosine |
| HKFY-35 | His(benzhydryl)- Lys(caprylic)-d- Phe(4-methyl)- Tyr(SO3H) | (S)-2-((R)-2-((S)-2-((S)-2-amino-3-(1-benzhydryl-1H-imidazol-4-yl)propanamido)-6-octanamidohexanamido)-3-(p-tolyl)propanamido)-3-(4-(sulfooxy)phenyl)propanoic acid |
| HKFY-36 | His(benzhydryl)- Lys(caprylic)-d- Phe(4-methyl)- Tyr(2,6-di-methyl) | (S)-2-((R)-2-((S)-2-((S)-2-amino-3-(1-benzhydryl-1H-imidazol-4-yl)propanamido)-6-octanamidohexanamido)-3-(p-tolyl)propanamido)-3-(4-hydroxy-2,6-dimethylphenyl)propanoic acid |
| HKFY-37 | Gly-Ser-His(trt)- Lys(caprylic)-Phe-Tyr | N2-(Na-glycyl-L-seryl-Nt-trityl-L-histidyl)-N6-octanoyl-L-lysyl-L-phenylalanyl-L-tyrosine |
| HKFY-38 | His(trt)- Lys(caprylic)-Phe- Tyr-Ser-Asp-Gln- Gln-Ala-Arg-Phe | (3S,6S,9S,12S,15S)-15-((S)-2-amino-3-(1-trityl-1H-imidazol-4-yl)propanamido)-12-benzyl-3-(((6S,9S,12S,15S)-1,18-diamino-6-(((S)-1-amino-1-oxo-3-phenylpropan-2-yl)carbamoyl)-12-(3-amino-3-oxopropyl)-1-imino-9-methyl-8,11,14,18-tetraoxo-2,7,10,13 -tetraazaoctadecan-15-yl)carbamoyl)-9-(4-hydroxybenzyl)-6-(hydroxymethyl)-5,8,11,14,21-pentaoxo-4,7,10,13,20-pentaazaoctacosanoic acid |
| HKFY-39 | His(trt)- Lys(caprylic)-Phe- Tyr-Ser-Gln-Asn- Gly-Ala-Arg-Phe | (3S,6S,9S,12S,15S,18S)-3-((2-(((S)-1-(((S)-1-(((S)-1-amino-1-oxo-3-phenylpropan-2-yl)amino)-5-guanidino-1-oxopentan-2-yl)amino)-1-oxopropan-2-yl)amino)-2-oxoethyl)carbamoyl)-18-((S)-2-amino-3-(1-trityl-1H-imidazol-4-yl)propanamido)-6-(3-amino-3-oxopropyl)-15-benzyl-12-(4-hydroxybenzyl)-9-(hydroxymethyl)-5,8,11,14,17,24-hexaoxo-4,7,10,13,16,23-hexaazahentriacontanoic acid |
| HKFY-40 | His(trt)- Lys(caprylic)-Phe- Tyr-Ser-His-Gln-Gln- Ala-Arg-Phe | (S)-2-((2S,5S,8S,11S,14S)-2-((1H-imidazol-4-yl)methyl)-14-((S)-2-amino-3-(1-trityl-1H-imidazol-4-yl)propanamido)-11-benzyl-8-(4-hydroxybenzyl)-5-(hydroxymethyl)-4,7,10,13,20-pentaoxo-3,6,9,12,19-pentaazaheptacosanamido)-N1-((S)-5-amino-1-(((S)-1-(((S)-1-(((S)-1-amino-1-oxo-3-phenylpropan-2-yl)amino)-5-guanidino-1-oxopentan-2-yl)amino)-1-oxopropan-2-yl)amino)-1,5-dioxopentan-2-yl)pentanediamide |
| HKFY-41 | His(trt)- Lys(caprylic)-Phe- Tyr-Gln-Asn-Gly- Ala-Arg-Phe | (3S,6S,9S,12S,15S,18S)-3-((2-(((S)-1-(((S)-1-(((S)-1-amino-1-oxo-3-phenylpropan-2-yl)amino)-5-guanidino-1-oxopentan-2-yl)amino)-1-oxopropan-2-yl)amino)-2-oxoethyl)carbamoyl)-18-((S)-2-amino-3-(1-trityl-1H-imidazol-4-yl)propanamido)-6-(3-amino-3-oxopropyl)-15-benzyl-12-(4-hydroxybenzyl)-9-(hydroxymethyl)-5,8,11,14,17,24-hexaoxo-4,7,10,13,16,23-hexaazahentriacontanoic acid |
| HKFY-42 | His(trt)- Lys(caprylic)-Phe- Tyr-His-Gln-Gln-Ala- Arg-Phe | (S)-2-((2S,5S,8S,11S)-2-((1H-imidazol-4-yl)methyl)-11-((S)-2-amino-3-(1-trityl-1H-imidazol-4-yl)propanamido)-8-benzyl-5-(4-hydroxybenzyl)-4,7,10,17-tetraoxo-3,6,9,16-tetraazatetracosanamido)-N1-((S)-5-amino-1-(((S)-1-(((S)-1-(((S)-1-amino-1-oxo-3-phenylpropan-2-yl)amino)-5-guanidino-1-oxopentan-2-yl)amino)-1-oxopropan-2-yl)amino)-1,5-dioxopentan-2-yl)pentanediamide |
| HKFY-43 | His(trt)- Lys(caprylic)-Phe- Tyr-Gln-Asn-Gln- Ala-Arg-Phe | (S)-N1-((S)-1-(((S)-1-(((S)-1-amino-1-oxo-3-phenylpropan-2-yl)amino)-5-guanidino-1-oxopentan-2-yl)amino)-1-oxopropan-2-yl)-2-((2S,5S,8S,11S,14S)-2-(2-amino-2-oxoethyl)-14-((S)-2-amino-3-(1-trityl-1H-imidazol-4-yl)propanamido)-5-(3-amino-3-oxopropyl)-11-benzyl-8-(4-hydroxybenzyl)-4,7,10,13,20-pentaoxo-3,6,9,12,19-pentaazaheptacosanamido)pentanediamide |
| HKFY-44 | His(trt)- Lys(caprylic)-Phe- Tyr-Trp | N6-octanoyl-N2-(Nt-trityl-L-histidyl)-L-lysyl-L-phenylalanyl-L-tyrosyl-L-tryptophan |
| HKFY-45 | Biotin-His(trt)- Lys(caprylic)- Ala(biphenyl)-Tyr | ((S)-3-([1,1'-biphenyl]-4-yl)-2-((S)-6-octanamido-2-((S)-2-(5-((3aS,4S,6aR)-2-oxohexahydro-1H-thieno[3,4-d]imidazol-4-yl)pentanamido)-3-(1-trityl-1H-imidazol-4-yl)propanamido)hexanamido)propanoyl)-L-tyrosine |
| HKFY-46 | His(trt)- Lys(caprylic)-Phe- Trp | N6-octanoyl-N2-(Nt-trityl-L-histidyl)-L-lysyl-L-phenylalanyl-L-tryptophan |
| HKFY-47 | His(trt)- Lys(caprylic)-Tyr-Phe | N6-octanoyl-N2-(Nt-trityl-L-histidyl)-L-lysyl-L-tyrosyl-L-phenylalanine |
| HKFY-48 | His(trt)- Lys(caprylic)-Tyr-Tyr | N6-octanoyl-N2-(Nt-trityl-L-histidyl)-L-lysyl-L-tyrosyl-L-tyrosine |
| HKFY-49 | His(trt)- Lys(caprylic)-Phe | N6-octanoyl-N2-(Nt-trityl-L-histidyl)-L-lysyl-L-phenylalanine |
| HKFY-50 | His(trt)- Lys(caprylic)-Phe-Ser | N6-octanoyl-N2-(Nt-trityl-L-histidyl)-L-lysyl-L-phenylalanyl-L-serine |
| HKFY-51 | His(trt)- Lys(caprylic)-Leu- Tyr | N6-octanoyl-N2-(Nt-trityl-L-histidyl)-L-lysyl-L-leucyl-L-tyrosine |
| HKFY-52 | His(trt)-Phe-Tyr-Asp- Gln-Gln-Ala-Arg-Phe | (6S,9S,12S,15S,18S)-1-amino-6-(((S)-1-amino-1-oxo-3-phenylpropan-2-yl)carbamoyl)-18-((S)-2-((S)-2-((S)-2-amino-3-(1-trityl-1H-imidazol-4-yl)propanamido)-3-phenylpropanamido)-3-(4-hydroxyphenyl)propanamido)-12,15-bis(3-amino-3-oxopropyl)-1-imino-9-methyl-8,11,14,17-tetraoxo-2,7,10,13,16-pentaazaicosan-20-oic acid |
| HKFY-53 | His(trt)-Gly-Ser- Lys(caprylic)-Phe-Tyr | N6-octanoyl-N2-(Nt-trityl-L-histidylglycyl-L-seryl)-L-lysyl-L-phenylalanyl-L-tyrosine |
| HKFY-54 | Lys(caprylic)-Phe-Tyr | N6-octanoyl-L-lysyl-L-phenylalanyl-L-tyrosine |
| HKFY-55 | Lys(caprylic)- His(trt)-Phe-Tyr | Na-(N6-octanoyl-L-lysyl)-Nt-trityl-L-histidyl-L-phenylalanyl-L-tyrosine |
| HKFY-56 | His(trt)-Lys(caproic)- Phe-Tyr-Asp-Gln- Gln-Ala-Arg-Phe | (6S,9S,12S,15S,18S)-1-amino-6-(((S)-1-amino-1-oxo-3-phenylpropan-2-yl)carbamoyl)-18-((S)-2-((S)-2-((S)-2-((S)-2-amino-3-(1-trityl-1H-imidazol-4-yl)propanamido)-6-(non-1-en-2-ylamino)hexanamido)-3-phenylpropanamido)-3-(4-hydroxyphenyl)propanamido)-12,15-bis(3-amino-3-oxopropyl)-1-imino-9-methyl-8,11,14,17-tetraoxo-2,7,10,13,16-pentaazaicosan-20-oic acid |
| HKFY-57 | His(2-phenyl)- Lys(caprylic)-d- Phe(4-methyl)-Tyr | ((R)-2-((S)-2-((S)-2-amino-3-(2-phenyl-1H-imidazol-4-yl)propanamido)-6-octanamidohexanamido)-3-(p-tolyl)propanoyl)-L-tyrosine |
| HKFY-58 | His(1-phenyl)- Lys(caprylic)-d- Phe(4-methyl)-Tyr | ((R)-2-((S)-2-((S)-2-amino-3-(1-phenyl-1H-imidazol-4-yl)propanamido)-6-octanamidohexanamido)-3-(p-tolyl)propanoyl)-L-tyrosine |
| HKFY-59 | His(1,2-diphenyl)- Lys(caprylic)-d- Phe(4-methyl)-Tyr | ((R)-2-((S)-2-((S)-2-amino-3-(1 ,2-diphenyl-1H-imidazol-4-yl)propanamido)-6-octanamidohexanamido)-3-(p-tolyl)propanoyl)-L-tyrosine |
| HKFY-60 | His(2-(4-tert- butyl)phenyl)- Lys(caprylic)-d- Phe(4-methyl)-Tyr | ((R)-2-((S)-2-((S)-2-amino-3-(2-(4-(tert-butyl)phenyl)-1H-imidazol-4-yl)propanamido)-6-octanamidohexanamido)-3-(p-tolyl)propanoyl)-L-tyrosine |
| HKFY-61 | His(1-(4-tert- butyl)phenyl)- Lys(caprylic)-d- Phe(4-methyl)-Tyr | ((R)-2-((S)-2-((S)-2-amino-3-(1-(4-(tert-butyl)phenyl)-1H-imidazol-4-yl)propanamido)-6-octanamidohexanamido)-3-(p-tolyl)propanoyl)-L-tyrosine |
| HKFY-62 | d-His(benzhydryl)- Lys(caprylic)-d- Phe(4-methyl)-Tyr | ((R)-2-((S)-2-((R)-2-amino-3-(1-benzhydryl-1H-imidazol-4-yl)propanamido)-6-octanamidohexanamido)-3-(p-tolyl)propanoyl)-L-tyrosine |
| HKFY-63 | His(thiophene)- Lys(caprylic)-d- Phe(4-methyl)-Tyr | ((R)-2-((S)-2-((S)-2-amino-3-(2-(thiophen-2-yl)-1H-imidazol-4-yl)propanamido)-6-octanamidohexanamido)-3-(p-tolyl)propanoyl)-L-tyrosine |
| HKFY-64 | His(4- methoxybenzhydryl)- Lys(caprylic)-d- Phe(4-methyl)-Tyr | ((2R)-2-((2S)-2-((2S)-2-amino-3-(1-((4-methoxyphenyl)(phenyl)methyl)-1H-imidazol-4-yl)propanamido)-6-octanamidohexanamido)-3-(p-tolyl)propanoyl)-L-tyrosine |
| HKFY-65 | His(4- hydroxybenzhydryl)- Lys(caprylic)-d- Phe(4-methyl)-Tyr | ((2R)-2-((2S)-2-((2S)-2-amino-3-(1-((4-hydroxyphenyl)(phenyl)methyl)-1H-imidazol-4-yl)propanamido)-6-octanamidohexanamido)-3-(p-tolyl)propanoyl)-L-tyrosine |
| HKFY-66 | His(benzhydryl)- Lys(caprylic)-d- Phe(4-methyl)-Tyr- NH2 | N-((S)-5-((S)-2-amino-3-(1-benzhydryl-1H-imidazol-4-yl)propanamido)-6-(((R)-1-(((S)-1-amino-3-(4-hydroxyphenyl)-1-oxopropan-2-yl)amino)-1-oxo-3-(p-tolyl)propan-2-yl)amino)-6-oxohexyl)octanamide |
| HKFY-67 | His(benzhydryl)- Lys(caprylic)-d- Phe(4-methyl)-Tyr(3- chloro) | (S)-2-((R)-2-((S)-2-((S)-2-amino-3-(1-benzhydryl-1H-imidazol-4-yl)propanamido)-6-octanamidohexanamido)-3-(p-tolyl)propanamido)-3-(3-chloro-4-hydroxyphenyl)propanoic acid |
| HKFY-68 | His(benzhydryl)- Lys(caprylic)-d- Phe(4-methyl)-Tyr(3- nitro) | (S)-2-((R)-2-((S)-2-((S)-2-amino-3-(1-benzhydryl-1H-imidazol-4-yl)propanamido)-6-octanamidohexanamido)-3-(p-tolyl)propanamido)-3-(4-hydroxy-3-nitrophenyl)propanoic acid |
| HKFY-69 | His(benzhydryl)- Lys(caprylic)-d- Phe(4-methyl)- Tyr(3,5-nitro) | (S)-2-((R)-2-((S)-2-((S)-2-amino-3-(1-benzhydryl-1H-imidazol-4-yl)propanamido)-6-octanamidohexanamido)-3-(p-tolyl)propanamido)-3-(4-hydroxy-3,5- dinitrophenyl)propanoic acid |
| HKFY-70 | His(benzhydryl)- Lys(caprylic)-d- Phe(4-methyl)-Tyr(3- amino) | (S)-2-((R)-2-((S)-2-((S)-2-amino-3-(1-benzhydryl-1H-imidazol-4-yl)propanamido)-6-octanamidohexanamido)-3-(p-tolyl)propanamido)-3-(3-amino-4-hydroxyphenyl)propanoic acid |
| HKFY-71 | His(benzhydryl)- Orn(caprylic)-d- Phe(4-methyl)-Tyr | ((R)-2-((S)-2-((S)-2-amino-3-(1-benzhydryl-1H-imidazol-4-yl)propanamido)-5-octanamidopentanamido)-3-(p-tolyl)propanoyl)-L-tyrosine |
| HKFY-72 | Biotin- His(benzhydryl)- Lys(caprylic)-d- Phe(4-methyl)-Tyr | ((S)-2-((S)-2-((S)-3-(1-benzhydryl-1H-imidazol-4-yl)-2-(5-((3aS,4S,6aR)-2-oxohexahydro-1H-thieno[3,4-d]imidazol-4-yl)pentanamido)propanamido)-6-octanamidohexanamido)-3-(p-tolyl)propanoyl)-L-tyrosine |
| HKFY-73 | His(trt)- Lys(caprylic)-d-3Pal- Tyr | ((R)-2-((S)-2-((S)-2-amino-3-(1-trityl-1H-imidazol-4-yl)propanamido)-6-octanamidohexanamido)-3-(pyridin-3-yl)propanoyl)-L-tyrosine |
| HKFY-74 | His(trt)- Lys(caprylic)-d-2Nal- Tyr | ((R)-2-((S)-2-((S)-2-amino-3-(1-trityl-1H-imidazol-4-yl)propanamido)-6-octanamidohexanamido)-3-(naphthalen-2-yl)propanoyl)-L-tyrosine |
| HKFY-75 | His(tosyl)- Lys(caprylic)-d- Phe(4-methyl)-Tyr | ((R)-2-((S)-2-((S)-2-amino-3-(1-tosyl-1H-imidazol-4-yl)propanamido)-6-octanamidohexanamido)-3-(p-tolyl)propanoyl)-L-tyrosine |
| HKFY-76 | His(trt)- Lys(caprylic)-d- Phe(4-CF3)-Tyr | ((R)-2-((S)-2-((S)-2-amino-3-(1-trityl-1H-imidazol-4-yl)propanamido)-6-octanamidohexanamido)-3-(4-(trifluoromethyl)phenyl)propanoyl)-L-tyrosine |
| HKFY-77 | His(tbm)- Lys(caprylic)-d- Phe(4-CF3)-Tyr | ((R)-2-((S)-2-((S)-3-(1-((2'-(1H-tetrazol-5-yl)-[1,1'-biphenyl]-4-yl)methyl)-1H-imidazol-4-yl)-2-aminopropanamido)-6-octanamidohexanamido)-3-(p-tolyl)propanoyl)-L-tyrosine |
| HKFY-78 | Boc-Trp- Lys(caprylic)-d- Phe(4-methyl)-Tyr | ((R)-2-((S)-2-((S)-2-((tert-butoxycarbonyl)amino)-3-(1H-indol-3-yl)propanamido)-6-octanamidohexanamido)-3-(p-tolyl)propanoyl)-L-tyrosine |
| HKFY-79 | His(benzhydryl)Lys(c aprylic) | N2-(Nt-benzhydryl-L-histidyl)-N6-octanoyl-L-lysine |

### Experimental Example 1. Analysis of HKFY derivatives

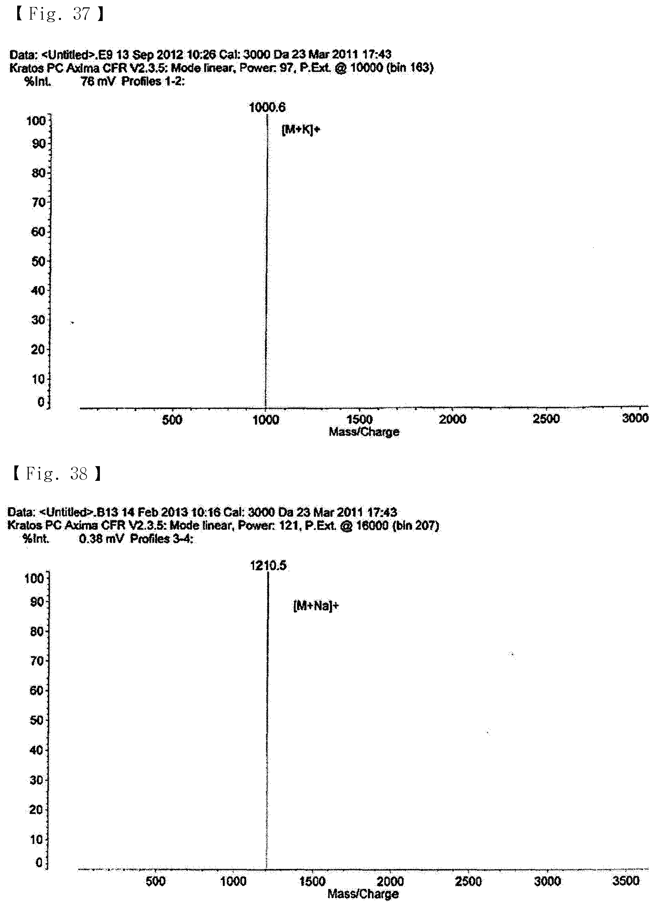

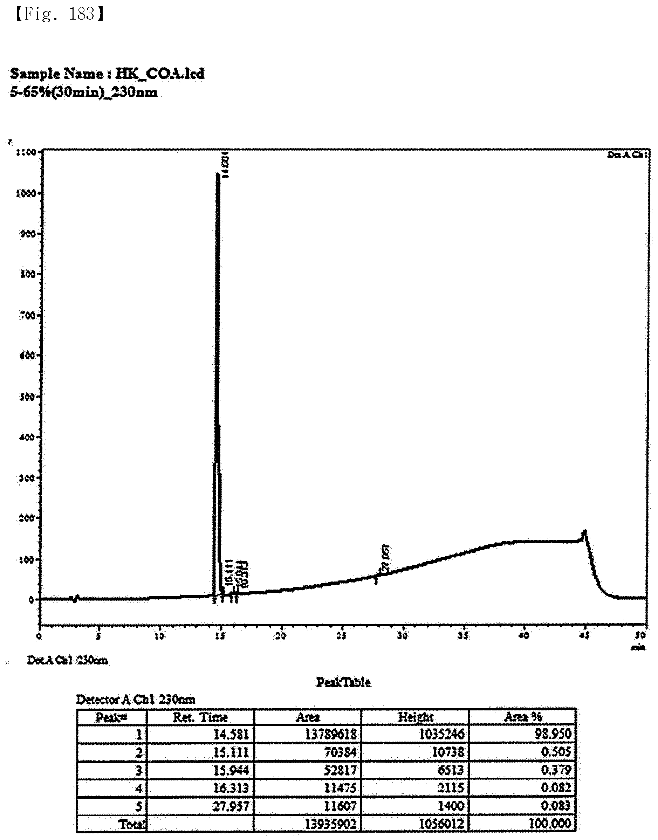

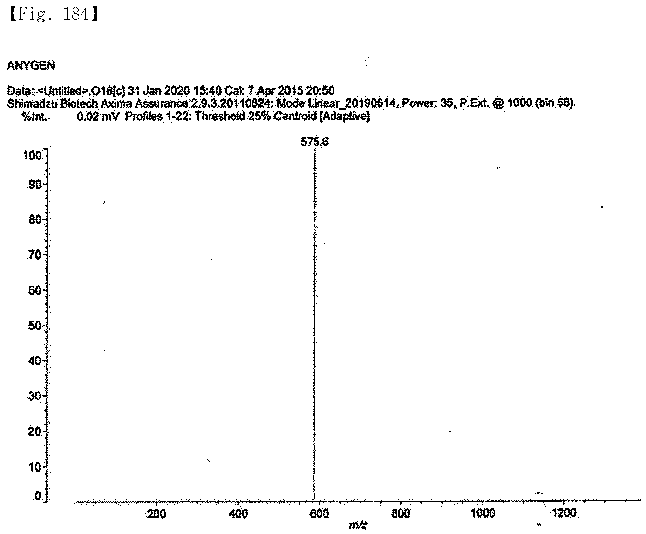

The HKFY derivatives prepared in Examples 1.1. to 1.79. were analyzed for purity and molecular weight using HPLC and Ion-Mass. The results are shown in Table 2 below, and the HPLC analysis results of the HKFY derivatives are shown in Figs. 1 to 36, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179, 181 and 183, and the Ion-Mass analysis results of the HKFY derivatives are shown in Figs. 37 to 72, 100, 102, 104, 106, 108, 110, 112, 114, 117, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178, 180, 182 and 184.

**[Table 2]**

| No. | Purity (%) | Molecular weight |
|---|---|---|
| HKFY-1 | 96.1 | 1000.6 |
| HKFY-2 | 94.8 | 1210.5 |
| HKFY-3 | 93.5 | 810.1 |
| HKFY-4 | 92.0 | 1000.5 |
| HKFY-5 | 86.4 | 976.2 |
| HKFY-6 | 95.2 | 956.6 |
| HKFY-7 | 99.2 | 844.9 |
| HKFY-8 | 93.4 | 969.1 |
| HKFY-9 | 98.7 | 823.3 |
| HKFY-10 | 98.5 | 873.1 |
| HKFY-11 | 97.5 | 829.1 |
| HKFY-12 | 96.3 | 1001.1 |
| HKFY-13 | 96.9 | 1018.9 |
| HKFY-14 | 96.6 | 1034.1 |
| HKFY-15 | 98.3 | 1034.0 |
| HKFY-16 | 95.3 | 1035.3 |
| HKFY-17 | 98.3 | 914.5 |
| HKFY-18 | 98.6 | 898.2 |
| HKFY-19 | 98.7 | 836.4 |
| HKFY-20 | 94.6 | 709.9 |
| HKFY-21 | 98.3 | 709.5 |
| HKFY-22 | 94.2 | 709.6 |
| HKFY-23 | 69.7 | 709.8 |
| HKFY-24 | 93.4 | 989.6 |
| HKFY-25 | 95.2 | 1019.1 |
| HKFY-26 | 89.1 | 1045.5 |
| HKFY-27 | 98.1 | 1073.6 |
| HKFY-28 | 96.5 | 980.6 |
| HKFY-29 | 96.2 | 1043.1 |
| HKFY-30 | 97.0 | 977.1 |
| HKFY-31 | 92.9 | 976.7 |
| HKFY-32 | 90.4 | 935.48 |
| HKFY-33 | 98.1 | 884.9 |
| HKFY-34 | 93.6 | 899.3 |
| HKFY-35 | 93.9 | 979.4 |
| HKFY-36 | 92.6 | 928.6 |
| HKFY-37 | 96.9 | 1128.1 |
| HKFY-38 | 95.6 | 1793.9 |
| HKFY-39 | 95.4 | 1721.4 |
| HKFY-40 | 96.1 | 1815.1 |
| HKFY-41 | 91.8 | 1634.7 |
| HKFY-42 | 87.0 | 1728.9 |
| HKFY-43 | 88.5 | 1705.6 |
| HKFY-44 | 94.2 | 1171.3 |
| HKFY-45 | 93.7 | 1287.0 |
| HKFY-46 | 96.7 | 986.1 |
| HKFY-47 | 97.4 | 962.8 |
| HKFY-48 | 98.0 | 978.9 |
| HKFY-49 | 93.3 | 799.1 |
| HKFY-50 | 88.9 | 886.6 |
| HKFY-51 | 95.1 | 928.2 |
| HKFY-52 | 96.6 | 1452.0 |
| HKFY-53 | 95.9 | 1105.2 |
| HKFY-54 | 98.2 | 583.3 |
| HKFY-55 | 99.0 | 962.7 |
| HKFY-56 | 95.6 | 1793.9 |
| HKFY-57 | 97.6 | 809.12 |
| HKFY-58 | 90.3 | 809.12 |
| HKFY-59 | 98.5 | 885.22 |
| HKFY-60 | 95.6 | 865.23 |
| HKFY-61 | 96.1 | 865.23 |
| HKFY-62 | 97.1 | 899.43 |
| HKFY-63 | 96.6 | 813.42 |
| HKFY-64 | 96.4 | 929.51 |
| HKFY-65 | 96.5 | 915.49 |
| HKFY-66 | 95.2 | 898 |
| HKFY-67 | 99.2 | 932.9 |
| HKFY-68 | 99.2 | 944 |
| HKFY-69 | 99.5 | 990 |
| HKFY-70 | 98.5 | 914.51 |
| HKFY-71 | 98.6 | 885.48 |
| HKFY-72 | 93.2 | 1126.21 |
| HKFY-73 | 95.5 | 963.19 |
| HKFY-74 | 94.4 | 1012.36 |
| HKFY-75 | 93.7 | 888.09 |
| HKFY-76 | 96.8 | 1030.2 |
| HKFY-77 | 98.2 | 968.17 |
| HKFY-78 | 98.9 | 883.1 |
| HKFY-79 | 95.2 | 575.35 |

### Experimental Example 2. Confirmation of binding capacity of HKFY derivatives to GPR39 receptor

In order to investigate the binding capacity of the HKFY derivatives prepared in Examples 1.1. to 1.79. above to GPR39, a luciferase assay system was used. The luciferase assay system is a method for confirming the degree of activity of a receptor in a cell through the binding of a ligand to the receptor in the cell.

Specifically, the fibroblast cell line CV-1 (1×10⁵ cells/mL, Korea Cell Line Bank) was cultured in a 96 well cell culture plate for 24 hours, and then treated with GPR39 and SRE (serum response element) luciferase inserted into adenovirus at 50 MOI (multiplicity of infection) and 25 MOI virus for 3 hours, respectively, and then cultured for 24 hours. In order to give a fasting process, after 24 hours, the medium was replaced with a serum-free medium and cultured for 16 hours. Thereafter, it was treated with each HKFY and HKFY derivative for 6 hours and cultured, and then the expression level of luciferase, a reporter gene, by activated GPR39 was quantified using a luminometer (Table 3).

**[Table 3]**

| No. | EC₅₀ (µM) | ECmax (-fold induction over basal) |
|---|---|---|
| HKFY-1 | 0.15 ± 0.01 | 20.51 ± 0.57 |
| HKFY-2 | 0.14 ± 0.07 | 15 ± 1.87 |
| HKFY-3 | 4.13 ± 0.15 | 9.2 ±2.58 |
| HKFY-4 | 0.13 ± 0.07 | 16.8 ± 1.87 |
| HKFY-5 | 1.84 ± 0.2 | 16 ± 1.7 |
| HKFY-6 | 68.4 ± 1.17 | 15 ± 2.12 |
| HKFY-7 | 1.56 ± 0.27 | 16 ±2.2 |
| HKFY-8 | 33.65 ±1.3 | 17.3 ± 1.22 |
| HKFY-9 | 42.7 ± 1.2 | 21 ± 2.52 |
| HKFY-10 | 46.5 ± 1.1 | 14 ± 2.12 |
| HKFY-11 | 57.8 ± 1.5 | 22 ± 2.2 |
| HKFY-12 | 0.035 ± 0.002 | 20.7 ± 2.3 |
| HKFY-13 | 0.014 ± 0.002 | 25.7 ± 2.4 |
| HKFY-14 | 0.02 ± 0.003 | 20.9 ± 1.4 |
| HKFY-15 | 0.098 ± 0.005 | 24.7 ± 2.6 |
| HKFY-16 | 0.24 ± 0.02 | 27.9 ± 3.4 |
| HKFY-17 | 8.12 ± 0.07 | 20 ± 0.57 |
| HKFY-18 | 2.29 ± 0.15 | 10 ± 1.48 |
| HKFY-19 | 24.6 ± 2.6 | 18 ± 1.7 |
| HKFY-20 | 159 ± 2.3 | 20.26 ± 2.7 |
| HKFY-21 | 136 ± 1.27 | 18.3 ± 2.5 |
| HKFY-22 | 59.8 ± 2.1 | 21.3 ± 1.8 |
| HKFY-23 | 2.6 ± 0.17 | 15 ± 1.37 |
| HKFY-24 | 0.24 ± 0.07 | 7.03 ± 0.57 |
| HKFY-25 | 0.63 ± 0.07 | 7.72 ± 0.57 |
| HKFY-26 | 0.28 ± 0.02 | 19.82 ± 0.57 |
| HKFY-27 | 0.56 ± 0.06 | 20.4 ± 0.57 |
| HKFY-28 | 0.074 ± 0.004 | 22.95 ± 2.58 |
| HKFY-29 | 0.31 ± 0.003 | 26.8 ± 1.87 |
| HKFY-30 | 0.008 ± 0.001 | 23.88 ± 3.04 |
| HKFY-31 | 0.02 ± 0.003 | 20.9 ± 2.51 |
| HKFY-32 | 0.002 ± 0.001 | 21.5 ± 2.7 |
| HKFY-33 | 0.15 ± 0.07 | 20 ± 0.57 |
| HKFY-34 | 0.007 ± 0.001 | 22.39 ± 4.59 |
| HKFY-35 | 0.28 ± 0.07 | 18.9 ± 0.41 |
| HKFY-36 | 0.15 ± 0.05 | 9.9 ± 0.28 |
| HKFY-37 | 1.84 ± 0.2 | 16 ± 1.7 |
| HKFY-38 | 3.5 ± 0.02 | 16.5 ± 0.02 |
| HKFY-39 | 0.78 ± 0.02 | 12.1 ± 0.02 |
| HKFY-40 | 1.3 ± 0.02 | 13.4 ± 0.02 |
| HKFY-41 | 9.55 ± 0.02 | 12.2 ± 0.02 |
| HKFY-42 | 1.58 ± 0.02 | 11.8 ± 0.02 |
| HKFY-43 | 1.21 ± 0.02 | 12.1 ± 0.02 |
| HKFY-44 | 0.37 ± 0.07 | 20 ± 0.47 |
| HKFY-45 | 0.14 ± 0.05 | 21 ± 2.58 |
| HKFY-46 | 0.26 ± 0.15 | 20 ± 0.28 |
| HKFY-47 | 0.3 ± 0.02 | 13 ± 1.7 |
| HKFY-48 | 0.33 ± 0.02 | 14.4 ± 2.7 |
| HKFY-49 | 0.32 ± 0.02 | 18.3 ± 1.8 |
| HKFY-50 | 0.7 ± 0.05 | 16.2 ± 1.5 |
| HKFY-51 | 0.12 ± 0.02 | 14.4 ± 1.9 |
| HKFY-52 | 56.4 ± 0.02 | 15.5 ± 2.2 |
| HKFY-53 | 0.34 ± 0.07 | 8 ± 2.12 |
| HKFY-54 | 25.9 ± 0.27 | 7.8 ± 2.5 |
| HKFY-55 | 2.49 ± 0.12 | 16.8 ± 2.5 |
| HKFY-56 | 0.03 ± 0.002 | 14.9 ± 1.8 |
| HKFY-57 | 0.26 ± 0.01 | 8.5 ± 2.3 |
| HKFY-58 | 0.02 ± 0.002 | 9.12 ± 2.1 |
| HKFY-59 | 0.55 ± 0.02 | 6.76 ± 2.2 |
| HKFY-60 | 0.19 ± 0.01 | 28.2 ± 2.5 |
| HKFY-61 | 0.02 ± 0.003 | 9.88 ± 1.8 |
| HKFY-62 | 0.83 ± 0.03 | 28.1 ± 2.1 |
| HKFY-63 | 0.006 ± 0.001 | 13.5 ± 1.8 |
| HKFY-64 | 0.007 ± 0.001 | 16 ± 1.5 |
| HKFY-65 | 0.003 ± 0.002 | 18.3 ± 2.2 |
| HKFY-66 | 0.011 ± 0.001 | 20.2 ± 3.5 |
| HKFY-67 | 0.043 ± 0.003 | 10.2 ± 1.51 |
| HKFY-68 | 0.013 ± 0.001 | 10.8 ± 1.41 |
| HKFY-69 | 0.015 ± 0.002 | 12.2 ± 2.5 |
| HKFY-70 | 0.02 ± 0.001 | 10.1 ± 1.6 |
| HKFY-71 | 0.012 ± 0.003 | 16.4 ± 2.51 |
| HKFY-72 | 0.011 ± 0.002 | 10.4 ± 1.12 |
| HKFY-73 | 0.023 ± 0.001 | 11.6 ± 2.3 |
| HKFY-74 | 0.008 ± 0.001 | 18.8 ± 3.2 |
| HKFY-75 | 0.001 ± 0.002 | 15.9 ± 4.12 |
| HKFY-76 | 0.007 ± 0.001 | 14.2 ± 3.75 |
| HKFY-77 | 0.007 ± 0.002 | 11.6 ± 2.14 |
| HKFY-78 | 0.009 ± 0.001 | 11.4 ± 1.54 |
| HKFY-79 | 0.97 ± 0.03 | 11.2 ± 1.4 |

As a result, referring to Table 3, it was confirmed that all HKFY derivatives had high activity at nM to µM level for GPR39. In addition, among the analogs in which various fatty acids were conjugated to Lys, the analog conjugated to caprylic acid showed the most excellent activity. Derivatives exhibiting an activity at nM level are considered to have highly selective and specific activity for GPR39.

On the other hand, HKFY-30, HKFY-34, HKFY-63, HKFY-64, and HKFY-65 derivatives exhibited a higher activity level than other HKFY derivatives. In particular, HKFY-30 and HKFY-34 composed of d-Phe(methyl) exhibited the most excellent activity and high ECmax value.

### Experimental Example 3. Confirmation of selectivity and specificity of HKFY derivatives for GPR39

In order to confirm the selectivity and specificity of the HKFY derivatives for GPR39, the degree of activity was confirmed by a luciferase assay system and a calcium influx assay system (Fig. 73 and Table 4).

Specifically, the luciferase assay system analyzed the degree of activity using HKFY-34 among the HKFY derivatives in Experimental Example 2 above (top in Fig. 73). In the calcium influx assay system, the fibroblast cell line CV-1 (1×10⁵ cells/mL) was transfected with a mixture of human GPR39 (pCDNA3.1_hGPR39, American Type Culture Collection), Gαq (pCDNA3.1_Gαq, American Type Culture Collection), Aequrin (pCDNA3.1_aequrin, American Type Culture Collection) and lipofectamine (American Type Culture Collection). Thereafter, the medium was replaced with a serum-free medium, and after 16 hours of fasting, treated with HKFY-34 for 6 hours and cultured. After culturing, the degree of binding of calcium activated by activated GPR39 to aequrin was quantified using a luminometer (bottom in Fig. 73).

**[Table 4]**

| Item | Reporter gene assay | | Ca²⁺ influx assay | |
|---|---|---|---|---|
| | EC₅₀ (nM) | ECmax (-fold induction over basal) | EC₅₀ (nM) | ECmax (-fold induction over basal) |
| HKFY-34 | 6.7 ± 0.7 | 22.39 ± 4.59 | 2.7 ± 0.5 | 2.4 ± 4.59 |
| HKFY | N.D | - | - | - |

As a result, referring to Fig. 73 and Table 4, it was confirmed that when treated with HKFY-34, the binding capacity and activity to the GPR39 receptor were excellent.

In addition, through the activity test of the HKFY derivatives for similar receptors to GPR39, ghrelin receptor family (ghrelin receptor (GHSR), motilin receptor (MTLR), neurotensin receptor (NTSR1 & NTSR2), neuromedin U receptor (NMU1 & NMU2), TRH (thyrotropin releasing hormone) receptor (TRHR)), the selectivity and specificity for the GPR39 receptor were confirmed.

Specifically, the degree of activity between the intrinsic ligand and HKFY-34 for each receptor was analyzed using a luciferase assay system (Fig. 74).

As a result, referring to Fig. 74, it was confirmed that the activity of HKFY-34 for GHSR, MTLR, NTSR1 & NTSR2, NMU1 & NMU2 and TRHR was very low.

These results suggest that the HKFY derivative can be used as an agonist that selectively and specifically binds to the GPR39 receptor.

### Experimental Example 4. Evaluation of promoting activity of HKFY-34 on intestinal tissue cell growth

It is known from studies using GPR39 deficient mice that GPR39 is involved in the growth and differentiation of intestinal tissue cells (Phil. Trans. R. Soc. B., 2016, 371:20150420). Therefore, in order to confirm the effect of HKFY-34 on the growth of intestinal tissue cells, the degree of cell proliferation by HKFY-34 was measured using HT-29 cells (Korea Cell Line Bank), which are intestinal epithelial cells.

Specifically, HT-29 cells were cultured in a 96 well plate and then treated with HKFY-34 at different concentrations for 24, 48, and 72 hours. The cell proliferation of the cultured cells was measured using a Cellvia kit (AB frontier) and a BrdU cell proliferation kit (abcam). As a result, the proliferation of HT-29 cells was increased by HKFY-34 in a dose-dependent manner (Fig. 75).

It is known from studies using GPR39 deficient mice that calcium signaling is involved in the growth of intestinal tissue cells by GPR39 (Phil. Trans. R. Soc. B. 2016, 371:20150420). Therefore, it was confirmed whether calcium signaling is involved in the cell growth promoted by HKFY-34.

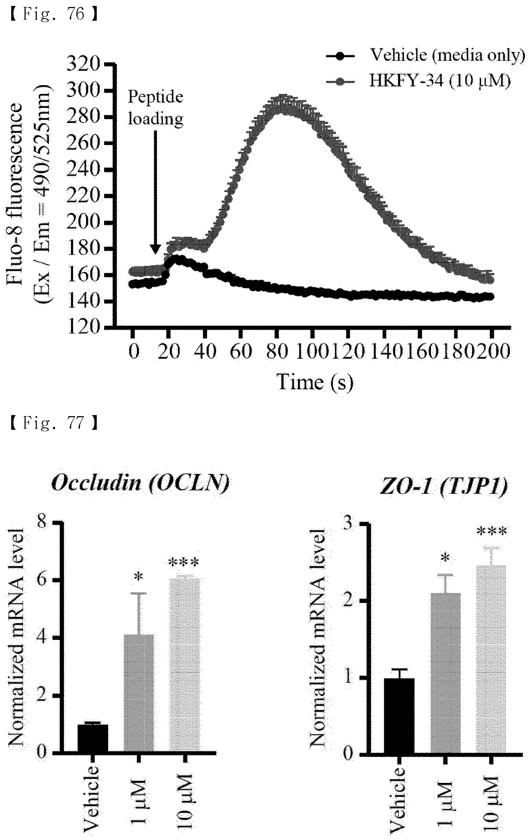

Specifically, HT-29 cells were cultured in a 96-well black culture plate and then treated with HKFY-34, and the calcium ion concentration in the cells was measured using a Fluo-8 direct^{™} calcium assay kit (Thermo Fisher Scientific). As a result, the calcium ion concentration in HT-29 cells was increased by HKFY-34 compared to the untreated group (vehicle) (Fig. 76).

### Experimental Example 5. Evaluation of promoting activity of HKFY-34 on tight junction

It is known that GPR39 is involved in the protection of the gastrointestinal tract (Phil. Trans. R. Soc. B., 2016, 371:20150420). Therefore, in order to confirm the effect of HKFY-34 on the gastrointestinal tract protective function by GPR39, the promoting activity of HKFY-34 on tight junction was evaluated using HT-29 cells, which are important for intestinal tissue formation.

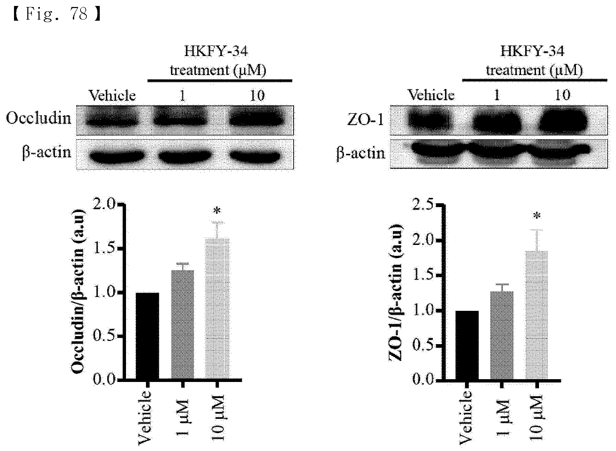

Specifically, the HT-29 cells were treated with HKFY-34 at different concentrations, and then the expression levels of mRNA (Fig. 77) and protein (Fig. 78) of ZO-1 and occludin, which are important factors of tight junction in intestinal barrier function, were confirmed through quantitative RT-PCR (qPCR) and Western blot. As a result, the expression levels of mRNA and protein of ZO-1 and occludin were significantly increased by HKFY-34 in a dose-dependent manner compared to the untreated group (vehicle) (Fig. 77 and Fig. 78).

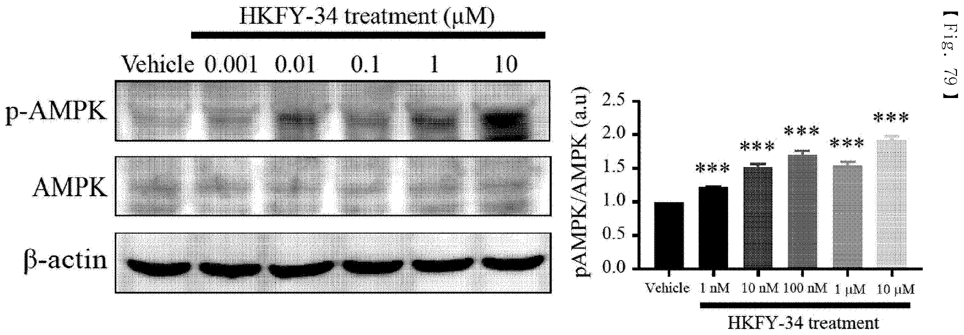

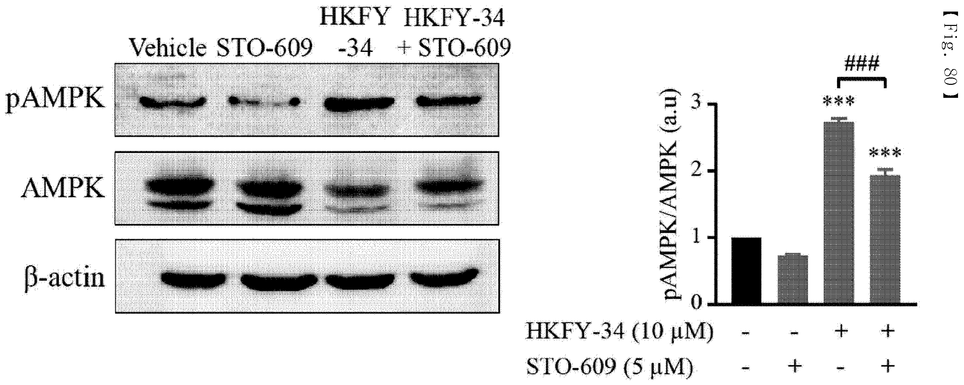

It is known that tight junctions are mediated by a signaling mechanism due to phosphorylation of AMPK protein. Therefore, in order to confirm whether AMPK is involved in the increase in tight junctions by HKFY-34, the expression of p-AMPK in the cells was confirmed using HT-29 cells (Figs. 79 and 80).

As a result, the expression of the p-AMPK protein was increased by HKFY-34 in a dose-dependent manner compared to the untreated group (vehicle) (Fig. 79). In addition, when treated with HKFY-34 after pretreatment with an AMPK inhibitor (STO-609), it was confirmed that the expression level of p-AMPK, which was increased due to HKFY-34 treatment, was decreased (Fig. 80). These results indicate that HKFY-34 acts as a GPR39 agonist, thereby enhancing tight junction.

### Experimental Example 6. Evaluation of anti-inflammatory activity of HKFY-34 in cell model of inflammatory bowel disease

In order to confirm the effect of HKFY-34 on anti-inflammatory action, which is one of the gastrointestinal tract protective function (barrier function) by GPR39, the anti-inflammatory activity of HKFY-34 was evaluated in a cell model of inflammatory bowel disease (IBD).

Specifically, the HT-29 cells were treated with 1 µg/mL LPS to induce inflammation and prepare an inflammatory bowel disease model, and were treated with HKFY-34 at different concentrations. Thereafter, the expression levels of mRNA (Fig. 81) and protein (Fig. 82) of inflammation factors for ulcerative colitis were measured through qPCR and ELISA.

As a result, the mRNA expression levels of IL-8, IL-6, IL-1β and TNF-α, which are pro-inflammatory cytokines increased by LPS treatment, were significantly decreased by HKFY-34 treatment in a dose-dependent manner (Fig. 81). In addition, the secretion levels of IL-8, IL-6, IL-1β and TNF-α in HT-29 cells treated with LPS were also significantly decreased by HKFY-34 treatment in a dose-dependent manner (Fig. 82).

These results indicate that HKFY-34, which acts as a GPR39 agonist, may have an effect of alleviating and treating inflammation in inflammatory bowel disease.

### Experimental Example 7. Evaluation of anti-inflammatory activity of HKFY-34 in animal model of inflammatory bowel disease

The anti-inflammatory activity of HKFY-34 in an animal model of inflammatory bowel disease was evaluated.

Specifically, an animal model of ulcerative colitis among inflammatory bowel diseases was prepared by feeding C57BL6/J mice (Central Lab. Animal Inc.) with water containing 3% DSS (dextran sodium sulfate).

The untreated (vehicle) mice were set as a control group, the mice fed with only DSS (vehicle+3% DSS) were set as a negative control group, and the mice administered intraperitoneally with SASP (sulfasalazine) at 50 mg/kg after treatment with DSS (SASP 50 mg/kg+3% DSS) were set as a positive control group. As a test group, the mice were intraperitoneally administered with HKFY-34 at 0.1, 1, and 10 mg/kg, respectively. HKFY-34 was administered once a day for 5 days, and during the administration period, the stool condition and body weight loss were measured in the subjects. Stool condition was observed for the degree of stool softness (stool consistency, diarrhea) and the degree of blood in stool (fecal bleeding), and a score was assigned from the lowest 0 to the highest 3 in proportion to the severity compared to the control group.

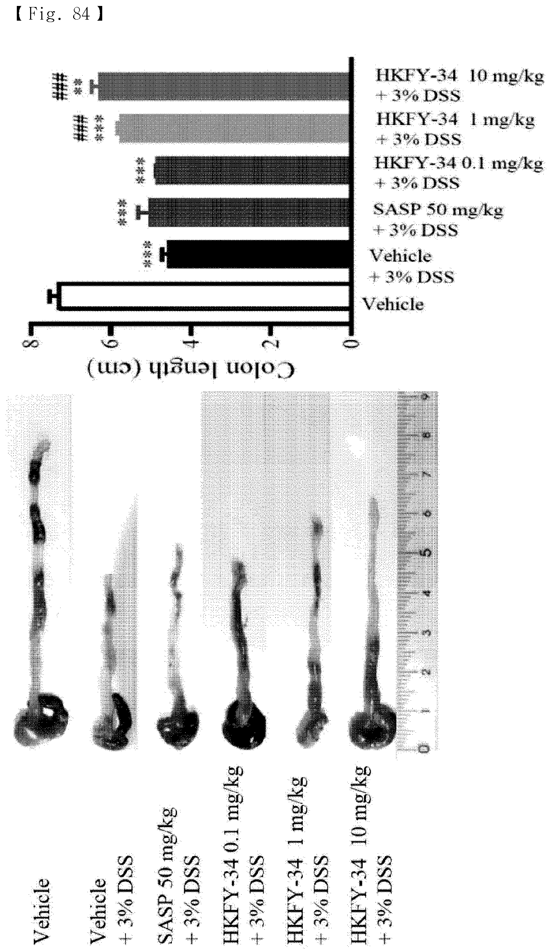

As a result, body weight loss, a symptom of ulcerative colitis, was significantly inhibited by HKFY-34 in a dose-dependent manner in the test group administered with HKFY-34 compared to the positive control group, SASP (Fig. 83). In addition, as a result of measuring the length of the colon of all subjects who completed the administration, the length contraction of the colon due to inflammatory damage was reduced by HKFY-34 in a dose-dependent manner in the HKFY-34 test group compared to the negative control group (Fig. 84).

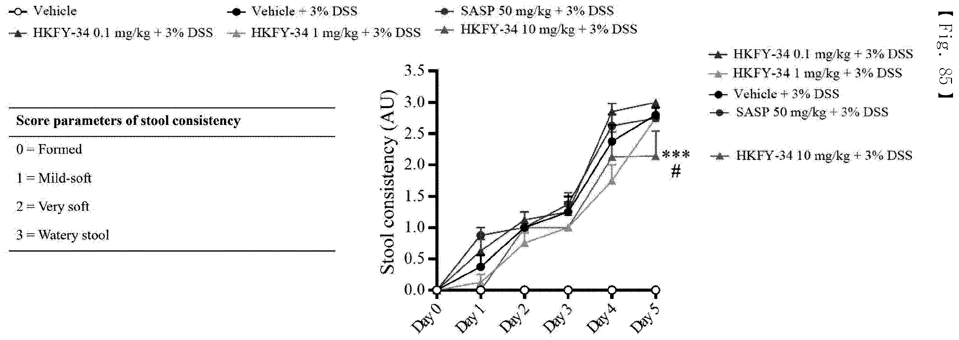

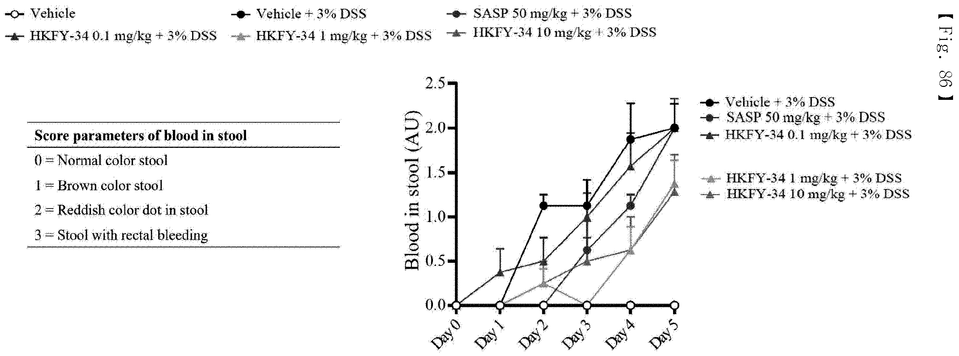

As a result of observing the stool in each subject, it was also confirmed that ulcerative colitis was alleviated in a dose-dependent manner in the test group administered with HKFY-34 (Figs. 85 and 86).

In order to confirm the anti-inflammatory action of HKFY-34 administration on colon tissue, the mRNA expression levels of pro-inflammatory cytokines in colon tissue for each group were confirmed by qPCR. As a result, the mRNA expression levels of IL-8, IL-6, IL-1β and TNF-α in the intestinal tissue were significantly decreased in the group administered with HKFY-34 compared to the negative control group (Fig. 87). In addition, the concentrations of IL-1β and TNF-α in the blood were significantly decreased in the HKFY-34 test group compared to the negative control group (Fig. 88).

For histological analysis of the colon tissues of each group, H&E (hematoxylin and eosin) staining was performed on the colon tissues of the negative control group, the positive control group, and the test group. For the stained tissue, the infiltration rate of immune cells (macrophages) in the colonic mucosa, the loss rate of mucus secreting cells, the structural collapse rate of colonic villi, and the loss rate of secretory glands were confirmed, and the degree of damage was analyzed by comparing it with the control group to assign a score. As a result, it was confirmed that the degree of damage caused by inflammation of the colon tissue was alleviated in the test group administered with HKFY-34 compared to the negative control group (Fig. 89).

In addition, as a result of confirming the expression levels of important factors for tight junction of the colon tissue in each subject, the mRNA expression levels of ZO-1 and occludin were significantly increased by HKFY-34 in a dose-dependent manner in the subject treated with HKFY-34 (Fig. 90).

### Experimental Example 8. Evaluation of anti-inflammatory activity of HKFY-34 in inflammatory bowel disease-induced GPR39 deficient mouse model

In order to confirm the effect of HKFY-34 in an inflammatory bowel disease model using GPR39 as a target substance, a disease model of ulcerative colitis was prepared using GPR39 deficient mice (GPR39 knock out (KO), *Gpr39*^{*-*/}*⁻).* At this time, wild type mice born from a litter were set as a control group for each treated group of GPR39 deficient mice.

Specifically, an animal model of ulcerative colitis was prepared by feeding GPR39 deficient mice (Lexicon pharmaceuticals) and wild type mice with water containing 3% DSS in the same manner as in Experimental Example 7. GPR39 KO mice fed with only DSS (vehicle+3% DSS) were set as a negative control group, and GPR39 KO mice administered with HKFY-34 (10 mg/kg) (HKFY-34 10 mg/kg+3% DSS) were set as a test group. Intraperitoneal administration was performed once a day for 5 days.

As a result, body weight loss was inhibited by administration of HKFY-34 in ulcerative colitis-induced wild type mice. On the other hand, no inhibitory effect of HKFY-34 on body weight loss was observed in the ulcerative colitis-induced GPR39 deficient mice (Fig. 91).

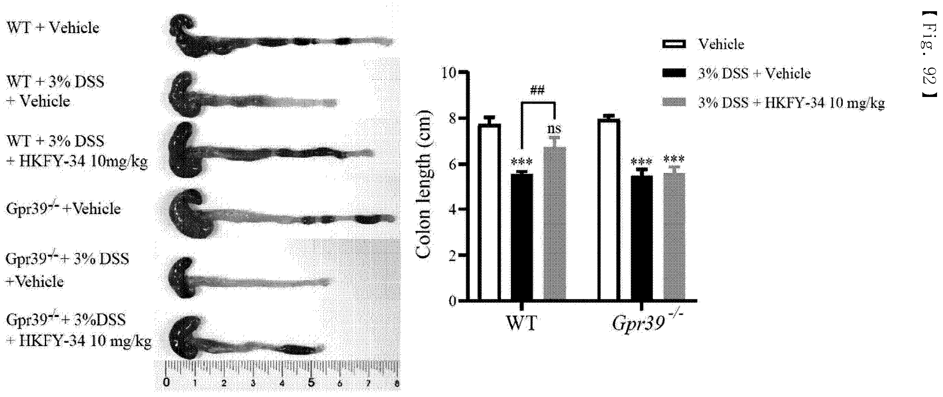

In addition, the decrease in colon length was inhibited by administration of HKFY-34 in the ulcerative colitis-induced wild type mice. On the other hand, the inhibitory effect of HKFY-34 on the reduction of colon length was not observed in the ulcerative colitis-induced GPR39 deficient mice (Fig. 92).

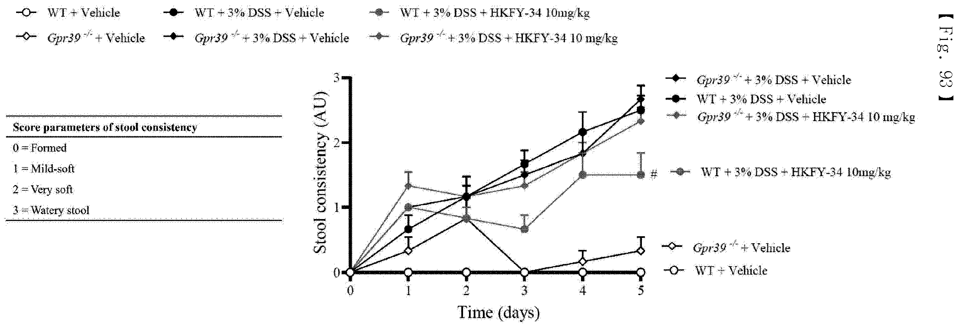

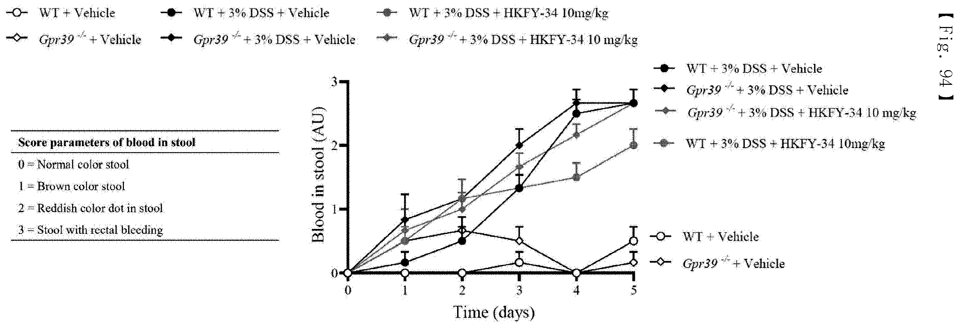

As a result of observing the stool conditions of individual GPR39 deficient mice in which ulcerative colitis was induced, the effect of alleviating stool consistency and blood in stool was not observed in the test group administered with HKFY-34 compared to the negative control group (Figs. 93 and 94).

As a result of analyzing histological morphology through H&E (hematoxylin and eosin) staining, it was observed that, in ulcerative colitis-induced wild type mice, damage due to inflammation of colon tissue was reduced in the test group administered with HKFY-34. On the other hand, in ulcerative colitis-induced GPR39 deficient mice, the effect of HKFY-34 reducing inflammatory damage was not observed (Fig. 95).

These results indicate that HKFY-34 did not mitigate ulcerative colitis in GPR39 deficient mice, indicating that HKFY-34 is an agonist targeting GPR39.

## Claims

1. A compound represented by Formula 1 below or a pharmaceutically acceptable salt thereof: 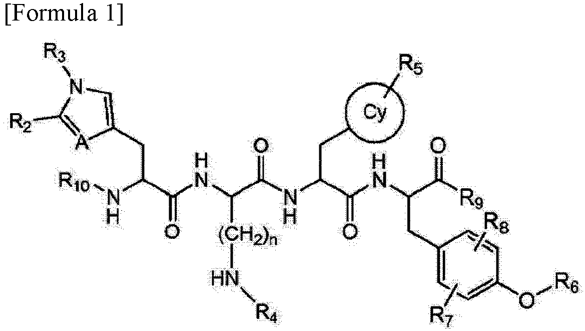 in Formula 1
A is -C(-R₀)-, -N= or -N(-R₁)-,
Cy is C₆₋₁₄ aryl or 5 to 6-membered heteroaryl,
R₁ and R₃ are each independently hydrogen, Ra, an amine protecting group, C₁₋₆ alkyl substituted with 1 to 3 Ra, C₃₋₁₀ cycloalkyl, or 5 to 6-membered heterocyclyl, wherein said heterocyclyl has or does not have 1 to 3 substituents selected from phenylethyl and cyclohexylethyl,
R₂ is hydrogen, Ra, or 5 to 6-membered heterocyclyl, and
R₀ is hydrogen, or R₀ and R₂ are linked to each other to form a benzene ring together with the two carbon atoms to which they are attached,
Ra is each independently C₃₋₁₀ cycloalkyl or C₆₋₁₄ aryl, wherein said aryl has or does not have one or more substituents selected from the group consisting of halogen, -OH, C₆₋₁₄ aryl substituted with 5 to 6-membered heteroaryl, C₁₋₆ alkyl and C₁₋₆ alkoxy,
n is 1 to 10,
R₄ is hydrogen, C₁₋₁₀ alkyl or C₁-C₂₀ alkylcarbonyl,
R₅ is hydrogen, halogen, CF₃ or C₁₋₆ alkyl,
R₆ is hydrogen, C₁₋₁₀ alkyl or -S(=O)zOH,
R₇ and R₈ are each independently hydrogen, halogen, nitro, amine or C₁₋₆ alkyl,
R₉ is -OH or -NH₂, and
R₁₀ is hydrogen, an amine protecting group or biotin,
wherein said heterocyclyl and heteroaryl each independently contain at least one heteroatom selected from the group consisting of N, S and O.

2. The compound or pharmaceutically acceptable salt thereof according to claim 1, wherein
R₁ is hydrogen,

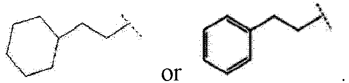

3. The compound or pharmaceutically acceptable salt thereof according to claim 1, wherein
R₂ is hydrogen, 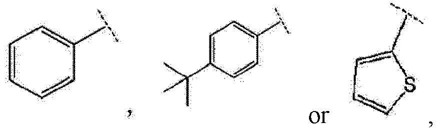
R₂ is hydrogen, Ra, or 5 to 6-membered heterocyclyl, and
R₀ is hydrogen, or R₀ and R₂ are linked to each other to form a benzene ring together with the two carbon atoms to which they are attached.

4. The compound or pharmaceutically acceptable salt thereof according to claim 1, wherein
R₃ is one selected from the group consisting of 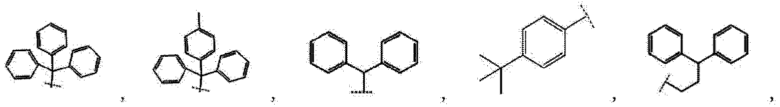 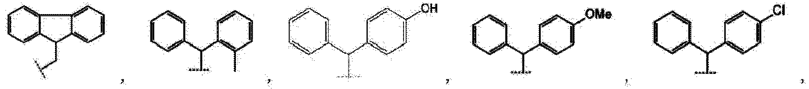 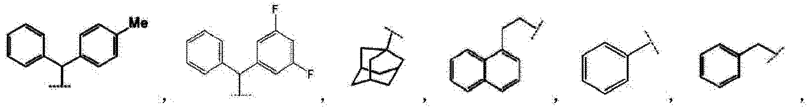 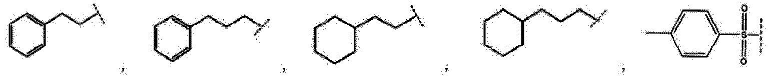 and

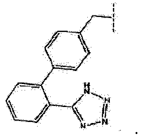

5. The compound or pharmaceutically acceptable salt thereof according to claim 1, wherein A is -C(-R₀)- or -N=.

6. The compound or pharmaceutically acceptable salt thereof according to claim 1, wherein
R₃ is

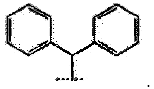

7. The compound or pharmaceutically acceptable salt thereof according to claim 1, wherein n is 3 or 4, and R₄ is heptylcarbonyl.

8. The compound or pharmaceutically acceptable salt thereof according to claim 1, wherein R₅ is methyl or CF₃.

9. The compound or pharmaceutically acceptable salt thereof according to claim 1, wherein R₆ is hydrogen or S(=O)₂OH.

10. The compound or pharmaceutically acceptable salt thereof according to claim 1, wherein R₇ and R₈ are each independently hydrogen or amine.

11. The compound or pharmaceutically acceptable salt thereof according to claim 1, wherein R₉ is -OH.

12. The compound or pharmaceutically acceptable salt thereof according to claim 1, wherein R₁₀ is hydrogen, p-toluenesulfonyl (Ts), t-butoxycarbonyl (Boc) or biotin.

13. The compound or pharmaceutically acceptable salt thereof according to claim 1, wherein said Cy is pyridyl, naphthyl or phenyl.

14. The compound or pharmaceutically acceptable salt thereof according to claim 1, wherein the compound is any one selected from the group consisting of:
1) His(trt)-Lys(caprylic)-Phe-Tyr,
2) Biotin-His(trt)-Lys(caprylic)-Phe-Tyr,
3) His(benzyl)-Lys(caprylic)-Phe-Tyr,
4) d-His(trt)-Lys(caprylic)-Phe-Tyr,
5) His(4-methyltrityl)-Lys(caprylic)-Phe-Tyr,
6) His(DAMP-3)-Lys(caprylic)-Phe-Tyr,
7) His(DAMP-5)-Lys(caprylic)-Phe-Tyr,
8) His(DAMP-2)-Lys(caprylic)-Phe-Tyr,
9) His(2-phenylethyl)-Lys(caprylic)-Phe-Tyr,
10) His(2-naphthalen-1-methyl)-Lys(caprylic)-Phe-Tyr,
11) His(2-cyclohexylethyl)-Lys(caprylic)-Phe-Tyr,
12) His(trt)-Lys(caprylic)-d-Phe-Tyr,
13) His(trt)-Lys(caprylic)-d-Phe(4-Cl)-Tyr,
14) d-His(trt)-Lys(caprylic)-d-Phe(4-Cl)-d-Tyr,
15) His(trt)-Lys(caprylic)-d-Phe(4-Cl)-d-Tyr,
16) His(trt)-d-Lys(caprylic)-d-Phe(4-Cl)-Tyr,
17) His(3,3-diphenylethyl)-Lys(caprylic)-Phe-Tyr,
18) His(Fm)-Lys(caprylic)-Phe-Tyr,
19) His(phenethyl)-Lys(caprylic)-Phe-Tyr,
20) His(4-methoxylbenzhydryl)-Lys(caprylic)-Phe-Tyr,
21) His(4-chlorobenzhydryl)-Lys(caprylic)-Phe-Tyr,
22) His(4-methylbenzhydryl)-Lys(caprylic)-Phe-Tyr,
23) His(adamantan-1-yl)-Lys(caprylic)-Phe-Tyr,
24) His(trt)-Lys(capric)-Phe-Tyr,
25) His(trt)-Lys(lauric)-Phe-Tyr,
26) His(trt)-Lys(myristic)-Phe-Tyr,
27) His(trt)-Lys(palmitic)-Phe-Tyr,
28) His(trt)-Lys(caprylic)-d-Phe(F)-Tyr,
29) His(trt)-Lys(caprylic)-d-Phe(Br)-Tyr,
30) His(trt)-Lys(caprylic)-d-Phe(4-methyl)-Tyr,
31) d-His(trt)-Lys(caprylic)-d-Phe(4-methyl)-Tyr,
32) His(1,3-difluorobenzhydryl)-Lys(caprylic)-d-Phe(4-methyl)-Tyr,
33) His(benzhydryl)-Lys(caprylic)-Phe-Tyr,
34) His(benzhydryl)-Lys(caprylic)-d-Phe(4-methyl)-Tyr,
35) His(benzhydryl)-Lys(caprylic)-d-Phe(4-methyl)-Tyr(SO₃H),
36) His(benzhydryl)-Lys(caprylic)-d-Phe(4-methyl)-Tyr(2,6-di-methyl),
57) His(2-phenyl)-Lys(caprylic)-d-Phe(4-methyl)-Tyr,
58) His(1-phenyl)-Lys(caprylic)-d-Phe(4-methyl)-Tyr,
59) His(1,2-diphenyl)-Lys(caprylic)-d-Phe(4-methyl)-Tyr,
60) His(2-(4-tert-butyl)phenyl)-Lys(caprylic)-d-Phe(4-methyl)-Tyr,
61) His(1-(4-tert-butyl)phenyl)-Lys(caprylic)-d-Phe(4-methyl)-Tyr,
62) d-His(benzhydryl)-Lys(caprylic)-d-Phe(4-methyl)-Tyr,
63) His(thiophene)-Lys(caprylic)-d-Phe(4-methyl)-Tyr,
64) His(4-methoxybenzhydryl)-Lys(caprylic)-d-Phe(4-methyl)-Tyr
65) His(4-hydroxybenzhydryl)-Lys(caprylic)-d-Phe(4-methyl)-Tyr
66) His(benzhydryl)-Lys(caprylic)-d-Phe(4-methyl)-Tyr-NH₂
67) His(benzhydryl)-Lys(caprylic)-d-Phe(4-methyl)-Tyr(3-chloro)
68) His(benzhydryl)-Lys(caprylic)-d-Phe(4-methyl)-Tyr(3-nitro)
69) His(benzhydryl)-Lys(caprylic)-d-Phe(4-methyl)-Tyr(3,5-nitro)
70) His(benzhydryl)-Lys(caprylic)-d-Phe(4-methyl)-Tyr(3-amino)
71) His(benzhydryl)-Orn(caprylic)-d-Phe(4-methyl)-Tyr
72) Biotin-His(benzhydryl)-Lys(caprylic)-d-Phe(4-methyl)-Tyr
73) His(trt)-Lys(caprylic)-d-3Pal-Tyr
74) His(trt)-Lys(caprylic)-d-2Nal-Tyr
75) His(tosyl)-Lys(caprylic)-d-Phe(4-methyl)-Tyr
76) His(trt)-Lys(caprylic)-d-Phe(4-CF3)-Tyr
77) His(tbm)-Lys(caprylic)-d-Phe(4-CF3)-Tyr
78) Boc-Trp-Lys(caprylic)-d-Phe(4-methyl)-Tyr.

15. The compound or pharmaceutically acceptable salt thereof according to claim 1, wherein two amino acids or derivatives thereof are further bound to the N terminus of the compound represented by Formula 1.

16. The compound or pharmaceutically acceptable salt thereof according to claim 15, wherein
the compound is (X₁)-(X₂)-[His(trt)-Lys(caprylic)-Phe-Tyr],
wherein X₁ and X₂ are each independently any one amino acid selected from 20 amino acids or derivatives thereof.

17. The compound or pharmaceutically acceptable salt thereof according to claim 16, wherein the compound is
37) Gly-Ser-His(trt)-Lys(caprylic)-Phe-Tyr.

18. The compound or pharmaceutically acceptable salt thereof according to claim 1, wherein 1 to 10 amino acids or derivatives thereof are further bound to the C terminus of the compound represented by Formula 1.

19. The compound or pharmaceutically acceptable salt thereof according to claim 18, wherein
the compound is [His(trt)-Lys(caprylic)-Phe-Tyr]-(X₃)ₐ-(X₄)_{b}-(X₅)_{c}-(X₆)_{d}-(X₇)ₑ-(X₈)f-(X₉)_{g},
wherein X₃ to X₉ are each independently any one amino acid selected from 20 amino acids or derivatives thereof, and
a to g are 0 or 1, and at least one of them is 1.

20. The compound or pharmaceutically acceptable salt thereof according to claim 19, wherein the compound is any one selected from the group consisting of:
38) His(trt)-Lys(caprylic)-Phe-Tyr-Ser-Asp-Gln-Gln-Ala-Arg-Phe,
39) His(trt)-Lys(caprylic)-Phe-Tyr-Ser-Gln-Asn-Gly-Ala-Arg-Phe,
40) His(trt)-Lys(caprylic)-Phe-Tyr-Ser-His-Gln-Gln-Ala-Arg-Phe,
41) His(trt)-Lys(caprylic)-Phe-Tyr-Gln-Asn-Gly-Ala-Arg-Phe,
42) His(trt)-Lys(caprylic)-Phe-Tyr-His-Gln-Gln-Ala-Arg-Phe,
43) His(trt)-Lys(caprylic)-Phe-Tyr-Gln-Asn-Gln-Ala-Arg-Phe, and
44) His(trt)-Lys(caprylic)-Phe-Tyr-Trp.

21. A compound or a pharmaceutically acceptable salt thereof, wherein the compound consists of [His(trt)-Lys(caprylic)]-(X₁₀)ₕ-(X11)ᵢ,
wherein X₁₀ and X₁₁ are each independently any one amino acid selected from 20 amino acids or derivatives thereof, and
h and i are 0 or 1, and at least one of them is 1.

22. The compound or pharmaceutically acceptable salt thereof according to claim 21, wherein the compound is any one selected from the group consisting of:
45) Biotin-His(trt)-Lys(caprylic)- Ala(biphenyl)-Tyr,
46) His(trt)-Lys(caprylic)-Phe-Trp,
47) His(trt)-Lys(caprylic)-Tyr-Phe,
48) His(trt)-Lys(caprylic)-Tyr-Tyr,
49) His(trt)-Lys(caprylic)-Phe,
50) His(trt)-Lys(caprylic)-Phe-Ser, and
51) His(trt)-Lys(caprylic)-Leu-Tyr.

23. A compound or a pharmaceutically acceptable salt thereof, wherein the compound is any one selected from the group consisting of:
52) His(trt)-Phe-Tyr-Asp-Gln-Gln-Ala-Arg-Phe,
53) His(trt)-Gly-Ser-Lys(caprylic)-Phe-Tyr,
54) Lys(caprylic)-Phe-Tyr,
55) Lys(caprylic)-His(trt)-Phe-Tyr
56) His(trt)-Lys(caproic)-Phe-Tyr-Asp-Gln-Gln-Ala-Arg-Phe, and
79) His(benzhydryl)Lys(caprylic).

24. A pharmaceutical composition for preventing or treating inflammatory bowel disease, comprising the compound or pharmaceutically acceptable salt thereof according to any one of claims 1 to 23 as an active ingredient.

25. A health functional food composition for preventing or mitigating inflammatory bowel disease, comprising the compound or cytologically acceptable salt thereof according to any one of claims 1 to 23 as an active ingredient.

26. A method for preventing or treating inflammatory bowel disease, comprising administering the compound or pharmaceutically acceptable salt thereof according to any one of claims 1 to 23 to a mammal.

27. Use of the compound or pharmaceutically acceptable salt thereof according to any one of claims 1 to 23, for the manufacture of a medicament for preventing or treating inflammatory bowel disease.
